# EUROPEAN PATENT APPLICATION

(11) **EP 4 353 309 A1**
(43) Date of publication of application: **17.04.2024**
(21) Application number: 22811378.3
(22) Date of filing: 26.05.2022
(51) Int. Cl.: A61P 1/02, A61P 3/02, A61P 5/06, A61P 9/00, C07D 211/14, A61K 31/4545

(54) **PHENYL UREA DERIVATIVE**

(30) Priority: 26.05.2021 JP 2021088034
(71) Applicant: Sumitomo Pharma Co., Ltd., Osaka 541-0045 (JP)
(72) Inventor: KOMIYA, Masafumi, Osaka-shi, Osaka 554-0022 (JP); UESUGI, Shunichiro, Osaka-shi, Osaka 554-0022 (JP); IDEUE, Eiji, Osaka-shi, Osaka 554-0022 (JP); LEE, Shoukou, Osaka-shi, Osaka 554-0022 (JP); TANAKA, Daisuke, Osaka-shi, Osaka 541-0045 (JP); FUNAKOSHI, Yuta, Osaka-shi, Osaka 554-0022 (JP)
(74) Representative: Carpmaels & Ransford LLP
(86) International application number: PCT/JP2022/021531
(87) International publication number: WO 2022/250108

(57) **Abstract**

The present invention relates to a medicament for treating or preventing a disease related to orexin receptor, especially orexin receptor type 2, comprising a new compound having a urea structure or a pharmaceutically acceptable salt thereof as an active ingredient. In more detail, the present invention relates to a medicament for treating or preventing a disease such as narcolepsy, idiopathic hypersomnia, hypersomnia, sleep apnea syndrome.

## Description

### TECHNICAL FIELD

The present invention relates to a medicament for treating or preventing a disease associated with orexin receptor, especially orexin receptor type 2, comprising a new compound having a urea structure or a pharmaceutically acceptable salt thereof as an active ingredient. Specifically, the present invention relates to a medicament for treating or preventing a disease such as narcolepsy, idiopathic hypersomnia, hypersomnia, sleep apnea syndrome.

### BACKGROUND ART

Orexin is a neuropeptide which is specifically produced in a specific neuron spreading across lateral hypothalamus and its adjacent region. Orexin is an endogenous ligand of orexin receptor that is a G-protein-coupled receptor existing mainly in brain, which binds to orexin receptor. It is known that orexin receptor has two subtypes, type 1 and type 2 (Non-Patent Document 1).

It was reported that a narcolepsy-like symptom in a transgenic mouse whose orexin neuron was denatured could be improved by intraventricular injection of an orexin peptide (Non-Patent Document 2), and a narcolepsy-like symptom could be initiated by knocking out (KO) prepro-orexin which is a precursor protein of orexin (Non-patent Reference 3), furthermore the orexin concentration in cerebrospinal fluid of narcolepsy patients was markedly lowered (Non-Patent Document 4). Thus, it is suggested that narcolepsy can be initiated due to lack of orexin.

In addition, it was reported that there was a mutation of orexin receptor type 2 in a dog suffering from hereditary narcolepsy (Non-Patent Document 5), which suggests that orexin receptor type 2 is involved in sleep-wake function. Furthermore, it was revealed that narcolepsy-like symptom was initiated in a KO mouse of orexin receptor type 2 (Non-Patent Document 6), which strongly suggests that the stimulation on orexin receptor type 2 is involved in sleep-wake function. Thus, an orexin receptor type 2 agonist is expected to be a hopeful therapy for a patient presenting with hypersomnia-like symptom such as narcolepsy.

Recently, a compound with orexin receptor type 2 agonistic effect was reported (Patent Document 1).

### PRIOR ART DOCUMENTS

### PATENT DOCUMENTS

Patent Document 1: WO 2017/135306

### NON-PATENT DOCUMENTS

Non-Patent Document 1: Cell, Vol.92, 573-585, 1998
Non-Patent Document 2: Proc. Natl. Acad. Sci. USA, Vol. 101, 4649-4654, 2004
Non-Patent Document 3: Cell, Vol. 98, 437-451, 1999
Non-Patent Document 4: THE LANCET, Vol. 355, 39-40, 2000
Non-Patent Document 5: Cell, Vol. 98, 365-376, 1999
Non-Patent Document 6: Neuron, Vol. 38, 715-730, 2003
Non-Patent Document 7: Brain, Vol. 130, 1577-1585, 2007
Non-Patent Document 8: Neuroscience Letters, Vol. 569, 68-73, 2014

### SUMMARY OF INVENTION

### (PROBLEM TO BE SOLVED BY THE INVENTION)

An object of the present invention is to provide a medicament for treating or preventing a disease associated with orexin receptor type 2, specifically a disease such as narcolepsy, idiopathic hypersomnia, hypersomnia, sleep apnea syndrome.

### (MEANS FOR SOLVING THE PROBLEMS)

The present inventors have extensively studied to reach the above object, and then have found that a compound of the following formula (1) or a pharmaceutically acceptable salt thereof (hereinafter, it may be referred to as "the present compound") has therapeutic and prophylactic effect for a disease associated with orexin receptor type 2, specifically, a disease such as narcolepsy, idiopathic hypersomnia, hypersomnia, sleep apnea syndrome. Based on the new findings, the present invention has been completed.

The present invention can show as follows.

### [Item 1]

A compound of formula (1): 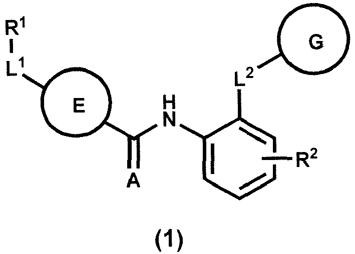 or a pharmaceutically acceptable salt thereof,
wherein
R¹ is optionally-substituted C₆₋₁₀ aromatic carbocyclyl group, optionally-substituted 5- to 10-membered aromatic heterocyclyl group, optionally-substituted C₃₋₆ saturated carbocyclyl group, optionally-substituted 4- to 10-membered saturated heterocyclyl group, or cyano;
L¹ and L² are each independently single bond, methylene (which may be optionally substituted with the same or different one or more C₁₋₆ alkyl groups), -NR⁶-, -C(=O)-, - OC(=O)-, -SO-, -SO₂-, -S-, or oxygen atom;
R² is hydrogen atom, hydroxy group, halogen atom, cyano, optionally-substituted C₁₋₆ alkyl, optionally-substituted C₁₋₆ alkoxy, C(=O)NR³R⁴ or C(=O)O-R⁵;
R³, R⁴, R⁵ and R⁶ are each independently hydrogen atom or optionally-substituted C₁₋₆ alkyl;
ring E is optionally-substituted 5- to 10-membered aromatic heterocyclyl group or optionally-substituted 4- to 10-membered saturated heterocyclyl group;
ring G is optionally-substituted C₆₋₁₀ aromatic carbocyclyl group, optionally-substituted 5- to 10-membered aromatic heterocyclyl group, optionally-substituted C₃₋₆ saturated carbocyclyl group, or optionally-substituted 4- to 10-membered saturated heterocyclyl group; and
A is oxygen atom or sulfur atom.

### [Item 2]

The compound according to Item 1 or a pharmaceutically acceptable salt thereof, wherein in R² to R⁶, the optional substituent of "optionally-substituted C₁₋₆ alkyl" is each independently the same or different one or more substituents selected from halogen atom, hydroxy group, C₁₋₆ alkoxy, or C₃₋₇ cycloalkyl, and the optional substituent of "optionally-substituted C₁₋₆ alkoxy" is each independently the same or different one or more substituents selected from halogen atom, hydroxy group, or C₃₋₇ cycloalkyl,
in R¹, the optional substituent of C₆₋₁₀ aromatic carbocyclyl group, 5- to 10-membered aromatic heterocyclyl group, C₃₋₆ saturated carbocyclyl group, and 4- to 10-membered saturated heterocyclyl group is each independently one or more substituents selected from the group consisting of halogen atom, hydroxy group, C₆₋₁₀ aromatic carbocyclyl group (which may be optionally substituted with the same or different one or more substituents selected from halogen atom, hydroxy group, C₁₋₆ alkyl, C₁₋₆ alkoxy or C₃₋₇ cycloalkyl), C₁₋₆ alkyl (which may be optionally substituted with the same or different one or more substituents selected from halogen atom, hydroxy group, C₁₋₆ alkoxy or C₃₋₇ cycloalkyl), C₃₋₇ cycloalkyl (which may be optionally substituted with the same or different one or more substituents selected from halogen atom, hydroxy group, C₁₋₆ alkyl, C₁₋₆ alkoxy or C₃₋₇ cycloalkyl), C₁₋₆ alkylamino (the alkyl group of which may be optionally substituted with halogen atom, hydroxy group or C₃₋₇ cycloalkyl), C₃₋₇ cycloalkoxy (which may be optionally substituted with the same or different one or more substituents selected from halogen atom, hydroxy group, C₁₋₆ alkyl, C₁₋₆ alkoxy or C₃₋₇ cycloalkyl), cyano, C₁₋₆ alkoxy (which may be optionally substituted with the same or different one or more substituents selected from halogen atom, hydroxy group or C₃₋₇ cycloalkyl), and 5- to 10-membered aromatic heterocyclyl group (which may be optionally substituted with the same or different one or more substituents selected from halogen atom, hydroxy group, C₁₋₆ alkyl, C₁₋₆ alkoxy or C₃₋₇ cycloalkyl), and
in ring E and ring G, the optional substituent of C₆₋₁₀ aromatic carbocyclyl group, 5- to 10-membered aromatic heterocyclyl group, C₃₋₆ saturated carbocyclyl group, and 4-to 10-membered saturated heterocyclyl group is each independently one or more substituents selected from the group consisting of halogen atom, C₁₋₆ alkyl (which may be optionally substituted with the same or different one or more substituents selected from halogen atom, hydroxy group, C₁₋₆ alkoxy or C₃₋₇ cycloalkyl), C₆₋₁₀ aromatic carbocyclyl group (which may be optionally substituted with the same or different one or more substituents selected from halogen atom, hydroxy group, C₁₋₆ alkyl, C₁₋₆ alkoxy or C₃₋₇ cycloalkyl), C₁₋₆ alkoxy (which may be optionally substituted with the same or different one or more substituents selected from halogen atom, hydroxy group or C₃₋₇ cycloalkyl), C₁₋₆ alkylamino (the alkyl group of which may be optionally substituted with halogen atom, hydroxy group or C₃₋₇ cycloalkyl), C₃₋₇ cycloalkyl (which may be optionally substituted with the same or different one or more substituents selected from halogen atom, hydroxy group, C₁₋₆ alkyl, C₁₋₆ alkoxy or C₃₋₇ cycloalkyl), and C₃₋₇ cycloalkoxy (which may be optionally substituted with the same or different one or more substituents selected from halogen atom, hydroxy group, C₁₋₆ alkyl, C₁₋₄ alkoxy or C₃₋₇ cycloalkyl), or when there are plural optional substituents, two of them may be taken together via C₁₋₆ alkylene to form a chemically-possible bicyclic structure selected from a fused ring, a spiro ring, or bridged ring.

### [Item 3]

The compound according to Item 1 or 2 or a pharmaceutically acceptable salt thereof, wherein the formula is represented by formula (2): 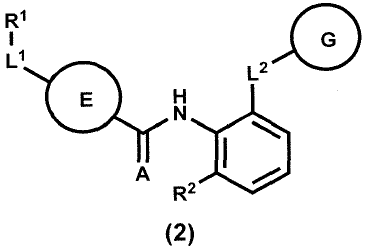 wherein
R² is hydroxy group, halogen atom, cyano, optionally-substituted C₁₋₄ alkyl, optionally-substituted C₁₋₄ alkoxy, C(=O)NR³R⁴ or C(=O)OR⁵; and
R¹, L¹, L², R³, R⁴, R⁵, ring E, ring G, and A are as defined in Item 1.

### [Item 4]

The compound according to any one of Items 1 to 3 or a pharmaceutically acceptable salt thereof, wherein R¹ is selected from cyano or the following formulae (1a-1) to (1a-6) : 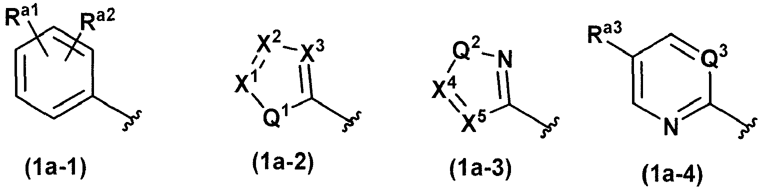 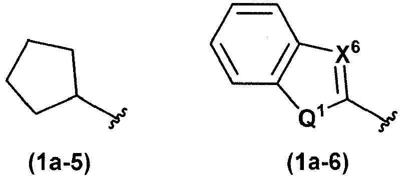 wherein
X¹ to X⁶ are each independently nitrogen atom or CR^{a4};
Q¹ and Q² are oxygen atom, -NR^{a5}- or sulfur atom;
Q³ is CH or nitrogen atom; and
R^{a1} to R^{a5} are each independently (if there are plural CR^{a4}, each R^{a4} is also independently) hydrogen atom, halogen atom, C₆₋₁₀ aromatic carbocyclyl group (which may be optionally substituted with the same or different one or more substituents selected from halogen atom, hydroxy group, C₁₋₆ alkyl or C₁₋₆ alkoxy), C₁₋₆ alkyl (which may be optionally substituted with the same or different one or more substituents selected from halogen atom, hydroxy group, C₁₋₄ alkoxy or C₃₋₇ cycloalkyl), C₃₋₇ cycloalkyl (which may be optionally substituted with the same or different one or more substituents selected from halogen atom, hydroxy group, C₁₋₆ alkyl which may be optionally substituted with the same or different one or more halogen atoms, or C₁₋₆ alkoxy), cyano, C₁₋₆ alkoxy (which may be optionally substituted with the same or different one or more substituents selected from halogen atom, hydroxy group or C₁₋₆ alkoxy), C₃₋₇ cycloalkoxy (which may be optionally substituted with the same or different one or more substituents selected from halogen atom, hydroxy group, C₁₋₆ alkyl or C₁₋₆ alkoxy), or 5- to 10-membered aromatic heterocyclyl group (which may be optionally substituted with the same or different one or more substituents selected from halogen atom, hydroxy group, C₁₋₆ alkyl or C₁₋₆ alkoxy).

### [Item 5]

The compound according to any one of Items 1 to 4 or a pharmaceutically acceptable salt thereof, wherein ring G is selected from the following formulae (1b-1) to (1b-7): 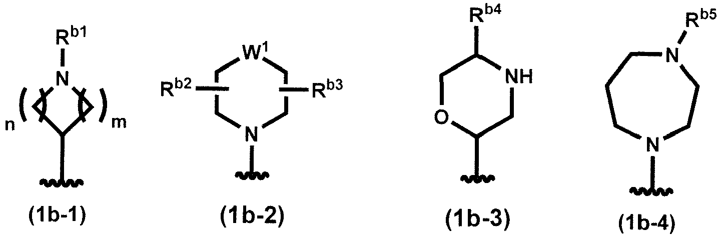 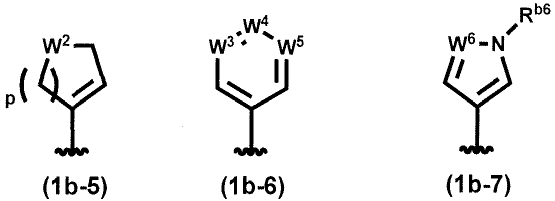 wherein
W¹ and W² are each independently NR^{b7}, oxygen atom or CR^{b8}R^{b9};
W³, W⁴, W⁵ and W⁶ are each independently nitrogen atom or CR^{b10};
R^{b1} to R^{b10} are each independently hydrogen atom, halogen atom, C₁₋₆ alkyl (which may be optionally substituted with the same or different one or more substituents selected from halogen atom or C₁₋₄ alkoxy), C₁₋₆ alkoxy (which may be optionally substituted with the same or different one or more halogen atoms), C₆₋₁₀ aromatic carbocyclyl group (which may be optionally substituted with the same or different one or more substituents selected from halogen atom, C₁₋₆ alkyl or C₁₋₆ alkoxy), C₃₋₇ cycloalkyl (which may be optionally substituted with the same or different one or more substituents selected from halogen atom, C₁₋₆ alkyl or C₁₋₆ alkoxy), 5- to 10-membered aromatic heterocyclyl group (which may be optionally substituted with the same or different one or more substituents selected from halogen atom, C₁₋₆ alkyl or C₁₋₆ alkoxy), or C₃₋₇ cycloalkoxy (which may be optionally substituted with the same or different one or more substituents selected from halogen atom, C₁₋₆ alkyl or C₁₋₆ alkoxy), and R^{b2} and R^{b3} may bind to the same carbon atom if chemically possible;
wherein R^{b2} and R^{b3} may be taken together via C₁₋₆ alkylene to form a fused ring or a bridged ring; and
n, m and p are each independently an integer of 1 or 2.

### [Item 6A]

The compound according to any one of Items 1 to 5 or a pharmaceutically acceptable salt thereof, wherein ring E is selected from the following formulae (1c-1) to (1c-4): 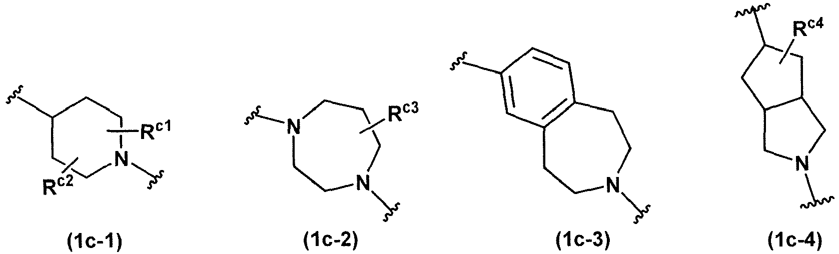 wherein
R^{c1}, R^{c2}, R^{c3} and R^{c4} are each independently hydrogen atom, halogen atom, cyano, C₁₋₆ alkyl (which may be optionally substituted with the same or different one or more substituents selected from halogen atom or C₁₋₆ alkoxy), C₁₋₆ alkoxy (which may be optionally substituted with the same or different one or more halogen atoms), C₆₋₁₀ aromatic carbocyclyl group (which may be optionally substituted with the same or different one or more substituents selected from halogen atom, C₁₋₆ alkyl or C₁₋₆ alkoxy), C₃₋₇ cycloalkyl (which may be optionally substituted with the same or different one or more substituents selected from halogen atom, C₁₋₆ alkyl or C₁₋₆ alkoxy), or C₃₋₇ cycloalkoxy (which may be optionally substituted with the same or different one or more substituents selected from halogen atom, C₁₋₆ alkyl or C₁₋₆ alkoxy), and R^{c1} and R^{c2} may bind to the same carbon atom if chemically possible; and
wherein R^{c1} and R^{c2} may be taken together via C₁₋₆ alkylene to form a fused ring or a bridged ring.

### [Item 6]

The compound according to any one of Items 1 to 5 or a pharmaceutically acceptable salt thereof, wherein ring E is selected from the following formulae (1c-1) to (1c-3): 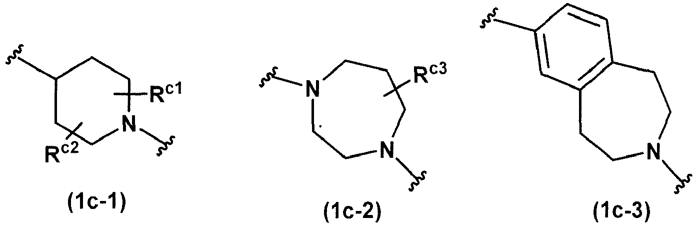 wherein
R^{c1}, R^{c2} and R^{c3} are each independently hydrogen atom, halogen atom, cyano, C₁₋₆ alkyl (which may be optionally substituted with the same or different one or more substituents selected from halogen atom or C₁₋₆ alkoxy), C₁₋₆ alkoxy (which may be optionally substituted with the same or different one or more halogen atoms), C₆₋₁₀ aromatic carbocyclyl group (which may be optionally substituted with the same or different one or more substituents selected from halogen atom, C₁₋₆ alkyl or C₁₋₆ alkoxy), C₃₋₇ cycloalkyl (which may be optionally substituted with the same or different one or more substituents selected from halogen atom, C₁₋₆ alkyl or C₁₋₆ alkoxy), or C₃₋₇ cycloalkoxy (which may be optionally substituted with the same or different one or more substituents selected from halogen atom, C₁₋₆ alkyl or C₁₋₆ alkoxy), and R^{c1} and R^{c2} may bind to the same carbon atom if chemically possible; and
wherein R^{c1} and R^{c2} may be taken together via C₁₋₆ alkylene to form a fused ring or a bridged ring.

### [Item 7A]

The compound according to any one of Items 1 to 6A and Item 6 or a pharmaceutically acceptable salt thereof, wherein ring E is selected from the following formulae (1c-11) to (1c-41): 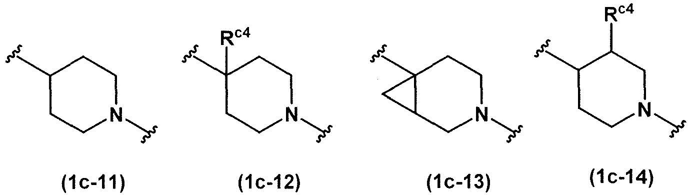 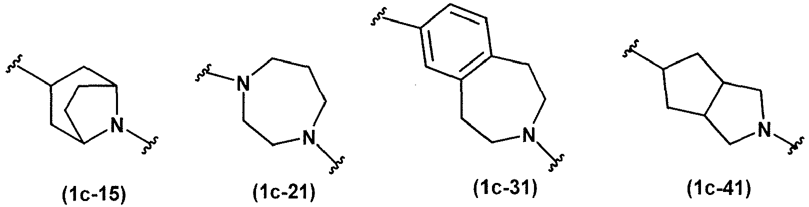 wherein
R^{c4} is each independently halogen atom, cyano, C₁₋₆ alkyl (which may be optionally substituted with the same or different one or more substituents selected from halogen atom or C₁₋₆ alkoxy), C₁₋₆ alkoxy (which may be optionally substituted with the same or different one or more halogen atoms), C₆₋₁₀ aromatic carbocyclyl group (which may be optionally substituted with one or more substituents selected from halogen atom, C₁₋₆ alkyl or C₁₋₆ alkoxy), C₃₋₇ cycloalkyl (which may be optionally substituted with the same or different one or more substituents selected from halogen atom, C₁₋₆ alkyl or C₁₋₆ alkoxy), or C₃₋₇ cycloalkoxy (which may be optionally substituted with the same or different one or more substituents selected from halogen atom, C₁₋₆ alkyl or C₁₋₆ alkoxy).

### [Item 7]

The compound according to any one of Items 1 to 6A and Item 6 or a pharmaceutically acceptable salt thereof, wherein ring E is selected from the following formulae (1c-11) to (1c-31): 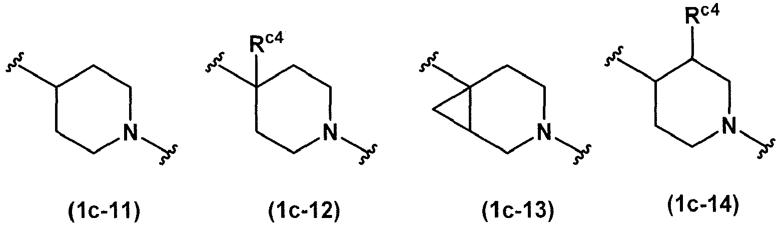 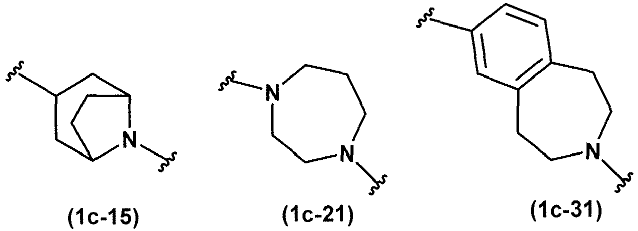 wherein
R^{c4} is each independently halogen atom, cyano, C₁₋₆ alkyl (which may be optionally substituted with the same or different one or more substituents selected from halogen atom or C₁₋₆ alkoxy), C₁₋₆ alkoxy (which may be optionally with the same or different one or more halogen atoms), C₆₋₁₀ aromatic carbocyclyl group (which may be optionally substituted with the same or different one or more substituents selected from halogen atom, C₁₋₆ alkyl or C₁₋₆ alkoxy), C₃₋₇ cycloalkyl (which may be optionally substituted with the same or different one or more substituents selected from halogen atom, C₁₋₆ alkyl or C₁₋₆ alkoxy), or C₃₋₇ cycloalkoxy (which may be optionally substituted with the same or different one or more substituents selected from halogen atom, C₁₋₆ alkyl or C₁₋₆ alkoxy).

### [Item 8]

The compound according to any one of Items 1 to 6A, Item 6, Item 7A and Item 7 or a pharmaceutically acceptable salt thereof, wherein the formula is represented by formula (3) : 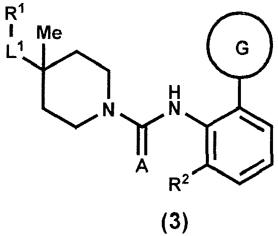 wherein
R² is C₁₋₆ alkoxy which may be optionally substituted with halogen atom; and
R¹, A, L¹ and ring G are as defined in Item 1.

### [Item 9]

The compound according to any one of Items 1 to 6A, Item 6, Item 7A, Item 7 and Item 8 or a pharmaceutically acceptable salt thereof, wherein L¹ is single bond, -CH₂- or oxygen atom.

### [Item 10]

The compound according to any one of Items 1 to 6A, Item 6, Item 7A and Items 7 to 9 or a pharmaceutically acceptable salt thereof, wherein R¹ is selected from the following formulae (1a-1) to (1a-3): 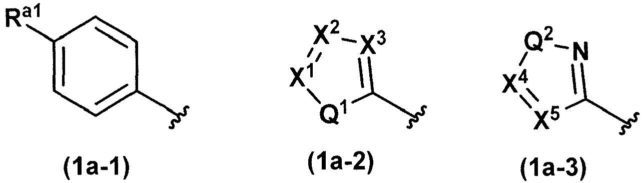 wherein
X¹ to X⁵ are each independently nitrogen atom or CR^{a4};
Q¹ and Q² are each independently oxygen atom or sulfur atom;
R^{a1} is hydrogen atom, halogen atom, cyano, C₁₋₆ alkyl (which may be optionally substituted with the same or different one or more substituents selected from halogen atom, hydroxy group or C₁₋₆ alkoxy), or C₁₋₄ alkoxy (which may be optionally substituted with the same or different one or more substituents selected from halogen atom, hydroxy group or C₁₋₆ alkoxy); and
R^{a4} is each independently (if there are plural CR^{a4}, each R^{a4} is independently) hydrogen atom, halogen atom, C₆₋₁₀ aromatic carbocyclyl group (which may be optionally substituted with the same or different one or more substituents selected from halogen atom, hydroxy group, C₁₋₆ alkyl or C₁₋₆ alkoxy), C₁₋₆ alkyl (which may be optionally substituted with the same or different one or more substituents selected from halogen atom, hydroxy group, C₁₋₆ alkoxy or C₃₋₇ cycloalkyl), C₃₋₇ cycloalkyl (which may be optionally substituted with the same or different one or more substituents selected from halogen atom, hydroxy group, C₁₋₆ alkyl or C₁₋₆ alkoxy), C₁₋₆ alkoxy (which may be optionally substituted with the same or different one or more substituents selected from halogen atom, hydroxy group or C₁₋₆ alkoxy), C₃₋₇ cycloalkoxy (which may be optionally substituted with the same or different one or more substituents selected from halogen atom, hydroxy group, C₁₋₆ alkyl or C₁₋₆ alkoxy), or 5- to 10-membered aromatic heterocyclyl group (which may be optionally substituted with the same or different one or more substituents selected from halogen atom, hydroxy group, C₁₋₆ alkyl or C₁₋₆ alkoxy).

### [Item 11]

The compound according to any one of Items 1 to 6A, Item 6, Item 7A and Items 7 to 10 or a pharmaceutically acceptable salt thereof, wherein ring G is selected from the following formulae (1b-1) to (1b-3): 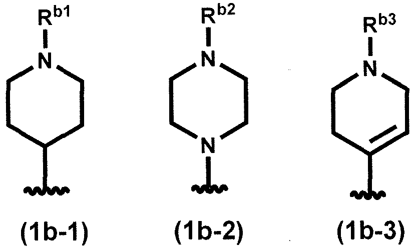 wherein
R^{b1} to R^{b3} are each independently hydrogen atom, C₁₋₆ alkyl (which may be optionally substituted with the same or different one or more substituents selected from halogen atom or C₁₋₆ alkoxy), C₁₋₆ alkoxy (which may be optionally substituted with the same or different one or more halogen atoms), 5- to 10-membered aromatic heterocyclyl group (which may be optionally substituted with the same or different one or more substituents selected from halogen atom, C₁₋₆ alkyl or C₁₋₆ alkoxy), C₆₋₁₀ aromatic carbocyclyl group (which may be optionally substituted with the same or different one or more substituents selected from halogen atom, C₁₋₆ alkyl or C₁₋₆ alkoxy), or C₃₋₇ cycloalkyl (which may be optionally substituted with the same or different one or more substituents selected from halogen atom, C₁₋₆ alkyl or C₁₋₆ alkoxy).

### [Item 12]

The compound according to any one of Items 1 to 6A, Item 6, Item 7A and Items 7 to 11 or a pharmaceutically acceptable salt thereof, wherein R² is halogen atom selected from F, Cl or Br, and A is oxygen atom.

### [Item 13]

The compound according to any one of Items 1 to 6A, Item 6, Item 7A and Items 7 to 12 or a pharmaceutically acceptable salt thereof, wherein R¹ is the following formula (1a-1): 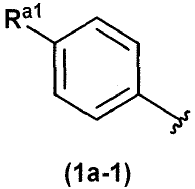 wherein
R^{a1} is hydrogen atom, halogen atom, cyano, C₁₋₆ alkyl (which may be optionally substituted with the same or different one or more substituents selected from halogen atom, hydroxy group or C₁₋₆ alkoxy), or C₁₋₄ alkoxy (which may be optionally substituted with the same or different one or more substituents selected from halogen atom, hydroxy group or C₁₋₆ alkoxy).

### [Item 14]

The compound according to any one of Items 1 to 6A, Item 6, Item 7A and Items 7 to 12 or a pharmaceutically acceptable salt thereof, wherein R¹ is the following formula (1a-2): 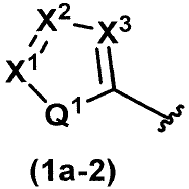 wherein
X¹ to X³ are each independently nitrogen atom or CR^{a4};
Q¹ is oxygen atom or sulfur atom;
R^{a4} is as defined in Item 10; and
   L¹ is single bond.

### [Item 15]

The compound according to any one of Items 1 to 6A, Item 6, Item 7A and Items 7 to 12 or a pharmaceutically acceptable salt thereof, wherein R¹ is selected from the following formulae (1a-21) to (1a-25): 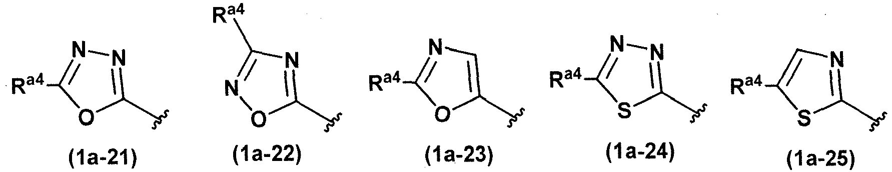 wherein R^{a4} is as defined in Item 10; and
L¹ is single bond.

### [Item 16]

The compound according to Item 15 or a pharmaceutically acceptable salt thereof, wherein R^{a4} is (if there are plural CR^{a4}, each R^{a4} is independently) C₆₋₁₀ aromatic carbocyclyl group (which may be optionally substituted with the same or different one or more substituents selected from halogen atom, hydroxy group, C₁₋₆ alkyl or C₁₋₆ alkoxy), C₁₋₆ alkyl (which may be optionally substituted with the same or different one or more substituents selected from halogen atom, hydroxy group, C₁₋₆ alkoxy or C₃₋₇ cycloalkyl), C₃₋₇ cycloalkyl (which may be optionally substituted with the same or different one or more substituents selected from halogen atom, hydroxy group, C₁₋₆ alkyl or C₁₋₆ alkoxy), or 5- to 10-membered aromatic heterocyclyl group (which may be optionally substituted with the same or different one or more substituents selected from halogen atom, hydroxy group, C₁₋₆ alkyl or C₁₋₆ alkoxy).

### [Item 17]

The compound according to any one of Items 1 to 6A, Item 6, Item 7A and Items 7 to 12 or a pharmaceutically acceptable salt thereof, wherein R¹ is the following formula (1a-3): 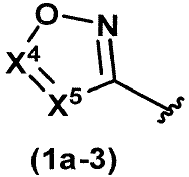 wherein X⁴ and X⁵ are as defined in Item 10; and
L¹ is single bond.

### [Item 18]

The compound according to Item 17 or a pharmaceutically acceptable salt thereof, wherein R¹ is selected from the following formulae (1a-31) to (1a-34): 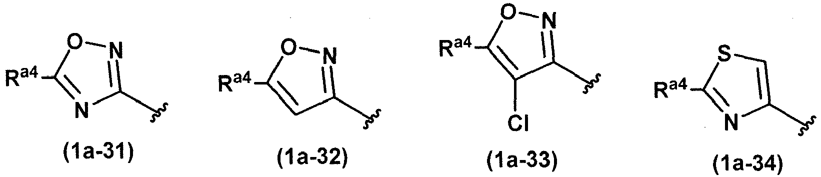 wherein R^{a4} is as defined in Item 10.

### [Item 19]

The compound according to Item 17 or 18 or a pharmaceutically acceptable salt thereof, wherein R^{a4} is (if there are plural CR^{a4}, each R^{a4} is independently) C₁₋₆ alkyl (which may be optionally substituted with the same or different one or more substituents selected from halogen atom, hydroxy group, C₁₋₆ alkoxy or C₃₋₇ cycloalkyl), C₃₋₇ cycloalkyl (which may be optionally substituted with the same or different one or more substituents selected from halogen atom, hydroxy group, C₁₋₆ alkyl or C₁₋₆ alkoxy), or C₁₋₆ alkoxy (which may be optionally substituted with the same or different one or more substituents selected from halogen atom, hydroxy group or C₁₋₆ alkoxy).

### [Item 20]

The compound according to any one of Items 1 to 6A, Item 6, Item 7A and Items 7 to 19 or a pharmaceutically acceptable salt thereof, wherein ring G is the following formula (1b-2): 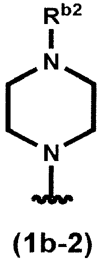 wherein R^{b2} is as defined in Item 11.

### [Item 21]

The compound according to any one of Items 1 to 6A, Item 6, Item 7A and Items 7 to 19 or a pharmaceutically acceptable salt thereof, wherein ring G is the following formula (1b-1): 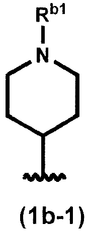 wherein
R^{b1} is C₁₋₆ alkyl (which may be optionally substituted with the same or different one or more substituents selected from halogen atom or C₁₋₆ alkoxy), 5- to 10-membered aromatic heterocyclyl group (which may be optionally substituted with the same or different one or more substituents selected from halogen atom, C₁₋₆ alkyl or C₁₋₆ alkoxy), or C₃₋₇ cycloalkyl (which may be optionally substituted with the same or different one or more substituents selected from halogen atom, C₁₋₆ alkyl or C₁₋₆ alkoxy).

### [Item 22]

The compound according to any one of Items 1 to 6A, Item 6, Item 7A and Items 7 to 19 or a pharmaceutically acceptable salt thereof, wherein ring G is the following formula (1b-3): 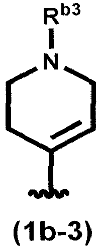 wherein
R^{b3} is C₁₋₆ alkyl (which may be optionally substituted with the same or different one or more substituents selected from halogen atom or C₁₋₆ alkoxy), 5- to 10-membered aromatic heterocyclyl group (which may be optionally substituted with the same or different one or more substituents selected from halogen atom, C₁₋₆ alkyl or C₁₋₆ alkoxy), or C₃₋₇ cycloalkyl (which may be optionally substituted with the same or different one or more substituents selected from halogen atom, C₁₋₆ alkyl or C₁₋₆ alkoxy).

### [Item 23]

The compound according to Item 1 or a pharmaceutically acceptable salt thereof, wherein the compound is selected from the following compounds:
Example 20: N-{2-fluoro-6-[4-(propan-2-yl)piperazin-1-yl]phenyl}-4-methyl-4-[5-(propan-2-yl)-1,2,4-oxadiazol-3-yl]piperidine-1-carboxamide
Example 22: N-{2-fluoro-6-[4-(propan-2-yl)piperazin-1-yl]phenyl}-4-methyl-4-(5-methyl-1,2,4-oxadiazol-3-yl)piperidine-1-carboxamide
Example 44: 4-(5-cyclopropyl-1,2,4-oxadiazol-3-yl)-N-{2-fluoro-6-[1-(propan-2-yl)-1,2,3,6-tetrahydropyridin-4-yl]phenyl}-4-methylpiperidine-1-carboxamide
Example 45: 4-(5-cyclopropyl-1,2,4-oxadiazol-3-yl)-N-{2-fluoro-6-[4-(propan-2-yl)p.iperazin-1-yl]phenyl}-4-methylpiperidine-1-carboxamide
Example 53: 4-(5-cyclopropyl-1,2-oxazol-3-yl)-N-{2-fluoro-6-[1-(propan-2-yl)piperidin-4-yl]phenyl}-4-methylpiperidine-1-carboxamide
Example 54: 4-(5-cyclopropyl-1,2-oxazol-3-yl)-N-{2-fluoro-6-[4-(propan-2-yl)piperazin-1-yl]phenyl}-4-methylpiperidine-1-carboxamide
Example 55: 4-(5-cyclopropyl-1,2-oxazol-3-yl)-N-{2-fluoro-6-[1-(propan-2-yl)-1,2,3,6-tetrahydropyridin-4-yl]phenyl}-4-methylpiperidine-1-carboxamide
Example 61: N-{2-chloro-6-[4-(propan-2-yl)piperazin-1-yl]phenyl}-4-(5-cyclopropyl-1,2-oxazol-3-yl)-4-methylpiperidine-1-carboxamide
Example 67: N-{2-chloro-6-[4-(propan-2-yl)piperazin-1-yl]phenyl}-4-(5-cyclopropyl-1,2,4-oxadiazol-3-yl)-4-methylpiperidine-1-carboxamide
Example 77: N-{2-chloro-6-[4-(propan-2-yl)piperazin-1-yl]phenyl}-4-methyl-4-(4-methylphenyl)piperidine-1-carboxamide.

### [Item 24]

The compound according to Item 1 or a pharmaceutically acceptable salt thereof, wherein the compound is selected from the following compounds:
Example 87: 4-(5-cyclopropyl-1,2,4-oxadiazol-3-yl)-4-ethyl-N-{2-fluoro-6-[4-(propan-2-yl)piperazin-1-yl]phenyl}piperidine-1-carboxamide
Example 89: N-{2-chloro-6-[4-(propan-2-yl)piperazin-1-yl]phenyl}-4-(5-cyclopropyl-1,2,4-oxadiazol-3-yl)-4-ethylpiperidine-1-carboxamide
Example 101: N-{2-chloro-6-[4-(propan-2-yl)piperazin-1-yl]phenyl}-4-(5-cyclopropyl-1,3-thiazol-2-yl)-4-methylpiperidine-1-carboxamide
Example 115: N-{2-chloro-6-[4-(propan-2-yl)piperazin-1-yl]phenyl}-4-{5-[(1S,2S)-2-fluorocyclopropyl]-1,2,4-oxadiazol-3-yl}-4-methylpiperidine-1-carboxamide
Example 116: N-{2-chloro-6-[4-(propan-2-yl)piperazin-1-yl]phenyl}-4-{5-[(1R,2R)-2-fluorocyclopropyl]-1,2,4-oxadiazol-3-yl}-4-methylpiperidine-1-carboxamide
Example 117: N-{2-chloro-6-[4-(propan-2-yl)piperazin-1-yl]phenyl}-4-methyl-4-{5-[(1S,2R)-2-methylcyclopropyl]-1,2,4-oxadiazol-3-yl}piperidine-1-carboxamide
Example 118: N-{2-chloro-6-[4-(propan-2-yl)piperazin-1-yl]phenyl}-4-methyl-4-{5-[(1R,2S)-2-methylcyclopropyl]-1,2,4-oxadiazol-3-yl}piperidine-1-carboxamide
Example 130: N-{2-chloro-6-[1-(propan-2-yl)-1,2,3,6-tetrahydropyridin-4-yl]phenyl}-4-(5-cyclopropyl-1,2,4-oxadiazol-3-yl)-4-methylpiperidine-1-carboxamide
Example 144: N-{2-chloro-6-[1-(propan-2-yl)-1,2,3,6-tetrahydropyridin-4-yl]phenyl}-4-(5-cyclopropyl-1,2-oxazol-3-yl)-4-methylpiperidine-1-carboxamide
Example 147: N-{2-chloro-6-[1-(propan-2-yl)-1,2,3,6-tetrahydropyridin-4-yl]phenyl}-4-methyl-4-(4-methylphenyl)piperidine-1-carboxamide.

### [Item 25]

A pharmaceutical composition comprising the compound according to any one of Items 1 to 6A, Item 6, Item 7A and Items 7 to 24 or a pharmaceutically acceptable salt thereof.

### [Item 26]

A medicament for treating a disease associated with orexin receptor type 2, comprising the compound according to any one of Items 1 to 6A, Item 6, Item 7A and Items 7 to 24 or a pharmaceutically acceptable salt thereof.

### [Item 27]

A medicament for treating narcolepsy, idiopathic hypersomnia, hypersomnia, sleep apnea syndrome, narcolepsy syndrome involving narcolepsy-like symptom, hypersomnia associated with Parkinson's disease, hypersomnia associated with dementia with Lewy body, hypersomnia syndrome involving daytime hypersomnia (for example, Kleine-Levin syndrome, major depression accompanied by hypersomnia, dementia with Lewy body, Parkinson's disease, progressive supranuclear palsy, Prader-Willi syndrome, Moebius syndrome, hypoventilation syndrome, Niemann-Pick disease type C, brain contusion, cerebral infarction, brain tumor, muscular dystrophy, multiple sclerosis, acute disseminated encephalomyelitis, Guillain-Barre syndrome, Rasmussen's encephalitis, Wernicke's encephalopathy, limbic encephalitis, Hashimoto encephalopathy), coma, loss of consciousness, obesity (for example, malignant mast cell, extrinsic obesity, hyperinsulinar obesity, hyperplasmic obesity, hypophysial obesity, hypoplasmic obesity, hypothyroid obesity, hypothalamic obesity, symptomatic obesity, childhood obesity, upper body obesity, alimentary obesity, gonadal obesity, systemic mastocytosis, primary obesity, central obesity), insulin resistance syndrome, Alzheimer, impaired consciousness such as coma, side effect or complication caused by anesthesia, sleep disturbance, sleep problem, insomnia, intermittent sleep, night myoclonus, REM sleep interruption, jet lag, jet lag syndrome, sleep disorder of shift workers, dyssomnia, sleep terror, depression, major depression, sleepwalking, enuresis, sleep disorder, Alzheimer's sundown syndrome, disease associated with circadian rhythm, fibromyalgia, condition resulting from decrease in sleeping quality, bulimia, obsessive eating disorder, obesity-related diseases, hypertension, diabetes, elevated plasma insulin level/insulin resistance, hyperlipemia, hyperlipidaemia, endometrial cancer, breast cancer, prostate cancer, colon cancer, cancer, osteoarthritis, obstructive sleep apnea, cholelithiasis, gallstone, heart disease, abnormal heartbeat, arrhythmia, myocardial infarction, congestive heart failure, coronary heart disease, cardiovascular disease, sudden death, polycystic ovary, craniopharyngioma, Prader-Willi syndrome, Froehlich syndrome, growth hormone deficiency, normal variant short stature, Turner syndrome, children suffering from acute lymphoblastic leukemia, syndrome X, reproductive hormone abnormality, decrease of fecundability, infertility, hypogonadism in men, sexual/reproductive-function dysfunction such as hirsutism in women, fetal defect associated with maternity obesity, gastrointestinal motility disorder such as obesity-related gastroesophageal reflux, obesity hypoventilation syndrome (Pickwickian syndrome), respiratory disease such as respiratory distress, inflammation such as vascular systemic inflammation, arteriosclerosis, hypercholesterolemia, hyperuricemia, low back pain, gallbladder disease, gout, renal cancer, secondary risk of obesity such as risk of left ventricle hypertrophy, migraine, headache, neuropathic pain, Parkinson's disease, psychosis, schizophrenia, facial flushing, night sweat, disease in genitalium/urinary system, disease associated with sexual function or fecundability, dysthymic disorder, bipolar disorder, bipolar I disorder, bipolar II disorder, cyclothymic disorder, acute stress disorder, agoraphobia, generalized anxiety disorder, obsessive-compulsive disorder, panic attack, panic disorder, posttraumatic stress disorder, separation anxiety disorder, social phobia, anxiety disorder, acute neurological and psychiatric disorder such as cerebral deficiency developed after heart bypass surgery or heart transplant, stroke, ischemic stroke, cerebral ischemia, spinal cord trauma, head injury, periparturient hypoxia, cardiac arrest, hypoglycemic nerve injury, Huntington's disease, amyotrophic lateral sclerosis, multiple sclerosis, eye damage, retinopathy, cognitive impairment, muscle spasm, tremor, epilepsy, disorder associated with muscle spasm, delirium, amnestic disorder, age-associated cognitive decline, schizoaffective disorder, paranoia, drug addiction, movement disorder, chronic fatigue syndrome, fatigue, medication-induced parkinsonian syndrome, Gilles de la Tourette syndrome, chorea, myoclonus, tic, restless legs syndrome, dystonia, dyskinesia, attention deficit hyperactivity disorder (ADHD), conduct disorder, urinary incontinence, withdrawal symptom, trigeminal neuralgia, hearing loss, tinnitus, nerve injury, retinopathy, macular degeneration, vomiting, cerebral edema, pain, bone pain, arthralgia, toothache, cataplexy, or traumatic brain injury, comprising the compound according to any one of Items 1 to 6A, Item 6, Item 7A and Items 7 to 24 or a pharmaceutically acceptable salt thereof.

### [Item 28]

A medicament for treating narcolepsy, idiopathic hypersomnia, hypersomnia, sleep apnea syndrome, narcolepsy syndrome involving narcolepsy-like symptom, hypersomnia associated with Parkinson's disease, or hypersomnia associated with dementia with Lewy body, comprising the compound according to any one of Items 1 to 6A, Item 6, Item 7A and Items 7 to 24 or a pharmaceutically acceptable salt thereof.

### [Item 29]

A medicament for treating narcolepsy, comprising the compound according to any one of Items 1 to 6A, Item 6, Item 7A and Items 7 to 24 or a pharmaceutically acceptable salt thereof.

### [Item 30]

A medicament for treating idiopathic hypersomnia, comprising the compound according to any one of Items 1 to 6A, Item 6, Item 7A and Items 7 to 24 or a pharmaceutically acceptable salt thereof.

### [Item 31]

A medicament for treating hypersomnia associated with Parkinson's disease, comprising the compound according to any one of Items 1 to 6A, Item 6, Item 7A and Items 7 to 24 or a pharmaceutically acceptable salt thereof.

### [Item 32]

A medicament for treating hypersomnia associated with dementia with Lewy body, comprising the compound according to any one of Items 1 to 6A, Item 6, Item 7A and Items 7 to 24 or a pharmaceutically acceptable salt thereof.

### [Item 33]

A method of treating narcolepsy, idiopathic hypersomnia, hypersomnia, sleep apnea syndrome, narcolepsy syndrome involving narcolepsy-like symptom, hypersomnia associated with Parkinson's disease, or hypersomnia associated with dementia with Lewy body, which comprises administering a therapeutically effective amount of the compound according to any one of Items 1 to 6A, Item 6, Item 7A and Items 7 to 24 or a pharmaceutically acceptable salt thereof to a patient in need thereof.

### [Item 34]

Use of the compound according to any one of Items 1 to 6A, Item 6, Item 7A and Items 7 to 24 or a pharmaceutically acceptable salt thereof, in the manufacture of a medicament for treating narcolepsy, idiopathic hypersomnia, hypersomnia, sleep apnea syndrome, narcolepsy syndrome involving narcolepsy-like symptom, hypersomnia associated with Parkinson's disease, or hypersomnia associated with dementia with Lewy body.

### [Item 35]

A pharmaceutical composition comprising the compound according to any one of Items 1 to 6A, Item 6, Item 7A and Items 7 to 24 or a pharmaceutically acceptable salt thereof for use in the treatment of narcolepsy, idiopathic hypersomnia, hypersomnia, sleep apnea syndrome, narcolepsy syndrome involving narcolepsy-like symptom, hypersomnia associated with Parkinson's disease, or hypersomnia associated with dementia with Lewy body.

### [Item 36]

The compound according to any one of Items 1 to 6A, Item 6, Item 7A and Items 7 to 24 or a pharmaceutically acceptable salt thereof for use in the treatment and/or prophylaxis of narcolepsy, idiopathic hypersomnia, hypersomnia, sleep apnea syndrome, narcolepsy syndrome involving narcolepsy-like symptom, hypersomnia associated with Parkinson's disease, or hypersomnia associated with dementia with Lewy body.

### DESCRIPTION OF EMBODIMENTS

Hereinafter, the present invention is explained in more detail. The number of carbon atoms in the definition of "substituent group" as used herein may be expressed as, for example, "C₁₋₆". Specifically, the expression "C₁₋₆ alkyl" means an alkyl group having 1 to 6 carbon atoms. As used herein, a substituent group which is not accompanied with the term "optionally-substituted" or "substituted" means an "unsubstituted" substituent group. For example, "C₁₋₆ alkyl" means "unsubstituted C₁₋₆ alkyl".

The substituent group as used herein may be expressed without the term "group". In case that "optionally-substituted" is used in the definition of substituent group, the number of the substituting group is not limited as long as the substitution is available, *i.e*., it is one or more. It means that the possible number of substituting groups is the substitution-available number on carbon atoms or carbon/nitrogen atoms in a substituent group which are acceptable for substitution. Unless otherwise specified, the definition of each substituent group also extends over the case that the substituent group is partially included in another substituent group or the case that the substituent group is attached to another substituent group.

Unless otherwise specified, the binding site of substituent groups is not limited as long as the site is chemically available to be bound. Also, in the structural formula as used herein, wedge solid and dashed bonds mean absolute configuration, and thick solid and dashed bonds mean relative configuration.

The "halogen" includes, for example, fluorine, chlorine, bromine, iodine, and the like. Preferably, it is fluorine or chlorine.

The "C₁₋₄ alkyl" means straight or branched chain saturated hydrocarbon group having 1 to 4 carbon atoms, and the "C₁₋₆ alkyl" means straight or branched chain saturated hydrocarbon group having 1 to 6 carbon atoms. Specific examples of the "C₁₋₄ alkyl" include methyl, ethyl, propyl, isopropyl, butyl, isobutyl, sec-butyl and tert-butyl, and specific examples of the "C₁₋₆ alkyl" include pentyl, isopentyl, neopentyl, 1-ethylpropyl, hexyl and a structural isomer thereof, besides the above C₁₋₄ alkyl. The "C₁₋₆ alkyl" or "C₁₋₄ alkyl" is preferably methyl, ethyl, propyl and isopropyl, more preferably methyl and isopropyl.

The "C₁₋₆ alkylene" means divalent straight or branched chain saturated hydrocarbon group having 1 to 6 carbon atoms. The "C₁₋₆ alkylene" includes preferably "C₁₋₄ alkylene", more preferably "C₁₋₃ alkylene". Specific examples of the "C₁₋₃ alkylene" include methylene, ·ethylene, propylene, trimethylene, and the like. Specific examples of the "C₁₋₄ alkylene" include butylene, 1,1-dimethylethylene, 1,2-dimethylethylene, 1-methyltrimethylene, 2-methyltrimethylene, and the like, besides the specific examples listed in the above "C₁₋₃ alkylene". Specific examples of the "C₁₋₆ alkylene" include pentylene, 1,1-dimethyltrimethylene, 1,2-dimethyltrimethylene, 1-methylbutylene, 2-methylbutylene, 1-methylpentylene, 2-methylpentylene, 3-methylpentylene, hexylene, and the like, besides the specific examples listed in the above "C₁₋₄ alkylene".

The "C₃₋₇ cycloalkyl" means a non-aromatic cyclic hydrocarbon group (*i.e*., saturated hydrocarbon group and partially-unsaturated hydrocarbon group) having 3 to 7 carbon atoms. Preferably, it is "C₃₋₆ cycloalkyl". The "C₃₋₇ cycloalkyl" also includes a bridged one. Specific examples of the "C₃₋₇ cycloalkyl" include cyclopropyl, cyclobutyl, cyclopentyl, cyclohexyl, cyclopentenyl, cyclohexenyl, cycloheptyl, and the like.

The "C₃₋₇ cycloalkyl" also includes a bi-cyclic condensed ring in which the "C₃₋₇ cycloalkyl" is fused with benzene ring or a 5- or 6-membered ring having one heteroatom selected from nitrogen, sulfur, or oxygen atom, or the same or different two or more (for example, 2 to 4) heteroatoms thereof (for example, "5- or 6-membered monocyclic aromatic heterocycle" mentioned below, and 5- or 6-membered ring in "4- to 10-membered saturated heterocycle" mentioned below).

The "C₁₋₄ alkoxy" means oxy group substituted with the above "C₁₋₄ alkyl", and the "C₁₋₆ alkoxy" means oxy group substituted with the above "C₁₋₆ alkyl". Specific examples of the "C₁₋₄ alkoxy" include methoxy, ethoxy, propoxy, isopropoxy, butoxy, isobutoxy, sec-butoxy, tert-butoxy, and the like, and specific examples of the "C₁₋₆ alkoxy" include pentyloxy, hexyloxy, and the like. Preferably, the "C₁₋₄ alkoxy" includes methoxy, ethoxy and isopropoxy.

The "C₃₋₇ cycloalkoxy" means oxy group substituted with the above "C₃₋₇ cycloalkyl". Preferably, it is "C₃₋₆ cycloalkoxy". Specific examples of the "C₃₋₇ cycloalkoxy" include cyclopropyloxy, cyclobutyloxy, cyclopentyloxy, cyclohexyloxy, and the like. Preferably, the "C₃₋₇ cycloalkoxy" is cyclohexyloxy.

The "C₆₋₁₀ aromatic carbocyclyl group" means an aromatic hydrocarbon group having 6 to 10 carbon atoms, which is also referred to as "C₆₋₁₀ aryl". More preferably, it is phenyl. Specific examples of the "C₆₋₁₀ aromatic carbocyclyl group" includes phenyl, 1-naphthyl, 2-naphthyl, and the like.

The "C₆₋₁₀ aromatic carbocyclyl group" also includes a condensed ring in which "phenyl" is fused with a 5- or 6-membered ring having one heteroatom selected from nitrogen, sulfur or oxygen atom, or the same or different two or more (for example, 2 to 4) heteroatoms thereof (for example, "5- or 6-membered monocyclic aromatic heterocycle" mentioned below, and 5- or 6-membered ring in "4- to 10-membered saturated heterocycle" mentioned below), or a 5- to 7-membered cycloalkyl ring (for example, cyclopentane, cyclohexane or cycloheptane).

The "5- to 10-membered aromatic heterocyclyl group" means a mono- or poly-cyclic 5- to 10-membered aromatic heterocyclyl group having one heteroatom selected from nitrogen, sulfur or oxygen atom, or the same or different two or more (for example, 2 to 4) heteroatoms thereof, besides carbon atoms as the ring atoms. Preferably, it is "5- or 6-membered monocyclic aromatic heterocyclyl group". The "5- or 6-membered monocyclic aromatic heterocyclyl group" means a monocyclic 5- or 6-membered aromatic heterocyclyl group within the "5- to 10-membered aromatic heterocyclyl group".

The polycyclic aromatic heterocyclyl group in the above "5- to 10-membered aromatic heterocyclyl group" includes, for example, a condensed ring in which the same or different two monocyclic aromatic heterocycles are fused, and a condensed ring in which a monocyclic aromatic heterocycle and an aromatic ring (for example, benzene) or a non-aromatic ring (for example, cyclohexane) are fused.

Specific examples of the "5- to 10-membered aromatic heterocyclyl group" include pyrazolyl, imidazolyl, pyridyl, pyrimidinyl, pyrazinyl and pyridazinyl. In another embodiment, it preferably includes benzofuranyl in which the binding site is on the heteroaryl (furan) ring, pyridyl, pyrimidinyl, pyrazinyl and pyridazinyl.

The "C₃₋₆ saturated carbocyclic ring" means a monocyclic saturated or partially-unsaturated hydrocarbon ring having 3 to 6 carbon atoms. Specific examples of the "C₃₋₆ saturated carbocyclic ring" include cyclopropane, cyclobutane, cyclopentane, cyclohexane, cyclopropene, cyclobutene, cyclopentene, cyclohexene, cyclohexadiene, and the like. Preferably, it includes cyclopropane and cyclobutane.

The "4- to 10-membered saturated heterocycle" means a monocyclic or bicyclic saturated heterocycle composed of 4 to 10 atoms, which has the same or different one or more (for example, 2 to 4, preferably 2 to 3, more preferably 2) heteroatoms selected from the group consisting of oxygen atom, nitrogen atom and sulfur atom, besides carbon atoms as the ring atoms. The heterocycle includes a partially-unsaturated heterocycle, a partially-bridged heterocycle and a partially-spiro heterocycle. Preferably, the heterocycle is 5- or 6-membered saturated heterocycle. The bicyclic saturated heterocycle also includes a condensed ring in which a monocyclic saturated heterocycle and benzene or a monocyclic 5- to 6-membered aromatic heterocycle are fused. Also, the saturated heterocycle may further comprise one or two carbonyl, thiocarbonyl, sulfinyl or sulfonyl, that is, the saturated heterocycle includes, for example, a cyclic group such as lactam, thiolactam, lactone, thiolactone, cyclic imide, cyclic carbamate, and cyclic thiocarbamate, which the number of atoms composing 4- to 10-membered ring (*i.e*., ring size) or the number of heteroatoms composing heterocycle does not count the oxygen atom in carbonyl, sulfinyl and sulfonyl, and the sulfur atom in thiocarbonyl. The "4- to 10-membered saturated heterocycle" includes preferably monocyclic or bicyclic "4- to 8-membered saturated heterocycle", more preferably monocyclic "4- to 6-membered saturated heterocycle", and furthermore preferably monocyclic "5- or 6-membered saturated heterocycle". Specific examples of the "4- to 10-membered saturated heterocycle" include piperazine, oxetane, azetidine, pyrrolidine, pyrazolidine, imidazolidine, piperidine, morpholine, homopiperidine, oxetane, thiomorpholine, dioxothiomorpholine, hexamethyleneimine, oxazolidine, thiazolidine, imidazolidine, oxoimidazolidine, dioxoimidazolidine, oxooxazolidine, dioxooxazolidine, dioxothiazolidine, tetrahydrofuran, tetrahydropyran, and tetrahydropyridine, and the like. Preferably, the 4- to 10-membered saturated heterocycle is pyrrolidine, piperidine, piperazine, and morpholine. Specific examples of the bicyclic saturated heterocycle include indoline, isoindoline, dihydropurine, dihydrothiazolopyrimidine, dihydrobenzodioxane, dihydroindazole, dihydropyrrolopyridine, tetrahydroquinoline, decahydroquinoline, tetrahydroisoquinoline, decahydroisoquinoline, tetrahydronapthyridine and tetrahydropyridoazepine, and the like.

The "4- to 10-membered saturated heterocyclyl group" means a monovalent substituent derived from the above "4- to 10-membered saturated heterocycle", and the "4- to 6-membered saturated heterocyclyl group" means a monovalent substituent derived from "4- to 6-membered saturated heterocycle" which belongs to the above "4- to 10-membered saturated heterocycle". Preferably, it includes azetidinyl, pyrrolidinyl, piperidinyl, piperazinyl, morpholinyl, oxetanyl, tetrahydrofuranyl and tetrahydropyranyl.

The compound of the present invention includes various hydrate, solvate, and crystal polymorph thereof.

The compound of the present invention may include one or more isotope atoms such as D, ³H, ¹¹C, ¹³C, ¹⁴C, ¹³N, ¹⁵N, ¹⁵O, ³⁵S, ¹⁸F and ¹²⁵I by substitution, and such isotope-substituted compound is also included in the compound of the present invention.

The "pharmaceutically acceptable salt" as used herein means a pharmaceutically usable acid addition salt and a pharmaceutically usable base addition salt. Examples of the "pharmaceutically acceptable salt" include an acid addition salt such as acetate, propionate, butyrate, formate, trifluoroacetate, maleate, fumarate, tartrate, citrate, stearate, succinate, ethylsuccinate, malonate, lactobionate, gluconate, glucoheptonate, benzoate, methanesulfonate, benzenesulfonate, p-toluenesulfonate (tosylate), laurylsulfate, malate, ascorbate, mandelate, saccharate, xinafoate, pamoate, cinnamate, adipate, cysteine, N-acetylcysteine, hydrochloride, hydrobromide, phosphate, sulfate, hydroiodide, nicotinate, oxalate, picrate, thiocyanate, undecanoate, polyacrylate and carboxy vinyl polymer; an inorganic base addition salt such as lithium salt, sodium salt, potassium salt and calcium salt; an organic base addition salt such as morpholine and piperidine; and an amino acid addition salt such as aspartate and glutamate, but are not limited thereto.

The compound of the present invention may be orally or parenterally administered directly or as a suitable formulation such as drug product, medicament, and pharmaceutical composition. Specific examples of the formulation thereof may include tablet, capsule, powder, granule, liquid, suspension, injection, patch and gel patch, but are not limited thereto. The formulation can be prepared with pharmaceutically acceptable additive agents in known means.

The additive agents may be chosen for any purpose, including an excipient, a disintegrant, a binder, a fluidizer, a lubricant, a coating agent, a solubilizer, a solubilizing agent, a thickener, dispersant, a stabilizing agent, a sweetening agent, a flavor, and the like. Specific examples thereof include lactose, mannitol, microcrystalline cellulose, low-substituted hydroxypropylcellulose, corn starch, partially-pregelatinized starch, carmellose calcium, croscarmellose sodium, hydroxypropylcellulose, hydroxypropyl methylcellulose, polyvinyl alcohol, magnesium stearate, sodium stearyl fumarate, polyethylene glycol, propylene glycol, titanium oxide, talc, and the like.

The dose of the compound of the present invention should be suitably determined depending on subject animal for administration, administration route, target disease, and age, body weight, and condition of patients. For example, in the case of oral administration, about 0.01 mg as minimum to 10000 mg as maximum may be administered a day for an adult in one to several portions.

The compound of the present invention is a compound with agonist activity on orexin receptor type 2. Hence, the compound of the present invention may be a medicament useful for preventing or treating a disease associated with orexin receptor. Specific examples of the disease include narcolepsy, idiopathic hypersomnia, hypersomnia, sleep apnea syndrome, narcolepsy syndrome involving narcolepsy-like symptom, hypersomnia associated with Parkinson's disease, hypersomnia associated with dementia with Lewy body, hypersomnia syndrome involving daytime hypersomnia (for example, Kleine-Levin syndrome, major depression accompanied by hypersomnia, dementia with Lewy body, Parkinson's disease, progressive supranuclear palsy, Prader-Willi syndrome, Moebius syndrome, hypoventilation syndrome, Niemann-Pick disease type C, brain contusion, cerebral infarction, brain tumor, muscular dystrophy, multiple sclerosis, acute disseminated encephalomyelitis, Guillain-Barre syndrome, Rasmussen's encephalitis, Wernicke's encephalopathy, limbic encephalitis, Hashimoto encephalopathy), coma, loss of consciousness, obesity (for example, malignant mast cell, extrinsic obesity, hyperinsulinar obesity, hyperplasmic obesity, hypophysial obesity, hypoplasmic obesity, hypothyroid obesity, hypothalamic obesity, symptomatic obesity, childhood obesity, upper body obesity, alimentary obesity, gonadal obesity, systemic mastocytosis, primary obesity, central obesity), insulin resistance syndrome, Alzheimer, impaired consciousness such as coma, side effect or complication caused by anesthesia, sleep disturbance, sleep problem, insomnia, intermittent sleep, night myoclonus, REM sleep interruption, jet lag, jet lag syndrome, sleep disorder of shift workers, dyssomnia, sleep terror, depression, major depression, sleepwalking, enuresis, sleep disorder, Alzheimer's sundown syndrome, disease associated with circadian rhythm, fibromyalgia, condition resulting from decrease in sleeping quality, bulimia, obsessive eating disorder, obesity-related diseases, hypertension, diabetes, elevated plasma insulin level/insulin resistance, hyperlipemia, hyperlipidaemia, endometrial cancer, breast cancer, prostate cancer, colon cancer, cancer, osteoarthritis, obstructive sleep apnea, cholelithiasis, gallstone, heart disease, abnormal heartbeat, arrhythmia, myocardial infarction, congestive heart failure, coronary heart disease, cardiovascular disease, sudden death, polycystic ovary, craniopharyngioma, Prader-Willi syndrome, Froehlich syndrome, growth hormone deficiency, normal variant short stature, Turner syndrome, children suffering from acute lymphoblastic leukemia, syndrome X, reproductive hormone abnormality, decrease of fecundability, infertility, hypogonadism in men, sexual/reproductive-function dysfunction such as hirsutism in women, fetal defect associated with maternity obesity, gastrointestinal motility disorder such as obesity-related gastroesophageal reflux, obesity hypoventilation syndrome (Pickwickian syndrome), respiratory disease such as respiratory distress, inflammation such as vascular systemic inflammation, arteriosclerosis, hypercholesterolemia, hyperuricemia, low back pain, gallbladder disease, gout, renal cancer, secondary risk of obesity such as risk of left ventricle hypertrophy, migraine, headache, neuropathic pain, Parkinson's disease, psychosis, schizophrenia, facial flushing, night sweat, disease in genitalium/urinary system, disease associated with sexual function or fecundability, dysthymic disorder, bipolar disorder, bipolar I disorder, bipolar II disorder, cyclothymic disorder, acute stress disorder, agoraphobia, generalized anxiety disorder, obsessive-compulsive disorder, panic attack, panic disorder, posttraumatic stress disorder, separation anxiety disorder, social phobia, anxiety disorder, acute neurological and psychiatric disorder such as cerebral deficiency developed after heart bypass surgery or heart transplant, stroke, ischemic stroke, cerebral ischemia, spinal cord trauma, head injury, periparturient hypoxia, cardiac arrest, hypoglycemic nerve injury, Huntington's disease, amyotrophic lateral sclerosis, multiple sclerosis, eye damage, retinopathy, cognitive impairment, muscle spasm, tremor, epilepsy, disorder associated with muscle spasm, delirium, amnestic disorder, age-associated cognitive decline, schizoaffective disorder, paranoia, drug addiction, movement disorder, chronic fatigue syndrome, fatigue, medication-induced parkinsonian syndrome, Gilles de la Tourette syndrome, chorea, myoclonus, tic, restless legs syndrome, dystonia, dyskinesia, attention deficit hyperactivity disorder (ADHD), conduct disorder, urinary incontinence, withdrawal symptom, trigeminal neuralgia, hearing loss, tinnitus, nerve injury, retinopathy, macular degeneration, vomiting, cerebral edema, pain, bone pain, arthralgia, toothache, cataplexy, or traumatic brain injury. Preferably, the disease includes narcolepsy, idiopathic hypersomnia, hypersomnia, sleep apnea syndrome, narcolepsy syndrome involving narcolepsy-like symptom, hypersomnia associated with Parkinson's disease, hypersomnia associated with dementia with Lewy body.

The "prophylaxis" as used herein means the administration of the present compound to a healthy subject who does not develop a disease. For example, the prophylaxis is intended to prevent the development of a disease. The "treatment" as used herein means the administration of the present compound to a person diagnosed with the development of a disease by a doctor (*i*.*e*., a patient). For example, the treatment is intended to alleviate a disease or symptom thereof or improve the condition of a patient to the previous condition before a disease is developed. Also, even if the present compound is administered for the purpose of preventing the worsening of a disease or symptom thereof, the administration is referred to as "treatment" when the subject to be administered is a patient.

Hereinafter, the processes for preparing the compound of formula (1) of the present invention are exemplified along with examples, but the processes of the present invention should not be limited to the examples.

### Preparation Process

The present compound may be synthesized according to each Preparation Process shown below or its combination with a known synthetic process.

Each compound in the following schemes may exist as a salt thereof, wherein the salt includes, for example, the "pharmaceutically acceptable salt" mentioned above. The following schemes are disclosed as just examples, thus it is also possible to optionally prepare the present compound by a different process based on the knowledge of a skilled person in synthetic organic chemistry field.

In each Preparation Process described below, protecting groups can be used as necessary, even if the use of protecting groups is not explicitly stated. And, the protecting groups can be deprotected after a reaction is completed or a series of reactions have been carried out to obtain the desired compound.

As such protecting groups, for example, general protecting groups described in T. W. Greene, and P. G. M. Wuts, "Protective Groups in Organic Synthesis", 3rd Ed., John Wiley & Sons, Inc., New York (1999*),* and the like may be used. Examples of amino-protecting group include *tert-*butoxycarbonyl, benzyloxycarbonyl, p-toluenesulfonyl, o-nitrobenzenesulfonyl, tetrahydropyranyl, and the like; examples of hydroxy-protecting group include trialkylsilyl, acetyl, benzyl, tetrahydropyranyl, methoxymethyl, and the like; examples of aldehyde-protecting group include dialkylacetal, cyclic alkylacetal, and the like; and examples of carboxyl-protecting group include *tert*-butyl ester, orthoester, amide, and the like.

The introduction and elimination of protecting groups can be carried out by a method commonly used in synthetic organic chemistry (for example, *see* T. W. Greene, and P. G. M. Wuts, "Protective Groups in Organic Synthesis", 3rd Ed., John Wiley & Sons, Inc., New York (1999*)*), or a similar method.

### Preparation Process 1:

In a compound of formula (1) or a pharmaceutically acceptable salt thereof, a compound of formula (1') can be prepared, for example, by the following process. 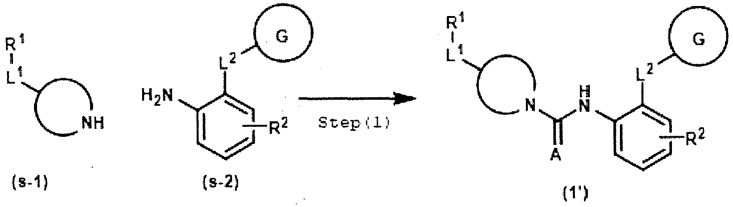 wherein R¹, R², L¹, L², ring G, and A are as defined in Item 1.

### Step (1):

Compound (1') can be prepared by reacting compound (s-1) and compound (s-2) in a suitable inert solvent under a reaction condition of urea-binding formulation. The present reaction condition includes, for example, using triphosgene, 4-nitrophenyl chloroformate or thiophosgene. A base is used in the present reaction, and the base used herein includes triethylamine and diisopropylethylamine. The solvent includes a halogenated carbon solvent such as chloroform and dichloromethane; an ether solvent such as diethyl ether, THF and 1,4-dioxane; an aromatic hydrocarbon solvent such as benzene, toluene and xylene; and an ester solvent such as ethyl acetate and methyl acetate. The reaction time is generally about 1 hour to 24 hours, and the reaction temperature is -20°C to boiling point of a solvent used herein.

### Preparation Process 2:

In a compound of formula (1) or a pharmaceutically acceptable salt thereof, a compound of formula (s-5) can be prepared, for example, by the following process. 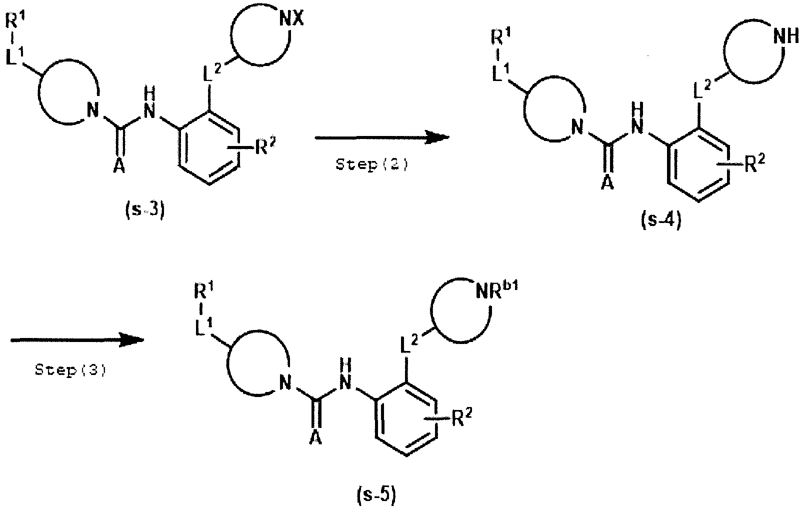 wherein R¹, R², L¹, L², and A are as defined in Item 1, R^{b1} is as defined in Item 5, X is an amino-protecting group. Compound (s-3) is a compound wherein ring G in Preparation Process 1 is a protected nitrogen-containing ring, and can be synthesized in the same manner as Step (1) in Preparation Process 1.

### Step (2):

Compound (s-4) can be prepared by reacting compound (s-3) in a suitable inert solvent under a commonly-used deprotection condition. For example, when X is Boc group, deprotection can be carried out by using an acid in the present reaction condition. Examples of the acid include hydrochloric acid, sulfuric acid, hydrobromic acid, trifluoroacetic acid, and the like. The solvent includes a halogenated carbon solvent such as chloroform and dichloromethane; an ether solvent such as diethyl ether, diisopropyl ether, tetrahydrofuran and 1,4-dioxane; and an ester solvent such as ethyl acetate and methyl acetate. The reaction time is generally about 1 hour to 24 hours, and the reaction temperature is -20°C to boiling point of a solvent used herein. When X is Cbz group or Bn group, deprotection can be carried out, for example, by catalytic reduction under hydrogenation condition. A heterogeneous catalyst such as palladium-carbon is used as the catalyst. The "under hydrogenation condition" means under hydrogen atmosphere or in the presence of formic acid, ammonium formate, and the like. The solvent includes methanol, ethanol, THF, ethyl acetate, and the like. The reaction time is 30 minutes to 24 hours, and the reaction temperature is 0°C to boiling point of a solvent used herein.

### Step (3):

Compound (s-5) can be prepared by reacting compound (s-4) in a suitable inert solvent under a commonly-used reductive amination condition. The present reaction condition includes, for example, using sodium triacetoxyborohydride, sodium cyanoborohydride, sodium borohydride, and the like. An acid is used in the present reaction, and the acid used herein includes acetic acid. The solvent includes a halogenated carbon solvent such as chloroform and dichloromethane; an ether solvent such as diethyl ether, THF and 1,4-dioxane; and an ester solvent such as ethyl acetate and methyl acetate. The reaction time is generally about 1 hour to 24 hours, and the reaction temperature is -20°C to boiling point of a solvent used herein.

### EXAMPLES

The present invention is explained in more detail in the following by referring to Reference Examples, Examples and Test Examples, but is not limited thereto. It should be understood that the names of compounds used in the following Reference Examples and Examples do not necessarily follow the IUPAC nomenclature.

In the present specification, the abbreviations shown below may be used.
CDCl₃: deuterochloroform
DMSO-d₆: deuterodimethylsulfoxide
Rt: retention time
min: minute
HATU: O-(7-aza-1H-benzotriazol-1-yl)-N,N,N',N'-tetramethyluronium hexafluorophosphate
THF: tetrahydrofuran
TFA: trifluoroacetic acid
DMF: N,N-dimethylformamide
Boc: tert-butoxycarbonyl
Ns: 2-nitrobenzenesulfonyl group
Tf: trifluoromethanesulfonyl group
Abs: Absolute Configuration; each chemical structure of compounds described along with Abs mark surrounded with a square flame is shown in absolute configuration with a wedged bond. However, not all compounds without Abs mark are shown in non-absolute configuration, *i.e.,* the configuration should be properly judged based on the disclosure about the subject compound in the present specification and its context, and a skilled person's technical knowledge, with or without Abs mark.
Rac: Racemic compound; It represents a compound in a state in which two types of enantiomers are present in equal amounts and no longer exhibit optical rotation.

In the column chromatography and amino chromatography used in Reference Examples and Examples, silica gel column and amino column made by YAMAZEN CORPORATION were used. The TLC (silica gel plate) used in the TLC purification was Silica gel 60F254 (Merck), and the TLC (NH silica gel plate) used therein was TLC plate NH (FujiSilysia).

In Reference Examples and Examples, the reactors shown below were used. The physicochemical data described in Reference Examples and Examples were obtained with the apparatuses below.
Microwave reactor: Biotage AB Initiator
¹H-NMR: JEOL JNM-AL400; JEOL JNM-ECS400; Brucker AVANCE 400 Spectrometer

The symbols used in NMR are defined as follows, s: singlet, d: doublet, dd: doublet of doublets, ddd: doublet of doublets of doublets, dddd: doublet of doublets of doublets of doublets, t: triplet, td: triplet of doublets, q: quartet, m: multiplet, br: broad singlet or multiplet, and J: coupling constant.

The LC/MS data of each compound in Examples and Reference Examples were obtained with any one of the apparatuses below.

### Method A

Detection apparatus: ACQUITY^{™} SQ detector (Waters Corporation)
HPLC: ACQUITY^{™} UPLC SYSTEM
Column: Waters ACQUITY^{™} UPLC BEH C18 (1.7 um, 2.1 mm × 30 mm)

### Method B

Detection apparatus: Shimadzu LCMS-2020
Column: Phenomenex Kinetex (C18, 1.7 pm, 2.1 mm × 50 mm)

### Method C

Detection apparatus: ACQUITY^{™} SQ detector (Waters Corporation)
HPLC: ACQUITY^{™} UPLC SYSTEM
Column: Waters ACQUITY^{™} UPLC BEH C18 (1.7 pm, 2.1 mm × 30 mm)

### Method D

Detection apparatus: Shimadzu LCMS-2020
Column: Waters ACQUITY UPLC^{™} C18 (1.8 µm 2.1 mm × 50 mm)

High-performance liquid chromatograph mass spectrometer; the measurement conditions of LC/MS are as follows, wherein the observed value [MS (m/z)] is denoted by [M+H]⁺ and the retention time is denoted by Rt (min). Each measured MS value shown below is accompanied by any one of A to D which were measurement methods used in the actual measurements.

### Method A

Solvent: A solution; 0.06 % formic acid/H₂O, B solution; 0.06 % formic acid/acetonitrile
Gradient condition: 0.0-1.3 min (linear gradient from B 2 % to B 96 %)
Flow rate: 0.8 mL/min; Detective UV: 220 nm and 254 nm; Temperature: 40°C

Hereinafter, the LC-MS data shown below were measured by Method A, unless otherwise indicated.

### Method B

Solvent: A solution; 0.05 % TFA/H₂O, B solution; acetonitrile Gradient condition: 0.0-1.7 min (linear gradient from B 10 % to B 99 %)
Flow rate: 0.5 mL/min; Detective UV: 220 nm; Temperature: 40°C

### Method C

Solvent: A solution; 0.05 % formic acid/H₂O, B solution; acetonitrile
Gradient condition: 0.0-1.3 min (linear gradient from B 10 % to B 95 %) 1.3-1.5 min (B 10 %)
Flow rate: 0.8 mL/min; Detective UV: 220 nm and 254 nm; Temperature: 40°C

### Method D

Solvent: A solution; 0.1 % formic acid/H₂O, B solution; 0.1 % formic acid/acetonitrile
Gradient condition: 0.01-4.0 min (linear gradient from B 5 % to B 99 %) 4.0-5.0 min (B 95 %)
Flow rate: 0.5 mL/min; Detective UV: 220 nm; Temperature: 25°C

Hereinafter, the data were measured by Method A, unless otherwise indicated.

### Reference Example 1: tert-Butyl 4-(2-{[4-(4-methylphenyl)piperidine-1-carbonyl]amino}phenyl)piperazine-1-carboxylate

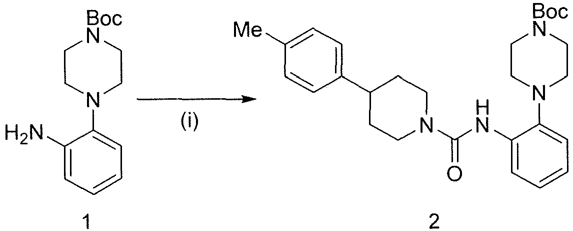

### Step (i) :

To a mixture of compound 1 (0.100 g), diisopropylethylamine (0.047 g) and chloroform (1.8 mL) was added 4-nitrophenylchloroformate (0.073 g) at 0°C and the mixture was stirred at the same temperature for 1 hour. To the reaction mixture was added 4-(4-methylphenyl)piperidine (0.070 g) at 0°C and the mixture was stirred at room temperature for 1 hour. Water was added to the reaction mixture and the mixture was extracted with ethyl acetate. The organic layer was dried over anhydrous sodium sulfate and concentrated *in vacuo.* The resulting residue was then purified by silica gel column chromatography (Eluate: hexane/ethyl acetate) to give the title compound 2 (0.152 g)
LCMS: [M+H]⁺/Rt(min): 479/1.27

### Reference Example 2: 2-[4-(Propan-2-yl)piperazin-1-yl]aniline

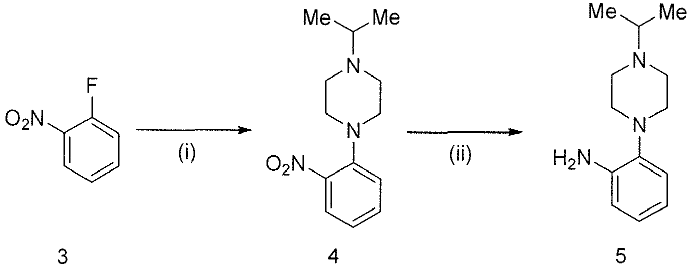

### Step (i):

A mixture of compound 3 (1.41 g), potassium carbonate (5.52 g), isopropylpiperazine (1.28 g) and THF (25 mL) was stirred at 50°C for 1 hour. Water was added to the reaction mixture and the mixture was extracted with ethyl acetate. The organic layer was dried over anhydrous sodium sulfate and concentrated *in vacuo.* The resulting residue was then used directly in the next step.

### Step (ii):

A mixutre of the compound 4 mixture, 10% palladium carbon (1.06 g) and methanol (25 mL) was stirred at room temperature under hydrogen gas atmosphere. The reaction mixture was filtrated with Celite, and the filtrate was concentrated *in vacuo.* The resulting residue was then purified by silica gel column chromatography (Eluate: chloroform/methanol) to give the title compound 5 (1.50 g). LCMS: [M+H]⁺/Rt(min): 220/0.35

### Reference Example 3: 4-Fluoro-2-[4-(propan-2-yl)piperazin-1-yl]aniline

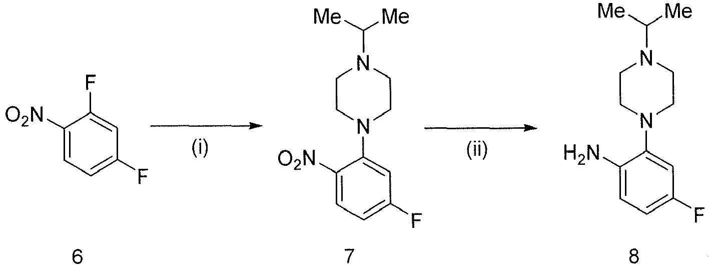

### Step (i):

The title compoud 7 mixture was prepared from compound 6 (0.159 g) in the same manner as Step (i) in Reference Example 2.

### Step (ii):

The title compound 8 (0.144 g) was prepared from the compound 7 mixture in the same manner as Step (ii) in Reference Example 2.
LCMS: [M+H]⁺/Rt(min): 238/0.39

### Reference Example 4: 2-Metoxy-6-[4-(propan-2-yl)piperazin-1-yl]aniline

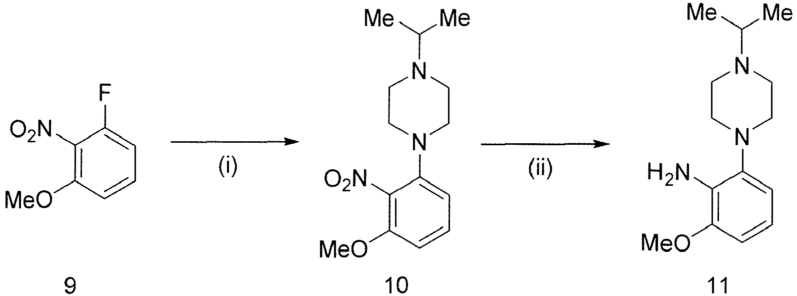

### Step (i) :

The title compound 10 mixutre was prepared from compound 9 (0.020 g) in the same manner as Step (i) in Reference Example 2.

### Step (ii):

The title compound 11 (0.019 g) was prepared from the compound 10 mixture in the same manner as Step (ii) in Reference Example 2.
LCMS: [M+H]⁺/Rt(min): 250/0.60

### Reference Example 5: 2-Fluoro-6-[4-(propan-2-yl)piperazin-1-yl]aniline

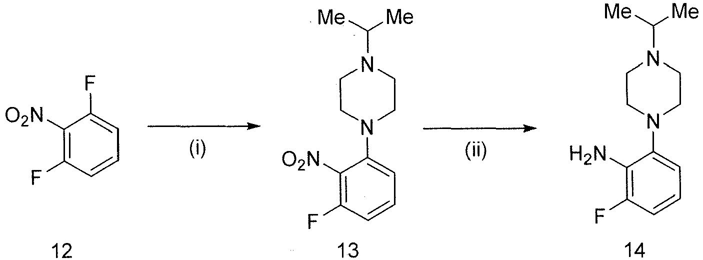

### Step (i) :

The title compound 13 mixture was prepared from compound 12 (0.159 g) in the same manner as Step (i) in Reference Example 2.

### Step (ii):

The title compound 14 (0.190 g) was prepared from the compound 13 mixture in the same manner as Step (ii) in Reference Example 2.
LCMS: [M+H]⁺/Rt(min): 238/1.16 (Method B)

### Reference Example 6: 3-Fluoro-2-[4-(propan-2-yl)piperazin-1-yl]aniline

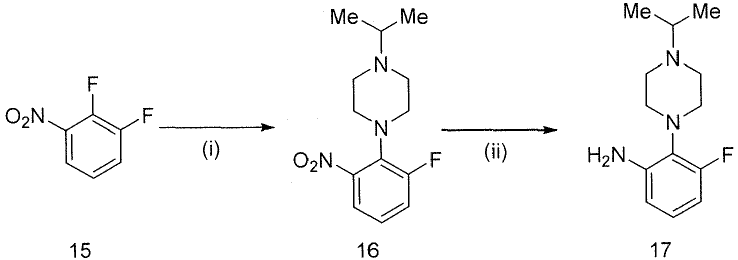

### Step (i):

The title compound 16 mixture was prepared from compound 15 (0.477 g) in the same manner as Step (i) in Reference Example 2.

### Step (ii):

The title compound 17 (0.443 g) was prepared from the compound 16 mixture in the same manner as Step (ii) in Reference Example 2.
LCMS: [M+H]⁺/Rt(min): 238/0.46

### Reference Example 7: 2-Methyl-6-[4-(propan-2-yl)piperazin-1-yl]aniline

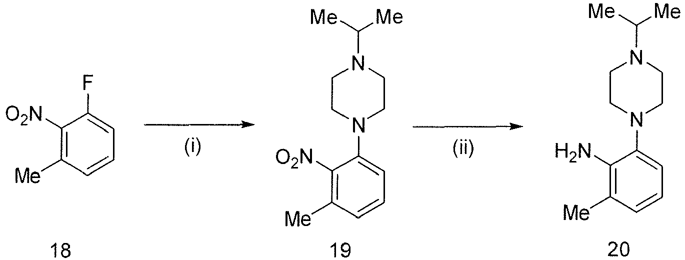

### Step (i) :

The title compound 19 mixture was prepared from compound 18 (0.310 g) in the same manner as Step (i) in Reference Example 2.

### Step (ii):

The title compound 20 (0.410 g) was prepared from the compound 19 mixture in the same manner as Step (ii) in Reference Example 2.
LCMS: [M+H]⁺/Rt(min): 234/0.61

### Reference Example 8: 2-[4-(Propan-2-yl)piperazin-1-yl]-6-(trifluoromethyl)aniline

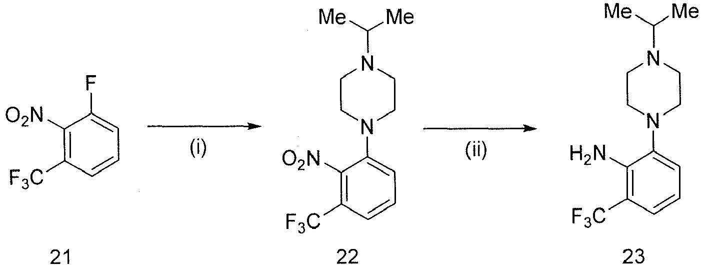

### Step (i):

The title compound 22 mixture was prepared from compound 21 (0.209 g) in the same manner as Step (i) in Reference Example 2.

### Step (ii):

The title compound 23 (0.220 g) was prepared from the compound 22 mixture in the same manner as Step (ii) in Reference Example 2.
LCMS: [M+H]⁺/Rt(min): 288/1.50 (Method B)

### Reference Example 9: 2-Chloro-6-[4-(propan-2-yl)piperazin-1-yl]aniline

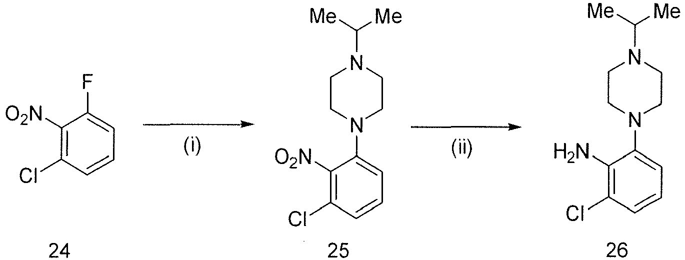

### Step (i) :

The title compound 25 mixture was prepared from compound 24 (4.00 g) in the same manner as Step (i) in Reference Example 2.

### Step (ii):

To a mixture of the compound 25 mixture, reduced iron (3.82g) and methanol (57 mL) was added 4N aqueous ammonium chloride solution (28.5 mL) at 90°C and the mixutre was stirred at the same temperature for 2 hours. The reaction mixture was cooled to room temperature and filtrated with Celite, and the filtrate was concentrated *in vacuo.* The resulting residue was then dissolved in ethyl acetate and washed with water and brine. The organic layer was dried over anhydrous sodium sulfate and concentrated *in vacuo.* The resulting residue was then purified by silica gel column chromatography (Eluate: chloroform/methanol) to give the title compound 26 (4.10 g).
LCMS: [M+H]⁺/Rt(min): 254/1.33 (Method B)

### Reference Example 10: 2-Chloro-6-(4-cyclopropylpiperazin-1-yl)aniline

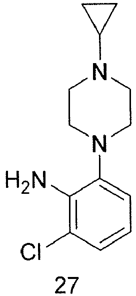

According to the process in the above Reference Example 9, compound 27 was synthesized by using cyclopropylpiperazine instead of isopropylpiperazine at Step (i) in Reference Example 9.
LCMS: [M+H]⁺/Rt(min): 252/0.60

### Reference Example 11: 2-Bromo-6-[4-(propan-2-yl)piperazin-1-yl]aniline

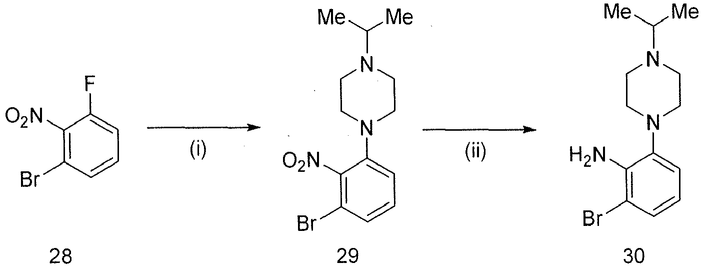

### Step (i):

The title compound 29 mixture was prepared from compound 28 (1.50 g) in the same manner as Step (i) in Reference Example 2.

### Step (ii):

The title compound 30 (1.64 g) was prepared from the compound 29 mixture in the same manner as Step (ii) in Reference Example 9.
LCMS: [M+H]⁺/Rt(min): 298,300/0.58

### Reference Example 12: 2-Amino-N,N-dimethyl-3-[4-(propan-2-yl)piperazin-1-yl]benzamide

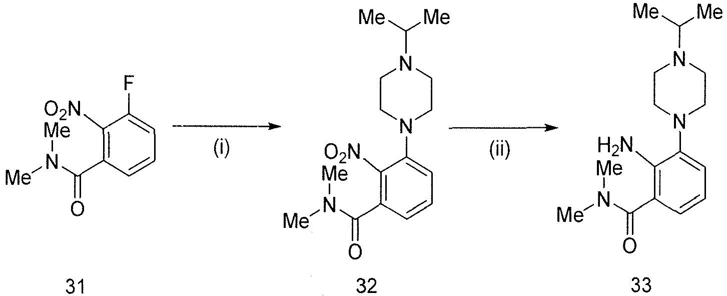

### Step (i) :

The title compound 32 mixture was prepared from compound 31 (0.212 g) in the same manner as Step (i) in Reference Example 2.

### Step (ii):

The title compound 33 (0.201 g) was prepared from the compound 32 mixture in the same manner as Step (ii) in Reference Example 9.
LCMS: [M+H]⁺/Rt(min): 291/1.07 (Method B)

### Reference Example 13: tert-Butyl (2R,5S)-2-(2-aminophenyl)-5-(propan-2-yl)morpholine-4-carboxylate

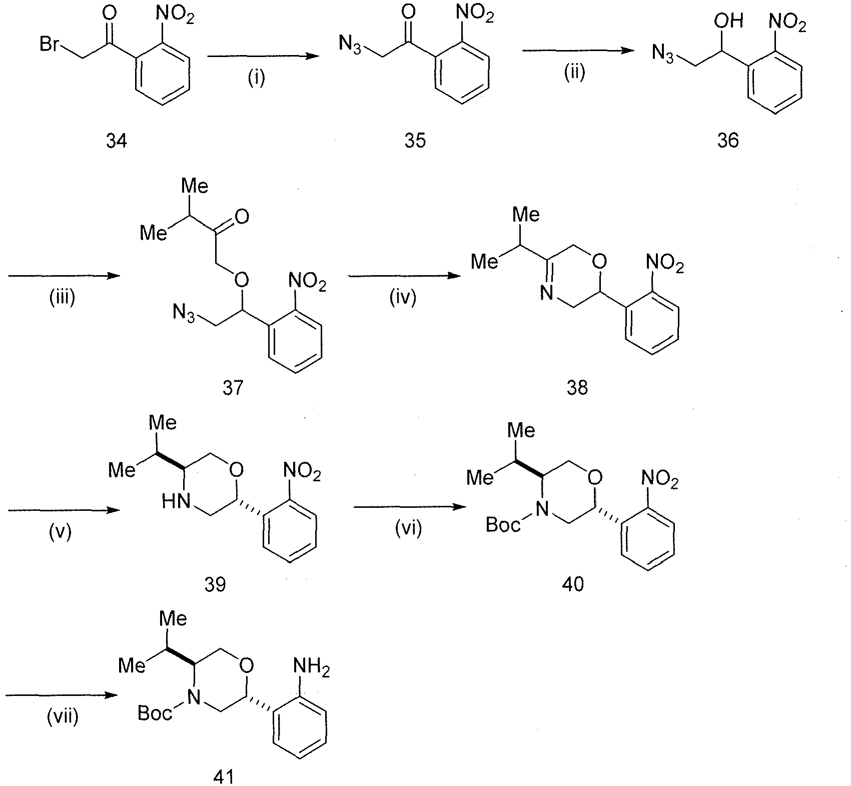

### Step (i) :

A mixutre of compound 34 (1.00 g), sodium azide (0.533 g), water (10 mL) and ethanol (20 mL) was stirred at room temperature for 4 hours. Water was added to the reaction mixture and the mixture was extracted with ethyl acetate. The organic layer was dried over anhydrous sodium sulfate and concentrated *in vacuo.* The resulting residue was then used directly in the next step.

### Step (ii):

A mixuture of the crude product of compound 35, sodium borohydride (0.127 g) and methanol was stirred at 0°C for 1 hour. To reaction mixture was added saturated aqueous ammonium chloride solution and the mixture was extracted with ethyl acetate. The organic layer was dried over anhydrous sodium sulfate and concentrated *in vacuo.* The resulting residue was then purified by silica gel column chromatography (Eluate: hexane/ethyl acetate) to give the title compound 36 (0.488 g).

### Step (iii):

A mixture of compound 36 (0.289 g), 1-bromo-3-methyl-2-butanone (0.641 g), caesium carbonate (1.176 g) and DMF (3 mL) was stirred at room temperature for 5 hours. Water was added to the reaction mixture and the mixture was extracted with ethyl acetate. The organic layer was dried over anhydrous sodium sulfate and concentrated *in vacuo.* The resulting residue was then purified by silica gel column chromatography (Eluate: hexane/ethyl acetate) to give the title compound 37 (0.402 g).
LCMS: [M+H]⁺/Rt(min): 293/1.05

### Step (iv):

A mixture of compound 37 (0.82 g), triphenylphosphine (0.184 g) and THF (2 mL) was stirred at room temperature for 10 hours. The reaction mixture was concentrated *in vacuo* and then the resulting residue was purified by silica gel column chromatography (Eluate: hexane/ethyl acetate) to give the title compound 38 (0.058 g).
LCMS: [M+H]⁺/Rt(min): 249/0.54

### Step (v):

A mixture of compound 38 (0.059 g), sodium triacetoxyborohydride (0.100 g) and dichloromethane (2 mL) was stirred at room temperature for 3 hours. To the reaction mixture was added saturated aqueous sodium hydrogen carbonate solution and the mixture was extracted with chloroform. The organic layer was dried over anhydrous sodium sulfate and concentrated *in vacuo.* The resulting residue was then purified by amino silica gel column chromatography (Eluate: hexane/ethyl acetate) to give the title compound 39 (0.049 g).
LCMS: [M+H]⁺/Rt(min): 251/0.63

### Step (vi):

A mixture of compound 39 (0.038 g), di-tert-butyl dicarbonate (0.040 g), N,N-dimethyl-4-aminopyridine (0.001 g) and acetonitrile (2 mL) was stirred at room temperature for 2 hours. Water was added to the reaction mixture and the mixture was extracted with chloroform. The organic layer was dried over anhydrous sodium sulfate and concentrated in *vacuo.* The resulting residue was then used directly in the next step.
LCMS: [M+H]⁺/Rt(min): 325/1.24

### Step (vii):

The title compound 41 (0.036 g) was prepared from the compound 40 mixture in the same manner as Step (ii) in Reference Example 9.
LCMS: [M+H]⁺/Rt(min): 321/1.13

### Reference Example 14: Rac-2-[3-methyl-4-(propan-2-yl)piperazin-1-yl]aniline

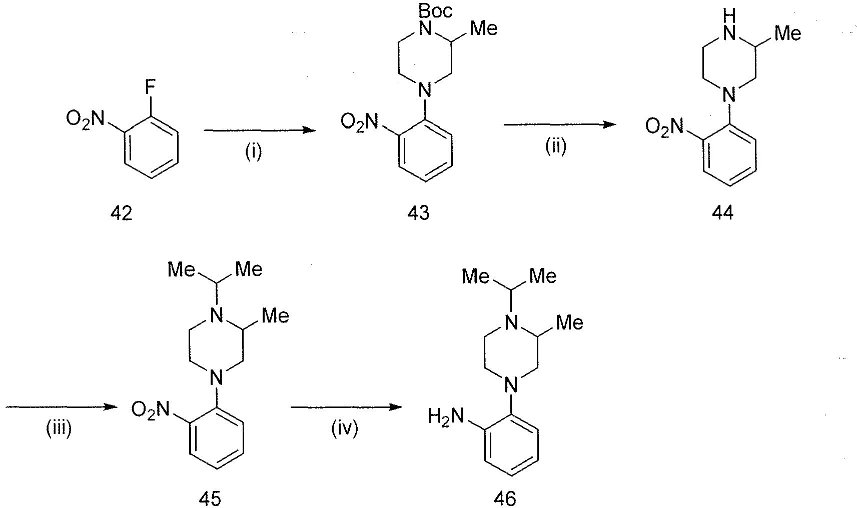

### Step (i):

The crude product of the title compound 43 was prepared from compound 42 (0.282 g) in the same manner as Step (i) in Reference Example 2.

### Step (ii):

A mixture of the crude product of compund 43, trifluoroacetic acid (1.80 g) and chloroform (1.0 mL) was stirred at room temperature for 1 hour. The reaction mixture was concentrated *in vacuo* and then the resulting residue was purified by amino silica gel column chromatography (Eluate: hexane/ethyl acetate) to give the title compound 44 (0.370 g).
LCMS: [M+H]⁺/Rt(min): 222/1.29 (Method B)

### Step (iii):

A mixutre of compound 44 (0.221 g), sodium triacetoxyborohydride (0.635 g), acetic acid (0.060 g), acetone (0.058 g) and THF (5 mL) was stirred at room temperature for 3 hour. To the reaction mixture was added saturated aqueous sodium hydrogen carbonate solution and the mixture was extracted with ethyl acetate. The oragnic layer was dried over anhydrous sodium sulfate and concentrated *in vacuo.* The resulting residue was then used directly in the next step.

### Step (iv):

The title compound 46 (0.170 g) was prepared from the crude product of compound 45 in the same manner as Step (ii) in Reference Example 2.
LCMS: [M+H]⁺/Rt(min): 234/0.48 (Method B)

### Reference Example 15: Rac-2-[2-methyl-4-(propan-2-yl)piperazin-1-yl]aniline

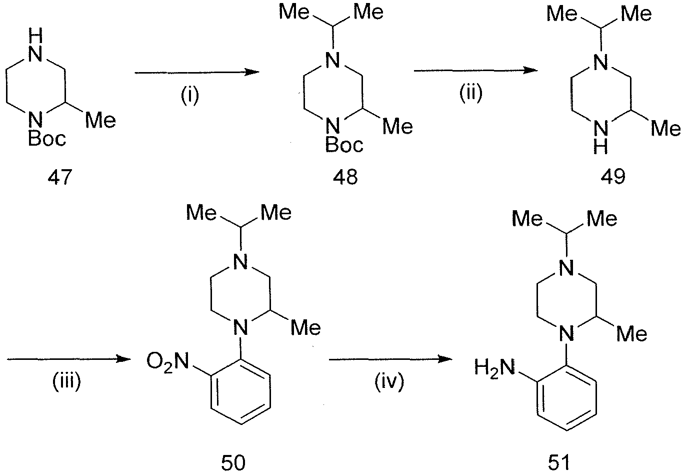

### Step (i):

The crude product of the title compound 48 was prepared from compound 47 (0.221 g) in the same manner as Step (iii) in Reference Example 14.

### Step (ii):

The title compound 49 (0.098 g) was prepared from the crude product of compound 48 in the same manner as Step (ii) in Reference Example 14.
LCMS: [M+H]⁺/Rt(min): 143/0.30

### Step (iii):

The crude product of the title compound 50 was prepared from compound 49 (0.071 g) in the same manner as Step (i) in Reference Example 2.

### Step (iv):

The title compound 51 (0.060 g) was prepared from the crude product of compound 50 in the same manner as Step (ii) in Reference Example 2.
LCMS: [M+H]⁺/Rt(min): 234/0.54

### Reference Example 16: Methyl 2-amino-3-[4-(propan-2-yl)piperazin-1-yl]benzoate

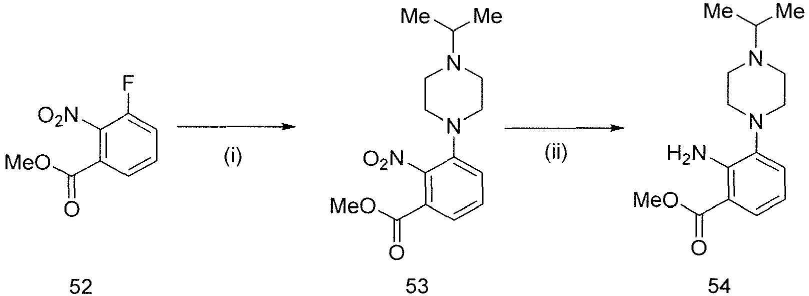

### Step (i):

The title compound 53 mixture was prepared from compound 52 (0.150 g) in the same manner as Step (i) in Reference Example 2.
LCMS: [M+H]⁺/Rt(min): 308/0.55

### Step (ii):

The title compound 54 (0.132 g) was prepared from the compound 53 mixture in the same manner as Step (ii) in Reference Example 2.
LCMS: [M+H]⁺/Rt(min): 278/0.58

### Reference Example 17: tert-Butyl 4-(2-{[4-(4-methylphenyl)piperidine-1-carbonyl]amino}phenyl)-1,4-diazepane-1-carboxylate

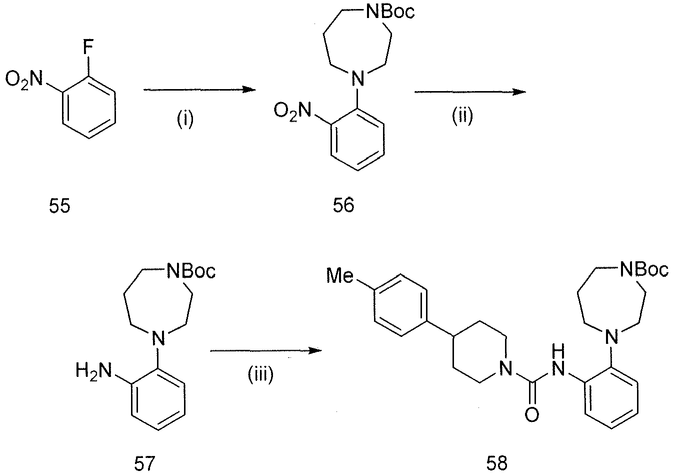

### Step (i) :

The title compound 56 (2.40 g) was prepared from compound 55 (1.06 g) in the same manner as Step (i) in Reference Example 2.
LCMS: [M+H]⁺/Rt(min): 322/1.08

### Step (ii):

The title compound 57 (1.77 g) was prepared from compound 56 (2.40 g) in the same manner as Step (ii) in Reference Example 2.
LCMS: [M+H]⁺/Rt(min): 292/0.96

### Step (iii):

The title compound 58 (0.153 g) was prepared from compound 57 (0.095 g) in the same manner as Step (i) in Reference Example 1.
LCMS: [M+H]⁺/Rt(min): 493/1.12

### Reference Example 18: tert-Butyl 4-(2-{[4-(5-cyclopropyl-1,2,4-oxadiazol-3-yl)-4-methylpiperidine-1-carbonyl]amino}phenyl)-1,4-diazepane-1-carboxylate

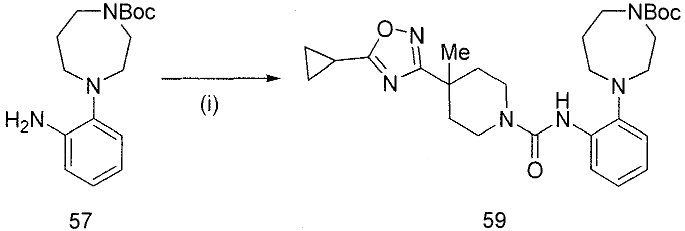

### Step (i):

The title compound 59 (0.154 g) was prepared from compound 57 (0.104 g) in the same manner as Step (i) in Reference Example 1.

### Reference Example 19: 2-(4-tert-Butylpiperazin-1-yl)-6-chloroaniline

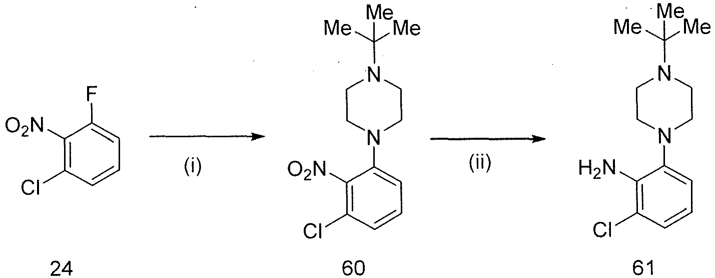

### Step (i) :

The crude product of the title compound 60 was prepared from compound 24 (0.300 g) in the same manner as Step (i) in Reference Example 2.

### Step (ii):

The title compound 61 (0.315 g) was prepared from the crude product of compound 60 in the same manner as Step (ii) in Reference Example 9.
LCMS: [M+H]⁺/Rt(min): 268/0.56

### Reference Example 20: 2-[1-(Propan-2-yl)piperidin-4-yl]aniline

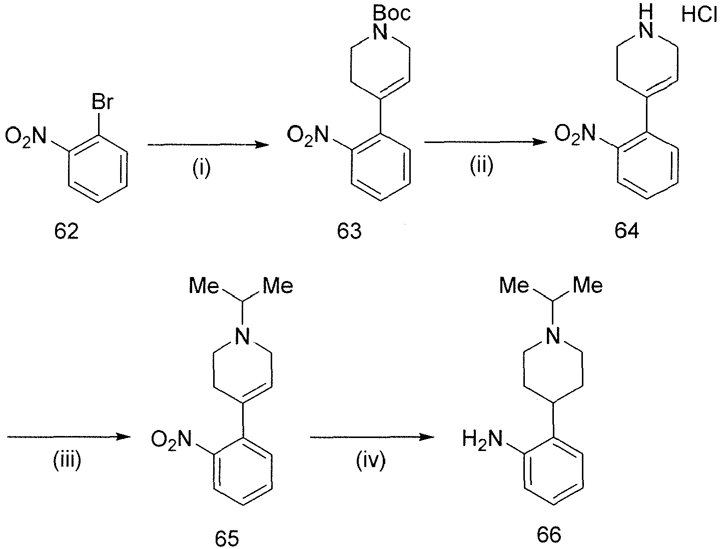

### Step (i):

A mixture of compound 62 (5.00 g), N-Boc-1,2,5,6-tetrahydropyridine-4-boronic acid pinacol ester (7.60 g), Pd(dppf)Cl₂CH₂Cl₂ (2.02 g), sodium carbonate (5.25 g), water (25 mL) and 1,4-dioxane (50 mL) was stirred at 110°C for 16 hours. Water was added to the reaction mixture and the mixture was extracted with ethyl acetate. The organic layer was dried over anhydrous sodium sulfate and concentrated *in vacuo.* The resulting residue was then purified by silica gel column chromatography (Eluate: hexane/ethyl acetate) to give the title compound 63 (7.51 g).
LCMS: [M+H]⁺/Rt(min): 305/4.24 (Method D)

### Step (ii):

A mixutre of compound 63 (7.51 g), 4N dioxane hydrochloride solution (31 mL) and methanol (150 mL) was stirred at room temperature for 1 hour. The reaction mixture was concentrated *in vacuo* to give the title compound 64 (5.13 g).
LCMS: [M+H]⁺/Rt(min): 205/2.15 (Method D)

### Step (iii):

A mixture of compound 64 (2.50 g), sodium triacetoxyborohydride (6.60 g), acetone (0.058 g) and dichloromethane (75 mL) was stirred at room temperature for 12 hours. To the reaction mixture was added saturated aqueous sodium hydrogen carbonate solution and the mixture was extracted with ethyl acetate. The organic layer was dried over anhydrous sodium sulfate and concentrated *in vacuo.* The resulting residue was then purified by silica gel column chromatography (Eluate: chloroform/methanol) to give the title compound 65 (1.60 g).
LCMS: [M+H]⁺/Rt(min): 247/2.17 (Method D)

### Step (iv):

The title compound 66 (0.675 g) was prepared from compound 65 (1.60 g) in the same manner as Step (ii) in Reference Example 2.
LCMS: [M+H]⁺/Rt(min): 219/1.85 (Method D)

### Reference Example 21: 2-Fluoro-6-[1-(propan-2-yl)piperidin-4-yl] aniline

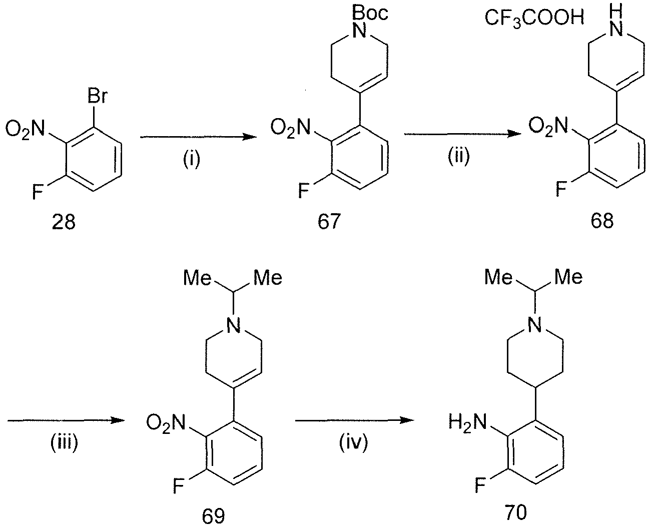

### Step (i):

The title compound 67 (0.234 g) was prepared from compound 28 (0.220 g) in the same manner as Step (i) in Reference Example 20.
LCMS: [M+H]⁺/Rt(min): 323/2.12 (Method B)

### Step (ii):

A mixture of compound 67 (0.230 g), trifluoroacetic acid (1.80 g) and chloroform (1.0 mL) was stirred at room temperature for 1 hour. The reaction mixture was concentrated *in vacuo* to give the crude product of the title compound 68.

### Step (iii):

A mixture of the crude product of compound 68, sodium triacetoxyborohydride (0.454 g), acetic acid (0.064 g), acetone (0.166 g) and THF (3 mL) was stirred at room temperature for 3 hours. To the reaction mixture was added saturated aqueous sodium hydrogen carbonate solution and the mixture was extracted with ethyl acetate. The organic layer was dried over anhydrous sodium sulfate and concentrated *in vacuo.* The resulting residue was then purified by amino silica gel column chromatography (Eluate: hexane/ethyl acetate) to give the title compound 69 (0.150 g).
LCMS: [M+H]⁺/Rt(min): 265/0.60 (Method B)

### Step (iv):

The title compound 70 (0.050 g) was prepared from compound 69 (0.100 g) in the same manner as Step (ii) in Reference Example 2.
LCMS: [M+H]⁺/Rt(min): 237/0.53 (Method B)

### Reference Example 22: 2-Fluoro-6-[1-(propan-2-yl)-1,2,3,6-tetrahydropyridin-4-yl]aniline

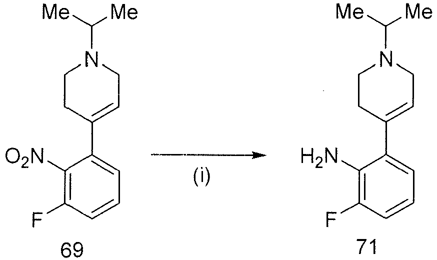

### Step (i) :

The title compound 71 (0.077 g) was prepared from compound 69 (0.100 g) in the same manner as Step (ii) in Reference Example 9.
LCMS: [M+H]⁺/Rt(min): 235/0.55 (Method B)

### Reference Example 23: tert-Butyl 4-(3-ethoxy-2-{[4-(4-methylphenyl)piperidine-1-carbonyl]amino}phenyl)piperidine-1-carboxylate

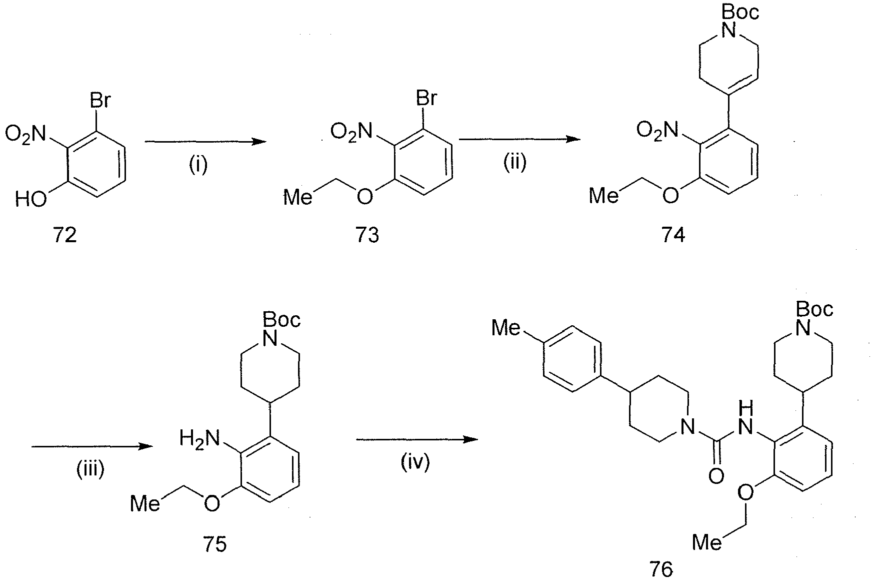

### Step (i):

A mixture of compound 72 (0.654 g), ethyl iodide (0.702 g), potassium carbonate (1.66 g) and THF (10 mL) was stirred at 50°C for 2 hours. Water was added to the reaction mixture and the mixture was extracted with ethyl acetate. The organic layer was dried over anhydrous sodium sulfate and concentrated *in vacuo.* The resulting residue was then purified by silica gel column chromatography (Eluate: hexane/ethyl acetate) to give the title compound 73 (0.621 g).

### Step (ii):

The title compound 74 (0.558 g) was prepared from compound 73 (0.500 g) in the same manner as Step (i) in Reference Example 20.
LCMS: [M+H]⁺/Rt(min): 349/2.24 (Method B)

### Step (iii):

The crude product of the title compound 75 (0.558 g) was prepared from compound 74 (0.300 g) in the same manner as Step (ii) in Reference Example 2.

### Step (iv):

To a mixture of the crude product of compound 75, diisopropylethylamine (0.278 g) and chloroform (4 mL) was added triphosgene (0.064 g) at 0°C and the mixture was stirred at the same temperature for 1 hour. To the reaction mixture was added 4-(4-methylphenyl)piperidine (0.084 g) at 0°C and the mixture was stirred for 1 hour. Water was added to the reaction mixture and the mixture was extracted with ethyl acetate. The organic layer was dried over anhydrous sodium sulfate and concentrated *in vacuo.* The resulting residue was then purified by silica gel column chromatography (Eluate: hexane/ethyl acetate) to give the title compound 76 (0.180 g).
LCMS: [M+H]⁺/Rt(min): 522/2.35 (Method B)

### Reference Example 24: tert-Butyl 4-(2-{[4-(4-methylphenyl)piperidine-1-carbonyl]amino}-3-[(propan-2-yl)oxy]phenyl)piperidine-1-carboxylate

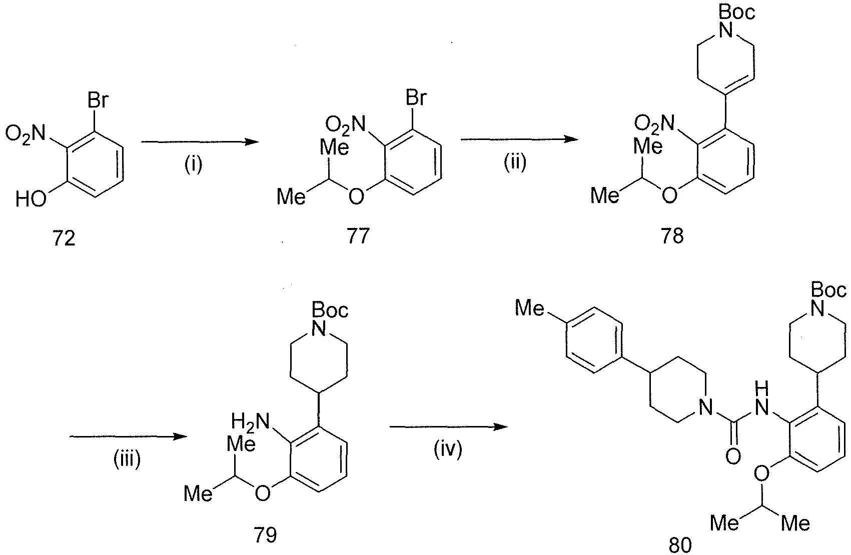

### Step (i):

The title compound 77 (0.522 g) was prepared from compound 72 (0.654 g) in the same manner as Step (i) in Reference Example 23.

### Step (ii):

The title compound 78 (0.611 g) was prepared from compound 77 (0.520 g) in the same manner as Step (i) in Reference Example 20.
LCMS: [M+H]⁺/Rt(min): 363/2.30 (Method B)

### Step (iii):

The crude product of the title compound 79 was prepared from compound 78 (0.300 g) in the same manner as Step (ii) in Reference Example 2.

### Step (iv):

The title compound 80 (0.299 g) was prepared from the crude product of compound 79 in the same manner as Step (iv) in Reference Example 23.
LCMS: [M+H]⁺/Rt(min): 536/2.40 (Method B)

### Reference Example 25: tert-Butyl 4-(3-chloro-2-{[4-(4-methylphenyl)piperidine-1-carbonyl]amino}phenyl)-3,6-dihydropyridine-1(2H)-carboxylate

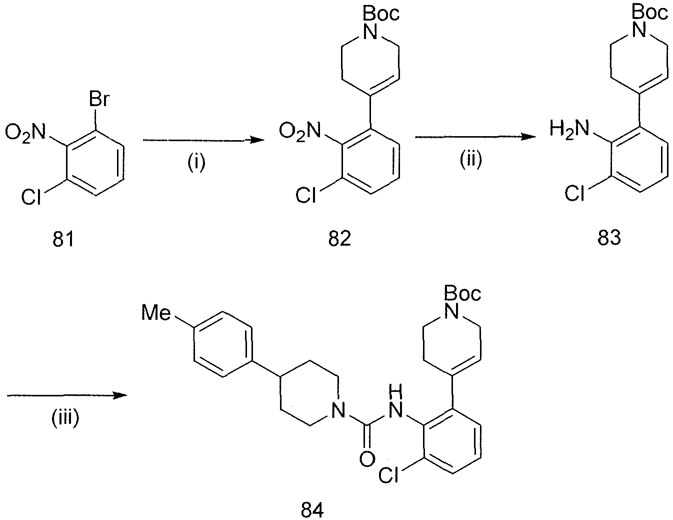

### Step (i) :

The title compound 82 (0.502 g) was prepared from compound 81 (0.709 g) in the same manner as Step (i) in Reference Example 20.
LCMS: [M+H]⁺/Rt(min): 339/2.26 (Method B)

### Step (ii):

The title compound 83 (0.070 g) was prepared from compound 82 (0.100 g) in the same manner as Step (ii) in Reference Example 9.
LCMS: [M+H]⁺/Rt(min): 309/2.22 (Method B)

### Step (iii):

The title compound 84 (0.082 g) was prepared from compound 83 (0.070 g) in the same manner as Step (iv) in Reference Example 23.
LCMS: [M+H]⁺/Rt(min): 511/2.27 (Method B)

### Reference Examples 26-29

According to the process in the above Reference Example 25, the compounds of Reference Examples 26-29 were synthesized by using each corresponding starting compound instead of 4-(4-methylphenyl)piperidine at Step (iii) in Reference Example 25.

**[Table 1]**

| Reference Example | Chemical Structural Formula | Instrumental Analysis Data |
|---|---|---|
| 26 | 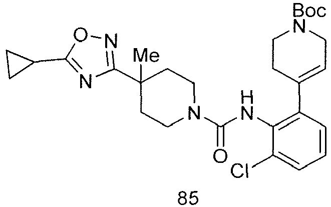 | LCMS: [M+H]⁺/Rt(min): 542/0.98 |
| 27 | 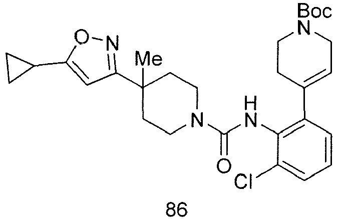 | LCMS: [M+H] + /Rt (min) : 542/1.68 (Method B) |
| 28 | 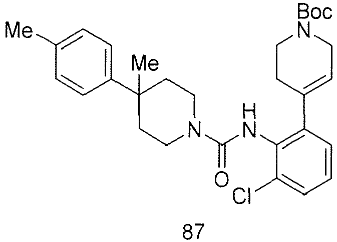 | LCMS: [M+H]⁺/Rt(min): 525/2.30 (Method B) |
| 29 | 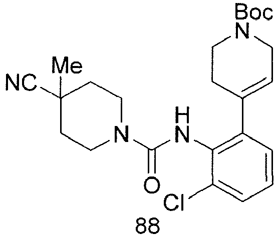 | LCMS: [M+H]⁺/Rt (min) : 459/0.94 (Method C) |

### Reference Example 26: tert-butyl 4-(3-chloro-2-{[4-(5-cyclopropyl-1,2,4-oxadiazol-3-yl)-4-methylpiperidine-1-carbonyl]amino}phenyl)-3,6-dihydropyridine-1(2H)-carboxylate

### Reference Example 27: tert-butyl 4-(3-chloro-2-{[4-(5-cyclopropyl-1,2-oxadiazol-3-yl)-4-methylpiperidine-1-carbonyl]amino}phenyl)-3,6-dihydropyridine-1(2H)-carboxylate

### Reference Example 28: tert-butyl 4-(3-chloro-2-{ [4-methyl-4-(4-methylphenyl)piperidine-1-carbonyl]amino}phenyl)-3,6-dihydropyridine-1(2H)-carboxylate

### Reference Example 29: tert-butyl 4-{3-chloro-2-[(4-cyano-4-methylpiperidine-1-carbonyl)amino]phenyl}-3,6-dihydropyridine-1(2H)-carboxylate

### Reference Example 30: tert-Butyl 4-(3-fluoro-2-{[4-(4-methylphenyl)piperidine-1-carbonyl]amino}phenyl)-3,6-dihydropyridine-1(2H)-carboxylate

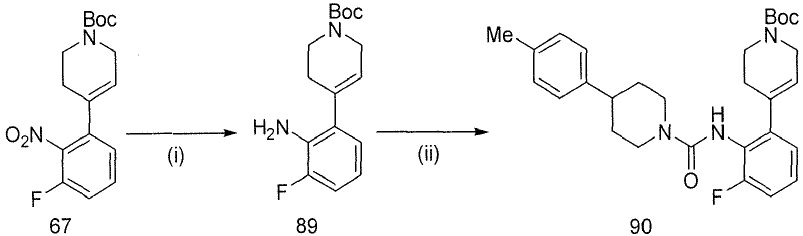

### Step (i):

The title compound 89 (0.750 g) was prepared from compound 67 (0.900 g) in the same manner as Step (ii) in Reference Example 9.
LCMS: [M+H]⁺/Rt(min): 293/2.10 (Method B)

### Step (ii):

The title compound 90 (0.130 g) was prepared from compound 89 (0.100 g) in the same manner as Step (iv) in Reference Example 23.
LCMS: [M+H]⁺/Rt(min): 494/2.27 (Method B)

### Reference Example 31: tert-Butyl 4-(3-fluoro-2-{[4-(4-methylphenyl)piperidine-1-carbonyl]amino}phenyl)piperidine-1-carboxylate

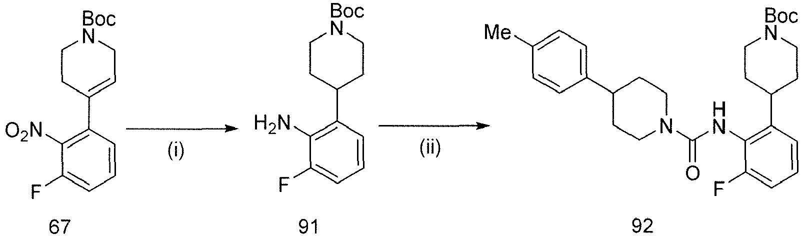

### Step (i):

The crude product of the title compound 91 was prepared from compound 67 (0.100 g) in the same manner as Step (ii) in Reference Example 2.

### Step (ii):

The title compound 92 (0.120 g) was prepared from the crude product of compound 91 in the same manner as Step (iv) in Reference Example 23.
LCMS: [M+H]⁺/Rt(min): 496/1.22

### Reference Example 32: 2-Methoxy-6-[1-(propan-2-yl)piperidin-4-yl]aniline

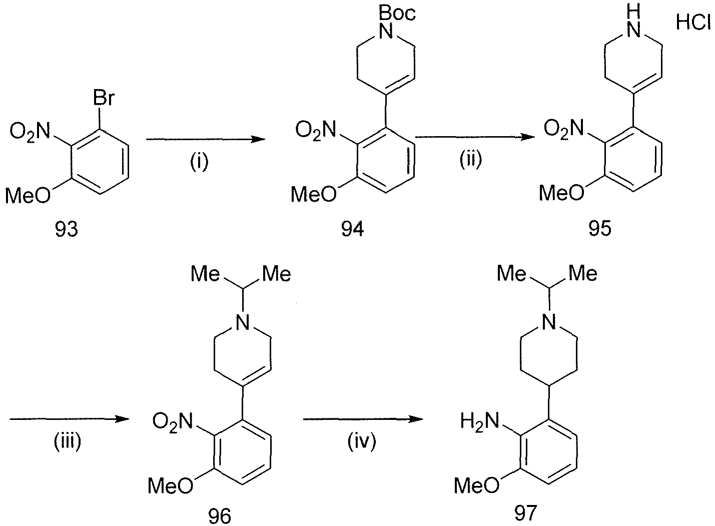

### Step (i):

The title compound 94 (3.78 g) was prepared from compound 93 (3.00 g) in the same manner as Step (i) in Reference Example 20.

### Step (ii):

The title compound 95 (2.80 g) was prepared from compound 94 (3.78 g) in the same manner as Step (ii) in Reference Example 20.
LCMS: [M+H]⁺/Rt(min): 235/2.31 (Method D)

### Step (iii):

The title compound 96 (2.80 g) was prepared from compound 95 (2.80 g) in the same manner as Step (iii) in Reference Example 20.
LCMS: [M+H]⁺/Rt(min): 277/2.42 (Method D)

### Step (iv):

The title compound 97 (1.45 g) was prepared from compound 96 (2.00 g) in the same manner as Step (ii) in Reference Example 2.
LCMS: [M+H]⁺/Rt(min): 249/3.57 (Method C)

### Reference Example 33: 2-Methoxy-6-[1-(propan-2-yl)-1,2,3,6-tetrahydropyridin-4-yl]aniline

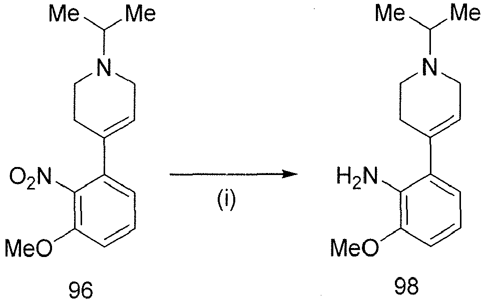

### Step (i):

The title compound 98 (0.237 g) was prepared from compound 96 (0.350 g) in the same manner as Step (ii) in Reference Example 9.
LCMS: [M+H]⁺/Rt(min): 247/5.02 (Method D)

### Reference Example 34: tert-Butyl 3-(3-fluoro-2-{[4-(4-methylphenyl)piperidine-1-carbonyl]amino}phenyl)-2,5-dihydro-1H-pyrrole-1-carboxylate

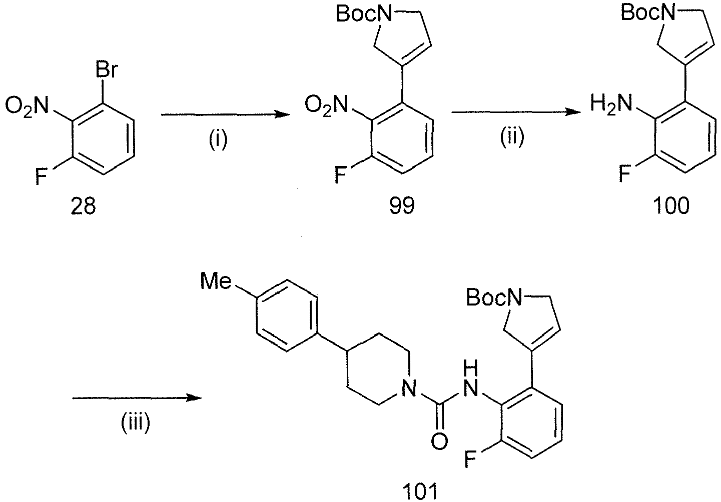

### Step (i):

The title compound 99 (0.113 g) was prepared from compound 28 (0.100 g) and corresponding boronic acid in the same manner as Step (i) in Reference Example 20.
¹H-NMR (CDCl₃) δ: 1.49 (9H, s), 4.20-4.34 (2H, m), 4.35-4.50 (2H, m), 5.90-6.04 (1H, m), 7.08-7.17 (1H, m), 7.17-7.25 (1H, m), 7.42-7.51 (1H, m).

### Step (ii):

The title compound 100 (0.065 g) was prepared from compound 99 (0.111 g) in the same manner as Step (ii) in Reference Example 9.
LCMS: [M+H]⁺/Rt(min): 279/0.99 (Method B)

### Step(iii):

The title compound 101 (0.030 g) was prepared from compound 100 (0.030 g) in the same manner as Step (iv) in Reference Example 23.
LCMS: [M+H]⁺/Rt(min): 480/2.21(shi) (Method C)

### Reference Example 35:

### 2-(3,6-Dihydro-2H-pyran-4-yl)-6-fluoroaniline

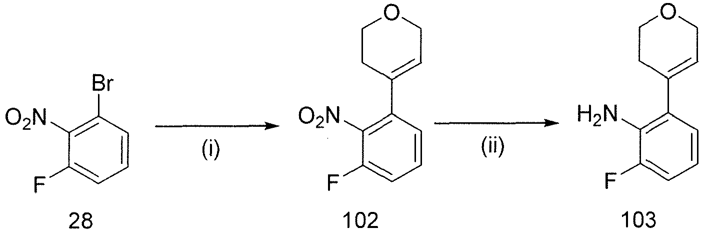

### Step (i):

The title compound 102 (0.056 g) was prepared from compound 28 (0.100 g) and corresponding boronic acid in the same manner as Step (i) in Reference Example 20.
¹H-NMR (CDCl₃) δ: 2.34-2.42 (2H, m), 3.86-3.92 (2H, m), 4.21-4.27 (2H, m), 5.79-5.86 (1H, m), 7.07-7.13 (1H, m), 7.13-7.21 (1H, m), 7.40-7.50 (1H, m).

### Step (ii):

The title compound 103 (0.032 g) was prepared from compound 102 (0.054 g) in the same manner as Step (ii) in Reference Example 9.
LCMS: [M+H]⁺/Rt(min): 194/0.70 (Method C)

### Reference Example 36: 2-(1-Cyclopropyl-1H-pyrazol-4-yl)-6-fluoroaniline

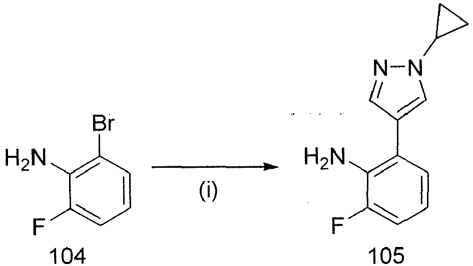

### Step (i):

The title compound 105 (0.007 g) was prepared from compound 104 (0.100 g) and corresponding boronic acid in the same manner as Step (i) in Reference Example 20.
LCMS: [M+H]⁺/Rt(min): 218/0.72 (Method C)

### Reference Example 37: 2-Fluoro-6-[6-(propan-2-yl)pyridin-3-yl]aniline

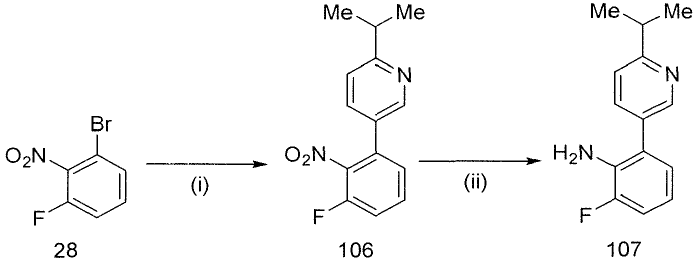

### Step (i):

The title compound 106 (0.110 g) was prepared from compound 28 (0.165 g) and corresponding boronic acid in the same manner as Step (i) in Reference Example 20.
LCMS: [M+H]⁺/Rt(min): 261/0.98

### Step (ii):

The title compound 107 (0.084 g) was prepared from compound 106 (0.110 g) in the same manner as Step (ii) in Reference Example 9.
LCMS: [M+H]⁺/Rt(min): 231/0.64

### Reference Example 38: 2-Fluoro-6-{[1-(propan-2-yl)piperidin-4-yl]oxy}aniline

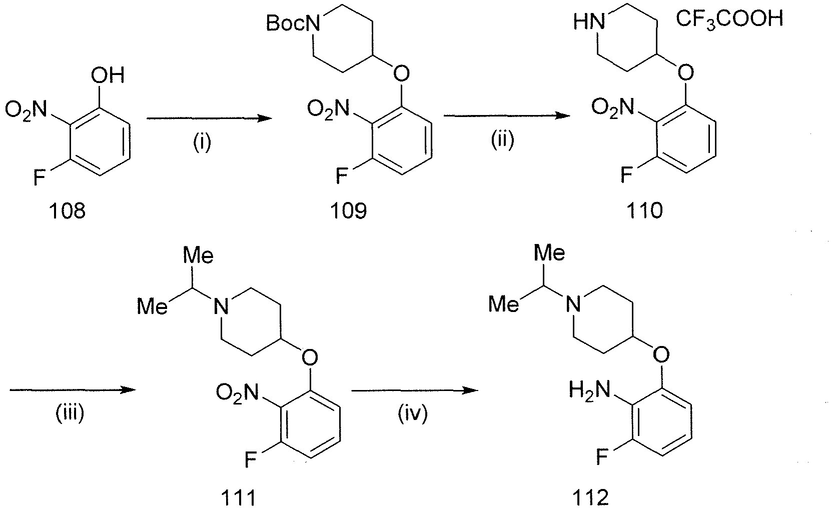

### Step (i) :

A mixture of compound 108 (0.157 g), tert-butyl 4-hydroxypiperidine-1-carboxylate (0.201 g), diisopropyl azodicarboxylate (0.242 g), triphenylphosphine (0.524 g) and THF (10 mL) was stirred at room temperature for 1 hour. Water was added to the reaction mixture and the mixture was extracted with ethyl acetate. The organic layer was dried over anhydrous sodium sulfate and concentrated *in vacuo.* The resulting residue was then purified by silica gel column chromatography (Eluate: hexane/ethyl acetate) to give the title compound 109 (0.233 g).
LCMS: [M+H]⁺/Rt(min): 341/2.17 (Method B)

### Step (ii):

The crude product of the title compound 110 was prepared from compound 109 (0.100 g) in the same manner as Step (ii) in Reference Example 21.

### Step (iii):

The title compound 111 (0.060 g) was prepared from the crude product of compound 110 in the same manner as Step (iii) in Reference Example 20.

### Step (iv):

The title compound 112 (0.030 g) was prepared from compound 111 (0.060 g) in the same manner as Step (ii) in Reference Example 2.

### Reference Example 39: Rac-2-chloro-6-{[1-(propan-2-yl)pyrrolidin-3-yl]oxy}aniline

According to the process in the above Reference Example 38, the compound shown below was synthesized by using each corresponding starting compound instead of compound 108 and tert-butyl 4-hydroxypiperidine-1-carboxylate at Step (i) in Reference Example 38.

**[Table 2]**

| Reference Example | Chemical Structural Formula | Instrumental Analysis Data |
|---|---|---|
| 39 | 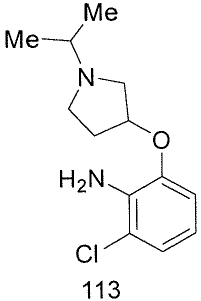 | LCMS: [M+H] ⁺/Rt (min) : 255/0.57 (Method C) |

### Reference Example 40: tert-Butyl 3-(3-chloro-2-{[4-(5-cyclopropyl-1,2,4-oxadiazol-3-yl)-4-methylpiperidine-1-carbonyl]amino}phenoxy)azetidine-1-carboxylate

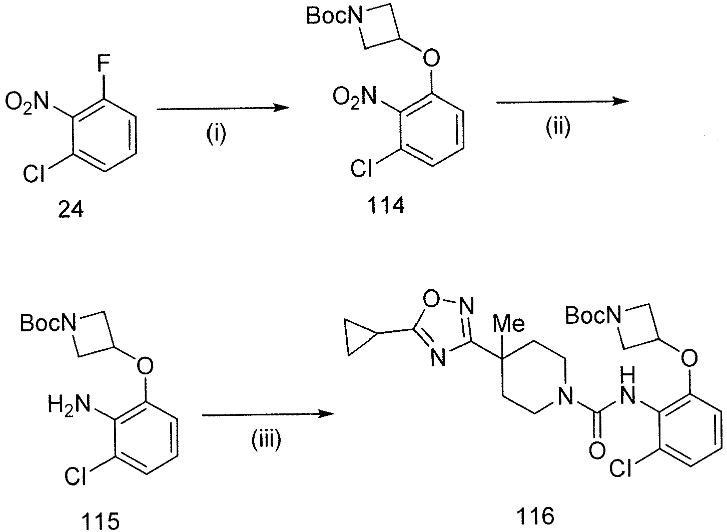

### Step (i) :

A mixture of tert-butyl 3-hydroxyazetidine-1-carboxylate (1.30 g), sodium hydride (0.41 g) and THF (21 mL) was stirred at 0°C for 30 minutes, and then compound 24 was added thereto and the mixture was stirred at room temperature for 3 hours. To the reaction was added saturated aqueous sodium hydrogen carbonate solution and the mixture was extracted with ethyl acetate. The organic layer was dried over anhydrous sodium sulfate and concentrated *in vacuo.* The resulting residue was then purifiled by silica gel column chromatography (Eluate: hexane/ethyl acetate) to give the title compound 114 (2.06 g).
LCMS: [M+H]⁺/Rt(min): 329/1.11

### Step (ii):

The title compound 115 (0.77 g) was prepared from compound 114 (1.00 g) in the same manner as Step (ii) in Reference Example 9.
LCMS: [M+H]⁺/Rt(min): 299/1.06

### Step (iii):

The title compound 116 (0.110 g) was prepared from compound 115 (0.060 g) in the same manner as Step (iv) in Reference Example 23.
LCMS: [M+H]⁺/Rt(min): 533/1.08

### Reference Example 41: 2-Methoxy-6-{ [4-(propan-2-yl)piperazin-1-yl]methyl}aniline

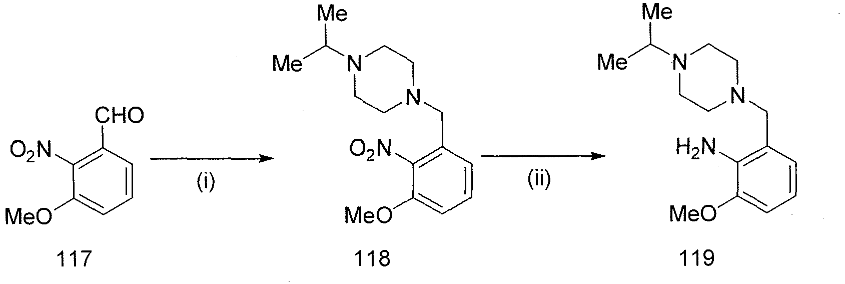

### Step (i):

The title compound 118 (0.231 g) was prepared from compound 117 (0.181 g) and isopropylpiperazine (0.128 g) in the same manner as Step (iii) in Reference Example 20.

### Step (ii):

The title compound 119 (0.140 g) was prepared from compound 118 (0.230 g) in the same manner as Step (ii) in Reference Example 2.

### Reference Example 42: 3-Fluoro-4'-(propan-2-yl)[1,1'-biphenyl]-2-amine

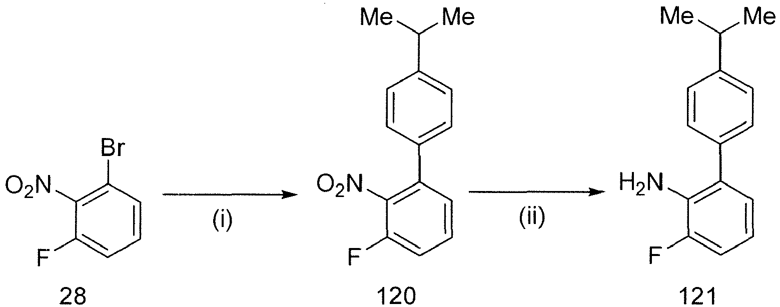

### Step (i) :

The title compound 20 (0.231 g) was prepared from compound 28 (0.220 g) and corresponding boronic acid in the same manner as Step (i) in Reference Example 20.

### Step (ii):

The title compound 121 (0.165 g) was prepared from compound 120 (0.230 g) in the same manner as Step (ii) in Reference Example 2.

### Reference Example 43: tert-Butyl 4-(3-chloro-2-{[4-(5-cyclopropyl-1,2,4-oxadiazol-3-yl)-4-methylpiperidine-1-carbonyl]amino}phenyl)piperidine-1-carboxylate

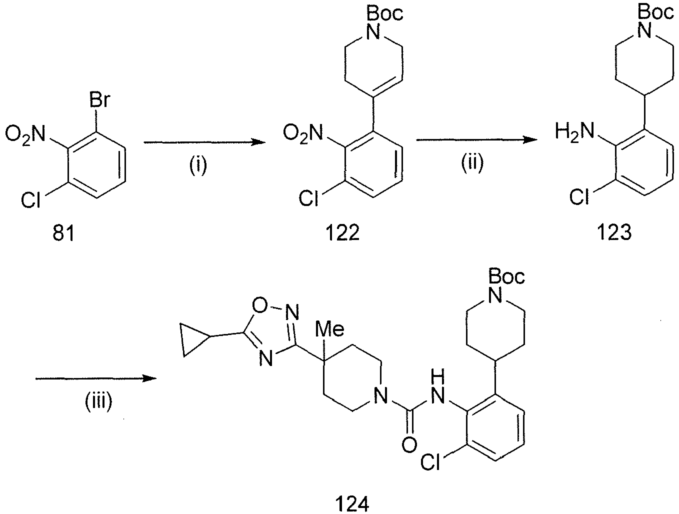

### Step (i):

The title compound 122 (0.502 g) was prepared from compound 81 (0.709 g) in the same manner as Step (i) in Reference Example 20.
LCMS: [M+H]⁺/Rt(min): 339/2.26 (Method B)

### Step (ii):

The title compound 123 (0.039 g) was prepared from compound 122 (0.10 g) in the same manner as Step (ii) in Reference Example 2.
LCMS: [M+H]⁺/Rt(min): 311/2.21 (Method B)

### Step (iii):

The title compound 124 (0.084 g) was prepared from compound 123 (0.070 g) in the same manner as Step (iv) in Reference Example 23.
LCMS: [M+H]⁺/Rt(min): 545/1.12 (Method C)

### Reference Examples 44-45

According to the process in the above Reference Example 43, the compounds shown below were synthesized by using each corresponding starting compound instead of 4-(4-methylphenyl)piperidine at Step (iii) in Reference Example 43.

**[Table 3]**

| Reference Example | Chemical Structural Formula | Instrumental Analysis Data |
|---|---|---|
| 44 | 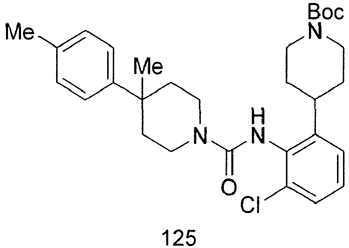 | LCMS: [M+H]+/Rt(min): 527/2.06 (Method B) |
| 45 | 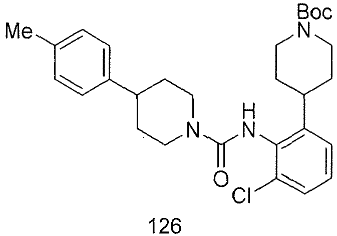 | LCMS: [M+H]⁺/Rt(min): 512/2.30 (Method B) |

Reference Example 44: tert-butyl 4-(3-chloro-2-{[4-methyl-4-(4-methylphenyl)piperidine-1-carbonyl]amino}phenyl)piperidine-1-carboxylate Reference Example 45: tert-butyl 4-(3-chloro-2-{[4-(4-methylphenyl)piperidine-1-carbonyl]amino}phenyl)piperidine-1-carboxylate

### Reference Example 46: 2-Fluoro-6-(6-methylpyridin-3-yl)aniline

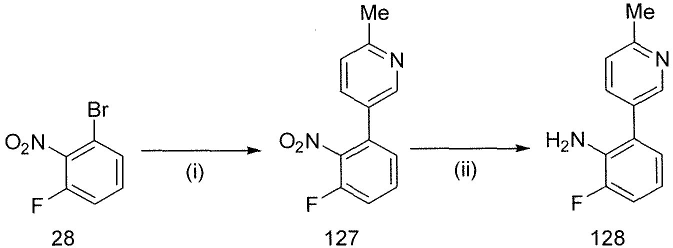

### Step (i):

The title compound 127 (0.198 g) was prepared from compound 28 (0.178 g) and corresponding boronic acid in the same manner as Step (i) in Reference Example 20.
LCMS: [M+H]⁺/Rt(min): 261/0.98

### Step (ii):

The title compound 128 (0.148 g) was prepared from compound 127 (0.184 g) in the same manner as Step (ii) in Reference Example 9.
LCMS: [M+H]⁺/Rt(min): 231/0.64

### Reference Example 47: 2-Chloro-6-[6-(propan-2-yl)pyridin-3-yl]aniline

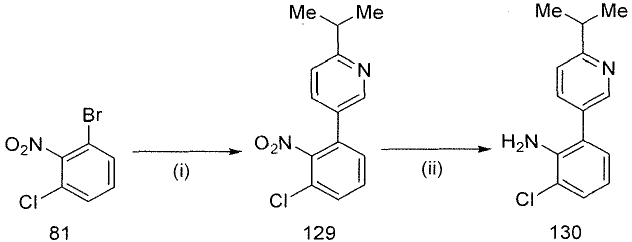

### Step(i):

The title compound 129 (0.147 g) was prepared from compound 81 (0.247 g) and corresponding boronic acid in the same manner as Step (i) in Reference Example 20.
LCMS: [M+H]⁺/Rt(min): 277/1.07

### Step (ii):

The title compound 130 (0.130 g) was prepared from compound 129 (0.142 g) in the same manner as Step (ii) in Reference Example 9.
LCMS: [M+H]⁺/Rt(min): 247/0.75

### Reference Example 48: 2-Fluoro-6-(5-methylpyridin-3-yl)aniline

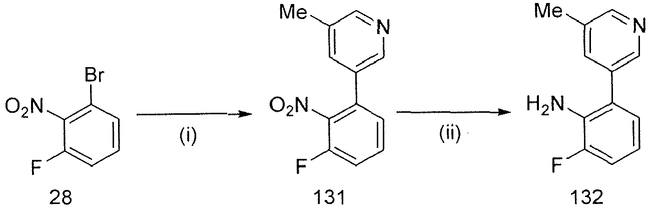

### Step (i) :

The title compound 131 (0.104 g) was prepared from compound 28 (0.143 g) and corresponding boronic acid in the same manner as Step (i) in Reference Example 20.
LCMS: [M+H]⁺/Rt(min): 233/0.80

### Step (ii):

The title compound 132 (0.072 g) was prepared from compound 131 (0.091 g) in the same manner as Step (ii) in Reference Example 9.
LCMS: [M+H]⁺/Rt(min): 203/0.53

### Reference Example 49: 2-Fluoro-6-[6-(trifluoromethyl)pyridin-3-yl]aniline

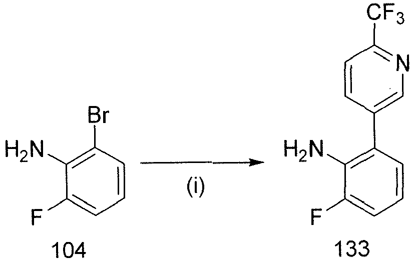

### Step (i) :

The title compound 133 (0.054 g) was prepared from compound 104 (0.248 g) and corresponding boronic acid in the same manner as Step (i) in Reference Example 20.
LCMS: [M+H]⁺/Rt(min): 257/0.90 (Method C)

### Reference Example 50: 2-Fluoro-6-[2-(propan-2-yl)pyrimidin-5-yl]aniline

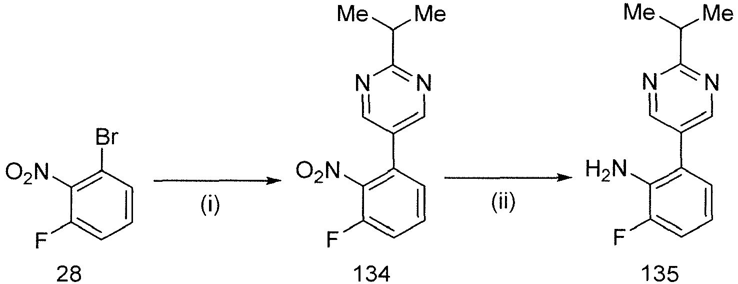

### Step (i):

The title compound 134 (0.038 g) was prepared from compound 28 (0.100 g) and corresponding boronic acid in the same manner as Step (i) in Reference Example 20.
LCMS: [M+H]⁺/Rt(min): 262/0.90 (Method C)

### Step (ii):

The title compound 135 (0.004 g) was prepared from compound 34 (0.036 g) in the same manner as Step (ii) in Reference Example 9.
¹H-NMR (CDCl₃) δ: 1.41 (6H, d, J = 6.8 Hz), 3.22-3.37 (1H, m), 3.79 (2H, br), 6.72-6.84 (1H, m), 6.84-6.94 (1H, m), 7.00-7.12 (1H, m), 8.81 (2H, s).

### Reference Example 51: tert-Butyl (1S,4S)-5-(3-fluoro-2-{[4-(4-methylphenyl)piperidine-1-carbonyl]amino}phenyl)-2,5-diazabicyclo[2.2.1]heptane-2-carboxylate

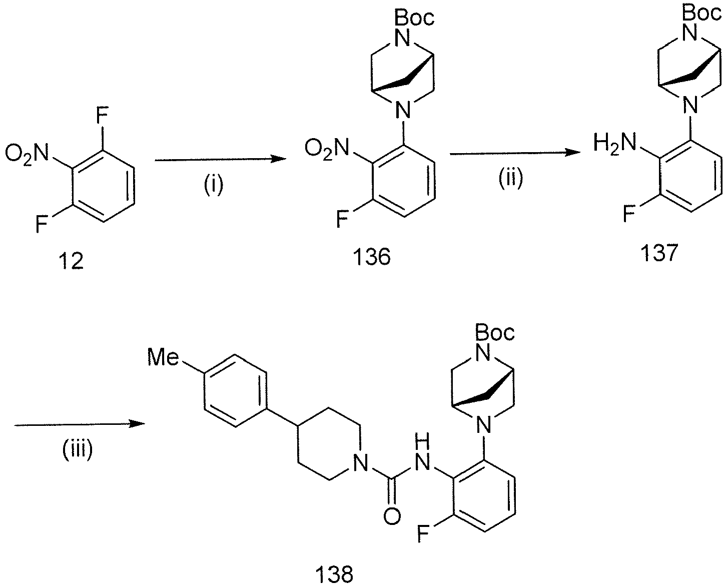

### Step (i) :

The title compound 136 (0.530 g) was prepared from compound 12 (0.324 g) in the same manner as Step (i) in Reference Example 2.
LCMS: [M+H]⁺/Rt(min): 338/1.08

### Step (ii):

The title compound 137 (0.385 g) was prepared from compound 136 (0.530 g) in the same manner as Step (ii) in Reference Example 2.
LCMS: [M+H]⁺/Rt(min): 308/1.01

### Step (iii):

The title compound 138 (0.080 g) was prepared from compound 137 (0.044 g) in the same manner as Step (iv) in Reference Example 23.
LCMS: [M+H]⁺/Rt(min): 509/1.15

### Reference Example 52: tert-Butyl (1S,4S)-5-(2-{[4-(5-cyclopropyl-1,2,4-oxadiazol-3-yl)-4-methylpiperidine-1-carbonyl]amino}-3-fluorophenyl)-2,5-diazabicyclo[2.2.1]heptane-2-carboxylate

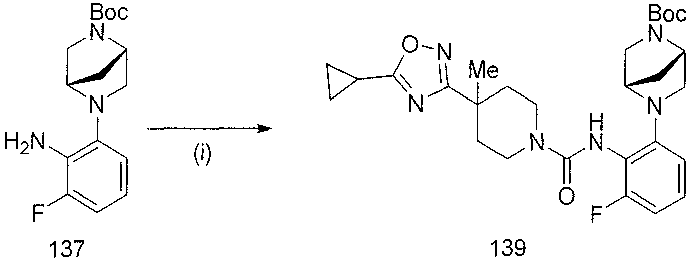

### Step (i):

The title compound 139 (0.189 g) was prepared from compound 137 (0.124 g) in the same manner as Step (iv) in Reference Example 23.
LCMS: [M+H]⁺/Rt(min): 541/1.01

### Reference Example 53: tert-Butyl (1R,4R)-5-(3-fluoro-2-{[4-(4-methylphenyl)piperidine-1-carbonyl]amino}phenyl)-2,5-diazabicyclo[2.2.1]heptane-2-carboxylate

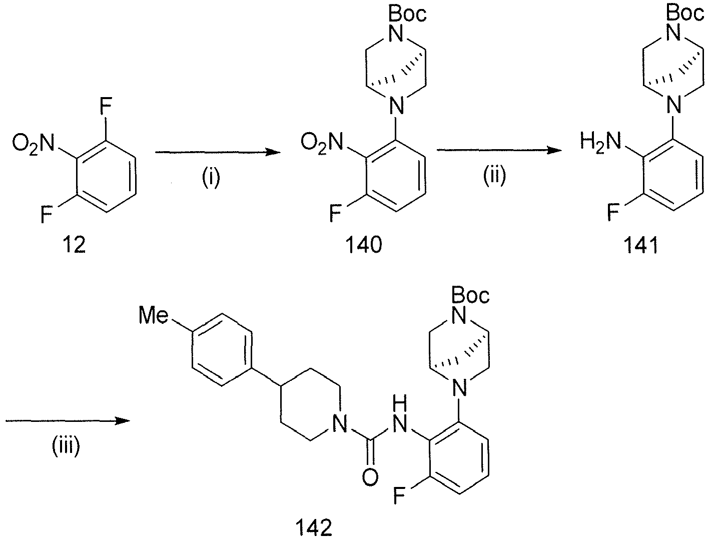

### Step (i):

The title compound 140 (0.410 g) was prepared from compound 12 (0.252 g) in the same manner as Step (i) in Reference Example 2.
LCMS: [M+H]⁺/Rt(min): 338/1.08

### Step (ii):

The title compound 141 (0.224 g) was prepared from compound 140 (0.404 g) in the same manner as Step (ii) in Reference Example 2.
LCMS: [M+H]⁺/Rt(min): 308/1.01

### Step (iii):

The title compound 142 (0.080 g) was prepared from compound 141 (0.037 g) in the same manner as Step (iv) in Reference Example 23.
LCMS: [M+H]⁺/Rt(min): 509/1.15

### Reference Example 54: tert-Butyl (1R,4R)-5-(2-{[4-(5-cyclopropyl-1,2,4-oxadiazol-3-yl)-4-methylpiperidine-1-carbonyl]amino}-3-fluorophenyl)-2,5-diazabicyclo[2.2.1]heptane-2-carboxylate

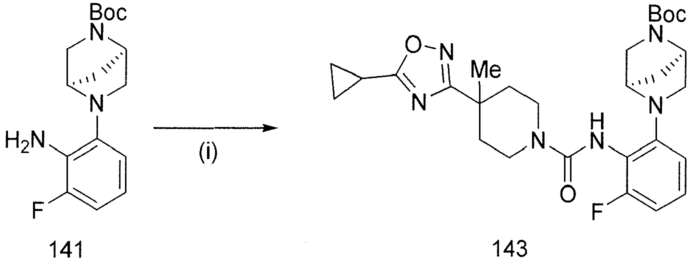

### Step (i):

The title compound 143 (0.088 g) was prepared from compound 141 (0.055 g) in the same manner as Step (iv) in Reference Example 23.
LCMS: [M+H]⁺/Rt(min): 541/1.01

### Reference Example 55: tert-Butyl 3-(3-fluoro-2-{[4-(4-methylphenyl)piperidine-1-carbonyl]amino}phenyl)-3,8-diazabicyclo[3.2.1]octane-8-carboxylate

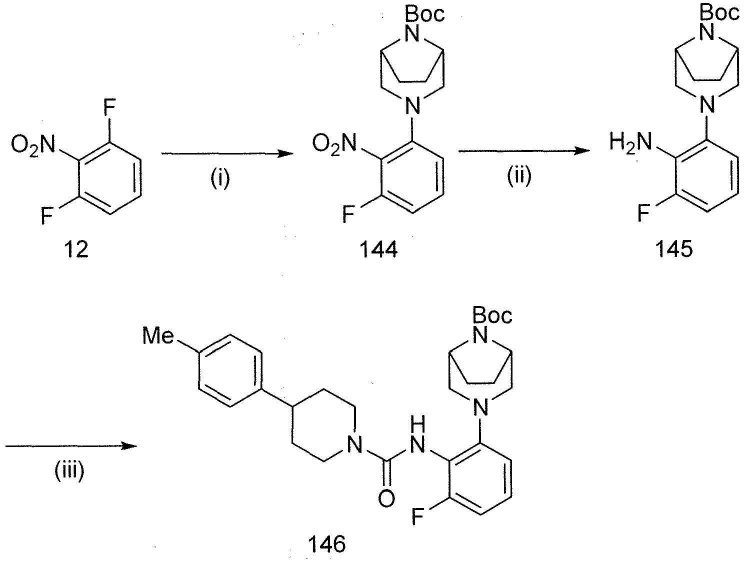

### Step (i) :

The title compound 144 (0.575 g) was prepared from compound 12 (0.382 g) in the same manner as Step (i) in Reference Example 2.

### Step (ii):

The title compound 145 (0.451 g) was prepared from compound 144 (0.563 g) in the same manner as Step (ii) in Reference Example 2.
LCMS: [M+H]⁺/Rt(min): 322/1.11

### Step (iii):

The title compound 146 (0.161 g) was prepared from compound 145 (0.111 g) in the same manner as Step (iv) in Reference Example 23.
LCMS: [M+H]⁺/Rt(min): 523/1.23

### Reference Example 56: tert-Butyl 3-(2-{[4-(5-cyclopropyl-1,2,4-oxadiazol-3-yl)-4-methylpiperidine-1-carbonyl]amino}-3-fluorophenyl)-3,8-diazabicyclo[3.2.1]octane-8-carboxylate

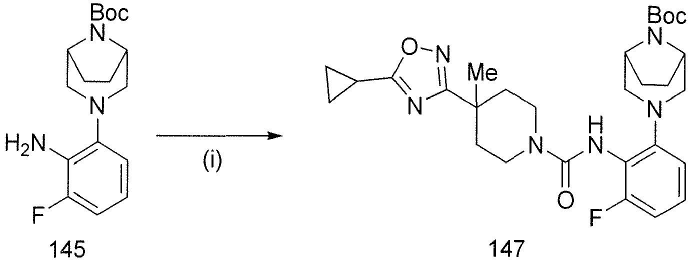

### Step (i):

The title compound 147 (0.189 g) was prepared from compound 145 (0.107 g) in the same manner as Step (iv) in Reference Example 23.
LCMS: [M+H]⁺/Rt(min): 556/1.12

### Reference Example 57: Benzyl 8-(3-fluoro-2-{[4-(4-methylphenyl)piperidine-1-carbonyl]amino}phenyl)-3,8-diazabicyclo[3.2.1]octane-3-carboxylate

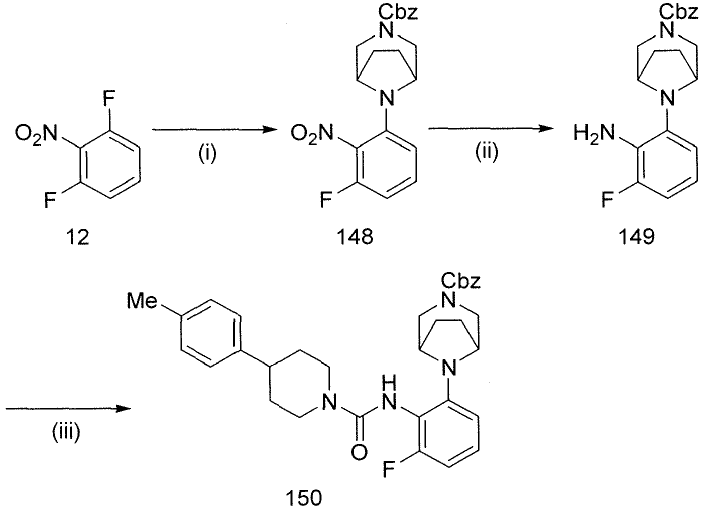

### Step (i):

The title compound 148 (0.338 g) was prepared from compound 12 (0.204 g) in the same manner as Step (i) in Reference Example 2.
LCMS: [M+H]⁺/Rt(min): 386/1.15

### Step (ii):

The title compound 149 (0.220 g) was prepared from compound 148 (0.307 g) in the same manner as Step (ii) in Reference Example 9.
LCMS: [M+H]⁺/Rt(min): 356/1.10

### Step (iii):

The title compound 150 (0.080 g) was prepared from compound 149 (0.073 g) in the same manner as Step (iv) in Reference Example 23.
LCMS: [M+H]⁺/Rt(min): 557/1.20

### Reference Example 58: Rac-tert-butyl (2R,5S)-2-(2-amino-3-chlorophenyl)-5-(propan-2-yl)morpholine-4-carboxylate

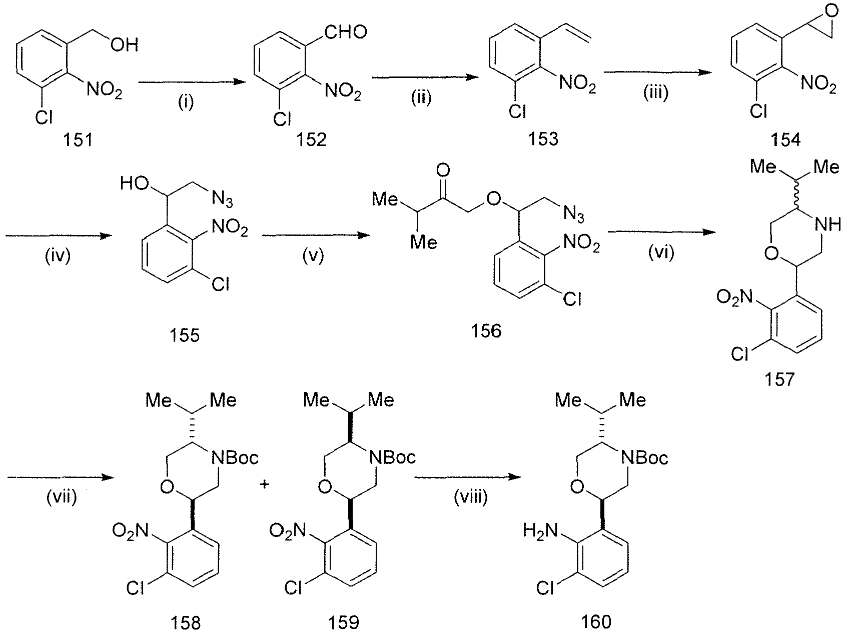

### Step (i):

A mixture of compound 151 (3.03 g), manganese dioxide (11.56 g) and chloroform (160 mL) was stirred with heating under reflux for 12 hours. The mixture was cooled, filtrated with Celite, and concentrated *in vacuo.* The resulting residue was then purified by silica gel column chromatography (Eluate: hexane/ethyl acetate) to give the title compound 152 (2.59 g).
LCMS: [M+H]⁺/Rt(min): 186/0.81

### Step (ii):

To a mixture of methyltriphenylphosphonium bromide (3.25 g) and THF (21 mL) was added n-butyllithium hexane solution (1.57M, 5.35 mL) at 0°C and the mixture was stirred at the same temperature for 1 hour. To the mixture was added dropwise a solution of compound 152 (1.30 g) in THF (7 mL) and the mixture was stirred at room temperature for 3 hours. To reaction mixture was added saturated aqueous ammonium chloride solution and the mixture was extracted with chloroform. The organic layer was dried over anhydrous sodium sulfate and concentrated *in vacuo.* The resulting residue was then purified by silica gel column chromatography (Eluate: hexane/ethyl acetate) to give the title compound 153 (0.756 g).

### Step (iii):

A mixture of compound 153 (0.333 g), m-chloroperoxybenzoic acid (0.542 g) and methylene chloride (9 mL) was stirred at room temperature for 4 hours. To the reaction mixture was added saturated aqueous sodium hydrogen carbonate solution and the mixture was extracted with chloroform. The organic layer was dried over anhydrous sodium sulfate and concentrated *in vacuo.* The resulting residue was then purified by silica gel column chromatography (Eluate: chloroform/methanol) to give the title compound 154 (0.141 g).

### Step (iv):

A mixture of compound 154 (0.141 g), sodium azide (0.046 g) and acetonitrile (3 mL) was stirred at 40°C for 3 hours. Water was further added thereto and the mixture was stirred with heating under reflux for 10 hours. The reaction mixture was concentrated *in vacuo* and then the resulting residue was purified by silica gel column chromatography (Eluate: hexane/ethyl acetate) to give the title compound 155 (0.084 g).

### Step (v):

A mixture of compound 155 (0.100 g), 1-bromo-3-methylbutan-2-one (0.086 g), caesium carbonate (0.226 g) and acetonitrile (3.5 mL) was stirred at room temperature for 2 hours. The reaction mixture was concentrated *in vacuo* and then the resulting residue was purified by silica gel column chromatography (Eluate: hexane/ethyl acetate) to give the title compound 156 (0.100 g).
LCMS: [M+H]⁺/Rt(min): 327/1.10

### Step (vi):

A mixture of compound 156 (0.100 g), triphenylphosphine (0.097 g) and THF (6 mL) was stirred at room temperature for 4 hours. To the reaction mixture was added sodium triacetoxyborohydride (0.098 g) and the mixture was stirred at room temperature for 16 hours. To the reaction mixture was added saturated aqueous sodium hydrogen carbonate solution and the mixture was extracted with chloroform. The organic layer was dried over anhydrous sodium sulfate and concentrated *in vacuo.* The resulting residue was then used directly in the next step.
LCMS: [M+H]⁺/Rt(min): 285/0.69, 285/0.74 (Method C)

### Step (vii):

A mixture of the crude product of compound 157, Boc₂O (0.067 g) and THF (6 mL) was stirred at room temperature for 16 hours. The title compound mixture (0.55 g) was prepared in the same manner as Step (ii) in Reference Example 2. To the reaction mixture was added saturated aqueous sodium hydrogen carbonate solution and the mixture was extracted with chloroform. The organic layer was dried over anhydrous sodium sulfate and concentrated *in vacuo.* The resulting residue was then purified by silica gel column chromatography (Eluate: hexane/ethyl acetate) to give the title compound 158 (0.031 g) and the title compound 159 (0.018).

### Step (vii):

The title compound 160 (0.029 g) was prepared from compound 158 (0.031 g) in the same manner as Step (ii) in Reference Example 9.
LCMS: [M+H]⁺/Rt(min): 355/1.29

### Reference Example 59: Rac-tert-butyl (2R,5R)-2-(2-amino-3-chlorophenyl)-5-(propan-2-yl)morpholine-4-carboxylate

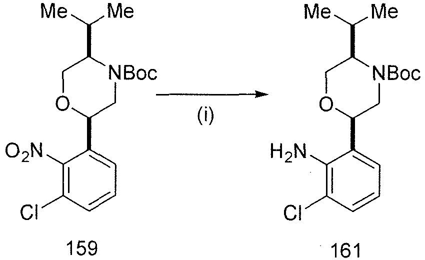

### Step (i) :

The title compound 161 (0.011 g) was prepared from compound 159 (0.018 g) in the same manner as Step (ii) in Reference Example 9.
LCMS: [M+H]⁺/Rt(min): 355/1.29

### Reference Example 60: Rac-6-(4-methylphenyl)-3-azabicyclo[4.1.0]heptane

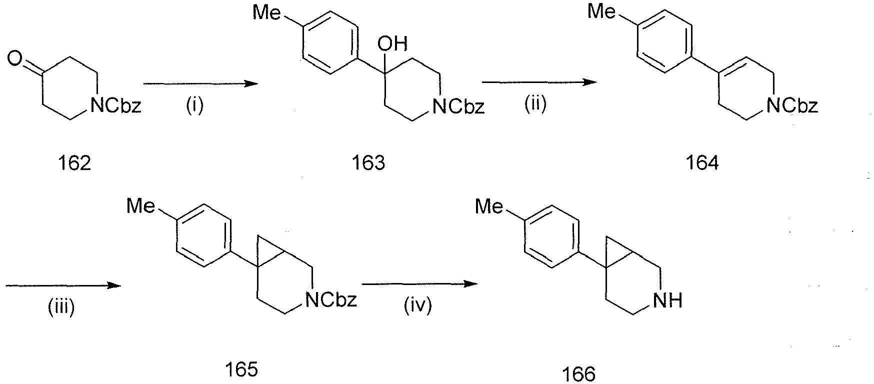

### Step (i) :

A mixture of compound 162 (1.42 g), p-tolylmagnesium bromide (1M, 8.52 mL) and THF (20 mL) was stirred at room temperature for 14 hours. Water was added to the reaction mixture and the mixture was extracted with ethyl acetate. The organic layer was dried over anhydrous sodium sulfate and concentrated *in vacuo.* The resulting residue was then purified by silica gel column chromatography (Eluate: hexane/ethyl acetate) to give the title compound 163 (1.62 g).
LCMS: [M+H]⁺/Rt(min): 326/1.01

### Step (ii):

A mixture of compound 163 (0.742 g), p-toluenesulfonic acid monohydrate (0.217 g) and toluene was stirred at 50°C for 1 hour. To the reaction mixture was added saturated aqueous sodium hydrogen carbonate solution and the mixture was extracted with ethyl acetate. The organic layer was dried over anhydrous sodium sulfate and concentrated *in vacuo.* The resulting residue was then purified by silica gel column chromatography (Eluate: hexane/ethyl acetate) to give the title compound 164 (0.561 g).
LCMS: [M+H]⁺/Rt(min): 308/2.23 (Method B)

### Step (iii):

To a mixture of diethylzinc (1M, 2.12 mL), diiodomethane (0.566 g) and dichloromethane (2 mL) was added dropwise a solution of compound 164 (0.130 g) in dichloromethane (0.8 mL) at 0°C and the mixture was stirred at room temperature for 14 hours. Water was added to the reaction mixture and the mixture was extracted with ethyl acetate. The organic layer was dried over anhydrous sodium sulfate, concentrated *in vacuo,* and then the resulting residue was dissolved in THF (2.8 mL) and water (2.8 mL). To the mixture were added N-methylmorphoine N-oxide (0.114 g) and osmium (VIII) oxide and the mixture was stirred at room temperature for 12 hours. To the reaction mixture were added THF (3 mL) and saturated aqueous sodium thiosulfate solution and the mixture was stirred at 0°C for 30 minutes. The mixture was extracted with ethyl acetate, and then the obtained organic layer was dried over anhydrous sodium sulfate and concentrated *in vacuo.* The resulting residue was then purified by silica gel column chromatography (Eluate: hexane/ethyl acetate) to give the title compound 165 (0.018 g).
LCMS: [M+H]⁺/Rt(min): 322/2.27 (Method B)

### Step (iv):

The title compound 166 (0.010 g) was prepared from compound 165 (0.017 g) in the same manner as Step (ii) in Reference Example 2.
LCMS: [M+H]⁺/Rt(min): 188/1.37 (Method B)

### Reference Example 61: 4-(2-Fluoro-4-methylphenyl)piperidine

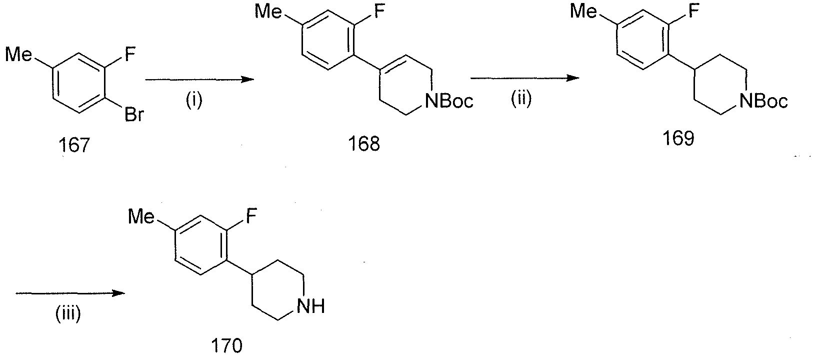

### Step (i) :

The title compound 168 (0.100 g) was prepared from compound 167 (0.095 g) in the same manner as Step (i) in Reference Example 20.
LCMS: [M+H]⁺/Rt(min): 292/2.26 (Method B)

### Step (ii):

The crude product of the title compound 169 was prepared from compound 168 (0.100 g) in the same manner as Step (ii) in Reference Example 2.

### Step (iii):

The title compound 170 (0.050 g) was prepared from the crude product of compound 169 in the same manner as Step (ii) in Reference Example 14.
LCMS: [M+H]⁺/Rt(min): 194/0.58

### Reference Example 62: 4-(5-Cyclopropyl-1,2,4-oxadiazol-3-yl)-4-methylpiperidine hydrochloride

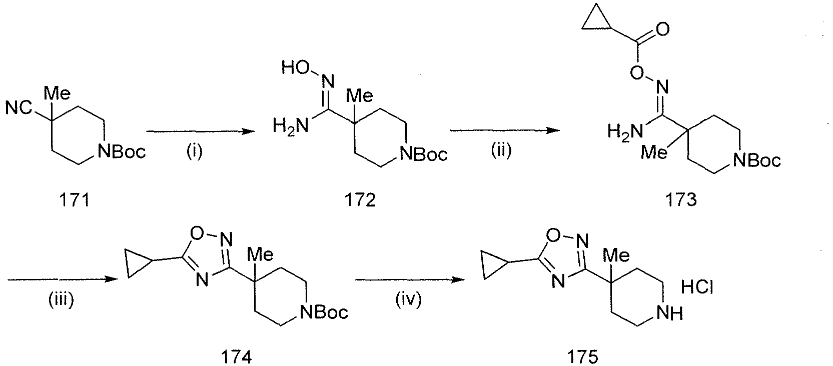

### Step (i) :

To a solution of compound 171 (50.0 g) in ethanol (446 mL) was addded aqueous 50% hydroxylamine solution (132 mL) and the mixture was stirred at 70°C for 8 hours. The mixture was cooled to room temperature, and then water (892 mL) was added to the reaction mixture and the mixture was stirred at room temperature for 30 minutes. The precipitated white crystal was collected on a filter, and then the obtained crsytal was suspended in water (344 mL) and stirred at room temperature for 30 minutes again. The precipitated white solid was collected on a filter and dried to give the title compound 172 (52.3 g).
LCMS: [M+H]⁺/Rt(min): 258/0.52 (Method C)

### Step (ii):

To a mixture of compound 172 (52.3 g), cyclopropanecarboxylic acid (18.4 g), HATU (85 g) and THF (406 mL) was added slowly dropwise triethylamine (142 mL) in ice bath and the mixture was stirred at room temperature for 12 hours. To the reaction mixture was added ethyl acetate (406 mL) and the mixture was washed with water (406 mL) and brine (406 mL). The organic layer was dried over anhydrous sodium sulfate and concentrated *in vacuo.* The resulting residue was then purified by silica gel column chromatography (Eluate: hexane/ethyl acetate) to give the title compound 173 (59.1 g).
LCMS: [M+H]⁺/Rt(min): 326/0.77 (Method C)

### Step (iii):

A mixture of compound 173 (59.1 g), DBU (54.2 mL) and toluene (727 mL) was stirred with heating under reflux for 1 hour. The reaction mixture was cooled to room temperature and then washed with water (727 mL). The organic layer was concentrated *in vacuo* and then the resulting residue was purified by silica gel column chromatography (eluate: hexane/ethyl acetate) to give the title compound 174 (54.5 g).
LCMS: [M+H]⁺/Rt(min): 308/1.11 (Method C)

### Step (iv):

The title compound 175 (35.3 g) was prepared from compound 174 (54.5 g) in the same manner as Step (ii) in Reference Example 20.
LCMS: [M+H]⁺/Rt(min): 208/0.30 (Method C)

### Reference Examples 63-87

According to the process in the above Reference Example 62, the compounds of Reference Examples 63-87 shown the table below were synthesized by using each corresponding starting compound instead of Compound 171 at Step (i) and cyclopropanecarboxylic acid at Step (ii) in Reference Example 62.

**[Table 4]**

| Reference Example | Chemical Structural Formula | Instrumental Analysis Data |
|---|---|---|
| 63 | 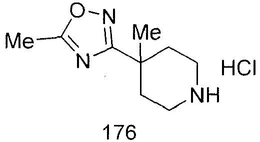 | LCMS: [M+H]⁺/Rt(min): 182/0.20 (Method C) |
| 64 | 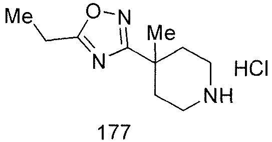 | LCMS: [M+H] ⁺/Rt (min) : 196/0.32 (Method C) |
| 65 | 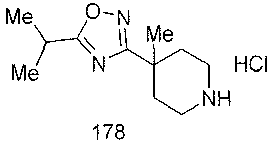 | LCMS: [M+H] ⁺/Rt (min) : 210/0.37 (Method C) |
| 66 | 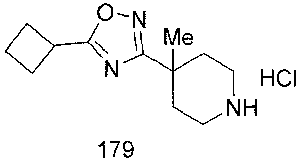 | LCMS: [M+H] ⁺/Rt (min) : 222/0.42 (Method C) |
| 67 | 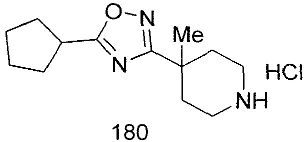 | LCMS: [M+H]⁺/Rt(min): 236/0.53 (Method C) |
| 68 | 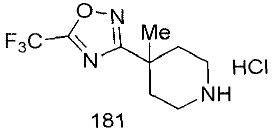 | LCMS: [M+H]⁺/Rt(min): 236/0.45 (Method C) |
| 69 | 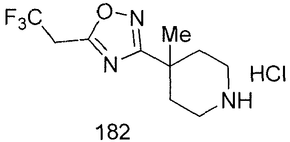 | LCMS: [M+H] ⁺/Rt (min) : 250/0.43 (Method C) |
| 70 | 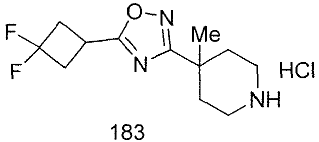 | LCMS: [M+H] ⁺/Rt (min) : 258/0.24 (Method C) |
| 71 | 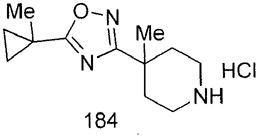 | LCMS: [M+H] ⁺/Rt (min) : 222/0.42 (Method C) |
| 72 | 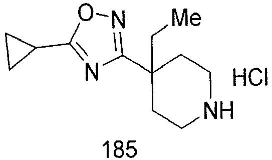 | LCMS: [M+H] ⁺/Rt (min) : 222/0.48 (Method C) |
| 73 | 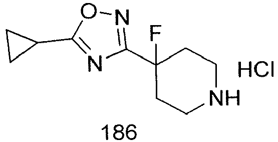 | LCMS: [M+H] ⁺/Rt (min) : 212/0.28 (Method C) |
| 74 | 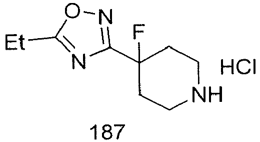 | LCMS: [M+H] ⁺/Rt (min) : 200/0.23 (Method C) |
| 75 | 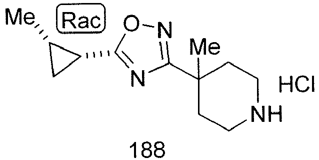 | LCMS: [M+H]⁺/Rt(min): 222/0.57 (Method C) |
| 76 | 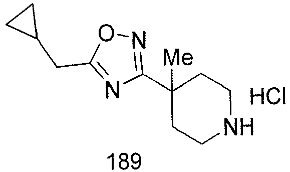 | LCMS: [M+H]⁺/Rt(min): 222/0.54 (Method C) |
| 77 | 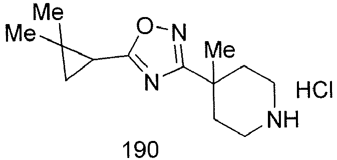 | LCMS: [M+H] ⁺/Rt (min) : 236/0.65 (Method C) |
| 78 | 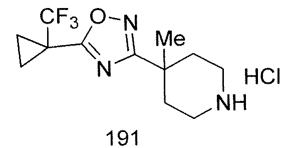 | LCMS: [M+H]⁺/Rt(min): 276/0.64 (Method C) |
| 79 | 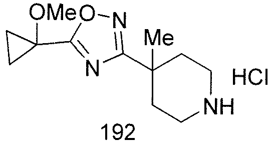 | LCMS: [M+H] ⁺/Rt (min) : 238/0.49 (Method C) |
| 80 | 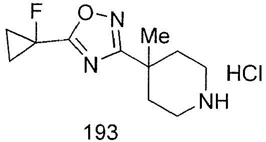 | LCMS: [M+H]⁺/Rt (min) : 226/0.37 (Method C) |
| 81 | 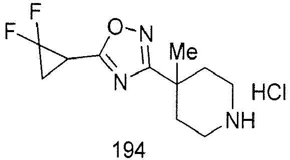 | LCMS: [M+H] ⁺/Rt (min) : 244/0.50 (Method C) |
| 82 | 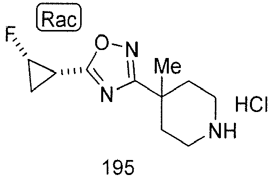 | LCMS: [M+H] ⁺/Rt (min) : 226/0.32 (Method C) |
| 83 | 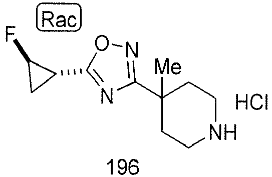 | LCMS: [M+H] ⁺/Rt (min) : 226/0.36 (Method C) |
| 84 | 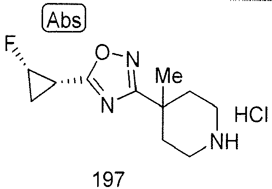 | LCMS: [M+H] ⁺/Rt (min) : 226/0.29 (Method C) |
| 85 | 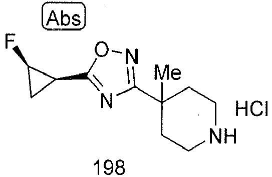 | LCMS: [M+H] ⁺/Rt (min) : 226/0.31 (Method C) |
| 86 | 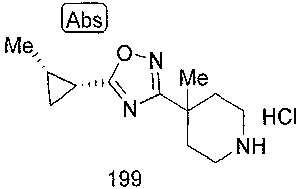 | LCMS: [M+H]+/Rt(min): 222/0.44 (Method C) |
| 87 | 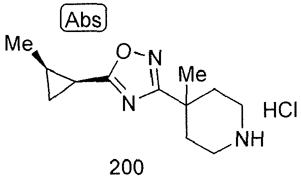 | LCMS: [M+H] ⁺/Rt (min) : 222/0.45 (Method C) |

Reference Example 63: 4-methyl-4-(5-methyl-1,2,4-oxadiazol-3-yl)piperidine hydrochloride
Reference Example 64: 4-(5-ethyl-1,2,4-oxadiazol-3-yl)-4-methylpiperidine hydrochloride
Reference Example 65: 4-methyl-4-[5-(propan-2-yl)-1,2,4-oxadiazol-3-yl]piperidine hydrochloride
Reference Example 66: 4-(5-cyclobutyl-1,2,4-oxadiazol-3-yl)-4-methylpiperidine hydrochloride
Reference Example 67: 4-(5-cyclopentyl-1,2,4-oxadiazol-3-yl)-4-methylpiperidine hydrochloride
Reference Example 68: 4-methyl-4-[5-(trifluoromethyl)-1,2,4-oxadiazol-3-yl]piperidine hydrochloride
Reference Example 69: 4-methyl-4-[5-(2,2,2-trifluoroethyl)-1,2,4-oxadiazol-3-yl]piperidine hydrochloride
Reference Example 70: 4-[5-(3,3-difluorocyclobutyl)-1,2,4-oxadiazol-3-yl]-4-methylpiperidine hydrochloride Reference Example 71: 4-methyl-4-[5-(1-methylcyclopropyl)-1,2,4-oxadiazol-3-yl]piperidine hydrochloride
Reference Example 72: 4-(5-cyclopropyl-1,2,4-oxadiazol-3-yl)-4-ethylpiperidine hydrochloride
Reference Example 73: 4-(5-cyclopropyl-1,2,4-oxadiazol-3-yl)-4-fluoropiperidine hydrochloride
Reference Example 74: 4-(5-ethyl-1,2,4-oxadiazol-3-yl)-4-fluoropiperidine hydrochloride
Reference Example 75: rac-4-metyl-4-{5-[(1R,2S)-2-methylcyclopropyl]-1,2,4-oxadiazol-3-yl}piperidine hydrochloride
Reference Example 76: 4-[5-(cyclopropylmethyl)-1,2,4-oxadiazol-3-yl]-4-methylpiperidine hydrochloride
Reference Example 77: 4-[5-(2,2-dimethylcyclopropyl)-1,2,4-oxadiazol-3-yl]-4-methylpiperidine hydrochloride
Reference Example 78: 4-methyl-4-{5-[1-(trifluoromethyl)cyclopropyl]-1,2,4-oxadiazol-3-yl}piperidine hydrochloride
Reference Example 79: 4-[5-(1-methoxycyclopropyl)-1,2,4-oxadiazol-3-yl]-4-methylpiperidine hydrochloride
Reference Example 80: 4-[5-(1-fluorocyclopropyl)-1,2,4-oxadiazol-3-yl]-4-methylpiperidine hydrochloride
Reference Example 81: 4-[5-(2,2-difluorocyclopropyl)-1,2,4-oxadiazol-3-yl]-4-methylpiperidine hydrochloride
Reference Example 82: rac-4-{5-[(1S,2S)-2-fluorocyclopropyl]-1,2,4-oxadiazol-3-yl}-4-methylpiperidine hydrochloride
Reference Example 83: rac-4-{5-[(1R,2S)-2-fluorocyclopropyl]-1,2,4-oxadiazol-3-yl}-4-methylpiperidine hydrochloride
Reference Example 84: 4-{5-[(1S,2S)-2-fluorocyclopropyl]-1,2,4-oxadiazol-3-yl}-4-methylpiperidine hydrochloride
Reference Example 85: 4-{5-[(1R,2R)-2-fluorocyclopropyl]-1,2,4-oxadiazol-3-yl}-4-methylpiperidine hydrochloride
Reference Example 86: 4-{5-[(1R,2S)-2-methylcyclopropyl]-1,2,4-oxadiazol-3-yl}-4-methylpiperidine hydrochloride
Reference Example 87: 4-{5-[(1S,2R)-2-methylcyclopropyl]-1,2,4-oxadiazol-3-yl}-4-methylpiperidine hydrochloride

### Reference Example 88: 3-(3-Cyclopropyl-1,2,4-oxadiazol-5-yl)-8-azabicyclo[3.2.1]octane

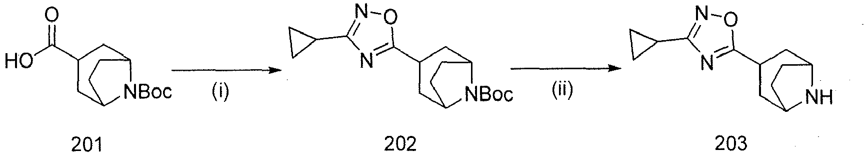

### Step (i) :

To a solution of compound 201 (0.100 g) in toluene (0.01 mL) was added CDI (0.070 g) and the mixture was stirred at room temperature for 3 hours. To the reaction mixture was added N'-hydroxycyclopropanecarboximidamide hydrochloride (0.059 mg) and the mixture was stirred heating under reflux for 2 hours with. The reaction mixture was purified by silica gel column chromatography (Eluate: hexane/ethyl acetate) to give the title compound 202 (0.123 g).
LCMS: [M+H]⁺/Rt(min): 320/1.01 (Method C)

### Step (ii):

The title compound 203 (0.091 g) was prepared from compound 202 (0.121 g) in the same manner as Step (ii) in Reference Example 14.
LCMS: [M+H]⁺/Rt(min): 220/0.30 (Method C)

### Reference Example 89: 4-(5-Cyclopropyl-1,2-oxazol-3-yl)-4-methylpiperidine hydrochloride

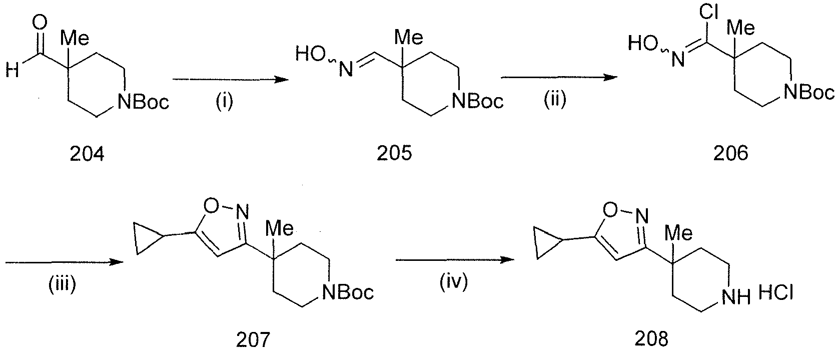

### Step (i):

To a mixture of compound 204 (900 mg), sodium acetate (650 mg) and methanol (5 mL) was added hydroxylamine hydrochloride (550 mg) and the mixture was stirred at room temperature for 24 hours. The reaction solution was cooled to 0°C, water was added thereto, and the mixture was extracted with chloroform. The organic layer was dried over anhydrous sodium sulfate and concentrated *in vacuo* to give the title compound 205 (1.23 g).

### Step (ii):

To a mixture of compound 205 (416 mg) and DMF (4 mL) was added N-chlorosuccinimide (252 mg) and the mixture was stirred for 3 hours. The reaction solution was cooled to 0°C, water (6 mL) was added thereto, and the precipitated solid was filtrated and dried to give the title compound 206 (326 mg).

### Step (iii):

To a mixture of ethynylcyclopropane (117 mg) and toluene (5 mL) were added compound 206 (326 mg) and sodium hydrogen carbonate (198 mg) and the mixture was stirred at room temperature. After the reaction was terminated as judged by the consumption of the starting material, water was added to the reaction mixture and the mixture was extracted with ethyl acetate. The organic layer was dried over anhydrous sodium sulfate and concentrated *in vacuo.* The resulting residue was then purified by silica gel column chromatography (Eluate: hexane/ethyl acetate) to give the title compound 207 (348 mg).
LCMS: [M+H]⁺/Rt(min): 307/1.13

### Step (iv):

The title compound 208 (307 mg) was prepared from compound 207 (337 mg) in the same manner as Step (ii) in Reference Example 20.
LCMS: [M+H]⁺/Rt(min): 207/0.49

### Reference Examples 90-91

According to the process in the above Reference Example 89, the compounds of Reference Examples 90-91 shown the table below were synthesized by using each corresponding starting compound instead of ethynylcyclopropane at Step (iii) in Reference Example 89.

**[Table 5]**

| Reference Example | Chemical Structural Formula | Instrumental Analysis Data |
|---|---|---|
| 90 | 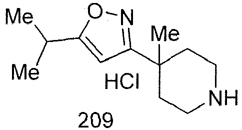 | LCMS: [M+H]⁺/Rt(min): 209/0.53 |
| 91 | 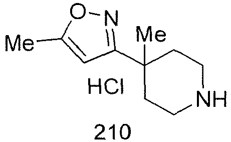 | LCMS: [M+H] ⁺/Rt (min) : 181/0.44 |

Reference Example 90: 4-methyl-4-[5-(propan-2-yl)-1,2-oxazol-3-yl]piperidine
Reference Example 91: 4-methyl-4-(5-methyl-1,2-oxazol-3-yl)piperidine

### Reference Example 92: 2-(4-Methylpiperidin-4-yl)-1,3-benzoxazole

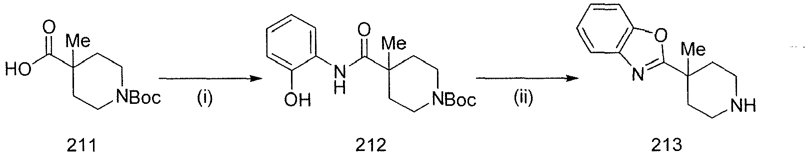

### Step (i) :

To a solution of compound 211 (1.46 g) in THF (30 mL) were added isobutyl chloroformate (819 mg) and diisopropylethylamine (3.88 g) under ice temperature and the mixture was stirred for 1 hour. To the mixture was then added 2-aminophenol (655 mg) under ice temperature and the mixture was heated and stirred at 70°C for 6 hours. The reation solution was directly purifed by amino silica gel column chromatography (Eluate: ethyl acetate/hexane) to give the title compound 212 (710 mg).
LCMS: [M+H]⁺/Rt(min): 335/2.28 (Method B)

### Step (ii):

A mixture of compound 212 (204 mg) and acetic acid (1.10 mL) was stirred at 90°C with heating for 2 hours and concetnrated *in vacuo.* The resulting residue was dissolved in chloroform (2 mL), trifluoromethanesulfonic acid (2.1 mL) was added thereto, and the mixture was stirred at room temperature for 1 hour. The reaction solution was concentrated *in vacuo,* ethyl acetate and aqueous sodium hydrogen carbonate solution were added thereto, and then the mixture was extraced with ethyl acetate. The organic layer was dried over anhydrous sodium sulfate and concentrated *in vacuo.* The resulting residue was then purfied by amino silica gel chromatography (Eluate: ethyl acetate/hexane) to give the title compound 213 (99 mg).
LCMS: [M+H]⁺/Rt(min): 217/1.36 (Method B)

### Reference Example 93: 4-(4-Chloro-5-cyclopropyl-1,2-oxazol-3-yl)-4-methylpiperidine hydrochloride

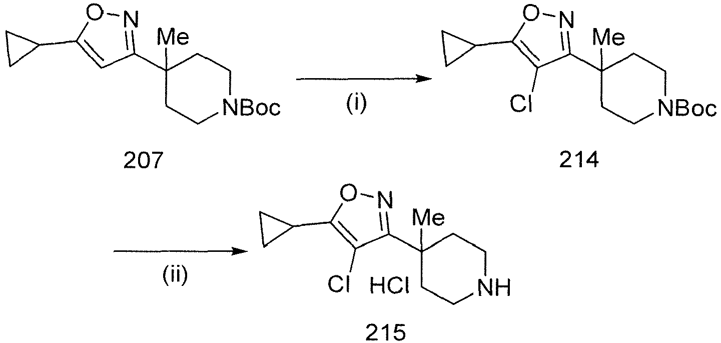

### Step (i):

A solution of compound 207 (500 mg) and N-chlorosuccinimide (240 mg) in DMF (3.3 mL) was stirred at room temperature for 3 days. Water was added to the reaction solution and the mixture was extracted with diethyl ether. The organic layer was washed with water, dried over sodium sulfate, and concentrated *in vacuo.* The resulting residue was then purified by silica gel column chromatography (Eluate: hexane/ethyl acetate) to give the title compound 214 (434 mg).

### Step (ii):

The title compound 215 (120.1 mg) was prepared from compound 214 (200 mg) in the same manner as Step (ii) in Reference Example 20.
LCMS: [M+H]⁺/Rt(min): 241/0.65

### Reference Example 94: 4-(4-Chloro-5-methyl-1,2-oxazol-3-yl)-4-methylpiperidine hydrochloride

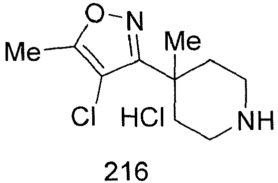

According to the process in the above Reference Example 93, compound 126 was synthesized by using compound 210 in Reference Example 91 instead of compound 207 at Step (i) in Reference Example 93.
LCMS: [M+H]⁺/Rt(min): 215/0.51

### Reference Example 95: 4-Cyclopentyl-4-methylpiperidine hydrochloride

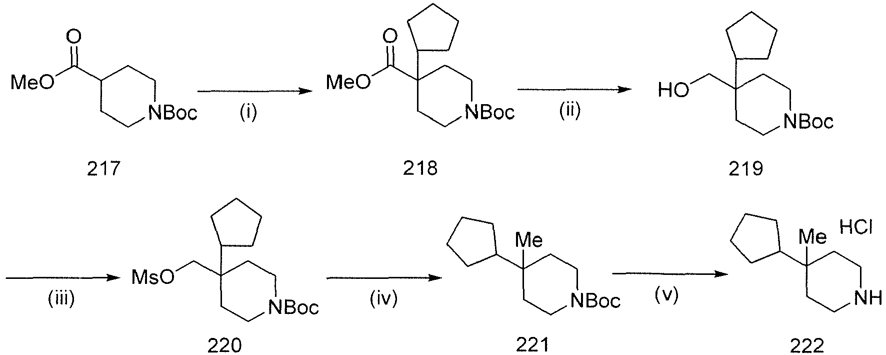

### Step (i):

To a mixture of compound 217 (700 mg) and THF (14 mL) was addded lithium diisopropylamide (2M, 5.18 mL) at -78°C and the mixture was stirred at the same temperature for 2 hours. To the reaction mixture were then added bromocyclopentane (1.23 mL) and potassium iodide (478 mg), the mixture was warmed to room temperature and stirred overnight, and then water was added thereto and the mixture was extracted with ethyl acetate. The organic layer was dried over anhydrous sodium sulfate and concentrated *in vacuo.* The resulting residue was then purified by silica gel column chromatography (Eluate: ethyl acetate/hexane) to give the title compound 218 (468 mg).
LCMS: [M+H]⁺/Rt(min): 312/1.26

### Step (ii):

To a mixture of lithium aluminium hydride (104 mg) and THF (3 mL) was added a mixture of compound 218 (371 mg) and THF (6 mL) under ice temperature and the mixture was stirred for 4 hours. After the reaction was terminated as judged by the consumption of the starting material, water (0.104 mL), 15% aqueous sodium hydroxide solution (0.104 ml), and then water (0.312 mL) were added to the reaction mixture at 0°C, and the mixture was stirred. The reaction solution was filtrated and the filtrate was concentrated *in vacuo.* The resulting residue was then purified by silica gel column chromatography (Eluate: ethyl acetate/hexane) to give the title compound 219 (320 mg).
LCMS: [M+H]⁺/Rt(min): 284/1.06

### Step (iii):

To a mixture of compound 219 (314 mg), triethylamine (0.309 mL) and THF (5 mL) was added methanesulfonyl chloride (0.104 mL) and the mixture was stirred at room temperature. After the reaction was terminated as judged by the consumption of the starting material, water was added to the reaction mixture and the mixture was extracted with chloroform. The organic layer was dried over anhydrous sodium sulfate and concentrated *in vacuo.* The resulting residue was then purified by silica gel column chromatography (Eluate: ethyl acetate/hexane) to give the title compound 220 (290 mg).
LCMS: [M+H]⁺/Rt(min): 362/1.15

### Step (iv):

To a mixture of compound 220 (278 mg) and THF (3 mL) was added lithium triethylborohydride (0.99 M, 1.55 mL) and the mixture was stirred at room temperature. The reaction solution was then heated to 70°C. After the reaction was terminated as judged by the consumption of the starting material, the reaction solution was cooled to 0°C and aqueous ammonium chloride solution was added thereto. The mixture was extracted with chloroform, the obtained organic layer was dried over anhydrous sodium sulfate and concentrated *in vacuo.* The resulting residue was then purifed by silica gel column chromatography (Eluate: hexane/ethyl acetate) to give the title compound 221 (100 mg).
LCMS: [M+H]⁺/Rt(min): 268/1.42

### Step (v) :

The title compound 222 (58.5 mg) was prepared from compound 221 (90 mg) in the same manner as Step (ii) in Reference Example 20.
LCMS: [M+H]⁺/Rt(min): 168/0.62

### Reference Example 96: 4-Methyl-4-(2-methyl-1,3-oxazol-5-yl)piperidine

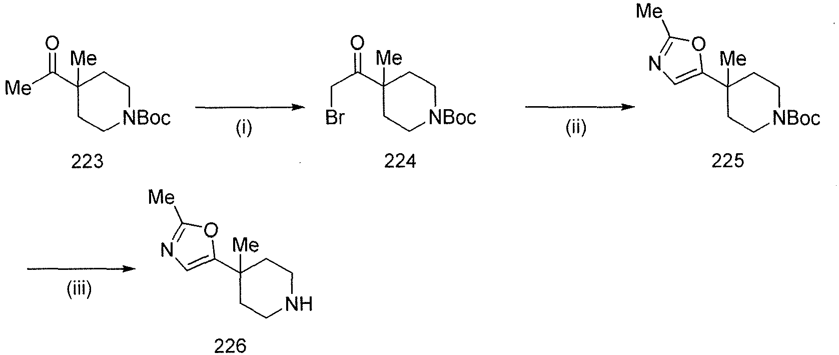

### Step (i):

To a solution of compound 223 (1.00 g) in THF (10 mL) cooled to -78°C was added dropwise a solution of LHMDS in toluene (1M, 4.35 mL) and the mixture was stirred for 1 hour. Trimethylchlorosilane (0.495 g) was added thereto and the mixture was warmed to 0°C andminutes. The mixture was cooled to -78°C again, and then bromine (0.662 g) was added thereto and the mixture was gradually warmed to room temperature. The reaction mixture was added to a mixture of 10% aqueous sodium hypochlorite solution (7.5 mL) and saturated aqueous ammonium chloride solution (7.5 mL) and the mixture was extracted with ethyl acetate. The obtained organic layer was concentrated in *vacuo* to give the title compound 224 (1.16 g).
LCMS: [M+H]⁺/Rt(min): 321/0.94

### Step (ii):

A mixture of compound 224 (0.10 g) and acetamide (0.020 g) was stirred at 130°C for 3 hours. The reaction mixture was purified by silica gel column chromatography (Eluate: hexane/ethyl acetate) to give the title compound 225 (0.007 g).
LCMS: [M+H]⁺/Rt(min): 281/0.97

### Step (iii):

The title compound 226 (0.005 g) was prepared from compound 225 (0.007 g) in the same manner as Step (ii) in Reference Example 14.
LCMS: [M+H]⁺/Rt(min): 181/0.22

### Reference Example 97: 4-Methyl-4-[2-(propan-2-yl)-1,3-oxazol-5-yl]piperidine

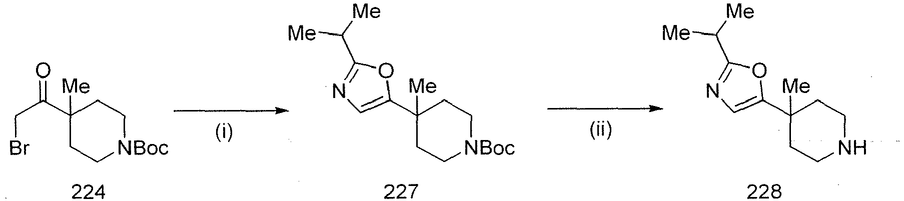

### Step (i):

The title compound 227 (0.012 g) was prepared from compound 224 (0.10 g) in the same manner as Step (ii) in Reference Example 96.
LCMS: [M+H]⁺/Rt(min): 309/1.18

### Step (ii):

The title compound 228 (0.009 g) was prepared from compound 227 (0.011 g) in the same manner as Step (ii) in Reference Example 14.
LCMS: [M+H]⁺/Rt(min): 209/0.38

### Reference Example 98: 4-(5-Cyclopropyl-1,3,4-thiadiazol-2-yl)-4-methylpiperidine

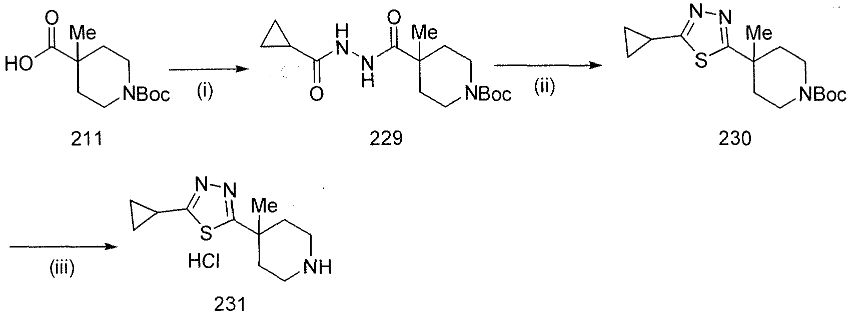

### Step (i):

To a mixture of compound 211 (399 mg), cyclopropanecarbohydrazide hydrochloride (269 mg) and DMF (5 mL) were added HATU (686 mg) and diisopropylethylamine (1.15 mL) and the mixture was stirred at room temperature for 3 hours. Water was added to the reaction solution and the mixture was extracted with ethyl acetate. The organic layer was dried over anhydrous sodium sulfate and concentrated *in vacuo.* The resulting residue was then purified by silica gel column chromatography (Eluate: hexane/ethyl acetate) to give the title compound 229 (520 mg).
LCMS: [M+H]⁺/Rt(min): 326/0.74

### Step (ii):

To a mixture of compound 229 (255 mg) and toluene (6 mL) was added Lawesson's reagent (349 mg) and the mixture was stirred with heating under reflux for 1 hour. The reaction solution was cooled to 0°C, and then aqueous sodium hydrogen carbonate solution was added thereto and the mixture was extracted with ethyl acetate. The organic layer was dried over anhydrous sodium sulfate and concentrated *in vacuo.* The resulting residue was then purified by silica gel column chromatography (Eluate: hexane/ethyl acetate) followed by amino silica gel column chromatography (Eluate: hexane/ethyl acetate) to give the title compound 230 (102 mg).
LCMS: [M+H]⁺/Rt(min): 324/1.08

### Step (iii):

The title compound 231 (78 mg) was prepared from compound 230 (92 mg) in the same manner as Step (ii) in Reference Example 20.
LCMS: [M+H]⁺/Rt(min): 224/0.45

### Reference Example 99: 4-(5-Cyclopropyl-1,3-thiazol-2-yl)-4-methylpiperidine hydrochloride

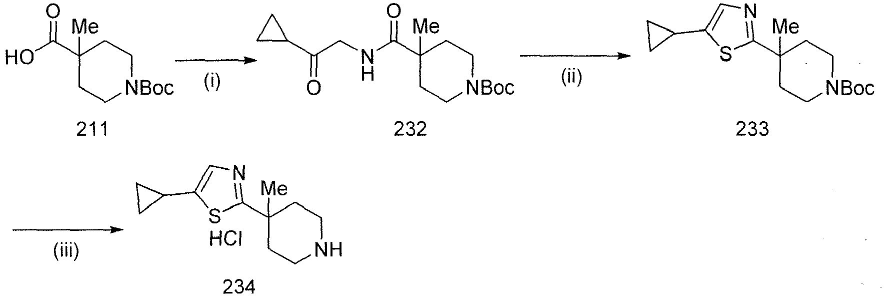

### Step (i):

The title compound 232 (796 mg) was prepared from compound 211 (718 mg) and 2-amino-1-cyclopropylethan-1-one hydrochloride (400 mg) in the same manner as Step (i) in Reference Example 98.
LCMS: [M+H]⁺/Rt(min): 325/0.83

### Step (ii):

To a mixture of compound 232 (127 mg), pyridine (0.063 mL) and toluene (3 mL) was added Lawesson's reagent (205 mg) and the mixture was stirred with heating under reflux for 14 hours. The reaction solution was cooled to room temperature, and then aqueous sodium hydrogen carbonate solution was added thereto and the mixture was extracted with ethyl acetate. The organic layer was dried over anhydrous sodium sulfate and concentrated *in vacuo.* The resulting residue was then purified by amino silica column chromatography (Eluate: hexane/ethyl acetate) to give the title compound 233 (76.3 mg) .
LCMS: [M+H]⁺/Rt(min): 323/1.43

### Step (iii):

The title compound 234 (66.5 mg) was prepared from compound 233 (77 mg) in the same manner as Step (ii) in Reference Example 20.
LCMS: [M+H]⁺/Rt(min): 223/0.67

### Reference Example 100: 4-(2-Cyclopropyl-1,3-thiazol-4-yl)-4-methylpiperidine hydrochloride

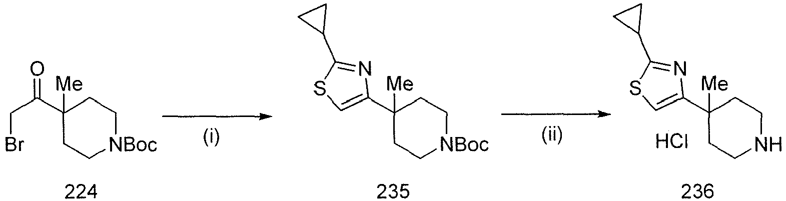

### Step (i) :

A soluiton of compound 224 (531.6 mg) and cyclopropanecarbothioamide (168 mg) in methanol (6 mL) was stirred with heating under reflux for 2 and a half hours. The resulting mixture was cooled to room temperature, and then saturated aqueous sodium hydrogen carbonate solution was added thereto and the mixture was extracted with chloroform. The organic layer was dried over sodium sulfate and concentrated *in vacuo.* The resulting residue was then purifed by silica gel column chromatography (Eluate: hexane/ethyl acetate) to give the title compound 235 (119 mg).

### Step (ii):

The title compound 236 (136.8 mg) was prepared from compound 235 (119 mg) in the same manner as Step (ii) in Reference Example 20.
LCMS: [M+H]⁺/Rt(min): 223/0.57

### Reference Example 101:

### Ethyl 4-methyl-1-({2-[4-(propan-2-yl)piperazin-1-yl]phenyl}carbamoyl)piperidine-4-carboxylate

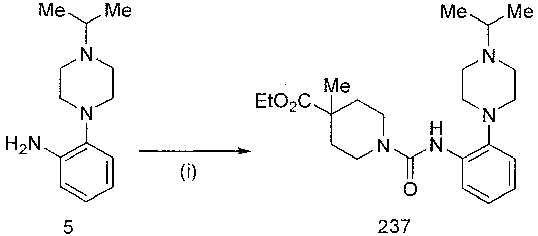

### Step (i) :

The title compound 237 (0.357 g) was prepared from compound 5 (0.237 g) in the same manner as Step (i) in Reference Example 1.
LCMS: [M+H]⁺/Rt(min): 417/1.61 (Method B)

### Reference Example 102:

### Ethyl 4-fluoro-1-({2-[4-(propan-2-yl)piperazin-1-yl]phenyl}carbamoyl)piperidine-4-carboxylate

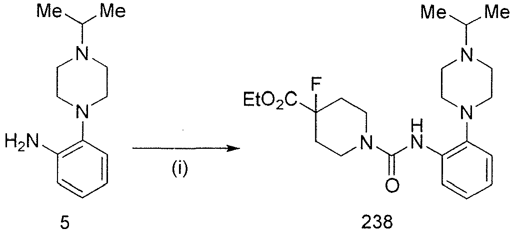

### Step (i) :

The title compound 238 (0.040 g) was prepared from compound 5 (0.179 g) in the same manner as Step (i) in Reference Example 1.
LCMS: [M+H]⁺/Rt(min): 421/1.57 (Method B)

### Reference Example 103 tert-Butyl (3aR,5s,6aS)-5-cyanohexahydrocyclopenta[c]pyrrole-2(1H)-carboxylate (compound A-2) and tert-butyl (3aR,5r,6aS)-5-cyanohexahydrocyclopenta[c]pyrrole-2 (1H)-carboxylate (compound A-3)

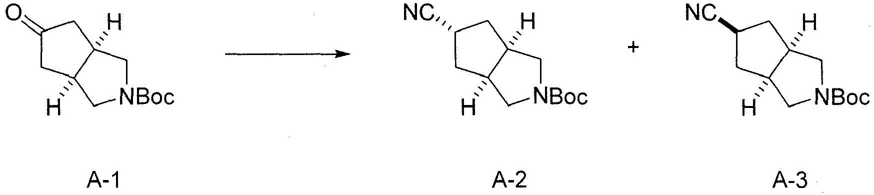

To a solution of compound A-1 (331 mg) in 1,2-dimethoxyethane (6.7 mL)/ethanol (0.67 mL) were added p-toluenesulfonylmethyl isocyanide (373 mg) and potassium t-butoxide (396 mg) at 0°C and the mixture was stirred at 40°C for 4 hours. The mixture was cooled to room temperature, and then the reaction solution was filtrated and the filtrate was concentrated *in vacuo.* The resulting residue was then purfiled by silica gel column chromatography (Eluate: hexane/ethyl acetate) to give the title compounds A-2 (80 mg) and A-3 (59.4 mg).
Compound A-2: LCMS: [M+H]⁺/Rt(min): 237/0.89
Compound A-3: LCMS: [M+H]⁺/Rt(min): 237/0.88

### Reference Examples 104-105

According to the process in the above Reference Example 62, the compounds of Reference Examples 104-105 shown in the table below were synthesized by using compound A-2 or A-3 in Reference Example 103 instead of compound 171 at Step (i) in Reference Example 62.

**[Table 6]**

| Reference Example | Chemical Structural Formula | Instrumental Analysis Data |
|---|---|---|
| 104 | 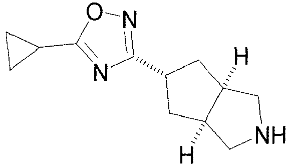 | LCMS: [M+H]⁺/Rt(min): 220/0.39 |
| 105 | 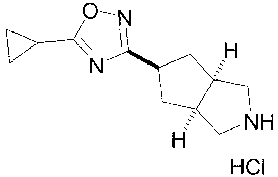 | LCMS: [M+H]⁺/Rt(min): 220/0.48 |

Reference Example 104: (3aR,5s,6aS)-5-(5-cyclopropyl-1,2,4-oxadiazol-3-yl)octahydrocyclopenta[c]pyrrole
Reference Example 105: (3aR,5r,6aS)-5-(5-cyclopropyl-1,2,4-oxadiazol-3-yl)octahydrocyclopenta[c]pyrrole

### Example 1: N-[2-(4-Ethylpiperazin-1-yl)phenyl]-4-(4-methoxyphenyl)piperidine-1-carboxamide

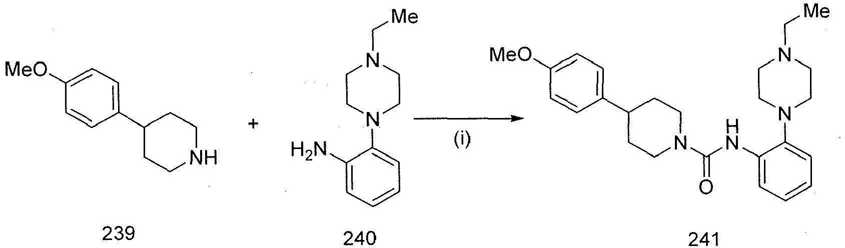

The title compound (0.031 g) was prepared from compound 239 (0.025 g) and compound 240 (0.021 g) in the same manner as Step (iv) in Reference Example 23.
¹H-NMR (CDCl₃) δ: 1.14 (3H, t, J = 7.2 Hz), 1.59-1.76 (3H, m), 1.94 (2H, d, J = 12.4 Hz), 2.48-2.75 (6H, m), 2.95-3.05 (6H, m), 3.80 (3H, s), 4.28 (2H, d, J = 13.2 Hz), 6.87 (2H, d, J = 7.2 Hz), 6.96 (1H, dt, J = 7.6, 5.6 Hz), 7.12-7.18 (4H, m), 8.22 (1H, dd, J = 8.4, 1.6 Hz), 8.26 (1H, s).

### Examples 2-120:

The compounds of Examples 2-120 shown in the table below were synthesized in the same manner as Example 1 by using each corresponding commercial compound or compound of Reference Example.

**[Table 7]**

| Example | Strucural Formula | Material A | Material B |
|---|---|---|---|
| | Spectral Data | | |
| 2 | | Commercial Product | Commercial Product |
| | 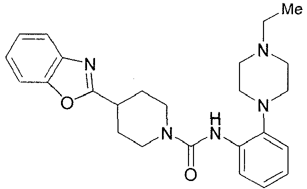 | 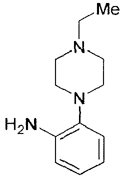 | 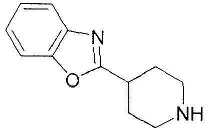 |
| | ¹H-NMR (CDCl₃) δ: 1.20 (3H, t, J = 6.4 Hz), 1.61 (2H, brs), 2.08 (1H, dd, J = 10.4, 4.0 Hz), 2.12 (1H, dd, J = 10.4, 4.0 Hz), 2.30 (2H, dd, J = 13.2, 4.0 Hz), 2.56-2.69 (4H, m), 3.00 (4H, brs), 3.27 (3H, dt, J = 11.2, 2.8 Hz), 4.18 (2H, dt, J = 14.0, 3.6 Hz), 7.00 (1H, dt, J = 7.6, 1.2 Hz), 7.15-7.22 (2H, m), 7.34-7.38 (2H, m), 7.54 (1H, dd, J = 3.6, 2.4 Hz), 7.73 (1H, dd, J = 3.6, 2.4 Hz), 8.23 (1H, dd, J = 8.4, 0.8 Hz), 8.29 (1H, brs) | | |
| 3 | | Commercial Product | Commercial Product |
| | 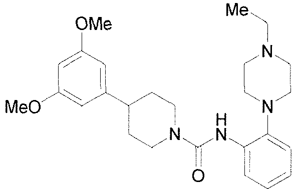 | 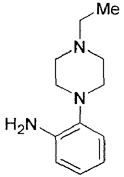 | 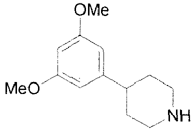 |
| | ¹H-NMR (CDCl₃) δ: 1.22 (3H, m), 1.73 (1H, dd, J = 12.4, 4.0 Hz), 1.78 (1H, dd, J = 12.4, 4.0 Hz), 1.97 (2H, d, J = 11.6 Hz), 2.76-2.58 (4H, m), 3.07-3.01 (5H, m), 3.82 (6H, s), 4.30 (2H, d, J = 13.2 Hz), 6.37 (1H, d, J = 2.4 Hz), 6.41 (2H, d, J = 2.4 Hz), 7.00 (1H, dt, J = 8.2, 1.0 Hz), 7.22-7.16 (2H, m), 8.23 (1H, d, J = 8.2 Hz) | | |
| 4 | | Commercial Product | Commercial Product |
| | 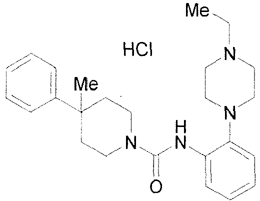 | 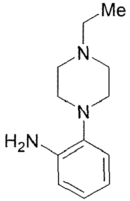 | 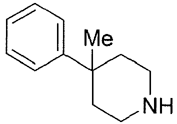 |
| | ¹H-NMR (CDCl₃) δ: 1.35 (3H, s), 1.53 (3H, t, J = 7.2 Hz), 2.13 (2H, t, J = 10.2 Hz), 2.47-2.43 (2H, m), 3.24-3.22 (2H, m), 3.40-3.78 (2H, m), 3.54-3.49 (4H, m), 3.69-3.65 (2H, m), 4.19 (4H, dd, J = 5.8, 1.0 Hz), 7.30-7.26 (2H, m), 7.41-7.34 (5H, m), 7.49 (1H, d, J = 7.6 Hz), 7.82 (1H, d, J = 7.6 Hz), 9.85 (1H, brs) | | |
| 5 | | Reference Example 2 | Commercial Product |
| | 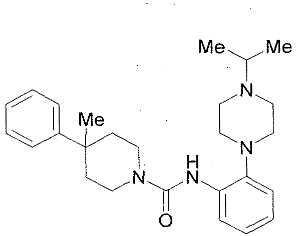 | 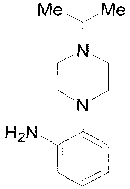 | 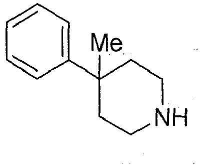 |
| | ¹H-NMR (CDCl₃) δ: 1.04 (6H, d, J = 6.1 Hz), 1.25 (3H, s), 1.70-1.76 (2H, m), 2.09-2.15 (2H, m), 2.55-2.68 (4H, m), 2.83 (4H, m), 3.41-3.59 (4H, m), 6.87 (1H, m), 7.03-7.09 (2H, m), 7.15-7.19 (3H, m), 7.29 (2H, m), 8.10 (1H, m), 8.17 (1H, brs) | | |
| 6 | | Reference Example 2 | Commercial Product |
| | 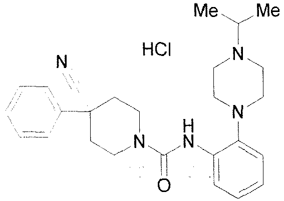 | 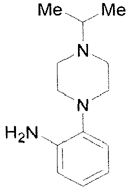 | 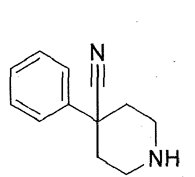 |
| | ¹H-NMR (CDCl₃) δ: 1.58 (6H, m), 2.26-2.20 (4H, m), 3.51-3.49 (2H, m), 3.64-3.63 (3H, m), 3.89-3.86 (2H, m), 4.59-4.45 (4H, m), 4.86-4.85 (2H, m), 7.41-7.37 (2H, m), 7.45 (2H, d, J = 8.4 Hz), 7.60-7.52 (4H, m), 7.64 (1H, d, J = 8.4 Hz), 9.14 (1H, brs) | | |
| 7 | | Reference Example 2 | Commercial Product |
| | 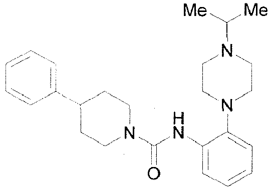 | 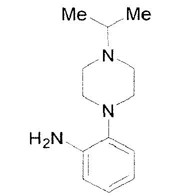 | 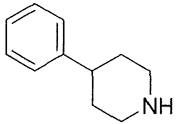 |
| | ¹H-NMR (CDCl₃) δ: 1.00 (6H, d, J = 6.1 Hz), 1.63-1.72 (2H, m), 1.87 (2H, m), 2.57-2.71 (6H, m), 2.84 (4H, m), 2.96 (2H, m), 4.23 (2H, m), 6.88 (1H, m), 7.05-7.10 (2H, m), 7.14-7.18 (3H, m), 7.24-7.28 (2H, m), 8.15 (1H, m), 8.27 (1H, brs) | | |
| 8 | | Reference Example 4 | Commercial Product |
| | 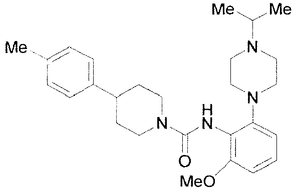 | 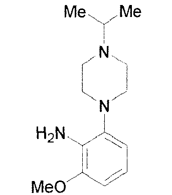 | 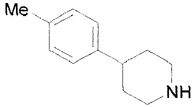 |
| | ¹H-NMR (CDCl₃) δ: 1.01 (6H, d, J = 6.7 Hz), 1.60-1.72 (2H, m), 1.83 (2H, m), 2.26 (3H, s), 2.57-2.69 (6H, m), 2.85-2.96 (6H, m), 3.79 (3H, s), 4.25 (2H, m), 6.12 (1H, s), 6.65 (2H, m), 7.00-7.08 (5H, m) | | |
| 9 | | Reference Example 5 | Commercial Product |
| | 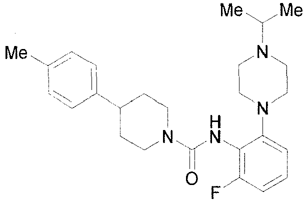 | 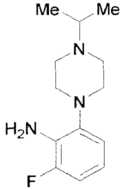 | 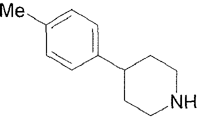 |
| | ¹H-NMR (CDCl₃) δ: 1.06 (6H, d, J = 6.7 Hz), 1.65-1.73 (2H, m), 1.91 (2H, m), 2.32 (3H, s), 2.57-2.69 (6H, m), 2.90 (4H, m), 3.01 (2H, m), 4.30 (2H, m), 6.35 (1H, s), 6.83-6.89 (2H, m), 7.01-7.14 (5H, m) | | |
| 10 | | Reference Example 3 | Commercial Product |
| | 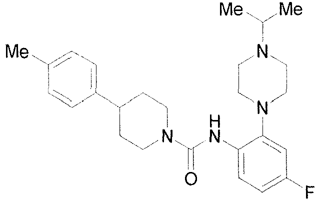 | 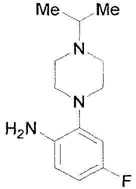 | 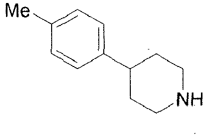 |
| | ¹H-NMR (CDCl₃) δ: 1.00 (6H, d, J = 6.7 Hz), 1.64-1.71 (2H, m), 1.85 (2H, m), 2.26 (3H, s), 2.58-2.69 (6H, m), 2.81 (4H, m), 2.95 (2H, m), 4.20 (2H, m), 6.74-6.83 (2H, m), 7.05 (4H, m), 7.89 (1H, s), 8.08 (1H, m) | | |
| 11 | | Reference Example 6 | Commercial Product |
| | 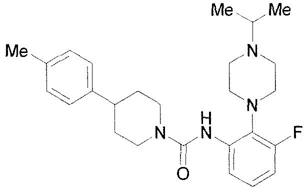 | 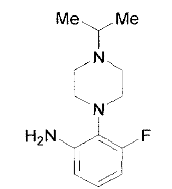 | 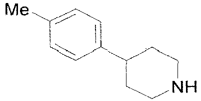 |
| | ¹H-NMR (CDCl₃) δ: 0.98 (6H, d, J = 6.7 Hz), 1.59-1.70 (2H, m), 1.85 (2H, m), 2.20-2.26 (5H, m), 2.60-2.75 (4H, m), 2.83-2.98 (4H, m), 3.33 (2H, m), 4.22 (2H, m), 6.55 (1H, m), 7.00-7.08 (5H, m), 7.97 (1H, m), 8.73 (1H, brs) | | |
| 12 | | Reference Example 2 | Commercial Product |
| | 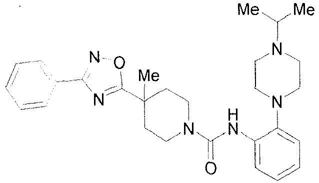 | 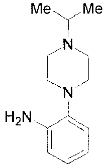 | 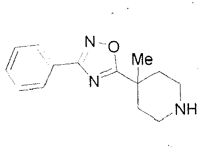 |
| | ¹H-NMR (CDCl₃) δ: 1.38 (6H, d, J = 6.8 Hz), 1.82 (2H, ddd, J = 14.0, 10.8, 3.2 Hz), 2.45 (2H, d, J = 13.6 Hz), 2.73-2.70 (4H, m), 2.80 (3H, s), 2.96-2.88 (4H, m), 3.27 (2H, ddd, J = 14.0, 10.8, 3.2 Hz), 3.96 (2H, dt, J = 14.0, 3.6 Hz), 6.96 (1H, dt, J = 8.0, 1.2 Hz), 7.24-7.11 (2H, m), 7.53-7.48 (3H, m), 8.11 (2H, dd, J = 7.2, 1.6 Hz), 8.17 (1H, dd, J = 7.2, 0.8 Hz), 8.27 (1H, brs) | | |
| 13 | | Reference Example 2 | Commercial Product |
| | 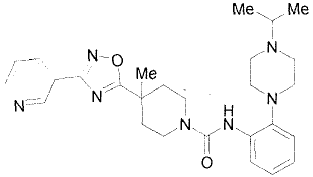 | 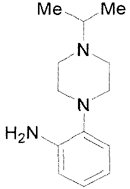 | 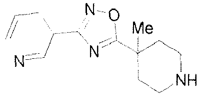 |
| | ¹H-NMR (CDCl₃) δ: 1.13 (6H, d, J = 6.4 Hz), 1.52 (3H, s), 1.84 (2H, ddd, J = 14.4, 10.0, 4.0 Hz), 2.46 (2H, dd, J = 11.2, 3.2 Hz), 2.77-2.66 (5H, m), 3.27 (2H, ddd, J = 14.4, 10.0, 4.0 Hz), 3.95 (2H, dt, J = 14.0, 4.0 Hz), 6.96 (1H, dt, J = 7.6, 1.2 Hz), 7.25-7.11 (3H, m), 7.44 (1H, ddd, J = 8.0, 4.8, 0.8 Hz), 8.17 (1H, dd, J = 8.0, 1.2 Hz), 8.28 (1H, brs), 8.37 (1H, dt, J = 8.0, 1.6 Hz), 8.76 (1H, dd, J = 4.8, 1.6 Hz), 9.33 (1H, dd, J = 2.0, 0.8 Hz) | | |
| 14 | | Reference Example 2 | Reference Example 60 |
| | 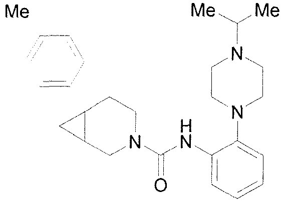 | 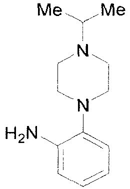 | 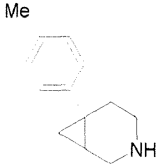 |
| | ¹H-NMR (CDCl₃) δ: 0.99 (1H, dd, J = 9.8, 5.2 Hz), 1.07 (1H, dd, J = 9.8, 5.2 Hz), 1.14 (6H, brs), 1.55-1.49 (2H, m), 2.21-2.17 (1H, m), 2.34-2.22 (1H, m), 2.36 (3H, s), 2.78-2.68 (4H, m), 2.95-2.94 (4H, m), 3.44-3.37 (1H, m), 3.66-3.62 (1H, m), 3.91 (1H, dd, J = 12.8, 2.4 Hz), 4.01 (1H, dd, J = 12.8, 4.8 Hz), 6.98 (1H, t, J = 8.0 Hz), 7.27-7.14 (6H, m), 8.25 (1H, dd, J = 8.0, 1.2 Hz) | | |
| 15 | | Reference Example 4 | Commercial Product |
| | 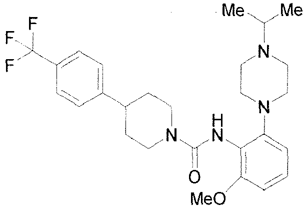 | 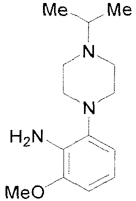 | 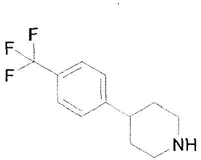 |
| | ¹H-NMR (CDCl₃) δ: 1.05 (6H, d, J = 6.7 Hz), 1.70-1.78 (2H, m), 1.90 (2H, m), 2.60-2.69 (5H, m), 2.75-2.95 (5H, m), 3.00 (2H, m), 3.85 (3H, s), 4.34 (2H, m), 6.18 (1H, s), 6.71 (2H, m), 7.08 (1H, m), 7.32 (2H, m), 7.56 (2H, m) | | |
| 16 | | Reference Example 4 | Commercial Product |
| | 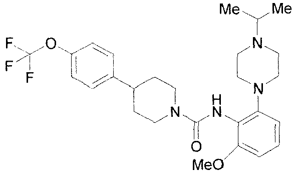 | 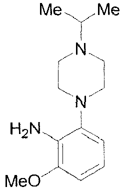 | 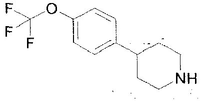 |
| | ¹H-NMR (CDCl₃) δ: 1.05 (6H, d, J = 6.1 Hz), 1.65-1.75 (2H, m), 1.90 (2H, m), 2.60-2.79 (6H, m), 2.90 (4H, m), 2.99 (2H, m), 3.85 (3H, s), 4.32 (2H, m), 6.18 (1H, s), 6.71 (2H, m), 7.08 (1H, m), 7.14 (2H, m), 7.22 (2H, m) | | |
| 17 | | Reference Example 7 | Commercial Product |
| | 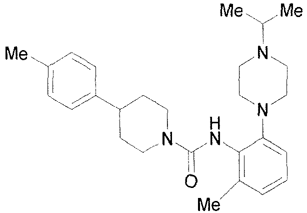 | 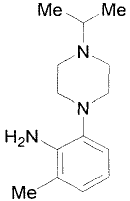 | 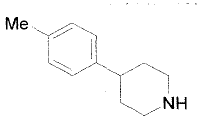 |
| | ¹H-NMR (CDCl₃) δ: 1.01 (6H, d, J = 6.7 Hz), 1.60-1.70 (2H, m), 1.85 (2H, m), 2.20 (3H, s), 2.26 (3H, s), 2.55-2.66 (6H, m), 2.82 (4H, m), 2.94 (2H, m), 4.25 (2H, m), 6.68 (1H, s), 6.85-6.99 (3H, m), 7.01-7.08 (4H, m) | | |
| 18 | | Reference Example 2 | Reference Example 61 |
| | 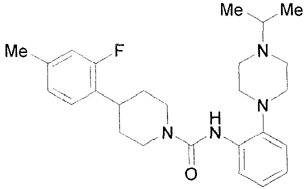 | 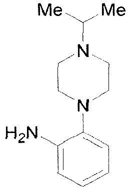 | 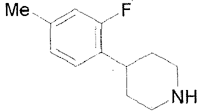 |
| | ¹H-NMR (CDCl₃) δ: 1.05 (6H, d, J = 6.7 Hz), 1.68-1.77 (2H, m), 1.87 (2H, m), 2.30 (3H, s), 2.56-2.69 (5H, m), 2.89 (4H, m), 3.02 (3H, m), 4.27 (2H, m), 6.82-6.95 (3H, m), 7.04-7.15 (3H, m), 8.19 (1H, m), 8.30 (1H, brs) | | |
| 19 | | Reference Example 2 | Reference Example 65 |
| | 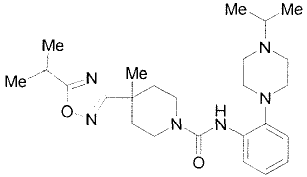 | 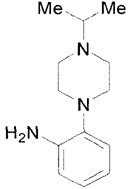 | 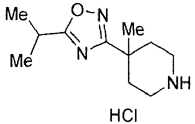 |
| | ¹H-NMR (CDCl₃) δ: 1.08 (6H, d, J = 6.7 Hz), 1.75 (2H, ddd, J = 25.4, 12.5, 4.0 Hz), 1.88-1.95 (2H, m), 2.33 (3H, s), 2.61-2.78 (6H, m), 2.87-2.94 (4H, m), 2.98-3.07 (2H, m), 3.87 (3H, s), 4.30-4.38 (2H, m), 7.05 (1H, dd, J = 7.9, 7.9 Hz), 7.14 (4H, s), 7.28 (1H, dd, J = 7.9, 1.5 Hz), 7.53 (1H, dd, J = 7.9, 1.5 Hz), 7.97 (1H, s). | | |
| 20 | | Reference Example 5 | Reference Example 65 |
| | 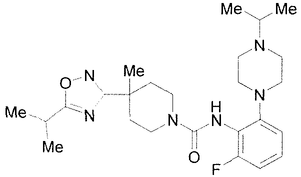 | 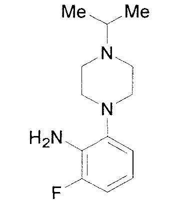 | 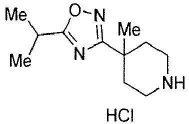 |
| | ¹H-NMR (CDCl₃) δ: 0.88-0.99 (2H, m), 0.99-1.07 (2H, m), 1.09 (6H, d, J = 6.7 Hz), 1.61-1.90 (6H, m), 1.96-2.07 (3H, m), 2.18-2.31 (2H, m), 2.68-2.85 (2H, m), 2.90-3.15 (5H, m), 4.09-4.16 (2H, m), 5.79 (1H, s), 5.85 (1H, s), 6.94 (1H, ddd, J = 9.6, 7.8, 1.4 Hz), 7.10-7.21 (2H, m). | | |
| 21 | | Reference Example 4 | Reference Example 65 |
| | 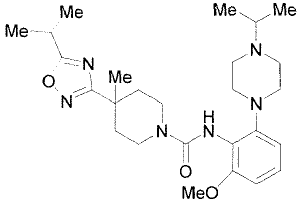 | 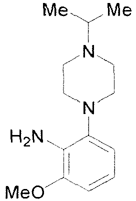 | 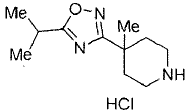 |
| | ¹H-NMR (CDCl₃) δ: 0.91-0.99 (2H, m), 1.00-1.07 (2H, m), 1.09 (6H, d, J = 6.1 Hz), 1.75 (2H, ddd, J = 25.1, 11.6, 4.3 Hz), 1.96-2.06 (3H, m), 2.61-2.79 (5H, m), 2.88-3.00 (5H, m), 3.09 (2H, ddd, J = 13.9, 11.6, 2.3 Hz), 4.16-4.26 (2H, m), 5.78 (1H, s), 6.35 (1H, s), 6.83-6.91 (2H, m), 7.05 (1H, ddd, J = 8.3, 8.3, 5.9 Hz). | | |
| 22 | | Reference Example 5 | Reference Example 63 |
| | 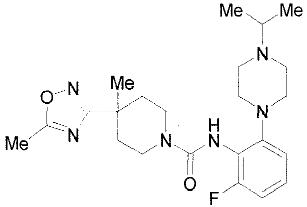 | 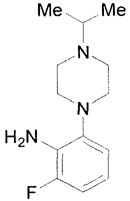 | 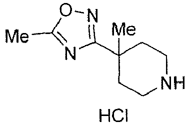 |
| | ¹H-NMR (CDCl₃) δ: 0.93-1.00 (2H, m), 1.00-1.08 (2H, m), 1.24 (3H, s), 1.32 (6H, d, J = 6.7 Hz), 1.51 (2H, ddd, J = 14.1, 10.4, 3.7 Hz), 1.96-2.05 (3H, m), 3.04-3.17 (3H, m), 3.60-3.68 (2H, m), 5.76 (1H, s), 5.78 (1H, s), 7.07-7.17 (2H, m), 7.21-7.26 (2H, m), 7.69 (1H, dd, J = 7.9, 2.1 Hz), 8.55 (1H, d, J = 2.1 Hz). | | |
| 23 | | Reference Example 4 | Reference Example 63 |
| | 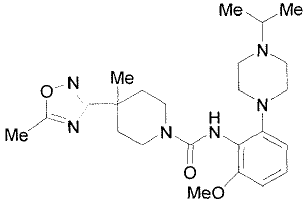 | 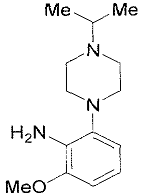 | 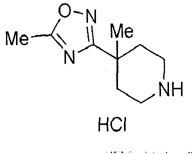 |
| | ¹H-NMR (CDCl₃) δ: 0.92-1.00 (2H, m), 1.00-1.09 (2H, m), 1.13 (3H, d, J = 6.7 Hz), 1.29 (3H, s), 1.61-1.73 (2H, m), 1.97-2.06 (1H, m), 2.06-2.18 (2H, m), 2.37-2.48 (2H, m), 2.72-2.83 (2H, m), 2.93-3.07 (1H, m), 3.20-3.35 (4H, m), 3.69-3.82 (2H, m), 4.33-4.62 (2H, m), 5.67 (1H, s), 5.79 (1H, s), 6.05 (1H, s), 7.04-7.11 (2H, m), 7.29 (1H, dd, J = 6.7, 2.4 Hz). | | |
| 24 | | Reference Example 5 | Reference Example 68 |
| | 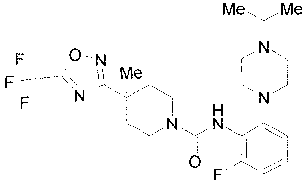 | 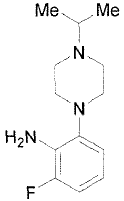 | 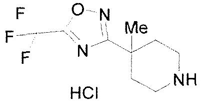 |
| | ¹H-NMR (CDCl₃) δ: 0.94-1.00 (2H, m), 1.00-1.08 (2H, m), 1.25 (3H, s), 1.51 (2H, ddd, J = 13.9, 10.2, 3.8 Hz), 1.96-2.05 (3H, m), 2.56 (3H, s), 3.13 (2H, ddd, J = 13.9, 10.9, 2.9 Hz), 3.61-3.69 (2H, m), 5.77 (1H, s), 5.84 (1H, s), 7.06-7.10 (1H, m), 7.13 (1H, ddd, J = 9.6, 8.4, 1.4 Hz), 7.17-7.27 (2H, m), 7.64 (1H, dd, J = 7.9, 2.1 Hz), 8.48 (1H, d, J = 2.1 Hz). | | |
| 25 | | Reference Example 4 | Reference Example 68 |
| | 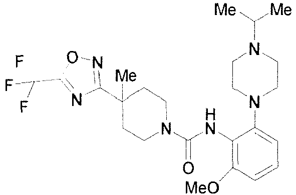 | 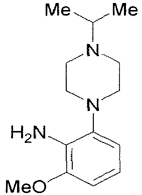 | 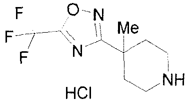 |
| | ¹H-NMR (CDCl₃) δ: 0.92-0.99 (2H, m), 0.99-1.07 (2H, m), 1.23 (3H, s), 1.30 (6H, d, J = 6.7 Hz), 1.47 (2H, ddd, J = 14.0, 10.4, 3.7 Hz), 1.94-2.04 (3H, m), 3.00-3.16 (3H, m), 3.59-3.68 (2H, m), 5.75 (1H, s), 6.02 (1H, s), 7.17-7.24 (3H, m), 7.43 (1H, dd, J = 6.7, 3.0 Hz), 7.68 (1H, dd, J = 7.9, 2.4 Hz), 8.52 (1H, d, J = 2.4 Hz). | | |
| 26 | | Reference Example 5 | Commercial Product |
| | 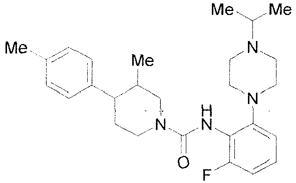 | 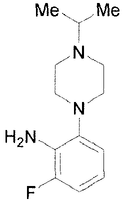 | 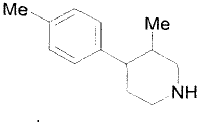 |
| | ¹H-NMR (CDCl₃) δ: 0.93-1.00 (2H, m), 1.00-1.09 (2H, m), 1.24 (3H, s), 1.50 (2H, ddd, J = 14.1, 10.7, 3.7 Hz), 1.94-2.06 (3H, m), 2.36 (3H, s), 3.12 (2H, ddd, J = 13.8, 10.7, 2.8 Hz), 3.60-3.69 (2H, m), 5.76 (1H, s), 5.78 (1H, s), 7.06-7.11 (1H, m), 7.11-7.18 (1H, m), 7.21-7.29 (1H, m), 7.54 (1H, s), 8.40 (1H, d, J = 1.8 Hz), 8.44 (1H, d, J = 1.8 Hz). | | |
| 27 | | Reference Example 5 | Commercial Product |
| | 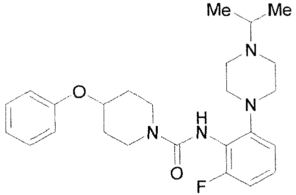 | 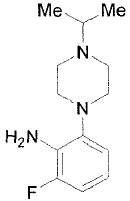 | 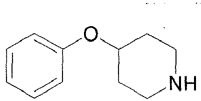 |
| | ¹H-NMR (CDCl₃) δ: 1.04 (3H, t, J = 7.0 Hz), 1.18-1.24 (4H, m), 1.33 (3H, s), 1.66 (2H, ddd, J = 14.1, 10.4, 3.7 Hz), 1.80 (1H, d, J = 9.8 Hz), 1.88 (1H, d, J = 9.8 Hz), 2.15-2.21 (1H, m), 2.22-2.30 (2H, m), 2.44-2.63 (2H, m), 2.71 (1H, d, J = 9.8 Hz), 2.92 (1H, dd, J = 9.8, 2.4 Hz), 3.13-3.23 (2H, m), 3.31 (1H, dd, J = 9.5, 2.4 Hz), 3.40 (1H, d, J = 9.5 Hz), 3.48-3.52 (1H, m), 3.74-3.84 (2H, m), 4.01-4.06 (1H, m), 5.66 (1H, s), 6.49 (1H, d, J = 8.6 Hz), 6.56 (1H, dd, J = 8.6, 8.6 Hz), 6.96-7.04 (1H, m). | | |
| 28 | | Reference Example 14 | Commercial Product |
| | 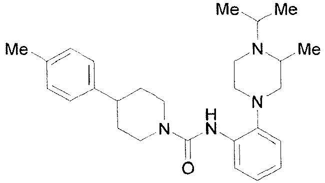 | 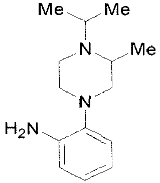 | 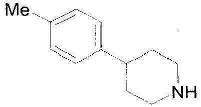 |
| | ¹H-NMR (CDCl₃) δ: 1.05 (6H, dd, J = 6.1, 1.2 Hz), 1.66-1.77 (2H, m), 1.82-1.95 (4H, m), 2.33 (3H, s), 2.54-2.76 (3H, m), 2.95-3.06 (2H, m), 3.13 (1H, dd, J = 9.2, 2.4 Hz), 3.33 (1H, dd, J = 9.8, 2.4 Hz), 3.46 (1H, d, J = 9.8 Hz), 3.70-3.74 (1H, m), 4.05-4.09 (1H, m), 4.18-4.28 (2H, m), 5.70 (1H, s), 6.51 (1H, d, J = 8.3 Hz), 6.60 (1H, ddd, J = 9.5, 8.3, 1.2 Hz), 6.99-7.06 (1H, m), 7.11 (2H, d, J = 8.6 Hz), 7.14 (2H, d, J = 8.6 Hz). | | |
| 29 | | Reference Example 5 | Commercial Product |
| | 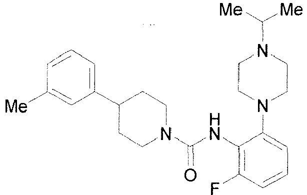 | 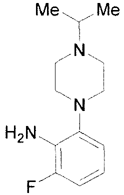 | 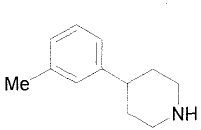 |
| | ¹H-NMR (CDCl₃) δ: 1.03 (6H, t, J = 6.1 Hz), 1.18-1.25 (4H, m), 1.33 (3H, s), 1.66-1.72 (2H, m), 1.83 (1H, d, J = 9.2 Hz), 1.91 (1H, d, J = 9.2 Hz), 2.14-2.22 (1H, m), 2.24-2.31 (2H, m), 2.53-2.60 (1H, m), 2.62 (1H, dd, J = 9.8, 1.2 Hz), 3.11 (1H, dd, J = 9.8, 2.4 Hz), 3.14-3.25 (2H, m), 3.29 (1H, dd, J = 9.8, 2.1 Hz), 3.41 (1H, d, J = 9.8 Hz), 3.67-3.72 (1H, m), 3.75-3.85 (2H, m), 4.02-4.06 (1H, m), 5.63 (1H, s), 6.49 (1H, d, J = 9.0 Hz), 6.57 (1H, ddd, J = 9.0, 7.9, 1.2 Hz), 6.98-7.05 (1H, m). | | |
| 30 | | Reference Example 15 | Commercial Product |
| | 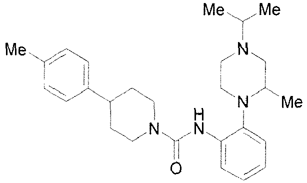 | 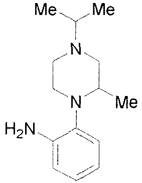 | 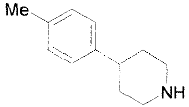 |
| | ¹H-NMR (CDCl₃) δ: 1.13 (6H, brs), 1.48-1.68 (2H, m), 1.76 (2H, ddd, J = 25.4, 12.8, 4.0 Hz), 1.81-2.04 (6H, m), 2.34 (3H, s), 2.57-2.77 (3H, m), 2.98-3.23 (3H, m), 3.53-3.67 (2H, m), 4.28-4.34 (2H, m), 6.38 (1H, s), 6.88 (1H, dd, J = 8.6, 8.6 Hz), 6.94 (1H, d, J = 8.6 Hz), 7.00-7.08 (1H, m), 7.12 (2H, d, J = 8.0 Hz), 7.15 (2H, d, J = 8.0 Hz). | | |
| 31 | | Reference Example 20 | Commercial Product |
| | 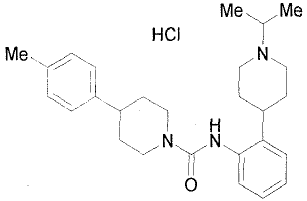 | 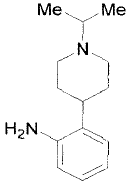 | 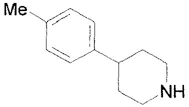 |
| | ¹H-NMR (CDCl₃) δ: 1.08 (6H, d, J = 6.1 Hz), 1.19-1.25 (4H, m), 1.34 (3H, s), 1.66-1.81 (4H, m), 1.89-1.97 (2H, m), 2.15-2.24 (1H, m), 2.26-2.33 (2H, m), 2.55-2.65 (2H, m), 2.67 (2H, dd, J = 11.3, 2.8 Hz), 3.04 (2H, d, J = 11.3 Hz), 3.23 (2H, ddd, J = 13.6, 10.9, 2.6 Hz), 3.47-3.57 (1H, m), 3.85-3.93 (2H, m), 6.34 (1H, s), 6.84 (1H, dd, J = 8.0, 1.5 Hz), 6.89 (1H, t, J = 8.0 Hz), 6.99-7.06 (1H, m). | | |
| 32 | | Reference Example 5 | Reference Example 88 |
| | 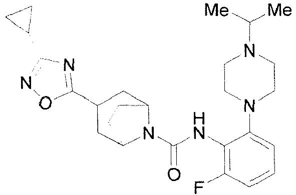 | 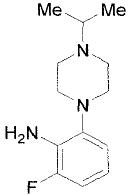 | 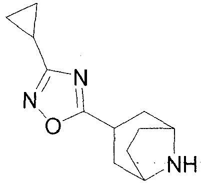 |
| | ¹H-NMR (CDCl₃) δ: 0.96-1.05 (4H, m), 1.07 (6H, d, J = 6.8 Hz), 1.81-1.90 (2H, m), 1.95-2.10 (3H, m), 2.12-2.25 (4H, m), 2.56-2.76 (5H, m), 2.88-3.00 (4H, m), 3.42-3.55 (1H, m), 4.44-4.55 (2H, m), 6.31 (1H, bs), 6.83-6.93 (2H, m), 7.03-7.12 (1H, m). | | |
| 33 | | Reference Example 12 | Commercial Product |
| | 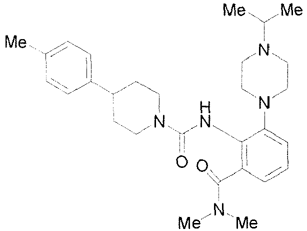 | 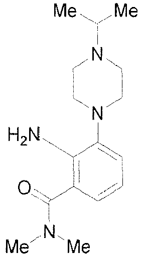 | 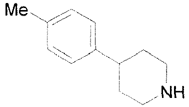 |
| | ¹H-NMR (CDCl₃) δ: 1.07 (6H, d, J = 6.7 Hz), 1.72-1.88 (4H, m), 2.33 (3H, s), 2.63-2.73 (6H, m), 2.91-3.02 (6H, m), 3.05 (3H, s), 3.09 (3H, s), 4.24 (2H, m), 6.99-7.05 (2H, m), 7.11-7.17 (5H, m), 7.48 (1H, brs) | | |
| 34 | | Reference Example 8 | Commercial Product |
| | 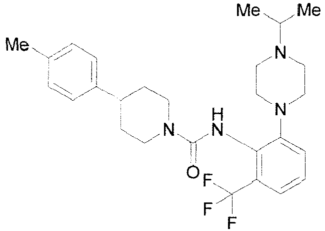 | 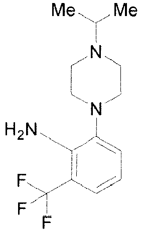 | 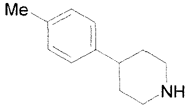 |
| | ¹H-NMR (CDCl₃) δ: 1.09 (6H, d, J = 6.7 Hz), 1.68-1.75 (2H, m), 1.90 (2H, m), 2.34 (3H, s), 2.65-2.75 (6H, m), 2.91-3.04 (6H, m), 4.30 (2H, m), 6.55 (1H, s), 7.11-7.16 (4H, m), 7.21-7.31 (2H, m), 7.40 (1H, m) | | |
| 35 | | Reference Example 9 | Commercial Product |
| | 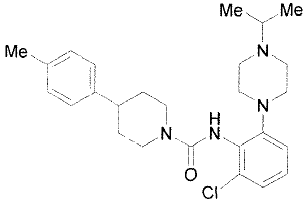 | 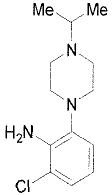 | 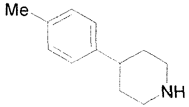 |
| | ¹H-NMR (CDCl₃) δ: 1.09 (6H, d, J = 6.7 Hz), 1.70-1.80 (2H, m), 1.92 (2H, m), 2.34 (3H, s), 2.63-2.77 (6H, m), 2.92 (4H, m), 3.04 (2H, m), 4.34 (2H, m), 6.43 (1H, s), 6.98 (1H, m), 7.06 (1H, m), 7.11-7.18 (5H, m) | | |
| 36 | | Reference Example 5 | Commercial Product |
| | 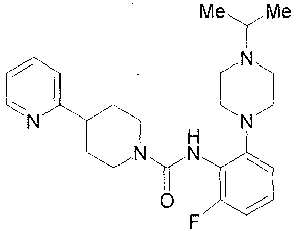 | 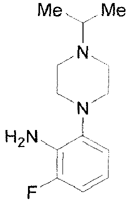 | 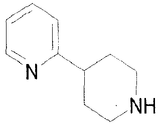 |
| | ¹H-NMR (CDCl₃) δ: 1.07 (6H, d, J = 6.7 Hz), 1.79-1.90 (2H, m), 2.03 (2H, m), 2.63-2.72 (5H, m), 2.88-3.01 (5H, m), 3.07 (2H, m), 4.32 (2H, m), 6.38 (1H, s), 6.84-6.89 (2H, m), 7.01-7.07 (1H, m), 7.12-7.18 (2H, m), 7.64 (1H, m), 8.53 (1H, m) | | |
| 37 | | Reference Example 5 | Commercial Product |
| | 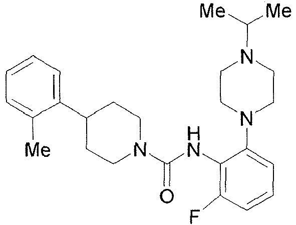 | 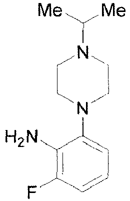 | 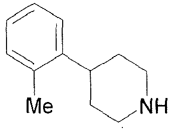 |
| | ¹H-NMR (CDCl₃) δ: 1.09 (6H, d, J = 6.1 Hz), 1.70-1.81 (2H, m), 1.86 (2H, m), 2.39 (3H, s), 2.65-2.75 (5H, m), 2.90-3.08 (7H, m), 4.35 (2H, m), 6.38 (1H, s), 6.86-6.91 (2H, m), 7.04-7.09 (1H, m), 7.11-7.21 (4H, m) | | |
| 38 | | Reference Example 16 | Commercial Product |
| | 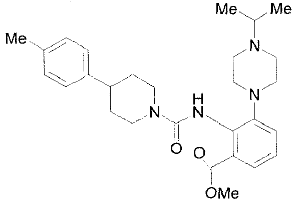 | 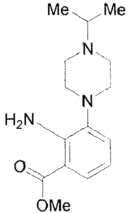 | 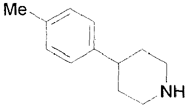 |
| | ¹H-NMR (CDCl₃) δ: 1.08 (6H, d, J = 6.7 Hz), 1.75 (2H, ddd, J = 25.4, 12.5, 4.0 Hz), 1.88-1.95 (2H, m), 2.33 (3H, s), 2.61-2.78 (6H, m), 2.87-2.94 (4H, m), 2.98-3.07 (2H, m), 3.87 (3H, s), 4.30-4.38 (2H, m), 7.05 (1H, dd, J = 7.9, 7.9 Hz), 7.14 (4H, s), 7.28 (1H, dd, J = 7.9, 1.5 Hz), 7.53 (1H, dd, J = 7.9, 1.5 Hz), 7.97 (1H, s). | | |
| 39 | | Reference Example 21 | Commercial Product |
| | 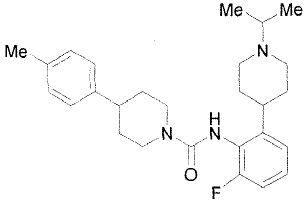 | 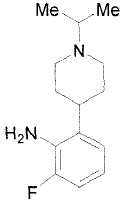 | 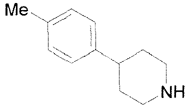 |
| | ¹H-NMR (CDCl₃) δ: 1.08 (6H, d, J = 6.7 Hz), 1.69-1.81 (4H, m), 1.89 (4H, m), 2.25 (2H, m), 2.34 (3H, s), 2.69-2.78 (3H, m), 3.04 (4H, m), 4.22 (2H, m), 5.84 (1H, s), 6.93-6.97 (1H, m), 7.10-7.21 (6H, m) | | |
| 40 | | Reference Example 5 | Commercial Product |
| | 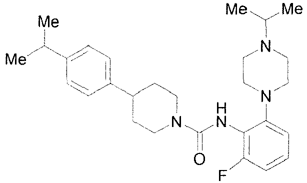 | 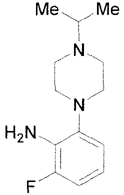 | 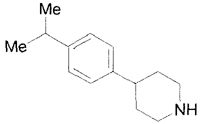 |
| | ¹H-NMR (CDCl₃) δ: 1.15 (6H, m), 1.25 (6H, d, J = 7.3 Hz), 1.56 (2H, m), 1.69-1.79 (2H, m), 1.93 (2H, m), 2.69-2.77 (4H, m), 2.90 (3H, m), 3.03 (4H, m), 4.30 (2H, m), 6.30 (1H, brs), 6.90 (2H, m), 7.04-7.11 (1H, m), 7.14-7.21 (4H, m) | | |
| 41 | | Reference Example 5 | Commercial Product |
| | 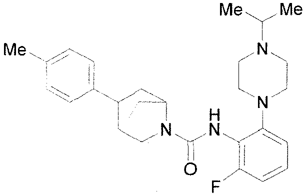 | 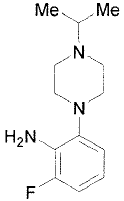 | 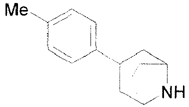 |
| | ¹H-NMR (CDCl₃) δ: 1.07 (6H, m), 1.60 (3H, m), 1.73 (2H, m), 2.15 (2H, m), 2.31 (3H, m), 2.56-2.77 (7H, m), 3.00 (4H, m), 4.44 (2H, m), 6.30 (1H, s), 6.87-6.92 (2H, m), 7.06-7.15 (5H, m) | | |
| 42 | | Reference Example 22 | Commercial Product |
| | 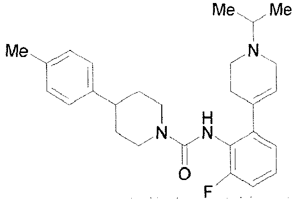 | 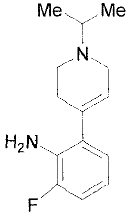 | 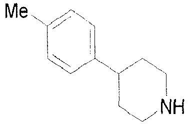 |
| | ¹H-NMR (CDCl₃) δ: 1.10 (6H, d, J = 6.1 Hz), 1.66-1.76 (2H, m), 1.88 (2H, m), 2.33 (3H, s), 2.46 (2H, m), 2.67-2.82 (4H, m), 2.99 (2H, m), 3.22 (2H, m), 4.22 (2H, m), 5.75 (1H, m), 5.96 (1H, s), 6.97-7.03 (2H, m), 7.07-7.15 (5H, m) | | |
| 43 | | Reference Example 21 | Reference Example 62 |
| | 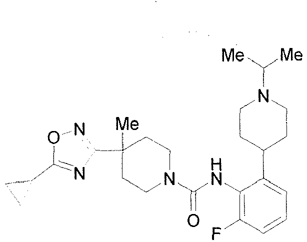 | 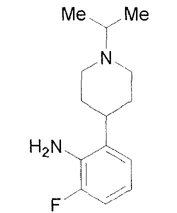 | 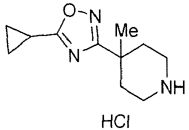 |
| | ¹H-NMR (CDCl₃) δ: 1.07 (6H, d, J = 6.8 Hz), 1.18-1.27 (4H, m), 1.35 (3H, s), 1.53-1.87 (6H, m), 2.15-2.25 (3H, m), 2.30 (2H, d, J = 14.0 Hz), 2.62-2.80 (2H, m), 2.93-3.06 (2H, m), 3.14-3.32 (2H, m), 3.76-3.88 (2H, m), 5.73-5.85 (1H, m), 6.88-6.99 (1H, m), 7.08-7.21 (2H, m). | | |
| 44 | | Reference Example 22 | Reference Example 62 |
| | 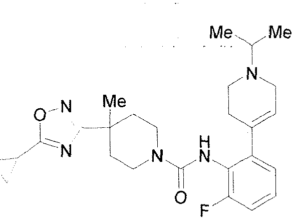 | 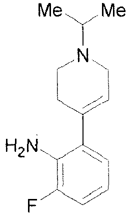 | 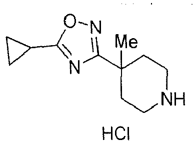 |
| | ¹H-NMR (CDCl₃) δ: 1.07-1.15 (6H, m), 1.16-1.25 (4H, m), 1.36 (3H, s), 1.62-1.74 (2H, m), 2.14-2.23 (1H, m), 2.23-2.31 (2H, m), 2.38-2.47 (2H, m), 2.67-2.74 (2H, m), 2.76-2.87 (1H, m), 3.12-3.26 (4H, m), 3.76-3.85 (2H, m), 5.69-5.75 (1H, m), 5.89-6.06 (1H, m), 6.93-7.04 (2H, m), 7.05-7.13 (1H, m). | | |
| 45 | | Reference Example 5 | Reference Example 62 |
| | 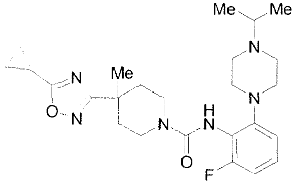 | 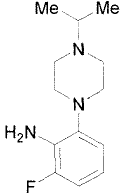 | 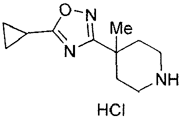 |
| | ¹H-NMR (CDCl₃) δ: 1.08 (6H, d, J = 6.8 Hz), 1.17-1.29 (4H, m), 1.34 (3H, s), 1.70 (2H, ddd, J = 14.0, 10.8, 4.0 Hz), 2.15-2.24 (1H, m), 2.29 (2H, d, J = 13.2 Hz), 2.55-2.78 (5H, m), 2.84-2.96 (4H, m), 3.23 (2H, ddd, J = 13.2, 10.8, 2.8 Hz), 3.88 (2H, dt, J = 14.0, 4.0 Hz), 6.32 (1H, bs), 6.81-6.91 (2H, m), 7.00-7.09 (1H, m). | | |
| 46 | | Reference Example 5 | Commercial Product |
| | 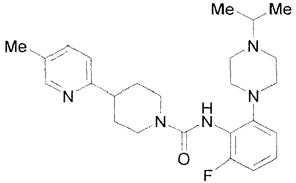 | 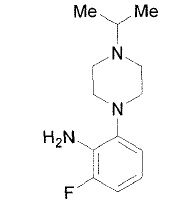 | 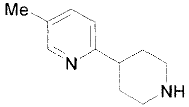 |
| | ¹H-NMR (CDCl₃) δ: 1.09 (6H, d, J = 6.7 Hz), 1.77-1.88 (2H, m), 2.02 (2H, m), 2.31 (3H, s), 2.63-2.78 (5H, m), 2.88-2.96 (5H, m), 3.06 (2H, m), 4.32 (2H, m), 6.37 (1H, s), 6.85-6.90 (2H, m), 7.02-7.08 (2H, m), 7.45 (1H, m), 8.37 (1H, m) | | |
| 47 | | Reference Example 33 | Commercial Product |
| | 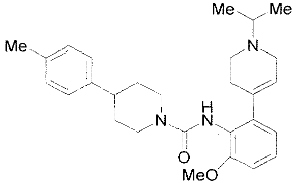 | 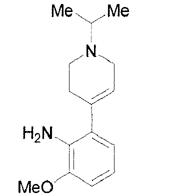 | 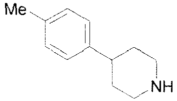 |
| | ¹H-NMR (CDCl₃) δ: 1.06 (6H, d, J = 6.7 Hz), 1.58-1.68 (2H, m), 1.80 (2H, m), 2.26 (3H, s), 2.47 (2H, m), 2.60-2.80 (4H, m), 2.89 (2H, m), 3.18 (2H, m), 3.77 (3H, s), 4.16 (2H, m), 5.64 (1H, m), 5.95 (1H, s), 6.72-6.78 (2H, m), 7.00-7.09 (5H, m) | | |
| 48 | | Reference Example 32 | Commercial Product |
| | 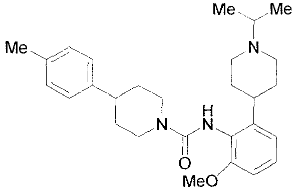 | 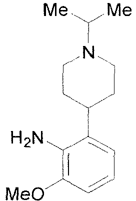 | 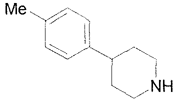 |
| | ¹H-NMR (CDCl₃) δ: 1.09 (6H, d, J = 6.7 Hz), 1.69-1.92 (8H, m), 2.26 (2H, m), 2.32 (3H, s), 2.68-2.82 (3H, m), 3.01 (4H, m), 3.79 (3H, s), 4.22 (2H, m), 5.94 (1H, s), 6.72 (1H, m), 6.97 (1H, m), 7.12-7.17 (5H, m) | | |
| 49 | | Reference Example 21 | Reference Example 92 |
| | 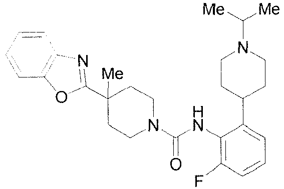 | 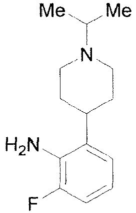 | 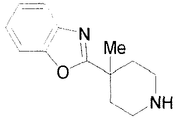 |
| | ¹H-NMR (CDCl₃) δ: 1.03 (6H, d, J = 6.7 Hz), 1.42 (3H, s), 1.72-1.79 (6H, m), 2.20 (2H, m), 2.43 (2H, m), 2.68 (2H, m), 2.97 (2H, m), 3.20 (2H, m), 3.83 (2H, m), 5.78 (1H, s), 6.84-6.89 (1H, m), 7.05-7.12 (2H, m), 7.25-7.30 (2H, m), 7.45-7.48 (1H, m), 7.64-7.66 (1H, m) | | |
| 50 | | Reference Example 22 | Reference Example 92 |
| | 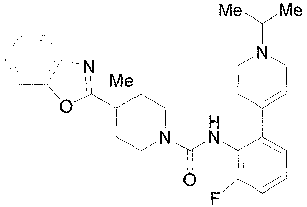 | 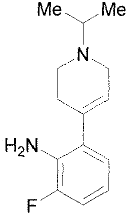 | 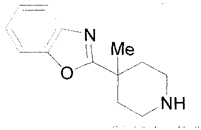 |
| | ¹H-NMR (CDCl₃) δ: 1.07 (6H, d, J = 6.1 Hz), 1.41 (3H, s), 1.70-1.77 (3H, m), 2.40 (3H, m), 2.70 (3H, m), 3.19 (4H, m), 3.85 (2H, m), 5.66 (1H, m), 6.08 (1H, brs), 6.86-7.04 (3H, m), 7.23-7.28 (2H, m), 7.43-7.46 (1H, m), 7.62-7.65 (1H, m) | | |
| 51 | | Reference Example 21 | Reference Example 89 |
| | 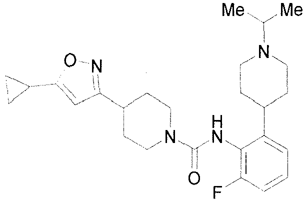 | 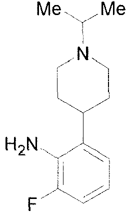 | 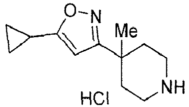 |
| | ¹H-NMR (CDCl₃) δ: 0.88-0.99 (2H, m), 0.99-1.07 (2H, m), 1.09 (6H, d, J = 6.7 Hz), 1.61-1.90 (6H, m), 1.96-2.07 (3H, m), 2.18-2.31 (2H, m), 2.68-2.85 (2H, m), 2.90-3.15 (5H, m), 4.09-4.16 (2H, m), 5.79 (1H, s), 5.85 (1H, s), 6.94 (1H, ddd, J = 9.6, 7.8, 1.4 Hz), 7.10-7.21 (2H, m). | | |
| 52 | | Reference Example 5 | Reference Example 89 |
| | 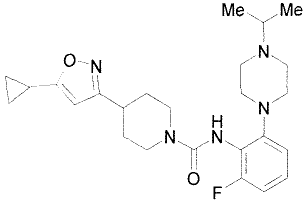 | 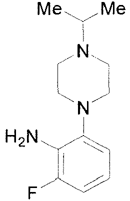 | 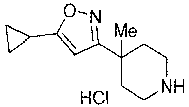 |
| | ¹H-NMR (CDCl₃) δ: 0.91-0.99 (2H, m), 1.00-1.07 (2H, m), 1.09 (6H, d, J = 6.1 Hz), 1.75 (2H, ddd, J = 25.1, 11.6, 4.3 Hz), 1.96-2.06 (3H, m), 2.61-2.79 (5H, m), 2.88-3.00 (5H, m), 3.09 (2H, ddd, J = 13.9, 11.6, 2.3 Hz), 4.16-4.26 (2H, m), 5.78 (1H, s), 6.35 (1H, s), 6.83-6.91 (2H, m), 7.05 (1H, ddd, J = 8.3, 8.3, 5.9 Hz). | | |
| 53 | | Reference Example 21 | Reference Example 89 |
| | 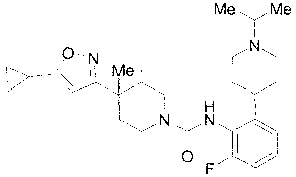 | 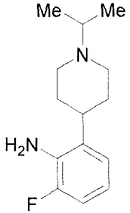 | 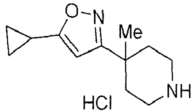 |
| | ¹H-NMR (CDCl₃) δ: 0.94-1.01 (2H, m), 1.01-1.08 (2H, m), 1.09 (6H, d, J = 6.7 Hz), 1.31 (3H, s), 1.70 (2H, ddd, J = 14.0, 10.4, 4.0 Hz), 1.77-1.89 (3H, m), 1.97-2.06 (1H, m), 2.10-2.19 (2H, m), 2.19-2.31 (2H, m), 2.66-2.84 (2H, m), 2.97-3.06 (2H, m), 3.28 (2H, ddd, J = 13.8, 10.4, 2.8 Hz), 3.72-3.82 (2H, m), 5.80 (1H, s), 5.80 (1H, s), 6.93 (1H, ddd, J = 9.5, 7.6, 1.8 Hz), 7.09-7.20 (2H, m). | | |
| 54 | | Reference Example 5 | Reference Example 89 |
| | 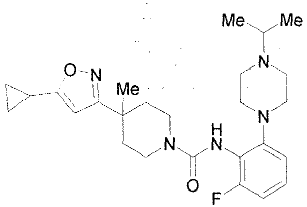 | 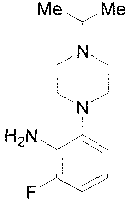 | 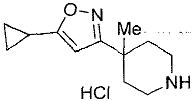 |
| | ¹H-NMR (CDCl₃) δ: 0.94-1.01 (2H, m), 1.01-1.09 (2H, m), 1.10 (6H, d, J = 6.4 Hz), 1.30 (3H, s), 1.70 (2H, ddd, J = 14.0, 10.4, 5.4 Hz), 1.98-2.06 (1H, m), 2.10-2.18 (2H, m), 2.53-2.82 (5H, m), 2.82-3.00 (4H, m), 3.31 (2H, ddd, J = 13.8, 10.7, 2.8 Hz), 3.81-3.89 (2H, m), 5.80 (1H, s), 6.30 (1H, s), 6.83-6.90 (2H, m), 7.01-7.08 (1H, m). | | |
| 55 | | Reference Example 22 | Reference Example 89 |
| | 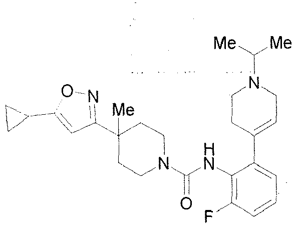 | 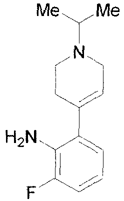 | 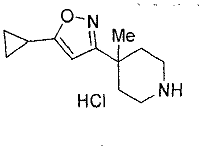 |
| | ¹H-NMR (CDCl₃) δ: 0.93-1.06 (4H, m), 1.07 (6H, d, J = 6.7 Hz), 1.27 (3H, s), 1.62-1.69 (2H, m), 2.00 (1H, m), 2.09 (2H, m), 2.40 (2H, m), 2.70 (2H, m), 2.77 (1H, - m), 3.17-3.26 (4H, m), 3.74 (2H, m), 5.70 (1H, m), 5.77 (1H, s), 5.86 (1H, brs), 6.93-6.99 (2H, m), 7.04-7.09 (1H, m) | | |
| 56 | | Reference Example 35 | Reference Example 62 |
| | 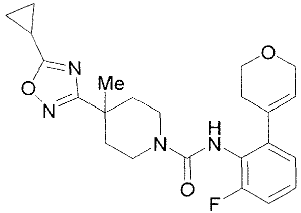 | 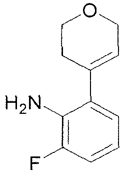 | 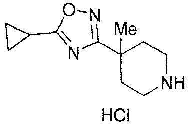 |
| | ¹H-NMR (CDCl₃) δ: 1.17-1.29 (4H, m), 1.34 (3H, s), 1.61-1.73 (2H, m), 2.15-2.24 (1H, m), 2.24-2.33 (2H, m), 2.35-2.42 (2H, m), 3.14-3.24 (2H, m), 3.74-3.84 (2H, m), 3.85-3.91 (2H, m), 4.23-4.30 (2H, m), 5.73-5.78 (1H, m), 5.83 (1H, bs), 6.93-6.98 (1H, m), 6.98-7.06 (1H, m), 7.09-7.16 (1H, m) . | | |
| 57 | | Reference Example 5 | Reference Example 62 |
| | 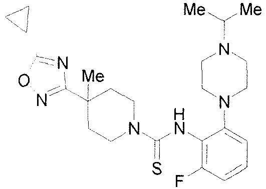 | 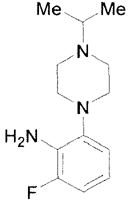 | 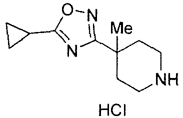 |
| | ¹H-NMR (CDCl₃) δ: 1.23 (6H, d, J = 6.8 Hz), 1.36 (3H, s), 1.41-1.70 (4H, m), 1.70-1.83 (2H, m), 2.14-2.24 (1H, m), 2.24-2.39 (2H, m), 2.51-3.56 (11H, m), 4.23-4.47 (2H, m), 6.76 (1H, bs), 6.82-6.93 (2H, m), 7.10-7.20 (1H, m). | | |
| 58 | | Reference Example 36 | Commercial Product |
| | 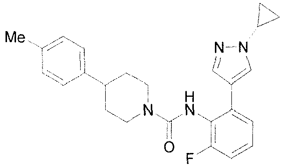 | 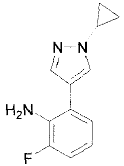 | 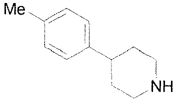 |
| | ¹H-NMR (CDCl₃) δ: 1.00-1.08 (2H, m), 1.11-1.19 (2H, m), 1.62-1.76 (2H, m), 1.84-1.95 (2H, m), 2.34 (3H, s), 2.63-2.76 (1H, m), 2.91-3.07 (2H, m), 3.56-3.66 (1H, m), 4.14-4.26 (2H, m), 5.87 (1H, bs), 6.97-7.08 (1H, m), 7.09-7.22 (6H, m), 7.65 (2H, s). | | |
| 59 | | Reference Example 37 | Reference Example 89 |
| | 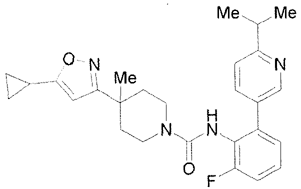 | 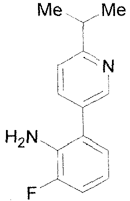 | 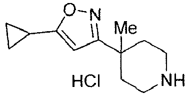 |
| | ¹H-NMR (CDCl₃) δ: 0.93-1.00 (2H, m), 1.00-1.08 (2H, m), 1.24 (3H, s), 1.32 (6H, d, J = 6.7 Hz), 1.51 (2H, ddd, J = 14.1, 10.4, 3.7 Hz), 1.96-2.05 (3H, m), 3.04-3.17 (3H, m), 3.60-3.68 (2H, m), 5.76 (1H, s), 5.78 (1H, s), 7.07-7.17 (2H, m), 7.21-7.26 (2H, m), 7.69 (1H, dd, J = 7.9, 2.1 Hz), 8.55 (1H, d, J = 2.1 Hz). | | |
| 60 | | Reference Example 37 | Reference Example 62 |
| | 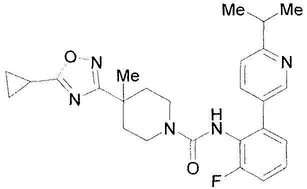 | 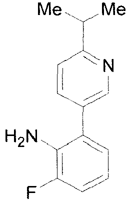 | 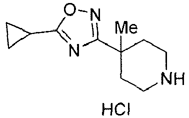 |
| | ¹H-NMR (CDCl₃) δ: 1.19-1.23 (4H, m), 1.28 (3H, s), 1.32 (6H, d, J = 6.7 Hz), 1.51 (2H, ddd, J = 14.2, 10.5, 3.5 Hz), 2.12-2.21 (3H, m), 2.99-3.14 (3H, m), 3.63-3.72 (2H, m), 5.81 (1H, s), 7.07-7.10 (1H, m), 7.13 (1H, ddd, J = 9.9, 8.1, 1.4 Hz), 7.21-7.26 (2H, m), 7.69 (1H, dd, J = 7.9, 2.1 Hz), 8.53 (1H, d, J = 2.1 Hz). | | |
| 61 | | Reference Example 9 | Reference Example 89 |
| | 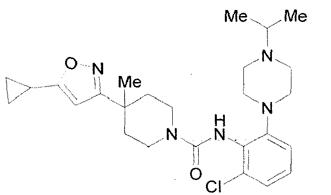 | 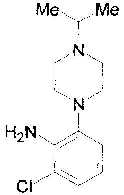 | 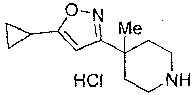 |
| | ¹H-NMR (CDCl₃) δ: 0.90-1.38 (7H, m), 0.92-1.00 (2H, m), 1.03-1.10 (2H, m), 1.31 (3H, s), 1.71 (2H, ddd, J = 14.0, 10.4, 3.6 Hz), 2.02 (1H, tt, J = 8.8, 5.2 Hz), 2.08-2.19 (2H, m), 2,51-3.18 (8H, m), 3.27-3.38 (2H, m), 3.77-3.91 (2H, m), 5.80 (1H, s), 6.11-6.45 (1H, brs), 6.98-7.10 (2H, m), 7.14-7.20 (1H, m). | | |
| 62 | | Reference Example 38 | Commercial Product |
| | 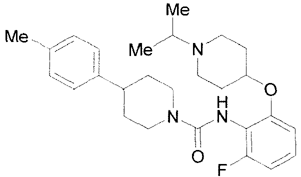 | 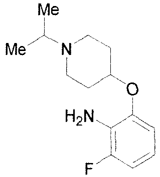 | 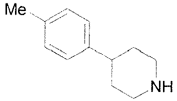 |
| | ¹H-NMR (CDCl₃) δ: 1.15 (6H, m), 1.64-1.75 (6H, m), 1.88 (3H, m), 2.32 (4H, s), 2.68 (1H, m), 2.77-2.99 (5H, m), 4.22 (2H, m), 4.44 (1H, m), 5.97 (1H, brs), 6.65-6.77 (2H, m), 7.01-7.14 (5H, m) | | |
| 63 | | Reference Example 38 | Commercial Product |
| | 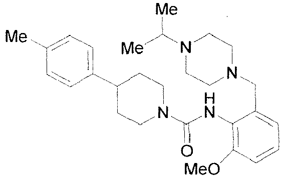 | 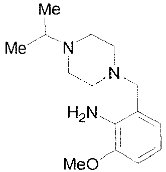 | 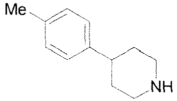 |
| | ¹H-NMR (CDCl₃) δ: 1.02 (6H, d, J = 6.7 Hz), 1.67-1.77 (2H, m), 1.86 (2H, m), 2.32 (3H, s), 2.29-2.70 (9H, m), 2.96 (2H, m), 3.87 (3H, s), 4.30 (2H, m), 6.72-6.80 (1H, m), 6.86-6.92 (1H, m), 7.01-7.14 (5H, m), 8.62 (1H, brs) | | |
| 64 | | Reference Example 5 | Commercial Product |
| | 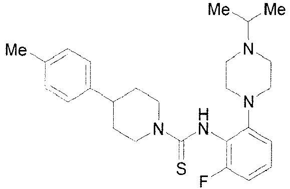 | 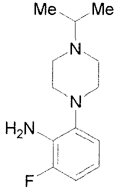 | 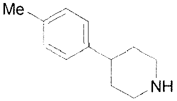 |
| | 1H-NMR (CD3OD) δ: 1.09-1.14 (2H, m), 1.38 (6H, d, J = 6.8 Hz), 1.67-1.81 (2H, m), 1.90-2.00 (2H, m), 2.30 (3H, s), 2.84-2.97 (1H, m), 4.90-5.01 (2H, m), 6.87-m), 3.49-3.76 (6H, m), 4.90-5.01 (2H, 6), 6.87- 6.99 (2H, m), 7.08-7.35 (5H, m). | | |
| 65 | | Reference Example 42 | Reference Example 62 |
| | 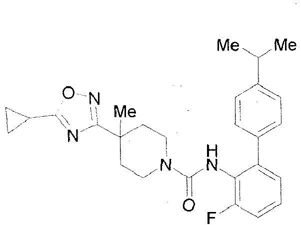 | 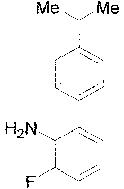 | 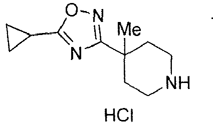 |
| | ¹H-NMR (CDCl₃) δ: 1.18-1.26 (13H, m), 1.47-1.53 (2H, m), 2.12-2.17 (3H, m), 2.92 (1H, m), 3.03 (2H, m), 3.64 (2H, m), 5.72 (1H, brs), 7.05-7.09 (2H, m), 7.15-7.19 (1H, m), 7.26-7.30 (4H, m) | | |
| 66 | | Reference Example 9 | Reference Example 92 |
| | 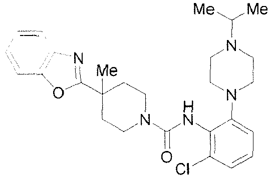 | 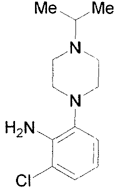 | 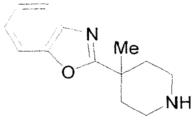 |
| | ¹H-NMR (CDCl₃) δ: 1.05 (6H, d, J = 6.7 Hz), 1.47 (3H, s), 1.81 (2H, m), 2.48 (2H, m), 2.58-2.76 (5H, m), 2.91 (4H, m), 3.27 (2H, m), 3.97 (2H, m), 6.34 (1H, brs), 6.96-7.06 (2H, m), 7.15 (1H, m), 7.31-7.33 (2H, m), 7.49-7.51 (1H, m), 7.68-7.70 (1H, m) | | |
| 67 | | Reference Example 9 | Reference Example 62 |
| | 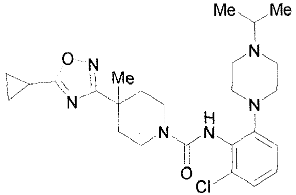 | 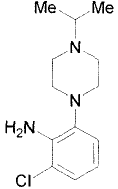 | 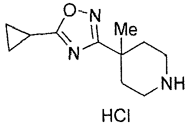 |
| | ¹H-NMR (CDCl₃) δ: 1.10 (6H, d, J = 6.1 Hz), 1.19-1.21 (3H, m), 1.33 (3H, s), 1.65-1.72 (3H, m), 2.17 (1H, m), 2.26 (2H, m), 2.60-2.82 (5H, m), 2.93 (4H, m), 3.22 (2H, m), 3.86 (2H, m), 6.31 (1H, brs), 6.96-7.05 (2H, m), 7.13 (1H, m) | | |
| 68 | | Reference Example 46 | Reference Example 89 |
| | 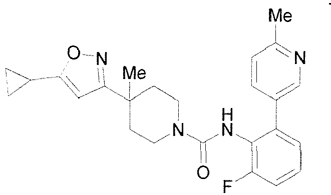 | 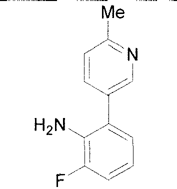 | 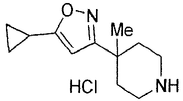 |
| | ¹H-NMR (CDCl₃) δ: 0.94-1.00 (2H, m), 1.00-1.08 (2H, m), 1.25 (3H, s), 1.51 (2H, ddd, J = 13.9, 10.2, 3.8 Hz), 1.96-2.05 (3H, m), 2.56 (3H, s), 3.13 (2H, ddd, J = 13.9, 10.9, 2.9 Hz), 3.61-3.69 (2H, m), 5.77 (1H, s), 5.84 (1H, s), 7.06-7.10 (1H, m), 7.13 (1H, ddd, J = 9.6, 8.4, 1.4 Hz), 7.17-7.27 (2H, m), 7.64 (1H, dd, J = 7.9, 2.1 Hz), 8.48 (1H, d, J = 2.1 Hz). | | |
| 69 | | Reference Example 47 | Reference Example 89 |
| | 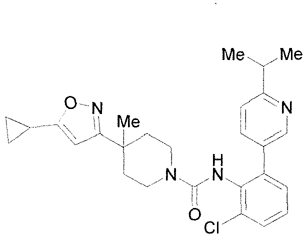 | 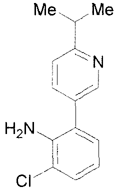 | 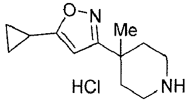 |
| | ¹H-NMR (CDCl₃) δ: 0.92-0.99 (2H, m), 0.99-1.07 (2H, m), 1.23 (3H, s), 1.30 (6H, d, J = 6.7 Hz), 1.47 (2H, ddd, J = 14.0, 10.4, 3.7 Hz), 1.94-2.04 (3H, m), 3.00-3.16 (3H, m), 3.59-3.68 (2H, m), 5.75 (1H, s), 6.02 (1H, s), 7.17-7.24 (3H, m), 7.43 (1H, dd, J = 6.7, 3.0 Hz), 7.68 (1H, dd, J = 7.9, 2.4 Hz), 8.52 (1H, d, J = 2.4 Hz). | | |
| 70 | | Reference Example 48 | Reference Example 89 |
| | 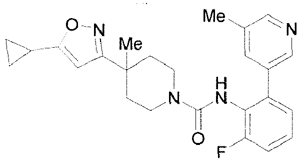 | 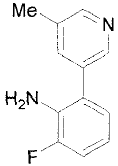 | 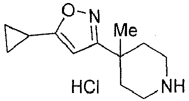 |
| | ¹H-NMR (CDCl₃) δ: 0.93-1.00 (2H, m), 1.00-1.09 (2H, m), 1.24 (3H, s), 1.50 (2H, ddd, J = 14.1, 10.7, 3.7 Hz), 1.94-2.06 (3H, m), 2.36 (3H, s), 3.12 (2H, ddd, J = 13.8, 10.7, 2.8 Hz), 3.60-3.69 (2H, m), 5.76 (1H, s), 5.78 (1H, s), 7.06-7.11 (1H, m), 7.11-7.18 (1H, m), 7.21-7.29 (1H, m), 7.54 (1H, s), 8.40 (1H, d, J = 1.8 Hz), 8.44 (1H, d, J = 1.8 Hz). | | |
| 71 | | Reference Example 49 | Reference Example 89 |
| | 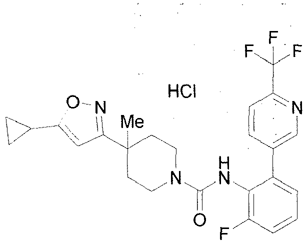 | 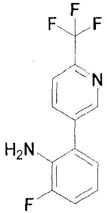 | 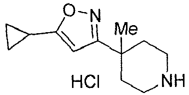 |
| | 1H-NMR (CD3OD) δ: 0.86-0.96 (2H, m), 1.02-1.11 (2H, m), 1.22 (3H, s), 1.35-1.49 (2H, m), 1.92-2.02 (2H, m), 2.02-2.13 (1H, m), 3.00-3.17 (2H, m), 3.68 (2H, d, J = 14.0 Hz), 6.04 (1H, s), 7.21-7.32 (2H, m), 7.37-7.46 (1H, m), 7.87 (1H, d, J = 8.0 Hz), 8.05 (1H, d, J = 8.0 Hz), 8.74 (1H, s). | | |
| 72 | | Reference Example 50 | Reference Example 62 |
| | 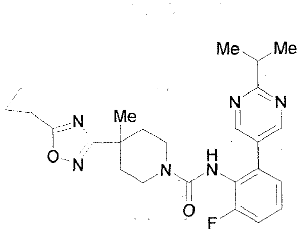 | 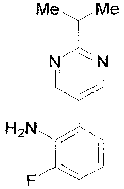 | 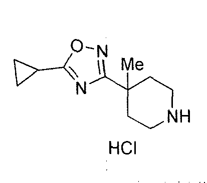 |
| | ¹H-NMR (CDCl₃) δ: 1.15-1.25 (4H, m), 1.30 (3H, s), 1.39 (6H, d, J = 7.6 Hz), 1.44-1.57 (2H, m), 2.12-2.24 (3H, m), 2.96-3.10 (2H, m), 3.24-3.38 (1H, m), 3.70 (2H, d, J = 14.0 Hz), 5.92 (1H, bs), 7.11 (1H, d, J = 8.0 Hz), 7.19 (1H, t, J = 8.0 Hz), 7.23-7.34 (3H, m). | | |
| 73 | | Reference Example 5 | Reference Example 67 |
| | 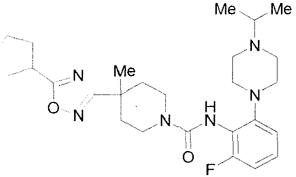 | 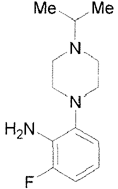 | 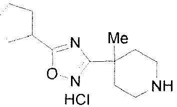 |
| | ¹H-NMR (CDCl₃) δ: 1.10 (6H, d, J = 6.0 Hz), 1.37 (3H, s), 1.66-1.77 (4H, m), 1.77-1.99 (4H, m), 2.07-2.19 (2H, m), 2.32 (2H, d, J = 14.0 Hz), 2.56-2.82 (5H, m), 2.82-3.02 (4H, m), 3.18-3.39 (3H, m), 3.88 (2H, d, J = 14.0 Hz), 6.32 (1H, bs), 6.81-6.92 (2H, m), 7.00-7.10 (1H, m). | | |
| 74 | | Reference Example 9 | Reference Example 67 |
| | 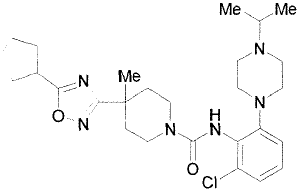 | 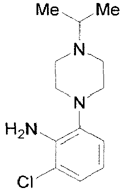 | 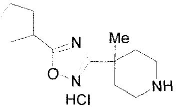 |
| | ¹H-NMR (CDCl₃) δ: 1.10 (6H, d, J = 6.4 Hz), 1.37 (3H, s), 1.66-1.78 (4H, m), 1.78-1.86 (2H, m), 1.86-1.99 (2H, m), 2.07-2.19 (2H, m), 2.32 (2H, d, J = 14.0 Hz), 2.55-2.81 (5H, m), 2.81-3.03 (4H, m), 3.19-3.39 (3H, m), 3.90 (2H, d, J = 14.0 Hz), 6.36 (1H, bs), 6.98 (1H, d, J = 8.0 Hz), 7.05 (1H, t, J = 8.0 Hz), 7.16 (1H, d, J = 8.0 Hz). | | |
| 75 | | Reference Example 5 | Reference Example 70 |
| | 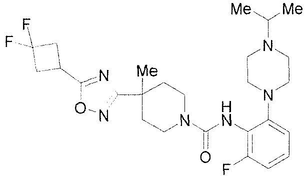 | 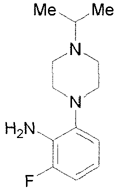 | 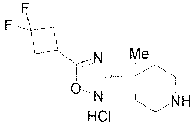 |
| | ¹H-NMR (CDCl₃) δ: 1.10 (6H, d, J = 8.4 Hz), 1.38 (3H, s), 1.74 (2H, ddd, J = 14.0, 10.8, 4.0 Hz), 2.32 (2H, d, J = 14.0 Hz), 2.58-2.83 (5H, m), 2.83-2.99 (4H, m), 2.99-3.16 (4H, m), 3.25 (2H, ddd, J = 14.0, 10.8, 4.0 Hz), 3.54-3.66 (1H, m), 3.88 (2H, dt, J = 14.0, 4.0 Hz), 6.32 (1H, bs), 6.82-6.93 (2H, m), 7.01-7.11 (1H, m). | | |
| 76 | | Reference Example 9 | Reference Example 70 |
| | 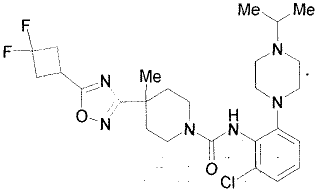 | 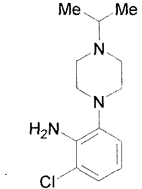 | 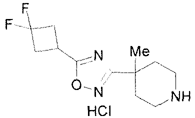 |
| | ¹H-NMR (CDCl₃) δ: 1.10 (6H, bs), 1.39 (3H, s), 1.75 (2H, ddd, J = 14.0, 10.8, 4.0 Hz), 2.32 (2H, d, J = 14.0 Hz), 2.56-2.81 (5H, m), 2.81-3.00 (4H, m), 3.00-3.16 (4H, m), 3.26 (2H, ddd, J = 14.0, 10.8, 4.0 Hz), 3.54-3.67 (1H, m), 3.90 (2H, dt, J = 14.0, 4.0 Hz), 6.36 (1H, bs), 6.99 (1H, dd, J = 8.0, 1.2 Hz), 7.06 (1H, t, J = 8.0 Hz), 7.16 (1H, dd, J = 8.0, 1.2 Hz). | | |
| 77 | | Reference Example 9 | Commercial Product |
| | 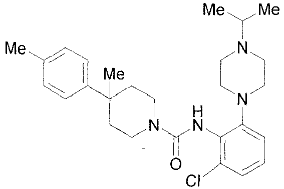 | 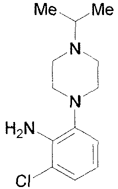 | 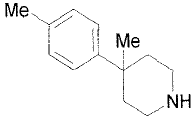 |
| | ¹H-NMR (CDCl₃) δ: 1.03 (6H, d, J = 6.7 Hz), 1.28 (3H, s), 1.74-1.80 (2H, m), 2.14-2.18 (2H, m), 2.32 (3H, s), 2.56-2.70 (5H, m), 2.87 (4H, m), 3.46-3.52 (2H, m), 3.62-3.68 (2H, m), 6.34 (1H, s), 6.94-7.05 (2H, m), 7.12-7.16 (3H, m), 7.23 (2H, m) | | |
| 78 | | Reference Example 9 | Reference Example 66 |
| | 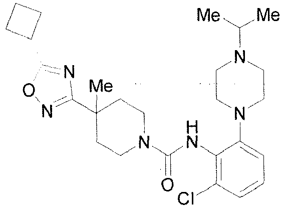 | 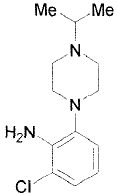 | 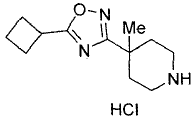 |
| | ¹H-NMR (CDCl₃) δ: 1.11 (6H, bs), 1.38 (3H, s), 1.74 (2H, ddd, J = 14.0, 10.8, 4.0 Hz), 1.99-2.21 (2H, m), 2.33 (2H, d, J = 13.6 Hz), 2.40-2.52 (4H, m), 2.55-2.82 (5H, m), 2.82-3.04 (4H, m), 3.26 (2H, ddd, J = 14.0, 10.8, 4.0 Hz), 3.69-3.81 (1H, m), 3.91 (2H, dt, J = 14.0, 4.0 Hz), 6.36 (1H, bs), 6.99 (1H, dd, J = 8.0, 1.2 Hz), 7.06 (1H, t, J = 8.0 Hz), 7.17 (1H, dd, J = 8.0, 1.2 Hz). | | |
| 79 | | Reference Example 5 | Reference Example 71 |
| | 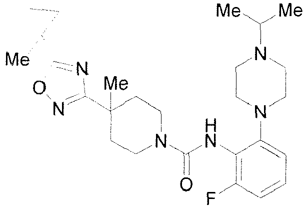 | 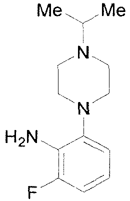 | 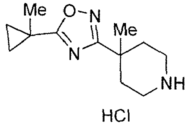 |
| | ¹H-NMR (CDCl₃) δ: 0.96-1.04 (2H, m), 1.12 (6H, bs), 1.35 (3H, s), 1.37-1.94 (2H, m), 1.45-1.76 (7H, m), 2.30 (2H, d, J = 14.0 Hz), 2.52-3.13 (7H, m), 3.18-3.32 (2H, m), 3.81-3.94 (2H, m), 6.30 (1H, bs), 6.81-6.94 (2H, m), 7.01-7.12 (1H, m). | | |
| 80 | | Reference Example 9 | Reference Example 71 |
| | 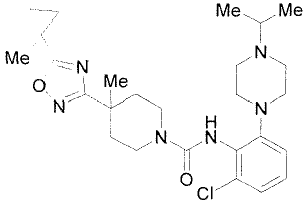 | 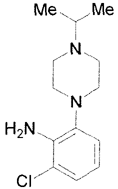 | 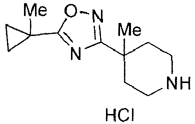 |
| | ¹H-NMR (CDCl₃) δ: 0.97-1.03 (2H, m), 1.12 (6H, bs), 1.35 (3H, s), 1.37-1.94 (2H, m), 1.48-1.76 (7H, m), 2.29 (2H, d, J = 14.0 Hz), 2.51-3.11 (7H, m), 3.19-3.32 (2H, m), 3.81-3.97 (2H, m), 6.34 (1H, bs), 6.95-7.10 (2H, m), 7.13-7.19 (1H, m) | | |
| 81 | | Reference Example 9 | Reference Example 63 |
| | 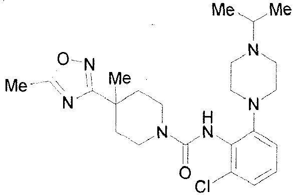 | 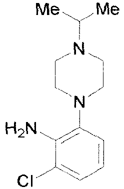 | 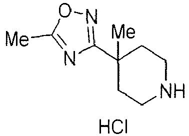 |
| | ¹H-NMR (CDCl₃) δ: 1.10 (6H, bs), 1.37 (3H, s), 1.74 (2H, ddd, J = 14.0, 10.4, 4.4 Hz), 2.31 (2H, d, J = 14.0 Hz), 2.60 (3H, s), 2.52-2.82 (5H, m), 2.82-3.02 (4H, m), 3.25 (2H, ddd, J = 14.0, 10.4, 3.6 Hz), 3.91 (2H, dt, J = 14.0, 3.6 Hz), 6.35 (1H, bs), 6.99 (1H, dd, J = 8.0, 1.2 Hz), 7.06 (1H, t, J = 8.0 Hz), 7.16 (1H, dd, J = 8.0, 1.2 Hz). | | |
| 82 | | Reference Example 5 | Reference Example 64 |
| | 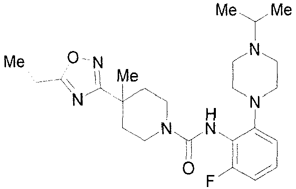 | 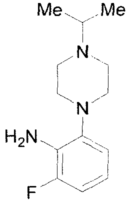 | 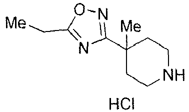 |
| | ¹H-NMR (CDCl₃) δ: 1.12 (6H, bs), 1.38 (3H, s), 1.40 (3H, t, J = 8.0 Hz), 1.73 (2H, ddd, J = 14.0, 10.4, 4.4 Hz), 2.32 (2H, d, J = 13.6 Hz), 2.56-2.81 (5H, m), 2.81-3.05 (4H, m), 2.92 (2H, q, J = 8.0 Hz), 3.25 (2H, ddd, J = 14.0, 10.4, 3.6 Hz), 3.88 (2H, dt, J = 14.0, 3.6 Hz), 6.30 (1H, bs), 6.83-6.92 (2H, m), 7.01-7.10 (1H. m). | | |
| 83 | | Reference Example 9 | Reference Example 64 |
| | 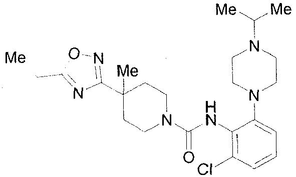 | 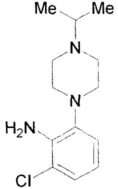 | 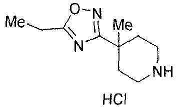 |
| | ¹H-NMR (CDCl₃) δ: 1.11 (6H, bs), 1.38 (3H, s), 1.40 (3H, t, J = 8.0 Hz), 1.74 (2H, ddd, J = 14.0, 10.4, 4.4 Hz), 2.32 (2H, d, J = 13.2 Hz), 2.56-2.82 (5H, m), 2.82-3.03 (4H, m), 2.92 (2H, q, J = 8.0 Hz), 3.26 (2H, ddd, J = 13.2, 10.4, 3.6 Hz), 3.91 (2H, dt, J = 14.0, 3.6 Hz), 6.35 (1H, bs), 6.99 (1H, dd, J = 8.0, 1.2 Hz), 7.06 (1H, t, J = 8.0 Hz), 7.16 (1H, dd, J = 8.0, 1.2 Hz). | | |
| 84 | | Reference Example 9 | Reference Example 91 |
| | 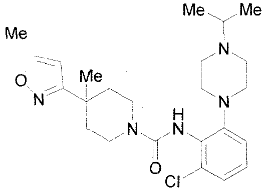 | 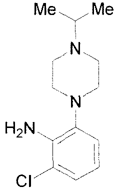 | 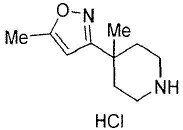 |
| | ¹H-NMR (CDCl₃) δ: 1.33 (3H, s), 1,41-1.59 (6H, m), 1.67-1.75 (2H, m), 1.81-1.89 (1H, m), 2.07-2.20 (2H, m), 2.42 (3H, s), 2,86-3.09 (2H, m), 3.10-3.29 (2H, m), 3.29-3.50 (4H, m), 3.67-3.85 (4H, m), 5.88 (1H, s), 5.97-6.05 (1H, brs), 7.09-7.16 (1H, m), 7.20-7.30 (2H, m). | | |
| 85 | | Reference Example 5 | Reference Example 91 |
| | 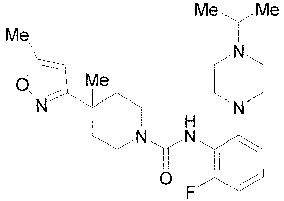 | 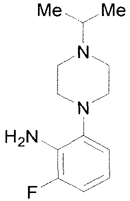 | 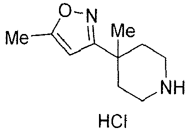 |
| | ¹H-NMR (CDCl₃) δ: 1.33 (3H, s), 1,43-1.58 (6H, m), 1.66-1.76 (2H, m), 1.81-1.89 (1H, m), 2.09-2.19 (2H, m), 2.42 (3H, s), 2,88-3.09 (2H, m), 3.11-3.25 (2H, m), 3.26-3.54 (4H, m), 3.68-3.85 (4H, m), 5.88 (1H, s), 5.92-6.00 (1H, brs), 6.87-7.02 (2H, m), 7.06-7.17 (1H, m). | | |
| 86 | | Reference Example 9 | Reference Example 68 |
| | 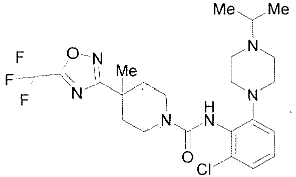 | 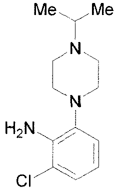 | 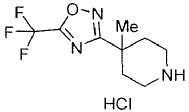 |
| | ¹H-NMR (CDCl₃) δ: 1.09 (6H, d, J = 6.8 Hz), 1.45 (3H, s), 1.82 (2H, ddd, J = 14.4, 10.4, 4.4 Hz), 2.34 (2H, dt, J = 9.6, 3.6 Hz), 2.55-2.80 (5H, m), 2.84-3.00 (4H, m), 3.29 (2H, ddd, J = 13.6, 10.4, 3.2 Hz), 3.91 (2H, dt, J = 13.6, 4.4 Hz), 6.38 (1H, bs), 6.99 (1H, dd, J = 8.0, 1.2 Hz), 7.06 (1H, t, J = 8.0 Hz), 7.17 (1H, dd, J = 8.0, 1.2 Hz). | | |
| 87 | | Reference Example 5 | Reference Example 72 |
| | 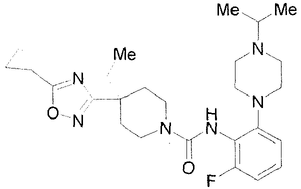 | 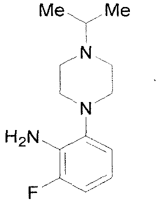 | 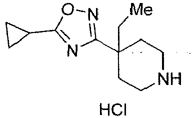 |
| | ¹H-NMR (CDCl₃) δ: 0.74 (3H, t, J = 7.2 Hz), 1.13 (6H, bs), 1.19-1.23 (1H, m), 1.24 (3H, s), 1.62-1.74 (4H, m), 2.15-2.25 (1H, m), 2.27-2.37 (2H, m), 2.57-2.86 (5H, m), 2.86-3.03 (4H, m), 3.03-3.15 (2H, m), 3.97 (2H, dt, J = 13.6, 4.0 Hz), 6.27 (1H, bs), 6.82-6.92 (2H, m), 7.00-7.09 (1H, m). | | |
| 88 | | Reference Example 11 | Reference Example 62 |
| | 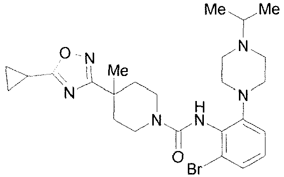 | 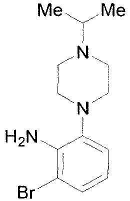 | 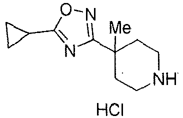 |
| | ¹H-NMR (CDCl₃) δ: 1.05 (6H, d, J = 6.1 Hz), 1.18-1.22 (4H, m), 1.33 (3H, s), 1.67-1.74 (2H, m), 2.14-2.19 (1H, m), 2.27 (2H, m), 2.60-2.70 (5H, m), 2.87 (4H, m), 3.22 (2H, m), 3.88 (2H, m), 6.38 (1H, brs), 6.94-7.01 (2H, m), 7.30-7.32 (1H, m) | | |
| 89 | | Reference Example 9 | Reference Example 72 |
| | 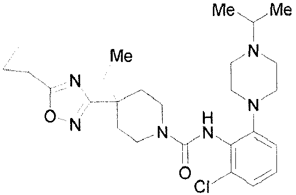 | 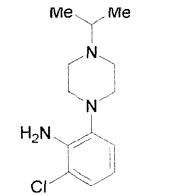 | 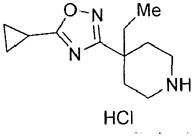 |
| | ¹H-NMR (CDCl₃) δ: 0.74 (3H, t, J = 7.6 Hz), 1.09 (6H, d, J = 6.4 Hz), 1.20-1.26 (4H, m), 1.64-1.74 (4H, m), 2.16-2.25 (1H, m), 2.27-2.38 (2H, m), 2.53-2.79 (5H, m), 2.83-2.99 (4H, m), 3.02-3.16 (2H, m), 4.00 (2H, dt, J = 13.6, 4.0 Hz), 6.36 (1H, bs), 6.98 (1H, dd, J = 8.0, 1.2 Hz), 7.05 (1H, t, J = 8.0 Hz), 7.16 (1H, dd, J = 8.0, 1.2 Hz). | | |
| 90 | | Reference Example 9 | Reference Example 94 |
| | 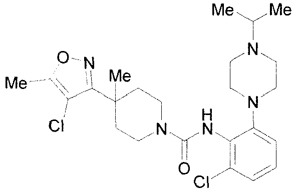 | 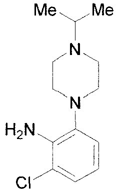 | 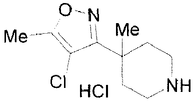 |
| | ¹H-NMR (CDCl₃) δ: 1.08 (6H, d, J = 6.4 Hz), 1.43 (3H, s), 1.70-1.77 (2H, m), 2.41 (3H, s), 2.43-2.51 (2H, m), 2.58-2.66 (4H, m), 2.66-2.76 (1H, m), 2.85-2.94 (4H, m), 3.29-3.41 (2H, m), 3.84-3.94 (2H, m), 6.38 (1H, s), 6.97 (1H, dd, J = 8.0, 1.6 Hz), 7.05 (1H, t, J = 8.0 Hz), 7.16 (1H, dd, J = 8.0, 1.6 Hz) | | |
| 91 | | Reference Example 5 | Reference Example 95 |
| | 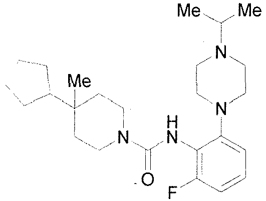 | 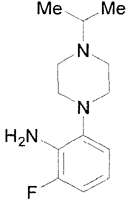 | 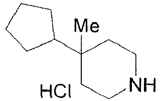 |
| | ¹H-NMR (CDCl₃) δ: 0.94 (3H, s), 1.09 (6H, d, J = 6.1 Hz), 1.20-1.34 (2H, m), 1.35-1.45 (2H, m), 1.46-1.63 (8H, m), 1.72-1.84 (1H, m), 2.60-2.68 (4H, m), 2.68-2.77 (1H, m), 2.86-2.94 (4H, m), 3.24 (2H, ddd, J = 13.6, 10.9, 2.9 Hz), 3.79-3.88 (2H, m), 6.31 (1H, s), 6.82-6.90 (2H, m), 7.00-7.07 (1H, m). | | |
| 92 | | Reference Example 9 | Reference Example 96 |
| | 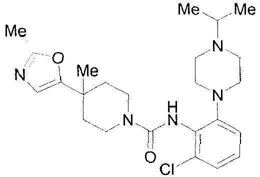 | 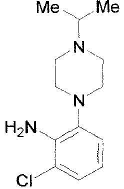 | 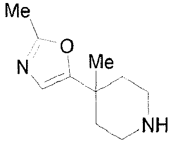 |
| | ¹H-NMR (CDCl₃) δ: 1.14 (6H, bs), 1.30 (3H, s), 1.63-1.75 (2H, m), 2.02-2.12 (2H, m), 2.45 (3H, s), 2.60-2.82 (5H, m), 2.87-3.11 (4H, m), 3.38-3.48 (2H, m), 3.66-3.82 (2H, m), 6.31 (1H, bs), 6.99 (1H, dd, J = 8.0, 1.2 Hz), 7.06 (1H, t, J = 8.0 Hz), 7.16 (1H, dd, J = 8.0, 1.2 Hz), 7.21 (1H, s). | | |
| 93 | | Reference Example 9 | Reference Example 97 |
| | 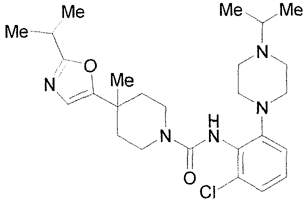 | 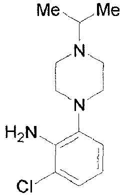 | 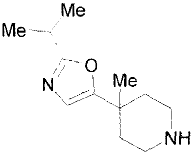 |
| | ¹H-NMR (CDCl₃) δ: 1.09 (6H, d, J = 6.4 Hz), 1.30 (3H, s), 1.32 (3H, s), 1.34 (3H, s), 1.63-1.74 (2H, m), 2.03-2.13 (2H, m), 2.58-2.82 (5H, m), 2.87-3.00 (4H, m), 3.02-3.13 (1H, m), 3.39-3.49 (2H, m), 3.66-3.76 (2H, m), 6.36 (1H, bs), 6.98 (1H, dd, J = 8.0, 1.2 Hz), 7.05 (1H, t, J = 8.0 Hz), 7.16 (1H, dd, J = 8.0, 1.2 Hz), 7.27 (1H, s). | | |
| 94 | | Reference Example 9 | Reference Example 73 |
| | 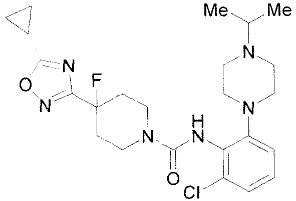 | 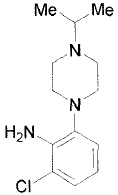 | 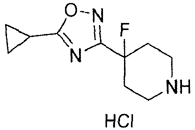 |
| | ¹H-NMR (CDCl₃) δ: 1.09 (6H, d, J = 6.8 Hz), 1.20-1.36 (5H, m), 2.17-2.35 (5H, m), 2.57-2.79 (4H, m), 2.87-2.98 (4H, m), 3.47-3.58 (2H, m), 4.03 (2H, dt, J = 13.6, 4.0 Hz), 6.43 (1H, bs), 6.99 (1H, dd, J = 8.0, 1.2 Hz), 7.07 (1H, t, J = 8.0 Hz), 7.16 (1H, dd, J = 8.0, 1.2 Hz). | | |
| 95 | | Reference Example 9 | Reference Example 74 |
| | 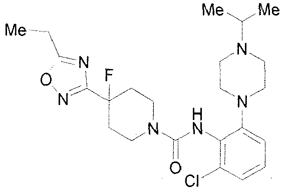 | 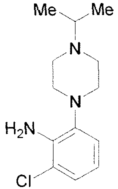 | 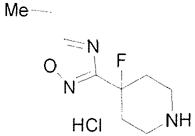 |
| | ¹H-NMR (CDCl₃) δ: 1.09 (6H, d, J = 6.4 Hz), 1.42 (3H, t, J = 8.0 Hz), 2.16-2.40 (4H, m), 2.60-2.69 (4H, m), 2.69-2.79 (1H, m), 2.87-2.96 (4H, m), 2.95 (2H, q, J = 8.0 Hz), 3.49-3.59 (2H, m), 4.05 (2H, dt, J = 13.2, 4.0 Hz), 6.44 (1H, bs), 7.00 (1H, dd, J = 8.0, 1.2 Hz), 7.07 (1H, t, J = 8.0 Hz), 7.17 (1H, dd, J = 8.0, 1.2 Hz). | | |
| 96 | | Reference Example 9 | Reference Example 100 |
| | 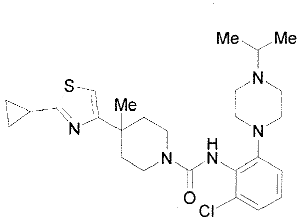 | 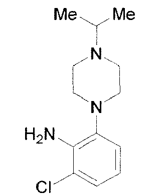 | 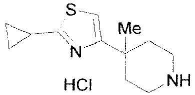 |
| | ¹H-NMR (CDCl₃) δ: 0.97-1.03 (2H, m), 1.04-1.15 (8H, m), 1.32 (3H, s), 1.74 (2H, ddd, J = 13.2, 8.8, 4.4 Hz), 2.19-2.34 (3H, m), 2.57-2.81 (5H, m), 2.85-3.00 (4H, m), 3.43 (2H, ddd, J = 13.2, 8.8, 4.0 Hz), 3.65-3.76 (2H, m), 6.35 (1H, s), 6.68 (1H, s), 6.98 (1H, dd, J = 8.0, 1.6 Hz), 7.05 (1H, t, J = 8.0 Hz), 7.16 (1H, dd, J = 8.0, 1.6 Hz). | | |
| 97 | | Reference Example 9 | Commercial Product |
| | 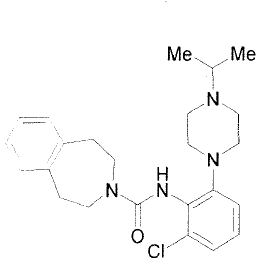 | 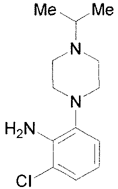 | 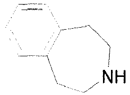 |
| | ¹H-NMR (CDCl₃) δ: 1.04 (6H, d, J = 6.8 Hz), 2.58-2.72 (5H, m), 2.89-2.96 (4H, m), 3.04-3.10 (4H, m), 3.71-3.77 (4H, m), 6.51 (1H, s), 7.01 (1H, dd, J = 8.0, 1.6 Hz), 7.07 (1H, t, J = 8.0 Hz), 7.15-7.20 (5H, m). | | |
| 98 | | Reference Example 9 | Commercial Product |
| | 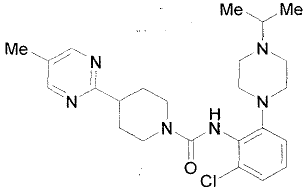 | 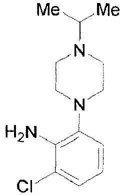 | 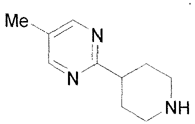 |
| | ¹H-NMR (CDCl₃) δ: 1.08 (6H, d, J = 6.4 Hz), 1.95 (2H, ddd, J = 24.8, 12.0, 4.0 Hz), 2.06-2.16 (2H, m), 2.30 (3H, s), 2.59-2.77 (5H, m), 2.87-2.98 (4H, m), 3.04-3.18 (3H, m), 4.25-4.36 (2H, m), 6.42 (1H, s), 6.98 (1H, dd, J = 8.0, 1.2 Hz), 7.05 (1H, t, J = 8.0 Hz), 7.16 (1H, dd, J = 8.0, 1.2 Hz), 8.51 (2H, s). | | |
| 99 | | Reference Example 9 | Commercial Product |
| | 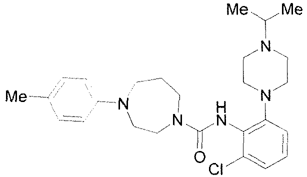 | 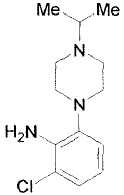 | 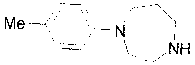 |
| | ¹H-NMR (CDCl₃) δ: 0.99-1.15 (6H, m), 2.10-2.22 (2H, m), 2.25 (3H, s), 2.50-2.74 (5H, m), 2.82-2.96 (4H, m), 3.42-3.49 (2H, m), 3.62-3.81 (6H, m), 6.33-6.45 (1H, m), 6.67 (2H, d, J = 8.8 Hz), 6.96-7.10 (4H, m), 7.14-7.19 (1H, m). | | |
| 100 | | Reference Example 19 | Reference Example 62 |
| | 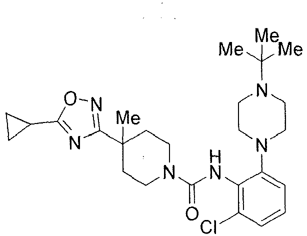 | 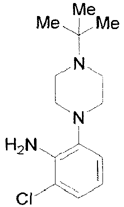 | 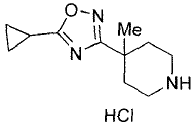 |
| | ¹H-NMR (CDCl₃) δ: 1.07 (9H, s), 1.18-1.22 (4H, m), 1.33 (3H, s), 1.67-1.73 (2H, m), 2.17 (1H, m), 2.27 (2H, m), 2.66 (4H, m), 2.86 (4H, m), 3.22 (2H, m), 3.88 (2H, m), 6.36 (1H, brs), 6.94-6.96 (1H, m), 7.00-7.04 (1H, m), 7.12-7.14 (1H, m) | | |
| 101 | | Reference Example 9 | Reference Example 99 |
| | 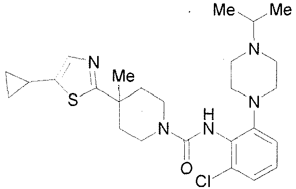 | 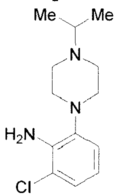 | 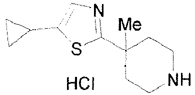 |
| | ¹H-NMR (CDCl₃) δ: 0.69-0.75 (2H, m), 0.99-1.05 (2H, m), 1.07 (6H, d, J = 6.8 Hz), 1.40 (3H, s), 1.82 (2H, ddd, J = 14.0, 9.6, 3.6 Hz), 1.98-2.08 (1H, m), 2.23-2.32 (2H, m), 2.54-2.66 (4H, m), 2.67-2.73 (1H, m), 2.85-2.92 (4H, m), 3.40 (2H, ddd, J = 13.2, 10.0, 3.2 Hz), 3.83 (2H, dt, J = 9.2, 4.4 Hz), 6.34-6.42 (1H, br), 6.97 (1H, dd, J = 8.0, 1.2 Hz), 7.05 (1H, t, J = 8.0 Hz), 7.13-7.18 (1H, m), 7.34 (1H, s). | | |
| 102 | | Reference Example 9 | Reference Example 98 |
| | 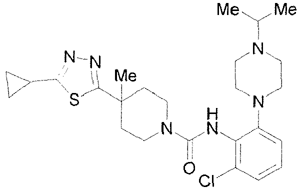 | 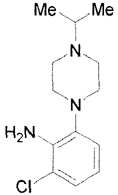 | 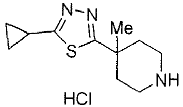 |
| | ¹H-NMR (CDCl₃) δ: 1.08 (6H, d, J = 6.0 Hz), 1.10-1.16 (2H, m), 1.18-1.29 (2H, m), 1.44 (3H, s), 1.88 (2H, ddd, J = 14.4, 9.6, 3.6 Hz), 2.24-2.33 (2H, m), 2.39 (1H, tt, J = 8.0, 4.8 Hz), 2.66-2.76 (5H, m), 2.85-2.92 (4H, m), 3.37 (2H, ddd, J = 14.0, 10.4, 3.2 Hz), 3.91 (2H, dt, J = 14.0, 4.4 Hz), 6.35-6.42 (1H, br), 6.98 (1H, dd, J = 8.0, 1.2 Hz), 7.05 (1H, t, J = 8.0 Hz), 7.16 (1H, dd, J = 8.0, 2.0 Hz). | | |
| 103 | | Reference Example 9 | Reference Example 93 |
| | 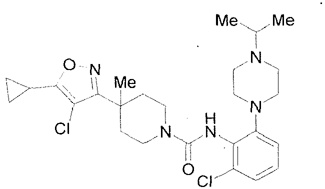 | 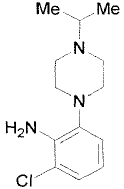 | 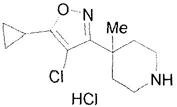 |
| | ¹H-NMR (CDCl₃) δ: 1.08 (6H, d, J = 6.8 Hz), 1.09-1.14 (2H, m), 1.14-1.20 (2H, m), 1.43 (3H, s), 1.73 (2H, ddd, J = 14.0, 10.4, 3.6 Hz), 2.02-2.13 (1H, m), 2.42-2.50 (2H, m), 2.58-2.76 (5H, m), 2.85-2.92 (4H, m), 3.35 (2H, ddd, J = 13.6, 10.4, 2.8 Hz), 3.88 (2H, dt, J = 9.2, 4.8 Hz), 6.35-6.40 (1H, br), 6.97 (1H, dd, J = 8.0, 1.2 Hz), 7.05 (1H, t, J = 8.0 Hz), 7.16 (1H, dd, J = 8.0, 1.2 Hz). | | |
| 104 | | Reference Example 9 | Reference Example 75 |
| | 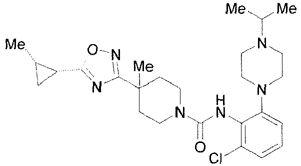 | 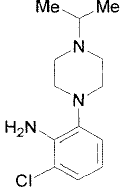 | 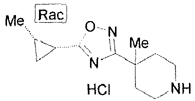 |
| | ¹H-NMR (CDCl₃) δ: 1.08 (6H, d, J = 6.8 Hz), , 1.10-1.18 (4H, m), 1.31-1.41 (4H, m), 1.46-1.60 (1H, m), 1.67-1.78 (2H, m), 2.24 (1H, td, J = 8.4, 5.2 Hz ), 2.28-2.37 (2H, m), 2.57-2.77 (5H, m), 2.85-2.93 (4H, m), 3.17-3.30 (2H, m), 3.87-3.97 (2H, m), 6.35-6.41 (1H, br), 6.97 (1H, dd, J = 8.0, 1.2 Hz), 7.05 (1H, t, J = 8.0 Hz), 7.16 (1H, dd, J = 8.0, 1.6 Hz). | | |
| 105 | | Reference Example 9 | Reference Example 76 |
| | 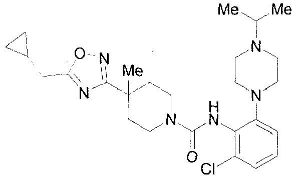 | 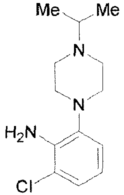 | 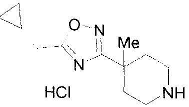 |
| | ¹H-NMR (CDCl₃) δ: 0.27-0.34 (2H, m), 0.60-0.67 (2H, m), 1.08 (6H, d, J = 6.8 Hz), 1.12-1.24 (1H, m), 1.39 (3H, s), 1.75 (2H, ddd, J = 14.4, 10.8, 4.0 Hz), 2.29-2.38 (2H, m), 2.57-2.77 (5H, m), 2.81 (2H, d, J = 6.8 Hz), 2.86-2.92 (4H, m), 3.26 (2H, ddd, J = 13.6, 10.4, 2.4 Hz), 3.92 (2H, dt, J = 13.2, 4.0 Hz), 6.37-6.41 (1H, br), 6.98 (1H, dd, J = 8.0, 1.2 Hz), 7.05 (1H, t, J = 8.0 Hz), 7.16 (1H, dd, J = 8.0, 2.0 Hz). | | |
| 106 | | Reference Example 9 | Reference Example 77 |
| | 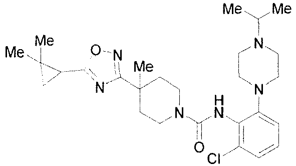 | 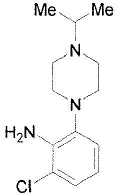 | 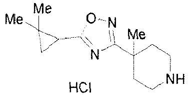 |
| | ¹H-NMR (CDCl₃) δ: 1.08 (6H, d, J = 6.8 Hz), 1.15-1.21 (4H, m), 1.27 (3H, s), 1.34 (1H, t, J = 5.2 Hz), 1.36 (3H, s), 1.67-1.77 (2H, m), 2.03 (1H, dd, J = 8,4, 5.6 Hz), 2.27-2.36 (2H, m), 2.57-2.75 (5H, m), 2.86-2.92 (4H, m), 3.16-3.28 (2H, m), 3.88-3.97 (2H, m), 6.36-6.40 (1H, br), 6.97 (1H, dd, J = 8.0, 1.2 Hz), 7.05 (1H, t, J = 8.0 Hz), 7.16 (1H, dd, J = 8.0, 1.2 Hz). | | |
| 107 | | Reference Example 9 | Reference Example 78 |
| | 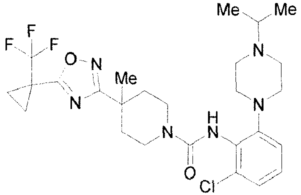 | 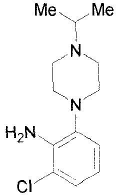 | 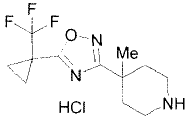 |
| | ¹H-NMR (CDCl₃) δ: 1.08 (6H, d, J = 6.8 Hz), 1.37 (3H, s), 1.63-1.79 (6H, m), 2.26-2.34 (2H, m), 2.57-2.76 (5H, m), 2.86-2.92 (4H, m), 3.26 (2H, ddd, J = 13.6, 10.4, 3.2 Hz), 3.90 (2H, dt, J = 14.8, 3.6 Hz), 6.37-6.40 (1H, br), 6.98 (1H, dd, J = 8.0, 1.6 Hz), 7.05 (1H, t, J = 8.0 Hz), 7.16 (1H, dd, J = 8.0, 1.2 Hz). | | |
| 108 | | Reference Example 9 | Reference Example 79 |
| | 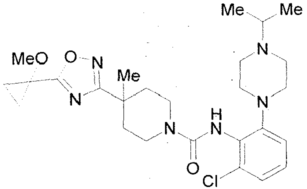 | 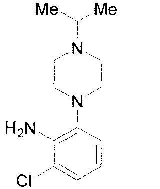 | 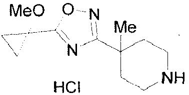 |
| | ¹H-NMR (CDCl₃) δ: 1.08 (6H, d, J = 6.8 Hz), 1.38 (3H, s), 1.42-1.47 (4H, m), 1.74 (2H, ddd, J = 14.0, 10.4, 3.6 Hz), 2.29-2.37 (2H, m), 2.57-2.76 (5H, m), 2.86-2.92 (4H, m), 3.26 (2H, ddd, J = 14.0, 10.8, 3.2 Hz), 3.54 (3H, s), 3.91 (2H, dt, J = 14.0, 4.0 Hz), 6.37-6.40 (1H, br), 6.98 (1H, dd, J = 8.0, 1.2 Hz), 7.05 (1H, t, J = 8.0 Hz), 7.16 (1H, dd, J = 8.0, 1.6 Hz). | | |
| 109 | | Reference Example 9 | Reference Example 80 |
| | 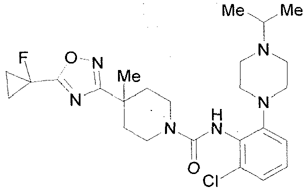 | 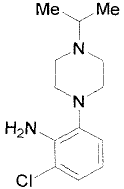 | 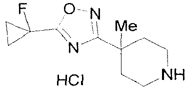 |
| | ¹H-NMR (CDCl₃) δ: 1.08 (6H, d, J = 6.8 Hz), 1.39 (3H, s), 1.48-1.60 (2H, m), 1.65-1.82 (4H, m), 2.27-2.37 (2H, m), 2.57-2.77 (5H, m), 2.85-2.93 (4H, m), 3.26 (2H, ddd, J = 13.6, 10.4, 2.4 Hz), 3.92 (2H, dt, J = 13.2, 4.0 Hz), 6.37-6.41 (1H, br), 6.98 (1H, dd, J = 8.0, 1.2 Hz), 7.05 (1H, t, J = 8.0 Hz), 7.16 (1H, dd, J = 8.0, 1.2 Hz). | | |
| 110 | | Reference Example 39 | Reference Example 62 |
| | 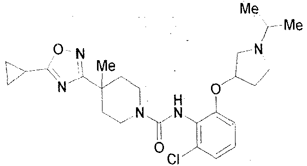 | 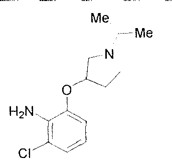 | 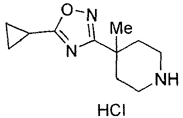 |
| | ¹H-NMR (CDCl₃) δ: 1.09 (3H, d, J = 8.0 Hz), 1.10 (3H, d, J = 8.0 Hz), 1.19-1.24 (4H, m), 1.33 (3H, s), 1.70 (2H, ddd, J = 14.0, 10.6, 3.7 Hz), 1.95-2.04 (1H, m), 2.15-2.22 (1H, m), 2.22-2.33 (3H, m), 2.37-2.43 (1H, m), 2.44-2.52 (1H, m), 2.79 (1H, dd, J = 10.6, 6.1 Hz), 2.84-2.93 (2H, m), 3.16-3.24 (2H, m), 3.87 (2H, ddd, J = 14.0, 4.6, 4.0 Hz), 4.76-4.82 (1H, m), 6.43 (1H, s), 6.70 (1H, dd, J = 6.1, 3.7 Hz), 6.99-7.05 (2H, m) . | | |
| 111 | | Reference Example 9 | Reference Example 75 |
| | 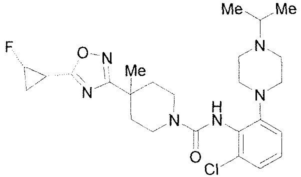 | 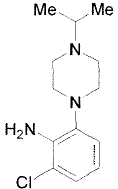 | 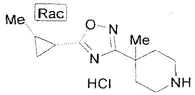 |
| | ¹H-NMR (CDCl₃) δ: 1.08 (6H, d, J = 6.4 Hz), 1.38 (3H, s), 1.44-1.55 (1H, m), 1.74 (2H, ddd, J = 14.0, 11.2, 4.4 Hz), 1.93-2.05 (1H, m), 2.27-2.43 (3H, m), 2.56-2.67 (4H, m), 2.67-2.76 (1H, m), 2.85-2.92 (4H, m), 3.24 (2H, ddd, J = 14.0, 11.2, 3.2 Hz), 3.87-3.98 (2H, m), 4.85 (0.5H, ddd, J = 6.0, 6.0, 3.6 Hz), 5.01 (0.5H, ddd, J = 6.0, 6.0, 3.6 Hz), 6.38 (1H, brs), 6.97 (1H, d, J = 8.0, 1.2 Hz), 7.05 (1H, t, J = 8.0 Hz), 7.16 (1H, dd, J = 8.0, 1.2 Hz). | | |
| 112 | | Reference Example 9 | Reference Example 83 |
| | 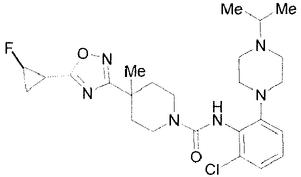 | 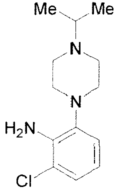 | 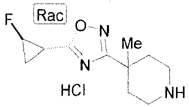 |
| | ¹H-NMR (CDCl₃) δ: 1.08 (6H, d, J = 6.8 Hz), 1.34 (3H, s), 1.50-1.58 (1H, m), 1.72 (2H, ddd, J = 14.0, 10.4, 3.6 Hz), 1.81 (1H, dddd, J = 21.6, 11.2, 7.6, 3.6 Hz), 2.28 (2H, dt, J = 14.0, 3.6 Hz), 2.57-2.76 (6H, m), 2.85-2.93 (4H, m), 3.23 (2H, ddd, J = 14.0, 10.4, 3.2 Hz), 3.90 (2H, dt, J = 14.0, 3.6 Hz), 4.91 (0.5H, ddd, J = 6.0, 3.6, 1.60 Hz), 5.07 (0.5H, ddd, J = 6.0, 3.6, 1.6 Hz), 6.38 (1H, brs), 6.98 (1H, d, J = 8.0, 1.2 Hz), 7.05 (1H, t, J = 8.0 Hz), 7.16 (1H, dd, J = 8.0, 1.2 Hz). | | |
| 113 | | Reference Example 10 | Reference Example 62 |
| | 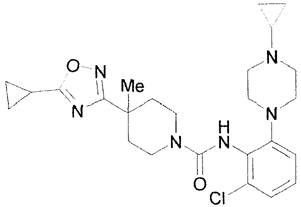 | 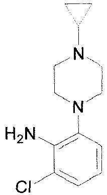 | 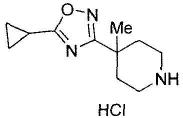 |
| | ¹H-NMR (CDCl₃) δ: 0.40-0.52 (4H, m), 1.19-1.27 (4H, m), 1.36 (3H, s), 1.62-1.78 (3H, m), 2.14-2.25 (1H, m), 2.26-2.36 (2H, m), 2.65-2.79 (4H, m), 2.80-2.88 (4H, m), 3.25 (2H, ddd, J = 13.6, 10.4, 2.4 Hz), 3.92 (2H, dt, J = 9.2, 4.4 Hz), 6.37-6.42 (1H, br), 6.95 (1H, dd, J = 8.0, 1.2 Hz), 7.04 (1H, t, J = 8.0 Hz), 7.16 (1H, dd, J = 8.0, 1.6 Hz). | | |
| 114 | | Reference Example 9 | Reference Example 81 |
| | 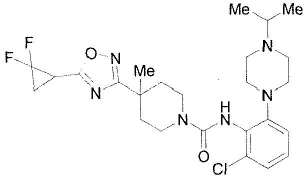 | 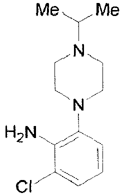 | 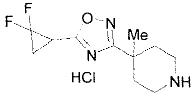 |
| | ¹H-NMR (CDCl₃) δ: 1.08 (6H, d, J = 6.4 Hz), 1.38 (3H, s), 1.69-1.81 (2H, m), 2.05-2.18 (1H, m), 2.23-2.36 (3H, m), 2.53-2.79 (5H, m), 2.82-2.96 (4H, m), 2.96-3.07 (1H, m), 3.17-3.30 (2H, m), 3.85-3.97 (2H, m), 6.38 (1H, s), 6.98 (1H, d, J = 8.0 Hz), 7.05 (1H, t, J = 8.0 Hz), 7.16 (1H, d, J = 8.0 Hz). | | |
| 115 | | Reference Example 9 | Reference Example 84 |
| | 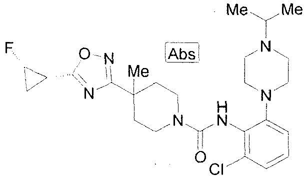 | 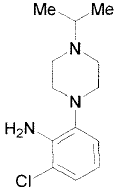 | 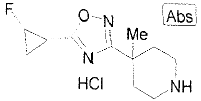 |
| | ¹H-NMR (CDCl₃) δ: 1.08 (6H, d, J = 6.4 Hz), 1.38 (3H, s), 1.44-1.54 (1H, m), 1.68-1.80 (2H, m), 1.92-2.05 (1H, m), 2.27-2.43 (3H, m), 2.54-2.77 (5H, m), 2.83-2.94 (4H, m), 3.18-3.29 (2H, m), 3.87-3.98 (2H, m), 4.82-4.88 (0.5H, m), 4.97-5.04 (0.5H, m), 6.38 (1H, s), 6.97 (1H, d, J = 8.0 Hz), 7.05 (1H, t, J = 8.0 Hz), 7.16 (1H, d, J = 8.0 Hz). | | |
| 116 | | Reference Example 9 | Reference Example 85 |
| | 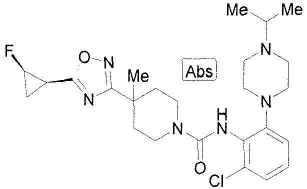 | 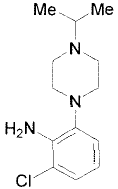 | 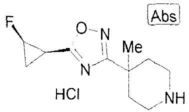 |
| | ¹H-NMR (CDCl₃) δ: 1.08 (6H, d, J = 6.4 Hz), 1.38 (3H, s), 1.44-1.54 (1H, m), 1.68-1.80 (2H, m), 1.92-2.05 (1H, m), 2.27-2.43 (3H, m), 2.54-2.77 (5H, m), 2.83-2.94 (4H, m), 3.18-3.29 (2H, m), 3.87-3.98 (2H, m), 4.82-4.88 (0.5H, m), 4.97-5.04 (0.5H, m), 6.38 (1H, s), 6.97 (1H, d, J = 8.0 Hz), 7.05 (1H, t, J = 8.0 Hz), 7.16 (1H, d, J = 8.0 Hz). | | |
| 117 | | Reference Example 9 | Reference Example 87 |
| | 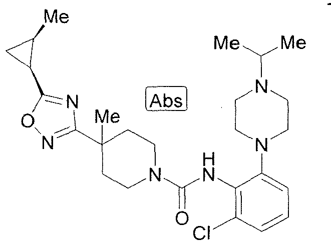 | 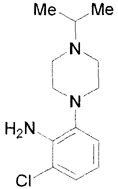 | 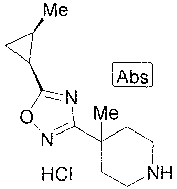 |
| | ¹H-NMR (CDCl₃) δ: 1.08 (6H, d, J = 6.4 Hz), 1.08-1.16 (1H, m), 1.16 (3H, d, J = 6.4 Hz), 1.31-1.39 (1H, m), 1.37 (3H, s), 1.45-1.60 (1H, m), 1.66-1.79 (2H, m), 2.20-2.28 (1H, m), 2.28-2.37 (2H, m), 2.56-2.67 (4H, m), 2.67-2.76 (1H, m), 2.82-2.95 (4H, m), 3.16-3.30 (2H, m), 3.86-3.99 (2H, m), 6.38 (1H, s), 6.97 (1H, d, J = 8.0 Hz), 7.05 (1H, t, J = 8.0 Hz), 7.16 (1H, d, J = 8.0 Hz). | | |
| 118 | | Reference Example 9 | Reference - Example 86 |
| | 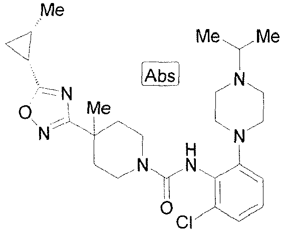 | 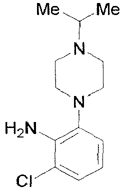 | 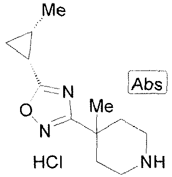 |
| | ¹H-NMR (CDCl₃) δ: 1.08 (6H, d, J = 6.8 Hz), 1.08-1.16 (1H, m), 1.16 (3H, d, J = 6.0 Hz), 1.31-1.39 (1H, m), 1.37 (3H, s), 1.45-1.60 (1H, m), 1.66-1.79 (2H, m), 2.20-2.28 (1H, m), 2.28-2.37 (2H, m), 2.56-2.67 (4H, m), 2.67-2.76 (1H, m), 2.82-2.95 (4H, m), 3.16-3.30 (2H, m), 3.86-3.99 (2H, m), 6.38 (1H, s), 6.97 (1H, d, J = 8.0 Hz), 7.05 (1H, t, J = 8.0 Hz), 7.16 (1H, d, J = 8.0 Hz). | | |
| 119 | | Reference Example 9 | Reference Example 69 |
| | 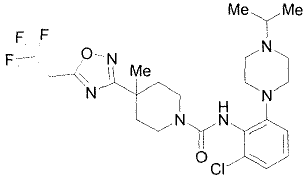 | 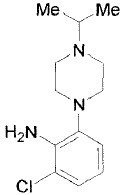 | 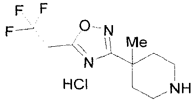 |
| | ¹H-NMR (CDCl₃) δ: 1.08 (6H, d, J = 6.0 Hz), 1.41 (3H, s), 1.72-1.91 (2H, m), 2.27-2.44 (2H, m), 2.58-2.83 (5H, m), 2.86-3.00 (4H, m), 3.22-3.42 (2H, m), 3.71-4.02 (4H, m), 6.33-6.47 (1H, m), 6.97-7.24 (3H, m). | | |
| 120 | | Reference Example 2 | Reference Example 63 |
| | 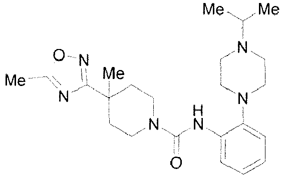 | 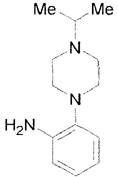 | 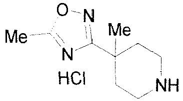 |
| | ¹H-NMR (CDCl₃) δ: 1.12 (6H, d, J = 6.4 Hz), 1.36 (3H, s), 1.49-1.66 (2H, m), 1.71 (2H, ddd, J = 14.0, 10.8, 4.0 Hz), 2.32 (2H, d, J = 14.0 Hz), 2.60 (3H, s), 2.62-2.82 (4H, m), 2.84-2.99 (3H, m), 3.22 (2H, ddd, J = 14.0, 10.8, 2.8 Hz), 3.87 (2H, dt, J = 13.6, 4.0 Hz), 6.94 (1H, dt, J = 8.0, 1.2 Hz), 7.08-7.20 (2H, m), 8.19 (1H, dd, J = 8.0, 1.2 Hz), 8.25 (1H, bs). | | |

Example 2: 4-(1,3-benzoxazol-2-yl)-N-[2-(4-ethylpiperazin-1-yl)phenyl]piperidine-1-carboxamide
Example 3: 4-(3,5-dimethoxyphenyl)-N-[2-(4-ethylpiperazin-1-yl)phenyl]piperidine-1-carboxamide
Example 4: N-[2-(4-ethylpiperazin-1-yl)phenyl]-4-methyl-4-phenylpiperidine-1-carboxamide hydrochloride
Example 5: 4-methyl-4-phenyl-N-{2-[4-(propan-2-yl)piperazin-1-yl]phenyl}piperidine-1-carboxamide
Example 6: 4-cyano-4-phenyl-N-{2-[4-(propan-2-yl)piperazin-1-yl]phenyl}piperidine-1-carboxamide
Example 7: 4-phenyl-N-{2-[4-(propan-2-yl)piperazin-1-yl]phenyl}piperidine-1-carboxamide
Example 8: N-{2-methoxy-6-[4-(propan-2-yl)piperazin-1-yl]phenyl}-4-(4-methylphenyl)piperidine-1-carboxamide
Example 9: N-{2-fluoro-6-[4-(propan-2-yl)piperazin-1-yl]phenyl}-4-(4-methylphenyl)piperidine-1-carboxamide
Example 10: N-{4-fluoro-2-[4-(propan-2-yl)piperazin-1-yl]phenyl}-4-(4-methylphenyl)piperidine-1-carboxamide
Example 11: N-{3-fluoro-2-[4-(propan-2-yl)piperazin-1-yl]phenyl}-4-(4-methylphenyl)piperidine-1-carboxamide
Example 12: 4-methyl-4-(3-phenyl-1,2,4-oxadiazol-5-yl)-N-{2-[4-(propan-2-yl)piperazin-1-yl]phenyl}piperidine-1-carboxamide
Example 13: 4-methyl-N-{2-[4-(propan-2-yl)piperazin-1-yl]phenyl}-4-[3-(pyridin-3-yl)-1,2,4-oxadiazol-5-yl]piperidine-1-carboxamide
Example 14: 6-(4-methylphenyl)-N-{2-[4-(propan-2-yl)piperazin-1-yl]phenyl}-3-azabicyclo[4.1.0]heptane-3-carboxamide
Example 15: N-{2-methoxy-6-[4-(propan-2-yl)piperazin-1-yl]phenyl}-4-[4-(trifluoromethyl)phenyl]piperidine-1-carboxamide
Example 16: N-{2-methoxy-6-[4-(propan-2-yl)piperazin-1-yl]phenyl}-4-[4-(trifluoromethoxy)phenyl]piperidine-1-carboxamide
Example 17: 4-(4-methylphenyl)-N-{2-methyl-6-[4-(propan-2-yl)piperazin-1-yl]phenyl}piperidine-1-carboxamide
Example 18: 4-(2-fluoro-4-methylphenyl)-N-{2-[4-(propan-2-yl)piperazin-1-yl]phenyl}piperidine-1-carboxamide
Example 19: 4-methyl-4-[5-(propan-2-yl)-1,2,4-oxadiazol-3-yl]-N-{2-[4-(propan-2-yl)piperazin-1-yl]phenyl}piperidine-1-carboxamide
Example 20: N-{2-fluoro-6-[4-(propan-2-yl)piperazin-1-yl]phenyl}-4-methyl-4-[5-(propan-2-yl)-1,2,4-oxadiazol-3-yl]piperidine-1-carboxamide
Example 21: N-{2-methoxy-6-[4-(propan-2-yl)piperazin-1-yl]phenyl}-4-methyl-4-[5-(propan-2-yl)-1,2,4-oxadiazol-3-yl]piperidine-1-carboxamide
Example 22: N-{2-fluoro-6-[4-(propan-2-yl)piperazin-1-yl]phenyl}-4-methyl-4-(5-methyl-1,2,4-oxadiazol-3-yl)piperidine-1-carboxamide
Example 23: N-{2-methoxy-6-[4-(propan-2-yl)piperazin-1-yl]phenyl}-4-methyl-4-(5-methyl-1,2,4-oxadiazol-3-yl)piperidine-1-carboxamide
Example 24: N-{2-fluoro-6-[4-(propan-2-yl)piperazin-1-yl]phenyl}-4-methyl-4-[5-(trifluoromethyl)-1,2,4-oxadiazol-3-yl]piperidine-1-carboxamide
Example 25: N-{2-methoxy-6-[4-(propan-2-yl)piperazin-1-yl]phenyl}-4-methyl-4-[5-(trifluoromethyl)-1,2,4-oxadiazol-3-yl]piperidine-1-carboxamide
Example 26: Rac-N-{2-fluoro-6-[4-(propan-2-yl)piperazin-1-yl]phenyl}-3-methyl-4-(4-methylphenyl)piperidine-1-carboxamide
Example 27: N-{2-fluoro-6-[4-(propan-2-yl)piperazin-1-yl]phenyl}-4-phenoxypiperidine-1-carboxamide
Example 28: Rac-4-(4-methylphenyl)-N-{2-[3-methyl-4-(propan-2-yl)piperazin-1-yl]phenyl}piperidine-1-carboxamide
Example 29: N-{2-fluoro-6-[4-(propan-2-yl)piperazin-1-yl]phenyl}-4-(3-methylphenyl)piperidine-1-carboxamide
Example 30: Rac-4-(4-methylphenyl)-N-{2-[2-methyl-4-(propan-2-yl)piperazin-1-yl]phenyl}piperidine-1-carboxamide
Example 31: 4-(4-methylphenyl)-N-{2-[1-(propan-2-yl)piperidin-4-yl]phenyl}piperidine-1-carboxamide hydrochloride
Example 32: 3-(3-cyclopropyl-1,2,4-oxadiazol-5-yl)-N-{2-fluoro-6-[4-(propan-2-yl)piperazin-1-yl]phenyl}-8-azabicyclo[3.2.1]octane-8-carboxamide
Example 33: N-{2-(dimethylcarbamoyl)-6-[4-(propan-2-yl)piperazin-1-yl]phenyl}-4-(4-methylphenyl)piperidine-1-carboxamide
Example 34: 4-(4-methylphenyl)-N-{2-[4-(propan-2-yl)piperazin-1-yl]-6-(trifluoromethyl)phenyl}piperidine-1-carboxamide
Example 35: N-{2-chloro-6-[4-(propan-2-yl)piperazin-1-yl]phenyl}-4-(4-methylphenyl)piperidine-1-carboxamide
Example 36: N-{2-fluoro-6-[4-(propan-2-yl)piperazin-1-yl]phenyl}-4-(pyridin-2-yl)piperidine-1-carboxamide
Example 37: N-{2-fluoro-6-[4-(propan-2-yl)piperazin-1-yl]phenyl}-4-(2-methylphenyl)piperidine-1-carboxamide
Example 38: methyl 2-{[4-(4-methylphenyl)piperidine-1-carbonyl]amino}-3-[4-(propan-2-yl)piperazin-1-yl]benzoate
Example 39: N-{2-fluoro-6-[1-(propan-2-yl)piperidin-4-yl]phenyl}-4-(4-methylphenyl)piperidine-1-carboxamide
Example 40: N-{2-fluoro-6-[4-(propan-2-yl)piperazin-1-yl]phenyl}-4-[4-(propan-2-yl)phenyl]piperidine-1-carboxamide
Example 41: N-{2-fluoro-6-[4-(propan-2-yl)piperazin-1-yl]phenyl}-3-(4-methylphenyl)-8-azabicyclo[3.2.1]octane-8-carboxamide
Example 42: N-{2-fluoro-6-[1-(propan-2-yl)-1,2,3,6-tetrahydropyridin-4-yl]phenyl}-4-(4-methylphenyl)piperidine-1-carboxamide
Example 43: 4-(5-cyclopropyl-1,2,4-oxadiazol-3-yl)-N-{2-fluoro-6-[1-(propan-2-yl)piperidin-4-yl]phenyl}-4-methylpiperidine-1-carboxamide
Example 44: 4-(5-cyclopropyl-1,2,4-oxadiazol-3-yl)-N-{2-fluoro-6-[1-(propan-2-yl)-1,2,3,6-tetrahydropyridin-4-yl]phenyl}-4-methylpiperidine-1-carboxamide
Example 45: 4-(5-cyclopropyl-1,2,4-oxadiazol-3-yl)-N-{2-fluoro-6-[4-(propan-2-yl)piperazin-1-yl]phenyl}-4-methylpiperidine-1-carboxamide
Example 46: N-{2-fluoro-6-[4-(propan-2-yl)piperazin-1-yl]phenyl}-4-(5-methylpyridin-2-yl)piperidine-1-carboxamide
Example 47: N-{2-methoxy-6-[1-(propan-2-yl)-1,2,3,6-tetrahydropyridin-4-yl]phenyl}-4-(4-methylphenyl)piperidine-1-carboxamide
Example 48: N-{2-methoxy-6-[1-(propan-2-yl)piperidin-4-yl]phenyl}-4-(4-methylphenyl)piperidine-1-carboxamide
Example 49: 4-(1,3-benzoxazol-2-yl)-N-{2-fluoro-6-[1-(propan-2-yl)piperidin-4-yl]phenyl}-4-methylpiperidine-1-carboxamide
Example 50: 4-(1,3-benzoxazol-2-yl)-N-{2-fluoro-6-[1-(propan-2-yl)-1,2,3,6-tetrahydropyridin-4-yl]phenyl}-4-methylpiperidine-1-carboxamide
Example 51: 4-(5-cyclopropyl-1,2-oxazol-3-yl)-N-{2-fluoro-6-[1-(propan-2-yl)piperidin-4-yl]phenyl}piperidine-1-carboxamide
Example 52: 4-(5-cyclopropyl-1,2-oxazol-3-yl)-N-{2-fluoro-6-[4-(propan-2-yl)piperazin-1-yl]phenyl}piperidine-1-carboxamide
Example 53: 4-(5-cyclopropyl-1,2-oxazol-3-yl)-N-{2-fluoro-6-[1-(propan-2-yl)piperidin-4-yl]phenyl}-4-methylpiperidine-1-carboxamide
Example 54: 4-(5-cyclopropyl-1,2-oxazol-3-yl)-N-{2-fluoro-6-[4-(propan-2-yl)piperazin-1-yl]phenyl}-4-methylpiperidine-1-carboxamide
Example 55: 4-(5-cyclopropyl-1,2-oxazol-3-yl)-N-{2-fluoro-6-[1-(propan-2-yl)-1,2,3,6-tetrahydropyridin-4-yl]phenyl}-4-methylpiperidine-1-carboxamide
Example 56: 4-(5-cyclopropyl-1,2,4-oxadiazol-3-yl)-N-[2-(3,6-dihydro-2H-pyran-4-yl)-6-fluorophenyl]-4-methylpiperidine-1-carboxamide
Example 57: 4-(5-cyclopropyl-1,2,4-oxadiazol-3-yl)-N-{2-fluoro-6-[4-(propan-2-yl)piperazin-1-yl]phenyl}-4-methylpiperidine-1-carbothioamide
Example 58: N-[2-(1-cyclopropyl-1H-pyrazol-4-yl)-6-fluoro-phenyl]-4-(4-methylphenyl)piperidine-1-carboxamide
Example 59: 4-(5-cyclopropyl-1,2-oxazol-3-yl)-N-{2-fluoro-6-[6-(propan-2-yl)pyridin-3-yl]phenyl}-4-methylpiperidine-1-carboxamide
Example 60: 4-(5-cyclopropyl-1,2,4-oxadiazol-3-yl)-N-{2-fluoro-6-[6-(propan-2-yl)pyridin-3-yl]phenyl}-4-methylpiperidine-1-carboxamide
Example 61: N-{2-chloro-6-[4-(propan-2-yl)piperazin-1-yl]phenyl}-4-(5-cyclopropyl-1,2-oxazol-3-yl)-4-methylpiperidine-1-carboxamide
Example 62: N-(2-fluoro-6-{[1-(propan-2-yl)piperidin-4-yl]oxy}phenyl)-4-(4-methylphenyl)piperidine-1-carboxamide
Example 63: N-(2-methoxy-6-{[4-(propan-2-yl)piperazin-1-yl]methyl}phenyl)-4-(4-methylphenyl)piperidine-1-carboxamide
Example 64: N-{2-fluoro-6-[4-(propan-2-yl)piperazin-1-yl]phenyl}-4-(4-methylphenyl)piperidine-1-carbothioamide
Example 65: 4-(5-cyclopropyl-1,2,4-oxadiazol-3-yl)-N-[3-fluoro-4'-(propan-2-yl)[1,1'-biphenyl]-2-yl]-4-methylpiperidine-1-carboxamide
Example 66: 4-(1,3-benzoxazol-2-yl)-N-{2-chloro-6-[4-(propan-2-yl)piperazin-1-yl]phenyl}-4-methylpiperidine-1-carboxamide
Example 67: N-{2-chloro-6-[4-(propan-2-yl)piperazin-1-yl]phenyl}-4-(5-cyclopropyl-1,2,4-oxadiazol-3-yl)-4-methylpiperidine-1-carboxamide
Example 68: 4-(5-cyclopropyl-1,2-oxazol-3-yl)-N-[2-fluoro-6-(6-methylpyridin-3-yl)phenyl]-4-methylpiperidine-1-carboxamide
Example 69: N-{2-chloro-6-[6-(propan-2-yl)pyridin-3-yl]phenyl}-4-(5-cyclopropyl-1,2-oxazol-3-yl)-4-methylpiperidine-1-carboxamide
Example 70: 4-(5-cyclopropyl-1,2-oxazol-3-yl)-N-[2-fluoro-6-(5-methylpiperidin-3-yl)phenyl]-4-methylpiperidine-1-carboxamide
Example 71: 4-(5-cyclopropyl-1,2-oxazol-3-yl)-N-{2-fluoro-6-[6-(trifluoromethyl)pyridin-3-yl]phenyl}-4-mehylpiperidine-1-carboxamidehydrochloride
Example 72: 4-(5-cyclopropyl-1,2,4-oxadiazol-3-yl)-N-{2-fluoro-6-[2-(propan-2-yl)pyrimidin-5-yl]phenyl}-4-methylpiperidine-1-carboxamide
Example 73: 4-(5-cyclopentyl-1,2,4-oxadiazol-3-yl)-N-{2-fluoro-6-[4-(propan-2-yl)piperaz-in-1-yl]phenyl}-4-methylpiperidine-1-carboxamide
Example 74: N-{2-chloro-6-[4-(propan-2-yl)piperazin-1-yl]phenyl}-4-(5-cyclopentyl-1,2,4-oxadiazol-3-yl)-4-methylpiperidine-1-carboxamide
Example 75: 4-[5-(3,3-difluorocyclobutyl)-1,2,4-oxadiazol-3-yl]-N-{2-fluoro-6-[4-(propan-2-yl)piperazin-1-yl]phenyl}-4-methylpiperidine-1-carboxamide
Example 76: N-{2-chloro-6-[4-(propan-2-yl)piperazin-1-yl]phenyl}-4-[5-(3,3-difluorocyclobutyl)-1,2,4-oxadiazol-3-yl]-4-methylpiperidine-1-carboxamide
Example 77: N-{2-chloro-6-[4-(propan-2-yl)piperazin-1-yl]phenyl}-4-methyl-4-(4-methylphenyl)piperidine-1-carboxamide
Example 78: N-{2-chloro-6-[4-(propan-2-yl)piperazin-1-yl]phenyl}-4-(5-cyclobutyl-1,2,4-oxadiazol-3-yl)-4-methylpiperidine-1-carboxamide
Example 79: N-{2-fluoro-6-[4-(propan-2-yl)piperazin-1-yl]phenyl}-4-methyl-4-[5-(1-methylcyclopropyl)-1,2,4-oxadiazol-3-yl]piperidine-1-carboxamide
Example 80: N-{2-chloro-6-[4-(propan-2-yl)piperazin-1-yl]phenyl}-4-methyl-4-[5-(1-methylcyclopropyl)-1,2,4-oxadiazol-3-yl]piperidine-1-carboxamide
Example 81: N-{2-chloro-6-[4-(propan-2-yl)piperazin-1-yl]phenyl}-4-methyl-4-(5-methyl-1,2,4-oxadiazol-3-yl)piperidine-1-carboxamide
Example 82: 4-(5-ethyl-1,2,4-oxadiazol-3-yl)-N-{2-fluoro-6-[4-(propan-2-yl)piperazin-1-yl]phenyl}-4-methylpiperidine-1-carboxamide
Example 83: N-{2-chloro-6-[4-(propan-2-yl)piperazin-1-yl]phenyl}-4-(5-ethyl-1,2,4-oxadiazol-3-yl)-4-methylpiperidine-1-carboxamide
Example 84: N-{2-chloro-6-[4-(propan-2-yl)piperazin-1-yl]phenyl}-4-methyl-4-(5-methyl-1,2-oxazol-3-yl)piperidine-1-carboxamide
Example 85: N-{2-fluoro-6-[4-(propan-2-yl)piperazin-1-yl]phenyl}-4-methyl-4-(5-methyl-1,2-oxazol-3-yl)piperidine-1-carboxamide
Example 86: N-.{2-chloro-6-[4-(propan-2-yl)piperazin-1-yl]phenyl}-4-methyl-4-[5-(trifluoromethyl)-1,2,4-oxadiazol-3-yl]piperidine-1-carboxamide
Example 87: 4-(5-cyclopropyl-1,2,4-oxadiazol-3-yl)-4-ethyl-N-{2-fluoro-6-[4-(propan-2-yl)piperazin-1-yl]phenyl}piperidine-1-carboxamide
Example 88: N-{2-bromo-6-[4-(propan-2-yl)piperazin-1-yl]phenyl}-4-(5-cyclopropyl-1,2,4-oxadiazol-3-yl)-4-methylpiperidine-1-carboxamide
Example 89: N-{2-chloro-6-[4-(propan-2-yl)piperazin-1-yl]phenyl}-4-(5-cyclopropyl-1,2,4-oxadiazol-3-yl)-4-ethylpiperidine-1-carboxamide
Example 90: 4-(4-chloro-5-methyl-1,2-oxazol-3-yl)-N-{2-chloro-6-[4-(propan-2-yl)piperazin-1-yl]phenyl}-4-methylpiperidine-1-carboxamide
Example 91: 4-cyclopentyl-N-{2-fluoro-6-[4-(propan-2-yl)piperazin-1-yl]phenyl}-4-methylpiperidine-1-carboxamide
Example 92: N-{2-chloro-6-[4-(propan-2-yl)piperazin-1-yl]phenyl}-4-methyl-4-(2-methyl-1,3-oxazol-5-yl)piperidine-1-carboxamide
Example 93: N-{2-chloro-6-[4-(propan-2-yl)piperazin-1-yl]phenyl}-4-methyl-4-[2-(propan-2-yl)-1,3-oxazol-5-yl]piperidine-1-carboxamide
Example 94: N-{2-chloro-6-[4-(propan-2-yl)piperazin-1-yl]phenyl}-4-(5-cyclopropyl-1,2,4-oxadiazol-3-yl)-4-fluoropiperidine-1-carboxamide
Example 95: N-{2-chloro-6-[4-(propan-2-yl)piperazin-1-yl]phenyl}-4-(5-ethyl-1,2,4-oxadiazol-3-yl)-4-fluoropiperidine-1-carboxamide
Example 96: N-{2-chloro-6-[4-(propan-2-yl)piperazin-1-yl]phenyl}-4-(2-cyclopropyl-1,3-thiazol-4-yl)-4-methylpiperidine-1-carboxamide
Example 97: N-{2-chloro-6-[4-(propan-2-yl)piperazin-1-yl]phenyl}-1,2,4,5-tetrahydro-3H-3-benzazepine-3-carboxamide
Example 98: N-{2-chloro-6-[4-(propan-2-yl)piperazin-1-yl]phenyl}-4-(5-methylpyrimidin-2-yl)piperidine-1-carboxamide
Example 99: N-{2-chloro-6-[4-(propan-2-yl)piperazin-1-yl]phenyl}-4-(4-methylphenyl)-1,4-diazepane-1-carboxamide
Example 100: N-[2-(4-tert-butylpiperazin-1-yl)-6-chlorophenyl]-4-(5-cyclopropyl-1,2,4-oxadiazol-3-yl)-4-methylpiperidine-1-carboxamide
Example 101: N-{2-chloro-6-[4-(propan-2-yl)piperazin-1-yl]phenyl}-4-(5-cyclopropyl-1,3-thiazol-2-yl)-4-methylpiperidine-1-carboxamide
Example 102: N-{2-chloro-6-[4-(propan-2-yl)piperazin-1-yl]phenyl}-4-(5-cyclopropyl-1,3,4-thiadiazol-2-yl)-4-methylpiperidine-1-carboxamide
Example 103: 4-(4-chloro-5-cyclopropyl-1,2-oxazol-3-yl)-N-{2-chloro-6-[4-(propan-2-yl)piperazin-1-yl]phenyl}-4-methylpiperidine-1-carboxamide
Example 104: Rac-N-{2-chloro-6-[4-(propan-2-yl)piperazin-1-yl]phenyl}-4-methyl-4-{5-[(1R,2S)-2-methylcyclopropyl]-1,2,4-oxadiazol-3-yl}piperidine-1-carboxamide
Example 105: N-{2-chloro-6-[4-(propan-2-yl)piperazin-1-yl]phenyl}-4-[5-(cyclopropylmethyl)-1,2,4-oxadiazol-3-yl]-4-methylpiperidine-1-carboxamide
Example 106: N-{2-chloro-6-[4-(propan-2-yl)piperazin-1-yl]phenyl}-4-[5-(2,2-dimethylcyclopropyl)-1,2,4-oxadiazol-3-yl]-4-methylpiperidine-1-carboxamide
Example 107: N-{2-chloro-6-[4-(propan-2-yl)piperazin-1-yl]phenyl}-4-methyl-4-{5-[1-(trifluoromethyl)cyclopropyl]-1,2,4-oxadiazol-3-yl}piperidine-1-carboxamide
Example 108: N-{2-chloro-6-[4-(propan-2-yl)piperazin-1-yl)phenyl}-4-[5-(1-methoxycyclopropyl)-1,2,4-oxadiazol-3-yl]-4-methylpiperidine-1-carboxamide
Example 109: N-{2-chloro-6-[4-(propan-2-yl)piperazin-1-yl]phenyl}-4-[5-(1-fluorocyclopropyl)-1,2,4-oxadiazol-3-yl]-4-methylpiperidine-1-carboxamide
Example 110: Rac-N-(2-chloro-6-{[1-(propan-2-yl)pyrrolidin-3-yl]oxy}phenyl)-4-(5-cyclopropyl-1,2,4-oxadiazol-3-yl)-4-methylpiperidine-1-carboxamide
Example 111: Rac-N-{2-chloro-6-[4-(propan-2-yl)piperazin-1-yl]phenyl}-4-{5-[(1S,2S)-2-fluorocyclopropyl]-1,2,4-oxadiazol-3-yl}-4-methylpiperidine-1-carboxamide
Example 112: Rac-N-{2-chloro-6-[4-(propan-2-yl)piperazin-1-yl]phenyl}-4-{5-[(1S,2R)-2-fluorocyclopropyl]-1,2,4-oxadiazol-3-yl}-4-methylpiperidine-1-carboxamide
Example 113: N-[2-chloro-6-(4-cyclopropylpiperazin-1-yl)phenyl]-4-(5-cyclopropyl-1,2,4-oxadiazol-3-yl)-4-methylpiperidine-1-carboxamide
Example 114: N-{2-chloro-6-[4-(propan-2-yl)piperazin-1-yl]phenyl}-4-[5-(2,2-difluorocyclopropyl)-1,2,4-oxadiazol-3-yl]-4-methylpiperidine-1-carboxamide
Example 115: N-{2-chloro-6-[4-(propan-2-yl)piperazin-1-yl]phenyl}-4-{5-[(1S,2S)-2-fluorocyclopropyl]-1,2,4-oxadiazol-3-yl}-4-methylpiperidine-1-carboxamide
Example 116: N-{2-chloro-6-[4-(propan-2-yl)piperazin-1-yl]phenyl}-4-{5-[(1R,2R)-2-fluorocyclopropyl]-1,2,4-oxadiazol-3-yl}-4-methylpiperidine-1-carboxamide
Example 117: N-{2-chloro-6-[4-(propan-2-yl)piperazin-1-yl]phenyl}-4-methyl-4-{5-[(1S,2R)-2-methylcyclopropyl]-1,2,4-oxadiazol-3-yl}piperidine-1-carboxamide
Example 118: N-{2-chloro-6-[4-(propan-2-yl)piperazin-1-yl]phenyl}-4-methyl-4-{5-[(1R,2S)-2-methylcyclopropyl]-1,2,4-oxadiazol-3-yl}piperidine-1-carboxamide
Example 119: N-{2-chloro-6-[4-(propan-2-yl)piperazin-1-yl]phenyl}-4-methyl-4-[5-(2,2,2-trifluoroethyl)-1,2,4-oxadiazol-3-yl]piperidine-1-carboxamide
Example 120: 4-methyl-4-(5-methyl-1,2,4-oxadiazol-3-yl)-N-{2-[4-(propan-2-yl)piperazin-1-yl]phenyl}piperidine-1-carboxamide

### Example 121: 4-(4-Methylphenyl)-N-[2-(4-methylpiperazin-1-yl)phenyl]piperidine-1-carboxamide

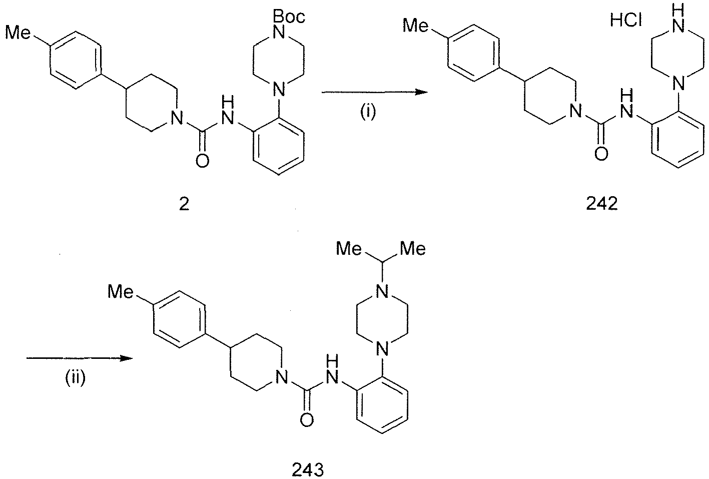

### Step (i):

The crude product of the title compound 242 was prepared from compound 2 (0.152 g) in the same manner as Step (ii) in Reference Example 20.
LCMS: [M+H]⁺/Rt(min): 208/0.30

### Step (ii):

The title compound 243 (0.202 g) was prepared from the crude product of compound 242 in the same manner as Step (iii) in Reference Example 20.
¹H-NMR (CDCl₃) 5: 1.21 (6H, d, J = 6.1 Hz), 1.62-1.71 (2H, m), 1.84 (2H, m), 2.26 (3H, s), 2.55-2.69 (6H, m), 2.85 (4H, m), 2.95 (2H, m), 4.22 (2H, m), 6.88 (1H, m), 7.03-7.11 (6H, m), 8.14 (1H, m), 8.25 (1H, brs)

### Examples 122-160:

The compounds of Examples 122-160 shown in the table below were prepared in the same manner as Example 121 by using each corresponding commerial compound or compound of Reference Examples.

**[Table 8]**

| Example | Structural Formula | Material |
|---|---|---|
| | Spectral Data | |
| 122 | | Reference Example 1 |
| | 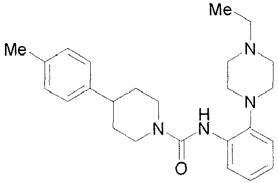 | 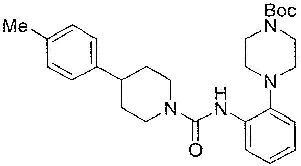 |
| | ¹H-NMR (CDCl₃) δ: 1.16 (3H, t, J = 6.6 Hz), 1.58 (1H, brs), 1.69 (1H, dd, J = 12.4, 4.2 Hz), 1.76 (1H, dd, J = 12.4, 4.2 Hz), 1.93 (1H, dd, J = 12.4, 1.6 Hz), 2.33 (3H, s), 2.49-2.52 (3H, m), 2.69-2.76 (3H, m), 2.97-3.06 (6H, m), 4.28 (2H, d, J = 13.2 Hz), 6.96 (1H, dt, J = 8.0, 1.6 Hz), 7.19-7.10 (6H, m), 8.22 (2H, dd, J = 8.2, 1.4 Hz) | |
| 123 | | Reference Example 1 |
| | 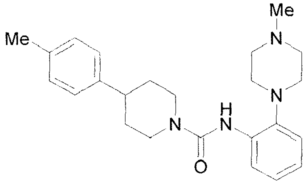 | 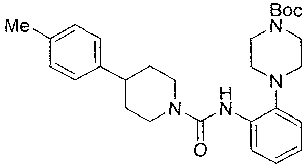 |
| | ¹H-NMR (CDCl₃) δ: 1.01 (6H, d, ), 1.64-1.68 (2H, m), 1.86 (2H, m), 2.26 (3H, s), 2.27 (3H, s), 2.49 (2H, m), 2.66 (1H, m), 2.84 (4H, m), 2.95 (2H, m), 4.21 (2H, m), 6.88 (1H, m), 7.04-7.10 (6H, m), 8.16 (2H, m) | |
| 124 | | Reference Example 1 |
| | 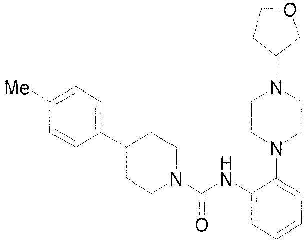 | 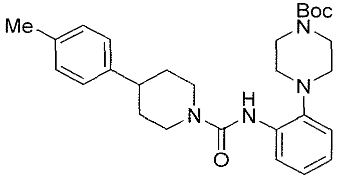 |
| | ¹H-NMR (CDCl₃) δ: 1.72 (2H, m), 1.91 (3H, m), 2.05 (1H, m), 2.31 (3H, s), 2.51-2.73 (4H, m), 2.89-3.05 (5H, m), 3.65 (1H, m), 3.78 (1H, m), 3.86-3.95 (2H, m), 4.25 (2H, m), 6.96 (1H, m), 7.08-7.15 (6H, m), 8.17 (1H, m), 8.24 (1H, brs | |
| 125 | | Reference Example 1 |
| | 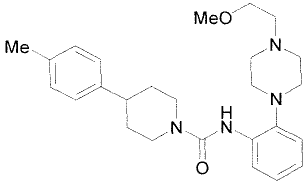 | 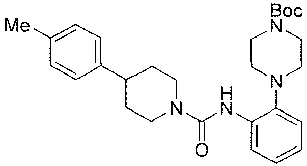 |
| | ¹H-NMR (CDCl₃) δ: 1.54-1.68 (2H, m), 1.85 (2H, m), 2.26 (3H, s), 2.53-2.66 (4H, m), 2.86 (4H, m), 2.94 (2H, m), 3.29 (3H, s), 3.46 (2H, m), 4.20 (2H, m), 6.87 (1H, m), 7.03-7.09 (6H, m), 8.15 (1H, m), 8.21 (1H, brs) | |
| 126 | | Reference Example 1 |
| | 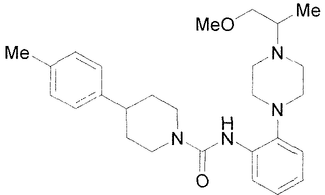 | 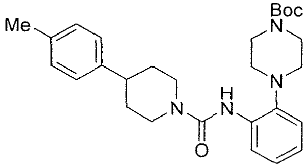 |
| | ¹H-NMR (CDCl₃) δ: 0.98 (3H, J = 6.7 Hz), 1.60-1.71 (2H, m), 1.85 (2H, m), 2.26 (3H, s), 2.60-2.85 (9H, m), 2.95 (2H, m), 3.24-3.28 (4H, m), 3.40 (1H, m), 4.22 (2H, m), 6.88 (1H, m), 7.03-7.09 (6H, m), 8.14 (1H, m), 8.26 (1H, brs) | |
| 127 | | Reference Example 23 |
| | 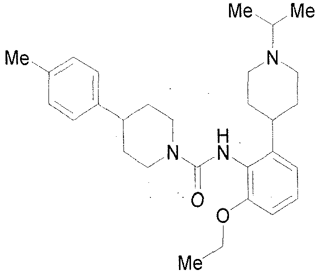 | 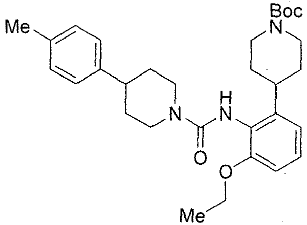 |
| | ¹H-NMR (CDCl₃) δ: 1.09 (6H, d, J = 6.7 Hz), 1.38 (3H, t, J = 7.0 Hz), 1. 70-1.92 (8H, m), 2.20-2.32 (5H, m), 2.69-2.82 (3H, m), 3.00 (4H, m), 4.00 (2H, q, J = 7.0 Hz), 4.24 (2H, m), 6.00 (1H, s), 6.69 (1H, m), 6.96 (1H, m), 7.09-7.13 (5H, m) | |
| 128 | | Reference Example 23 |
| | 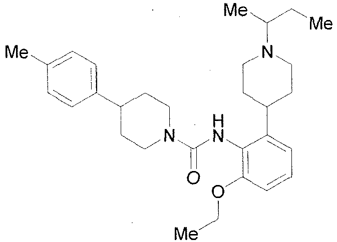 | 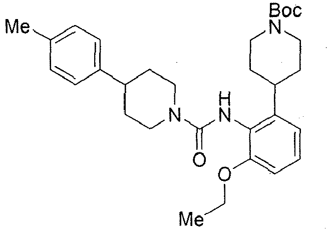 |
| | ¹H-NMR (CDCl₃) δ: 0.94 (3H, m), 1.37 (5H, m), 1.56 (5H, m), 1.68-1.77 (3H, m), 1.85-1.99 (5H, m), 2.31 (3H, s), 2.68-2.92 (3H, m), 3.01 (4H, m), 3.99 (2H, q, J = 6.9 Hz), 4.23 (2H, m), 6.05 (1H, brs), 6.70 (1H, m), 7.05-7.13 (6H, m) | |
| 129 | | Reference Example 24 |
| | 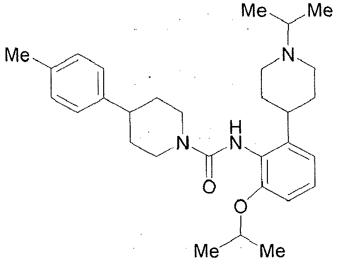 | 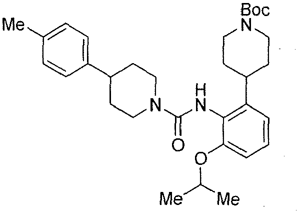 |
| | ¹H-NMR (CDCl₃) δ: 1.09 (6H, d, J = 6.7 Hz), 1.25 (6H, d, J = 6.1 Hz), 1.61-1.85 (8H, m), 2.15-2.28 (5H, m), 2.62-2.79 (3H, m), 2.95 (4H, m), 4.18 (2H, m), 4.44 (1H, m), 5.98 (1H, s), 6.66 (1H, m), 6.90 (1H, m), 7.02-7.07 (5H, m) | |
| 130 | | Reference Example 26 |
| | 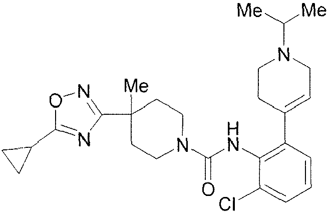 | 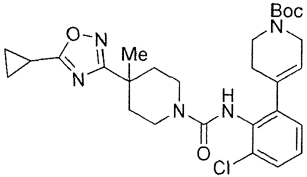 |
| | ¹H-NMR (CDCl₃) δ: 1.10 (6H, d, J = 6.8 Hz), 1.17-1.29 (3H, m), 1.34 (3H, s), 1.56-1.84 (6H, m), 2.14-2.23 (1H, m), 2.27 (2H, d, J = 14.0 Hz), 2.37-2.49 (2H, m), 2.63-2.74 (2H, m), 2.74-2.87 (1H, m), 3.12-3.27 (4H, m), 3.80 (2H, dt, J = 14.0, 4.0 Hz), 5.65-5.73 (1H, m), 6.04 (1H, bs), 7.04-7.13 (2H, m), 7.29 (1H, dd, J = 7.6, 2.4 Hz). | |
| 131 | | Reference Example 26 |
| | 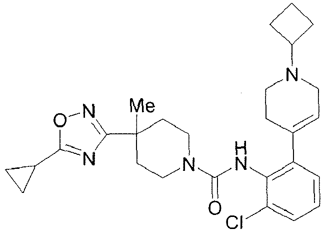 | 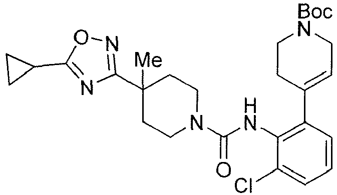 |
| | ¹H-NMR (CDCl₃) δ: 1.15-1.38 (5H, m), 1.34 (3H, s), 1.53-2.00 (5H, m), 2.01-2.12 (2H, m), 2.12-2.35 (3H, m), 2.37-2.46 (2H, m), 2.46-2.56 (2H, m), 2.76-2.88 (1H, m), 2.92-3.03 (2H, m), 3.19 (2H, ddd, J = 14.0, 10.8, 2.8 Hz), 3.79 (2H, dt, J = 14.0, 4.4 Hz), 5.64-5.72 (1H, m), 6.03 (1H, bs), 7.04-7.15 (2H, m), 7.29 (1H, dd, J = 7.2, 2.8 Hz) | |
| 132 | | Reference Example 28 |
| | 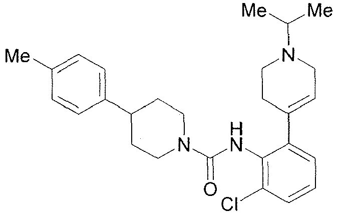 | 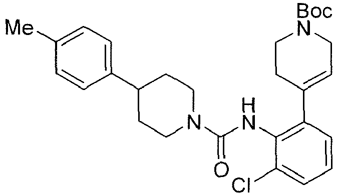 |
| | ¹H-NMR (CDCl₃) δ: 1.12 (6H, d, J = 6.1 Hz), 1.66-1.76 (2H, m), 1.86 (2H, m), 2.31 (3H, s), 2.50 (2H, m), 2.66-2.85 (4H, m), 2.98 (2H, m), 3.24 (2H, m), 4.22 (2H, m), 5.71 (1H, m), 6.15 (1H, brs), 7.04-7.13 (6H, m), 7.28-7.31 (1H, m) | |
| 133 | | Reference Example 31 |
| | 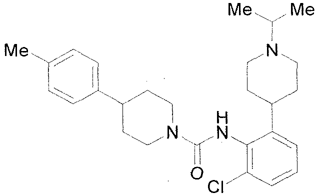 | 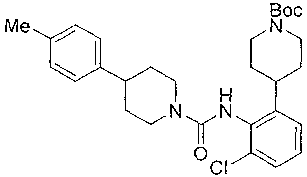 |
| | ¹H-NMR (CDCl₃) δ: 1.09 (6H, d, J = 6.1 Hz), 1.70-1.91 (8H, m), 2.27 (2H, m), 2.32 (3H, s), 2.69-2.79 (3H, m), 3.03 (4H, m), 4.22 (2H, m), 6.04 (1H, s), 7.01-7.17 (5H, m), 7.23-7.29 (2H, m) | |
| 134 | | Reference Example 30 |
| | 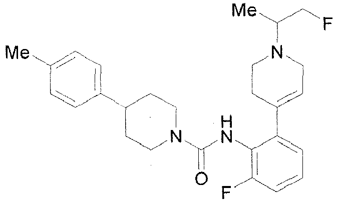 | 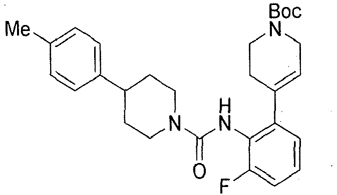 |
| | ¹H-NMR (CDCl₃) δ: 1.14 (3H, d, J = 6.7 Hz), 1.64-1.75 (3H, m), 1.86 (3H, m), 2.31 (3H, s), 2.46 (2H, m), 2.67 (1H, m), 2.84 (2H, m), 2.97 (3H, m), 3.34 (2H, m), 4.45 (1H, m), 4.56 (1H, m), 5.71 (1H, m), 6.00 (1H, brs), 6.93-7.13 (7H, m) | |
| 135 | | Reference Example 30 |
| | 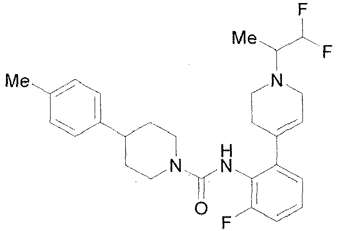 | 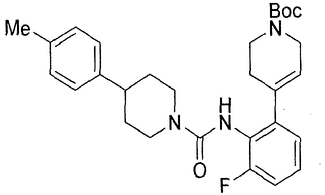 |
| | ¹H-NMR (CDCl₃) δ: 1.17 (3H, d, J = 6.7 Hz), 1.64-1.75 (3H, m), 1.87 (2H, m), 2.31 (3H, s), 2.42 (2H, m), 2.69 (1H, m), 2.85-3.02 (5H, m), 3.34 (2H, m), 4.21 (2H, m), 5.70 (1H, m), 5.89 (1H, brs), 6.92-7.13 (7H, m) | |
| 136 | | Reference Example 30 |
| | 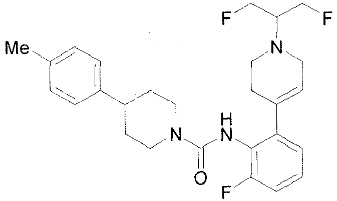 | 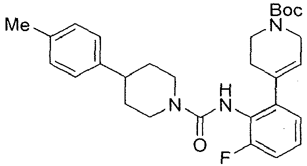 |
| | ¹H-NMR (CDCl₃) δ: 1. 64-1. 74 (3H, m), 1.87 (2H, m), 2.31 (3H, s), 2.44 (2H, m), 2.69 (1H, m), 2.90-3.01 (4H, m), 3.43 (2H, m), 4.20 (2H, m), 4.61-4.73 (4H, m), 5.70 (1H, m), 5.90 (1H, brs), 6.92-7.13 (7H, m) | |
| 137 | | Reference Example 31 |
| | 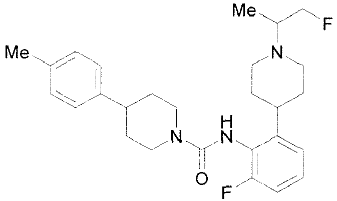 | 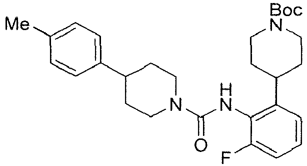 |
| | ¹H-NMR (CDCl₃) δ: 1.14 (3H, m), 1.68-1.79 (4H, m), 1.89 (4H, m), 2.32 (3H, s), 2.47 (2H, m), 2.69-2.75 (3H, m), 3.04 (4H, m), 4.20 (2H, m), 4.44 (1H, m), 4.56 (1H, m), 5.84 (1H, brs), 6.91-6.97 (1H, m), 7.10-7.19 (6H, m) | |
| 138 | | Reference Example 31 |
| | 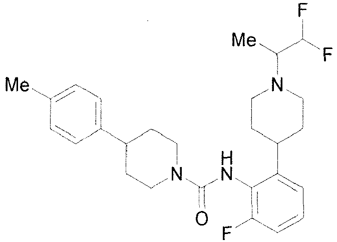 | 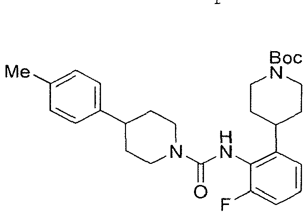 |
| | ¹H-NMR (CDCl₃) δ: 1.07 (3H, m), 1.64-1.87 (8H, m), 2.27 (3H, s), 2.46 (2H, m), 2.67 (3H, m), 2.88-3.01 (5H, m), 4.15 (2H, m), 5.76 (1H, brs), 6.89 (1H, m), 7.02-7.15 (6H, m) | |
| 139 | | Reference Example 34 |
| | 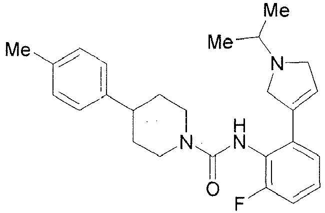 | 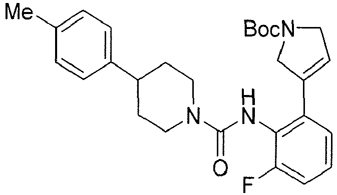 |
| | ¹H-NMR (CDCl₃) δ: 1.31 (6H, d, J = 6.1 Hz), 1.64-1.75 (3H, m), 1.89 (2H, m), 2.31 (3H, s), 2.69 (1H, m), 3.02 (3H, m), 3.90 (2H, m), 4.05 (2H, m), 4.26 (1H, m), 5.98 (1H, m), 6.35 (1H, brs), 6.98-7.15 (7H, m) | |
| 140 | | Reference Example 34 |
| | 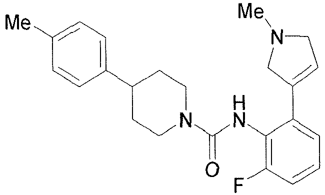 | 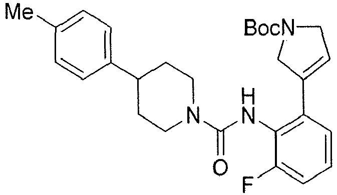 |
| | ¹H-NMR (CDCl₃) δ: 1.63-1.75 (2H, m), 1.89 (2H, m), 2.31 (3H, s), 2.63 (3H, s), 2.69 (1H, m), 2.99 (2H, m), 3.72-3.93 (4H, m), 4.22 (2H, m), 5.98 (1H, m), 6.06 (1H, brs), 6.99-7.13 (7H, m) | |
| 141 | | Reference Example 27 |
| | 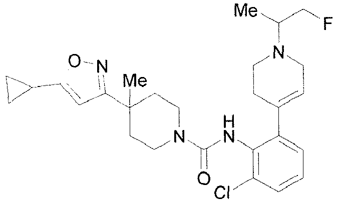 | 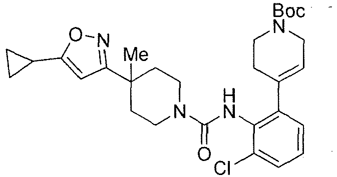 |
| | ¹H-NMR (CDCl₃) δ: 0.92-1. 00 (2H, m), 1.00-1.09 (2H, m), 1.13 (3H, d, J = 6.7 Hz), 1.29 (3H, s), 1.61-1.73 (2H, m), 1.97-2.06 (1H, m), 2.06-2.18 (2H, m), 2.37-2.48 (2H, m), 2.72-2.83 (2H, m), 2.93-3.07 (1H, m), 3.20-3.35 (4H, m), 3.69-3.82 (2H, m), 4.33-4.62 (2H, m), 5.67 (1H, s), 5.79 (1H, s), 6.05 (1H, s), 7.04-7.11 (2H, m), 7.29 (1H, dd, J = 6.7, 2.4 Hz). | |
| 142 | | Reference Example 26 |
| | 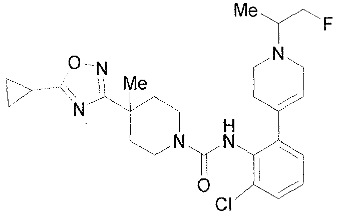 | 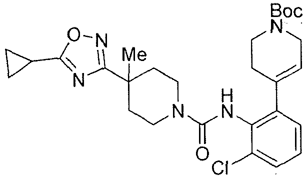 |
| | ¹H-NMR (CDCl₃) δ: 1.16-1.24 (7H, m), 1.32 (3H, s), 1.63-1.70 (3H, m), 2.14-2.26 (3H, m), 2.46 (2H, m), 2.83 (2H, m), 3.20 (3H, m), 3.33 (1H, m), 3.79 (2H, m), 4.46 (1H, m), 4.58 (1H, m), 5.66 (1H, m), 6.09 (1H, brs), 7.03-7.08 (2H, m), 7.27-7.29 (1H, m) | |
| 143 | | Reference Example 43 |
| | 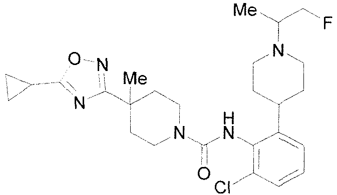 | 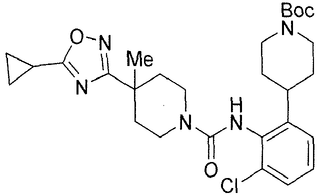 |
| | ¹H-NMR (CDCl₃) δ: 1.02-1.25 (7H, m), 1.36 (3H, s), 1,43-1.93 (4H, m), 1.72 (2H, ddd, J = 13.6, 11.2, 4.4 Hz), 2.20 (1H, tt, J = 7.6, 3.6 Hz), 2.25-2.35 (2H, m), 2.35-2.55 (2H, m), 2,69-3.13 (4H, m), 3.18-3.29 (2H, m), 3.83 (2H, ddd, J = 14.0, 4.4, 3.6), 4.24-4.65 (2H, m), 5.92-6.07 (1H, brs), 7.11-7.19 (1H, m), 7.20-7.30 (2H, m). | |
| 144 | | Reference Example 27 |
| | 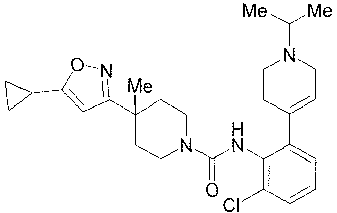 | 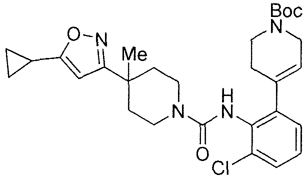 |
| | ¹H-NMR (CDCl₃) δ: 0.88-1.01 (4H, m), 1.06 (6H, d, J = 6.1 Hz), 1.22 (3H, s), 1.58-1.65 (2H, m), 1.91-2.07 (3H, m), 2.41 (2H, m), 2.66 (2H, m), 2.79 (1H, m), 3.13-3.24 (4H, m), 3.70 (2H, m), 5.62 (1H, m), 5.72 (1H, s), 6.07 (1H, brs), 6.98-7.03 (2H, m), 7.21-7.24 (1H, m) | |
| 145 | | Reference Example 43 |
| | 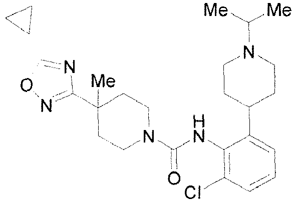 | 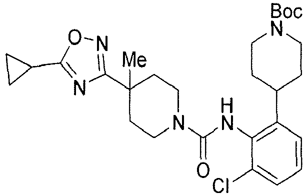 |
| | ¹H-NMR (CDCl₃) δ: 0.99-1.42 (10H, m), 1.36 (3H, s), 1.43-1.80 (5H, m), 1.82-2.07 (3H, m), 2.13-2.55 (4H, m), 2.72-3.33 (5H, m), 3.83 (2H, d, J = 14.0 Hz), 6.03 (1H, bs), 7.10-7.21 (1H, m), 7.21-7.29 (1H, m), 7.29-7.41 (1H, m). | |
| 146 | | Reference Example 43 |
| | 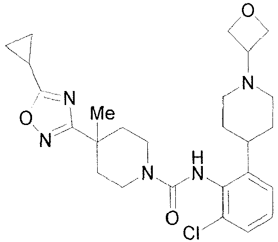 | 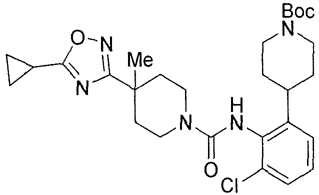 |
| | ¹H-NMR (CDCl₃) δ: 1.17-1.28 (5H, m), 1.37 (3H, s), 1.65-1.77 (3H, m), 1.79-2.09 (4H, m), 2.11-2.36 (4H, m), 3.17-3.35 (2H, m), 3.73-3.87 (2H, m), 4.52-4.61 (2H, m), 4.63-4.74 (2H, m), 4.78-4.85 (2H, m), 4.85-4.94 (1H, m), 6.04 (1H, bs), 7.14-7.22 (1H, m), 7.23-7.36 (2H, m). | |
| 147 | | Reference Example 28 |
| | 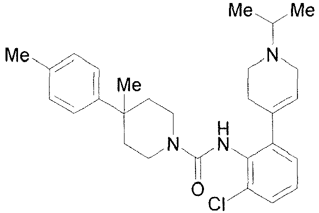 | 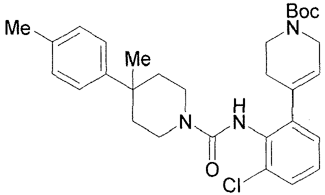 |
| | ¹H-NMR (CDCl₃) δ: 1.05 (6H, d, J = 6.1 Hz), 1.26 (3H, s), 1.72-1.77 (2H, m), 2.10-2.16 (2H, m), 2.32 (3H, s), 2.41 (2H, m), 2.62-2.75 (3H, m), 3.12 (2H, m), 3.40-3.44 (2H, m), 3.55-3.60 (2H, m), 5.66 (1H, m), 5.97 (1H, s), 7.03-7.09 (2H, m), 7.14 (2H, m), 7.21-7.28 (3H, m) | |
| 148 | | Reference Example 44 |
| | 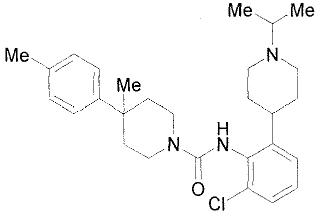 | 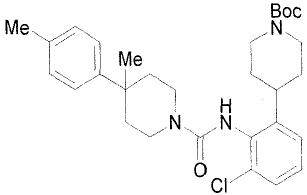 |
| | ¹H-NMR (CDCl₃) δ: 1.02 (6H, d, J = 6.7 Hz), 1.28 (3H, s), 1.62-1.84 (6H, m), 2.11-2.19 (4H, m), 2.33 (3H, s), 2.66-2.74 (2H, m), 2.95 (2H, m), 3.43-3.49 (2H, m), 3.58-3.64 (2H, m), 5.95 (1H, s), 7.10-7.17 (3H, m), 7.21-7.25 (4H, m) | |
| 149 | | Reference Example 52 |
| | 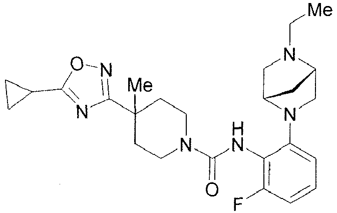 | 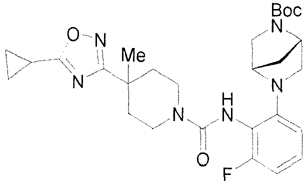 |
| | ¹H-NMR (CDCl₃) δ: 1.04 (3H, t, J = 7.0 Hz), 1.18-1.24 (4H, m), 1.33 (3H, s), 1.66 (2H, ddd, J = 14.1, 10.4, 3.7 Hz), 1.80 (1H, d, J = 9.8 Hz), 1.88 (1H, d, J = 9.8 Hz), 2.15-2.21 (1H, m), 2.22-2.30 (2H, m), 2.44-2.63 (2H, m), 2.71 (1H, d, J = 9.8 Hz), 2.92 (1H, dd, J = 9.8, 2.4 Hz), 3.13-3.23 (2H, m), 3.31 (1H, dd, J = 9.5, 2.4 Hz), 3.40 (1H, d, J = 9.5 Hz), 3.48-3.52 (1H, m), 3.74-3.84 (2H, m), 4.01-4.06 (1H, m), 5.66 (1H, s), 6.49 (1H, d, J = 8.6 Hz), 6.56 (1H, dd, J = 8.6, 8.6 Hz), 6.96-7.04 (1H, m). | |
| 150 | | Reference Example 51 |
| | 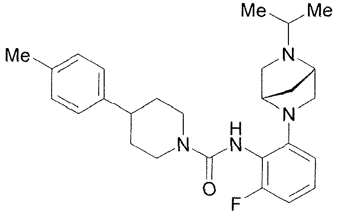 | 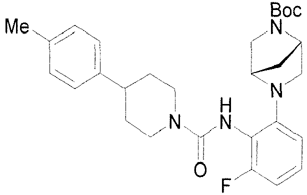 |
| | ¹H-NMR (CDCl₃) δ: 1.05 (6H, dd, J = 6.1, 1.2 Hz), 1.66-1.77 (2H, m), 1.82-1.95 (4H, m), 2.33 (3H, s), 2.54-2.76 (3H, m), 2.95-3.06 (2H, m), 3.13 (1H, dd, J = 9.2, 2.4 Hz), 3.33 (1H, dd, J = 9.8, 2.4 Hz), 3.46 (1H, d, J = 9.8 Hz), 3.70-3.74 (1H, m), 4.05-4.09 (1H, m), 4.18-4.28 (2H, m), 5.70 (1H, s), 6.51 (1H, d, J = 8.3 Hz), 6.60 (1H, ddd, J = 9.5, 8.3, 1.2 Hz), 6.99-7.06 (1H, m), 7.11 (2H, d, J = 8.6 Hz), 7.14 (2H, d, J = 8.6 Hz). | |
| 151 | | Reference Example 53 |
| | 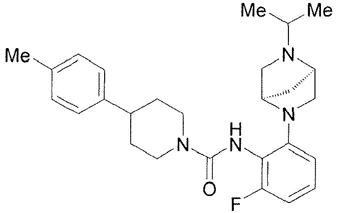 | 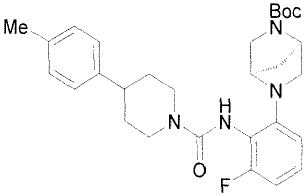 |
| | ¹H-NMR (CDCl₃) δ: 1.05 (6H, dd, J = 6.1, 1.2 Hz), 1.66-1.77 (2H, m), 1.82-1.95 (4H, m), 2.33 (3H, s), 2.54-2.76 (3H, m), 2.95-3.06 (2H, m), 3.13 (1H, dd, J = 9.2, 2.4 Hz), 3.33 (1H, dd, J = 9.8, 2.4 Hz), 3.46 (1H, d, J = 9.8 Hz), 3.70-3.74 (1H, m), 4.05-4.09 (1H, m), 4.18-4.28 (2H, m), 5.70 (1H, s), 6.51 (1H, d, J = 8.3 Hz), 6.60 (1H, ddd, J = 9.5, 8.3, 1.2 Hz), 6.99-7.06 (1H, m), 7.11 (2H, d, J = 8.6 Hz), 7.14 (2H, d, J = 8.6 Hz). | |
| 152 | | Reference Example 54 |
| | 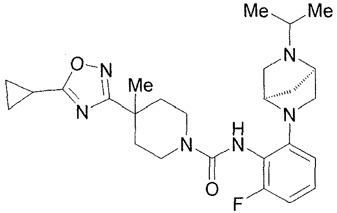 | 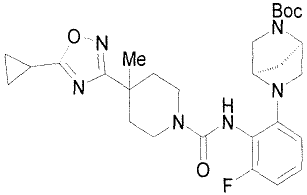 |
| | ¹H-NMR (CDCl₃) δ: 1.03 (6H, t, J = 6.1 Hz), 1.18-1.25 (4H, m), 1.33 (3H, s), 1.66-1.72 (2H, m), 1.83 (1H, d, J = 9.2 Hz), 1.91 (1H, d, J = 9.2 Hz), 2.14-2.22 (1H, m), 2.24-2.31 (2H, m), 2.53-2.60 (1H, m), 2.62 (1H, dd, J = 9.8, 1.2 Hz), 3.11 (1H, dd, J = 9.8, 2.4 Hz), 3.14-3.25 (2H, m), 3.29 (1H, dd, J = 9.8, 2.1 Hz), 3.41 (1H, d, J = 9.8 Hz), 3.67-3.72 (1H, m), 3.75-3.85 (2H, m), 4.02-4.06 (1H, m), 5.63 (1H, s), 6.49 (1H, d, J = 9.0 Hz), 6.57 (1H, ddd, J = 9.0, 7.9, 1.2 Hz), 6.98-7.05 (1H, m). | |
| 153 | | Reference Example 17 |
| | 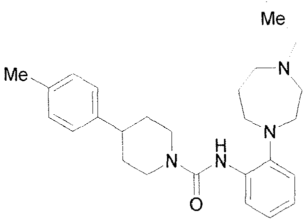 | 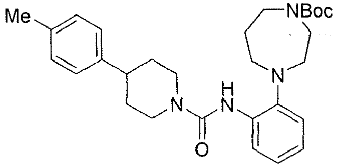 |
| | ¹H-NMR (CDCl₃) δ: 1.38-1.52 (3H, m), 1,52-1.76 (2H, m), 1.76-2.01 (4H, m), 2.32 (3H, s), 2.44-2.56 (1H, m), 2.65-2.85 (4H, m), 2.96-3.33 (4H, m), 3.40-3.47 (1H, m), 3.53 (1H, t, J = 6.4 Hz), 3.66-3.72 (1H, m), 3.77-4.17 (3H, m), 4.48-4.80 (1H, m), 7.04 (1H, d, J = 7.2 Hz), 7.07-7.22 (6H, m), 7.37 (1H, d, J = 6.8 Hz). | |
| 154 | | Reference Example 55 |
| | 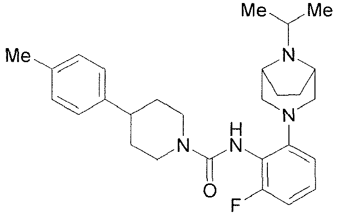 | 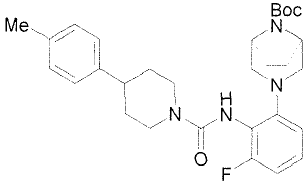 |
| | ¹H-NMR (CDCl₃) δ: 1. 13 (6H, brs), 1.48-1.68 (2H, m), 1.76 (2H, ddd, J = 25.4, 12.8, 4.0 Hz), 1.81-2.04 (6H, m), 2.34 (3H, s), 2.57-2.77 (3H, m), 2.98-3.23 (3H, m), 3.53-3.67 (2H, m), 4.28-4.34 (2H, m), 6.38 (1H, s), 6.88 (1H, dd, J = 8.6, 8.6 Hz), 6.94 (1H, d, J = 8.6 Hz), 7.00-7.08 (1H, m), 7.12 (2H, d, J = 8.0 Hz), 7.15 (2H, d, J = 8.0 Hz). | |
| 155 | | Reference Example 56 |
| | 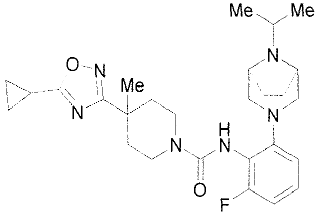 | 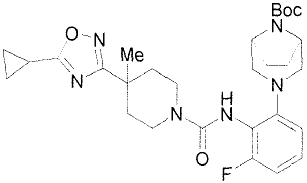 |
| | ¹H-NMR (CDCl₃) δ: 1.08 (6H, d, J = 6.1 Hz), 1.19-1.25 (4H, m), 1.34 (3H, s), 1.66-1.81 (4H, m), 1.89-1.97 (2H, m), 2.15-2.24 (1H, m), 2.26-2.33 (2H, m), 2.55-2.65 (2H, m), 2.67 (2H, dd, J = 11.3, 2.8 Hz), 3.04 (2H, d, J = 11.3 Hz), 3.23 (2H, ddd, J = 13.6, 10.9, 2.6 Hz), 3.47-3.57 (1H, m), 3.85-3.93 (2H, m), 6.34 (1H, s), 6.84 (1H, dd, J = 8.0, 1.5 Hz), 6.89 (1H, t, J = 8.0 Hz), 6.99-7.06 (1H, m). | |
| 156 | | Reference Example 18 |
| | 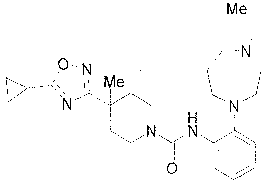 | 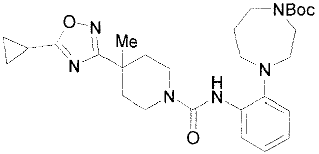 |
| | ¹H-NMR (CDCl₃) δ: 1.10 (3H, t, J = 7.2 Hz), 1.18-1.29 (4H, m), 1.34 (3H, s), 1.58-1.76 (2H, m), 1.92 (2H, tt, J = 6.4, 6.0 Hz), 2.14-2.25 (1H, m), 2.25-2.35 (2H, m), 2.61 (2H, q, J = 7.2 Hz), 2.72-2.85 (4H, m), 3.02-3.11 (4H, m), 3.16-3.28 (2H, m), 3.88 (2H, dt, J = 8.8, 4.4 Hz), 6.92 (1H, td, J = 7.6, 1.6 Hz), 7.05-7.12 (1H, m), 7.14 (1H, dd, J = 7.6, 1.6 Hz), 8.17 (1H, dd, J = 8.0, 1.6 Hz), 8.39 (1H, s). | |
| 157 | | Reference Example 18 |
| | 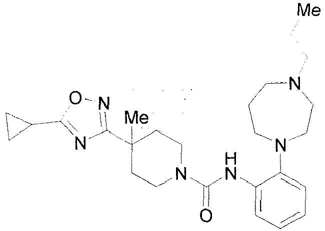 | 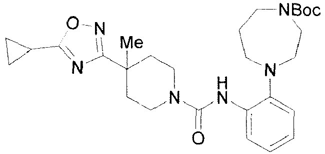 |
| | ¹H-NMR (CDCl₃) δ: 0.92 (3H, t, J = 7.2 Hz), 1.17-1.28 (4H, m), 1.33 (3H, s), 1.45-1.58 (2H, m), 1.68 (2H, ddd, J = 13.6, 10.8, 4.0 Hz), 1.90 (2H, tt, J = 6.4, 5.6 Hz), 2.14-2.24 (1H, m), 2.25-2.35 (2H, m), 2.45-2.53 (2H, m), 2.74-2.85 (4H, m), 3.01-3.09 (4H, m), 3.22 (2H, ddd, J = 14.0, 10.4, 2.8 Hz), 3.88 (2H, dt, J = 8.8, 4.4 Hz), 6.91 (1H, td, J = 7.6, 1.6 Hz), 7.05-7.11 (1H, m), 7.13 (1H, dd, J = 7.6, 1.6 Hz), 8.17 (1H, dd, J = 8.0, 1.6 Hz), 8.39 (1H, s). | |
| 158 | | Reference Example 29 |
| | 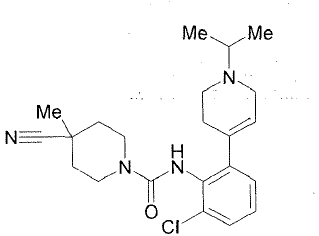 | 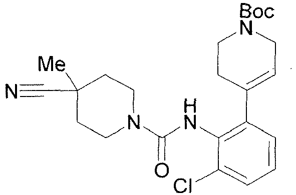 |
| | ¹H-NMR (CDCl₃) δ: 1.11 (6H, d, J = 6.8 Hz), 1.44 (3H, s), 1.52-1.64 (2H, m), 1.96 (2H, d, J = 11.2 Hz), 2.37-2.50 (2H, m), 2.63-2.76 (2H, m), 2.76-2.90 (1H, m), 3.13-3.30 (4H, m), 4.11 (2H, d, J = 14.0 Hz), 5.66-5.73 (1H, m), 6.14 (1H, bs), 7.07-7.14 (2H, m), 7.28-7.35 (1H, m). | |
| 159 | | Reference Example 29 |
| | 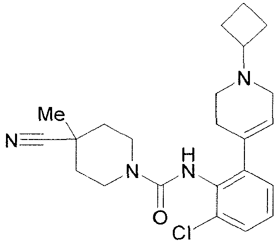 | 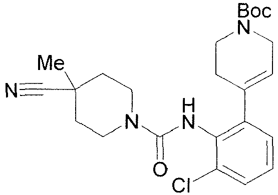 |
| | ¹H-NMR (CDCl₃) δ: 1.44 (3H, s), 1.52-1.82 (4H, m), 1.86-2.01 (4H, m), 2.03-2.15 (2H, m), 2.37-2.47 (2H, m), 2.47-2.57 (2H, m), 2.76-2.89 (1H, m), 2.94-3.03 (2H, m), 3.14-3.30 (2H, m), 4.10 (2H, d, J = 14.0 Hz), 5.66-5.71 (1H, m), 6.08 (1H, bs), 7.07-7.14 (2H, m), 7.27-7.34 (1H, m). | |
| 160 | | Reference Example 40 |
| | 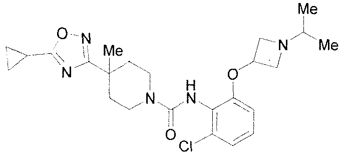 | 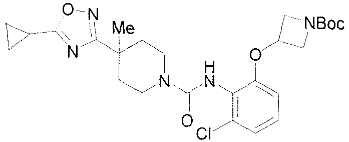 |
| | ¹H-NMR (CDCl₃) δ: 0.96 (6H, d, J = 6.0 Hz), 1.17-1.29 (4H, m), 1.35 (3H, s), 1.72 (2H, ddd, J = 14.8, 11.2, 4.4 Hz), 2.15-2.24 (1H, m), 2.24-2.33 (2H, m), 2.39 (1H, sept, J = 6.0 Hz), 3.08-3.14 (2H, m), 3.22 (2H, ddd, J = 13.2, 10.4, 2.4 Hz), 3.70-3.77 (2H, m), 3.86 (2H, dt, J = 9.0, 4.8 Hz), 4.74 (1H, quin, J = 5.6 Hz), 6.02-6.09 (1H, br), 6.51-6,58 (1H, m), 6.98-7.06 (2H, m). | |

Example 122: N-[2-(4-ethylpiperazin-1-yl)phenyl]-4-(4-methylphenyl)piperidine-1-carboxamide
Example 123: 4-(4-methylphenyl)-N-[2-(4-methylpiperazin-1-yl)phenyl]piperidine-1-carboxamide
Example 124: 4-(4-methylphenyl)-N-{2-[4-(oxolan-3-yl)piperazin-1-yl]phenyl}piperidine-1-carboxamide
Example 125: N-{2-[4-(2-methoxyethyl)piperazin-1-yl]phenyl}-4-(4-methylphenyl)piperidine-1-carboxamide
Example 126: N-{2-[4-(1-methoxypropan-2-yl)piperazin-1-yl]phenyl}-4-(4-methylphenyl)piperidine-1-carboxamide
Example 127: N-{2-ethoxy-6-[1-(propan-2-yl)piperidin-4-yl]phenyl}-4-(4-methylphenyl)piperidine-1-carboxamide
Example 128: N-{2-[1-(butan-2-yl)piperidin-4-yl]-6-ethoxyphenyl}-4-(4-methylphenyl)piperidine-1-carboxamide
Example 129: 4-(4-methylphenyl)-N-{2-[(propan-2-yl)oxy]-6-[1-(propan-2-yl)piperidin-4-yl]phenyl}piperidine-1-carboxamide
Example 130: N-{2-chloro-6-[1-(propan-2-yl)-1,2,3,6-tetrahydropyridin-4-yl]phenyl}-4-(5-cyclopropyl-1,2,4-oxadiazol-3-yl)-4-methylpiperidine-1-carboxamide
Example 131: N-[2-chloro-6-(1-cyclobutyl-1,2,3,6-tetrahydropyridin-4-yl)phenyl]-4-(5-cyclopropyl-1,2,4-oxadiazol-3-yl)-4-methylpiperidine-1-carboxamide
Example 132: N-{2-chloro-6-[1-(propan-2-yl)-1,2,3,6-tetrahydropyridin-4-yl]phenyl}-4-(4-methylphenyl)piperidine-1-carboxamide
Example 133: N-{2-chloro-6-[1-(propan-2-yl)piperidin-4-yl]phenyl}-4-(4-methylphenyl)piperidine-1-carboxamide
Example 134: N-{2-fluoro-6-[1-(1-fluoropropan-2-yl)-1,2,3,6-tetrahydropyridin-4-yl]phenyl}-4-(4-methylphenyl)piperidine-1-carboxamide
Example 135: N-{2-[1-(1,1-difluoropropan-2-yl)-1,2,3,6-tetrahydropyridin-4-yl]-6-fluorophenyl}-4-(4-methylphenyl)piperidine-1-carboxamide
Example 136: N-{2-[1-(1,3-difluoropropan-2-yl)-1,2,3,6-tetrahydropyridin-4-yl]-6-fluorophenyl}-4-(4-methylphenyl)piperidine-1-carboxamide
Example 137: N-{2-fluoro-6-[1-(1-fluoropropan-2-yl)piperidin-4-yl]phenyl}-4-(4-methylphenyl)piperidine-1-carboxamide
Example 138: N-{2-[1-(1,1-difluoropropan-2-yl)piperidin-4-yl]-6-fluorophenyl}-4-(4-methylphenyl)piperidine-1-carboxamide
Example 139: N-{2-fluoro-6-[1-(propan-2-yl)-2,5-dihydro-1H-pyrrol-3-yl]phenyl}-4-(4-methylphenyl)piperidine-1-carboxamide
Example 140: N-[2-fluoro-6-(1-methyl-2,5-dihydro-1H-pyrrol-3-yl)phenyl]-4-(4-methylphenyl)piperidine-1-carboxamide
Example 141: N-{2-chloro-6-[1-(1-fluoropropan-2-yl)-1,2,3,6-tetrahydropyridin-4-yl]phenyl}-4-(5-cyclopropyl-1,2-oxazol-3-yl)-4-methylpiperidine-1-carboxamide
Example 142: N-{2-chloro-6-[1-(1-fluoropropan-2-yl)-1,2,3,6-tetrahydropyridin-4-yl]phenyl}-4-(5-cyclopropyl-1,2,4-oxadiazol-3-yl)-4-methylpiperidine-1-carboxamide
Example 143: N-{2-chloro-6-[1-(1-fluoropropan-2-yl)piperidin-4-yl]phenyl}-4-(5-cyclopropyl-1,2,4-oxadiazol-3-yl)-4-methylpiperidine-1-carboxamide
Example 144: N-{2-chloro-6-[1-(propan-2-yl)-1,2,3,6-tetrahydropyridin-4-yl]phenyl}-4-(5-cyclopropyl-1,2-oxazol-3-yl)-4-methylpiperidine-1-carboxamide
Example 145: N-{2-chloro-6-[1-(propan-2-yl)piperidin-4-yl]phenyl}-4-(5-cyclopropyl-1,2,4-oxadiazol-3-yl)-4-methylpiperidine-1-carboxamide
Example 146: N-{2-chloro-6-[1-(oxetan-3-yl)piperidin-4-yl]phenyl}-4-(5-cyclopropyl-1,2,4-oxadiazol-3-yl)-4-methylpiperidine-1-carboxamide
Example 147: N-{2-chloro-6-[1-(propan-2-yl)-1,2,3,6-tetrahydropyridin-4-yl]phenyl}-4-methyl-4-(4-methylphenyl)piperidine-1-carboxamide
Example 148: N-{2-chloro-6-[1-(propan-2-yl)piperidin-4-yl]phenyl}-4-methyl-4-(4-methylphenyl)piperidine-1-carboxamide
Example 149: 4-(5-cyclopropyl-1,2,4-oxadiazol-3-yl)-N-{2-[(1S,4S)-5-ethyl-2,5-diazabicyclo[2.2.1]heptan-2-yl]-6-fluorophenyl}-4-methylpiperidine-1-carboxamide
Example 150: N-{2-fluoro-6-[(1S,4S)-5-(propan-2-yl)-2,5-diazabicyclo[2.2.1]heptan-2-yl]phenyl}-4-(4-methylphenyl)piperidine-1-carboxamide
Example 151: N-{2-fluoro-6-[(1R,4R)-5-(propan-2-yl)-2,5-diazabicyclo[2.2.1]heptan-2-yl]phenyl}-4-(4-methylphenyl)piperidine-1-carboxamide
Example 152: 4-(5-cyclopropyl-1,2,4-oxadiazol-3-yl)-N-{2-fluoro-6-[(1R,4R)-5-(propan-2-yl)-2,5-diazabicyclo[2.2.1]heptan-2-yl]phenyl}-4-methylpiperidine-1-carboxamide
Example 153: N-[2-(4-ethyl-1,4-diazepan-1-yl)phenyl]-4-(4-methylphenyl)piperidine-1-carboxamide
Example 154: N-{2-fluoro-6-[8-(propan-2-yl)-3,8-diazabicyclo[3.2.1]octan-3-yl]phenyl}-4-(4-methylphenyl)piperidine-1-carboxamide
Example 155: 4-(5-cyclopropyl-1,2,4-oxadiazol-3-yl)-N-{2-fluoro-6-[8-(propan-2-yl)-3,8-diazabicyclo[3.2.1]octan-3-yl]phenyl}-4-methylpiperidine-1-carboxamide
Example 156: 4-(5-cyclopropyl-1,2,4-oxadiazol-3-yl)-N-[2-(4-ethyl-1,4-diazepan-1-yl)phenyl]-4-methylpiperidine-1-carboxamide
Example 157: 4-(5-cyclopropyl-1,2,4-oxadiazol-3-yl)-4-methyl-N-[2-(4-propyl-1,4-diazepan-1-yl)phenyl]piperidine-1-carboxamide
Example 158: N-{2-chloro-6-[1-(propan-2-yl)-1,2,3,6-tetrahydropyridin-4-yl]phenyl}-4-cyano-4-methylpiperidine-1-carboxamide
Example 159: N-[2-chloro-6-(1-cyclobutyl-1,2,3,6-tetrahydropyridin-4-yl)phenyl]-4-cyano-4-methylpiperidine-1-carboxamide
Example 160: N-(2-chloro-6-{[1-(propan-2-yl)azetidin-3-yl]oxy}phenyl)-4-(5-cyclopropyl-1,2,4-oxadiazol-3-yl)-4-methylpiperidine-1-carboxamide

### Example 161: N-{2-Fluoro-6-[3-(propan-2-yl)-3,8-diazabicyclo[3.2.1]octan-8-yl]phenyl}-4-(4-methylphenyl)piperidine-1-carboxamide

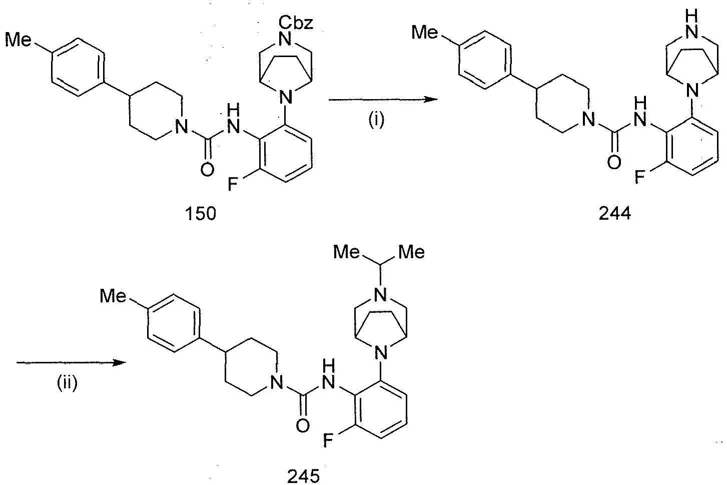

### Step (i) :

A mixture of compound 150 (0.079 g), palladium hydroxide (0.020 g) and ethanol (2 mL) was stirred at room temperature for 3 horus under hydrogen atmosphere. The reaction mixture was filtrated with Celite and the filtrate was concentrated *in vacuo.* The resulting residue was then used directly in the next step.

### Step (ii):

The title compound 245 (0.066 g) was prepared from the crude product of compound 244 in the same manner as Step (iii) in Reference Example 20.
¹H-NMR (CDCl₃) δ: 1.02 (6H, d, J = 6.7 Hz), 1.61 (3H, s), 1.74 (2H, ddd, J = 25.4, 13.1, 4.0 Hz), 1.83-1.96 (6H, m), 2.33 (3H, s), 2.55 (2H, d, J = 9.8 Hz), 2.62-2.76 (4H, m), 3.03 (2H, ddd, J = 13.1, 13.1, 2.2 Hz), 3.60-3.65 (2H, m), 4.29-4.37 (2H, m), 6.17 (1H, s), 6.66 (1H, d, J = 8.3 Hz), 6.77 (1H, dd, J = 8.6, 8.3 Hz), 6.94-7.01 (1H, m), 7.11 (2H, d, J = 8.6 Hz), 7.14 (2H, d, J = 8.6 Hz).

### Example 162: 4-Methyl-4-(3-methyl-1,2,4-oxadiazol-5-yl)-N-{2-[4-(propan-2-yl)piperazin-1-yl]phenyl}piperidine-1-carboxamide hydrochloride

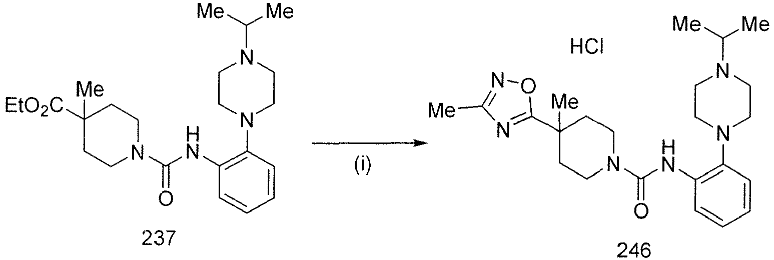

### Step (i):

A mixture of (Z)-N'-hydroxyacetimidamide (0.0125 g), sodium hydride (0.0134 g) and THF (1 mL) was stirred with heating under reflux for 2 hours. The mixture was cooled to room temperature, compound 237 in Reference Example 101 (0.035 g) was added thereto, and the mixture was stirred with heating under reflux for 5 hours. The mixture was cooled to room temperature, water was added to the reaction mixture at 0°C, and the mixture was extracted with chloroform. The organic layer was dried over anhydrous sodium sulfate and concentrated *in vacuo.* The resulting residue was then purified by amino silica gel column chromatography (Eluate: hexane/ethyl acetate) to give the title compound 246 (0.005 g).
LCMS: [M+H]⁺/Rt(min): 427/0.70

### Examples 163-164:

The compounds of Examples 163-164 shown in the table below were synthesized in the same manner as Example 162 by using each corresponding commercial compound or compound of Reference Example.

**[Table 9]**

| Example | Structural Formula | Material |
|---|---|---|
| | Spectral Data | |
| 163 | | Reference Example 101 |
| | 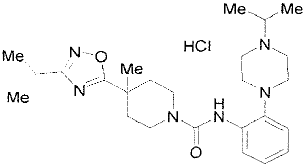 | 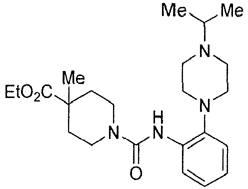 |
| | ¹H-NMR (CDCl₃) δ: 1.38 (8H, d, J = 6.8 Hz), 1.86-1.85 (3H, m), 2.10 (3H, s), 2.40 (2H, m), 3.13-3.08 (2H, m), 3.30-3.29 (3H, m), 3.59-3.47 (6H, m), 3.96-3.95 (4H, m), 4.39-4.37 (2H, m), 7.25-7.22 (1H, m), 7.39-7.35 (1H, m), 7.48 (1H, d, J = 8.0 Hz), 7.77 (1H, d, J = 8.0 Hz), 8.54 (1H, brs) | |
| 164 | | Reference Example 102 |
| | 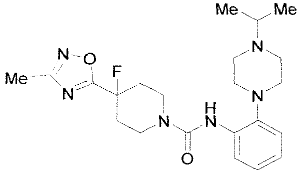 | 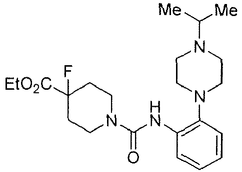 |
| | LCMS: [M+H]+/Rt = 431/1.52 min (Method B) | |

Example 163: 4-methyl-4-[3-(propan-2-yl)-1,2,4-oxadiazol-5-yl]-N-{2-[4-(propan-2-yl)piperazin-1-yl]phenyl}piperidine-1-carboxamide hydrochloride
Example 164: 4-fluoro-4-(3-methl-1,2,4-oxadiazol-5-yl)-N-{2-[4-(propan-2-yl)piperazin-1-yl]phenyl}piperidine-1-carboxamide

### Example 165: 4-Methyl-4 -(5-methyl-1,3,4-oxadiazol-2-yl)-N-{2-[4-(propan-2-yl)piperazin-1-yl]phenyl}piperidine-1-carboxamide

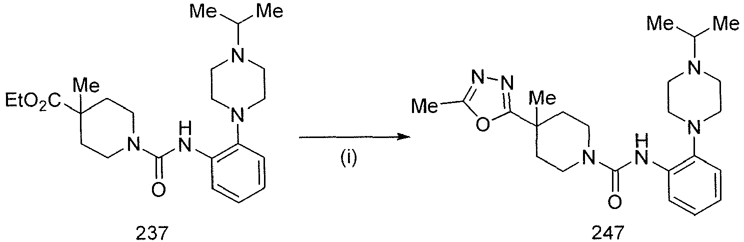

To a mixture of compound 237 in Reference Example 101 (0.056 g), THF (0.5 mL), MeOH (0.5 mL) and water (0.5 mL) was added sodium hydroxide (0.027 g) and the mixture was stirred at 60°C for 1 hour. The mixture was neutralized with 10% aqueous hydrochloric acid solution and extracted with chloroform/methanol (4:1) solution. The solvent was concentrated *in vacuo* and the resulting residue was dissolved in DMF (0.5 mL). Diisopropylethylamine (0.059 mL), acetylhydrazine (0.020 g) and HATU (0.128 g) were added thereto and the mixture was stirred at room temperature for 14 hours. To the reaction solution was saturated aqueous sodium hydrogen carbonate solution and the mixture was extracted with chloroform. The solvent was concentrated *in vacuo* and the resulting residue was dissolved in acetonitrile (0.5 mL). Triphenylphosphine (0.071 g), triethylamine (0.057 mL) and carbon tetrachloride (0.052 mL) were added thereto and the mixture was stirred with heating under reflux for 7 hours. The mixture was cooled and concentrated *in vacuo.* The resulting residue was then purified by amino silica gel column chromatography (Eluate: hexane/ethyl acetate) to give the title compound 247 (0.010 g).
LCMS: [M+H]⁺/Rt(min): 427/1.43

### Example 166: 4-(4-Methylphenyl)-N-{2-[(2R,5S)-5-(propan-2-yl)morpholin-2-yl]phenyl}piperidine-1-carboxamide

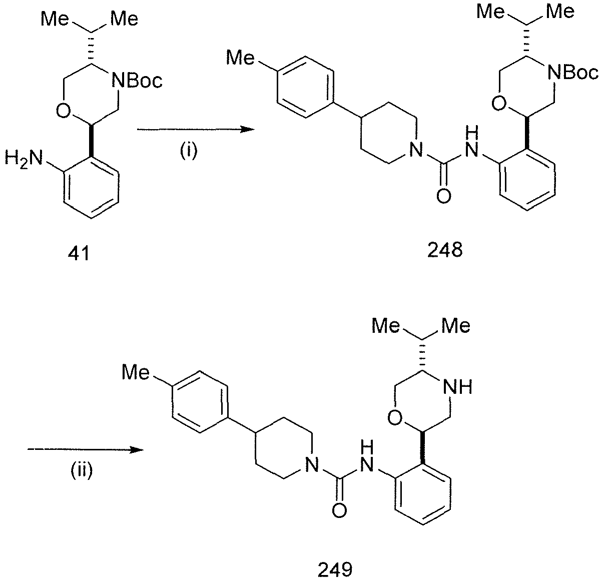

### Step (i) :

The title compound 248 (0.045 g) was prepared from compound 41 (0.036 g) in the same manner as Step (iv) in Reference Example 23.
LCMS: [M+H]⁺/Rt(min): 522/1.25

### Step (ii):

The title compound 249 (0.031 g) was synthesized from compound 248 (0.042 g) in the same manner as Step (ii) in Reference Example 14.
¹H-NMR (CDCl₃) 5: 0.92 (3H, d, J = 7.0 Hz), 0.97 (3H, d, J = 7.0 Hz), 1.51-1.62 (1H, m), 1.66-1.77 (2H, m), 1.86-1.94 (2H, m), 2.33 (3H, s), 2.53-2.60 (1H, m), 2.67-2.76 (1H, m), 2.95-3.04 (2H, m), 3.07 (1H, dd, J = 12.5, 2.7 Hz), 3.16 (1H, dd, J = 11.3, 11.3 Hz), 3.48 (1H, dd, J = 10.4, 10.4 Hz), 4.09 (1H, dd, J = 11.3, 2.7 Hz), 4.21-4.32 (2H, m), 4.53 (1H, dd, J = 10.4, 3.0 Hz), 6.95 (1H, dd, J = 7.3, 7.3 Hz), 7.06 (1H, d, J = 7.3 Hz), 7.11 (4H, d, J = 8.5 Hz), 7.14 (4H, d, J = 8.5 Hz), 7.27 (1H, dd, J = 7.3, 7.3 Hz), 8.10 (1H, d, J = 8.5 Hz), 8.71 (1H, s).

### Examples 167-168:

The compounds of Examples 167-168 shown in the table below were synthesized in the same manner as Example 166 by using each corresponding commercial compound or compound of Reference Example.

**[Table 10]**

| Example | Structural Formula | Material |
|---|---|---|
| | Spectral Data | |
| 167 | 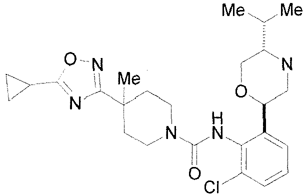 | Reference Example 58 |
| | | 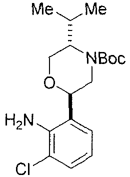 |
| | ¹H-NMR (CDCl₃) δ: (mixture of rotamers) 0.93 (3H, d, J = 6.8 Hz), 0.97 (3H, d, J = 6.8 Hz), 1.35 (3H, s), 1.50-1.80 (5H, m), 2.12-2.25 (2H, m), 2.25-2.35 (2H, m), 2.47-2.56 (1H, m), 2.88 (1H, dd, J = 12.4, 10.4 Hz), 2.94-3.05 (2H, m), 3.12-3.30 (2H, m), 3.34-3.46 (2H, m) 3.80-3.92 (2H, m), 4.05 (1H, dd, J = 11.2, 3.2 Hz), 4.53 (1H, dd, J = 10.4, 2.4 Hz), 6.89 (1H, br s), 7.13 (1H, t, J = 8.0 Hz), 7.21-7.29 (1H, m), 7.36 (1H, dd, J = 8.0, 1.6 Hz). | |
| 168 | 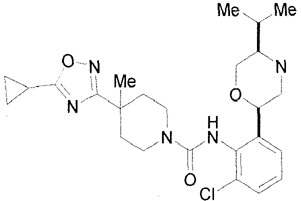 | Reference Example 59 |
| | | 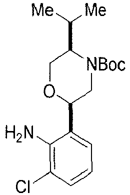 |
| | ¹H-NMR (CDCl₃) δ: (mixture of rotamers) 0.90-1.06 (6H, m), 1.35 (3H, s), 1.51-1.63 (1H, m) 1.62-1.81 (4H, m), 2.12-2.24 (2H, m), 2.24-2.35 (2H, m), 2.35-2.59 (1H, m), 2.87 (0.6H, dd, J = 12.0, 10.8 Hz), 2.90-3.97 (8.8H, m) 4.05 (0.6H, dd, J = 11.2, 3.2 Hz), 4.50-4.68 (1H, m), 4.97-5.03 (0.3H, m), 6.80-6.96 (0.7H, br s), 7.10-7.19 (1H, m), 7.22-7.29 (1H, m), 7.36 (0.7H, dd, J = 8.0, 1.2 Hz), 7.43 (0.3H, dd, J = 8.0, 1.2 Hz). | |

Example 167: Rac-N-{2-chloro-6-[(2R,5S)-5-(propan-2-yl)morpholin-2-yl]phenyl}-4-(5-cyclopropyl-1,2,4-oxadiazol-3-yl)-4-methylpiperidine-1-carboxamide
Example 168: Rac-N-{2-chloro-6-[(2R,5R)-5-(propan-2-yl)morpholin-2-yl]phenyl}-4-(5-cyclopropyl-1,2,4-oxadiazol-3-yl)-4-methylpiperidine-1-carboxamide

### Examples 169-170:

The compounds of Examples 169-170 shown in the table below were synthesized in the same manner as Example 1 by using each corresponding commercial compound or compound of Reference Example.

**[Table 11]**

| Example | Structural Formula | Material | |
|---|---|---|---|
| | Spectral Data | | |
| 169 | 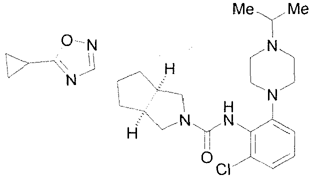 | Reference Example 9 | Reference Example 104 |
| | | 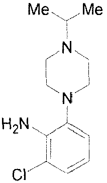 | 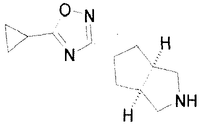 |
| | ¹H-NMR (CDCl₃) δ: 7.16 (1H, dd, J = 7.9, 1.2 Hz), 7.07 (1H, dd, J = 7.9, 7.9 Hz), 6.97 (1H, dd, J = 7.9, 1.2 Hz), 6.28 (1H, s), 3.81-3.75 (2H, m), 3.51-3.39 (3H, m), 3.04-2.96 (2H, m), 2.95-2.87 (4H, m), 2.75-2.59 (5H, m), 2.27-2.12 (3H, m), 2.05-1. 96 (2H, m), 1. 24-1.18 (4H, m), 1.07 (6H, d, J = 6.7 Hz). | | |
| 170 | 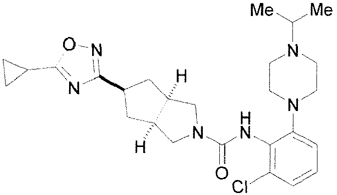 | Reference Example 9 | Reference Example 105 |
| | | 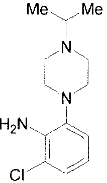 | 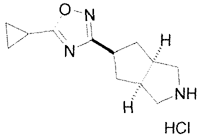 |
| | ¹H-NMR (CDCl₃) δ: 7.16 (1H, dd, J = 7.9, 1.2 Hz), 7.06 (1H, dd, J = 7.9, 7.9 Hz), 6.96 (1H, dd, J = 7.9, 1.2 Hz), 6.26 (1H, s), 3.74-3.68 (2H, m), 3.53 (2H, dd, J = 10.7, 3.4 Hz), 3.35-3.24 (1H, m), 2.95-2.82 (6H, m), 2.75-2.58 (5H, m), 2.43-2.35 (2H, m), 2.20-2.12 (1H, m), 1.88-1.78 (2H, m), 1.23-1.17 (4H, m), 1.07 (6H, d, J = 6.1 Hz). | | |

Example 169: (3aR,5s,6aS)-N-{2-chloro-6-[4-(propan-2-yl)piperazin-1-yl]phenyl}-5-(5-cyclopropyl-1,2,4-oxadiazol-3-yl)hexahydrocyclopenta[c]pyrrol-2(1H)-carboxamide
Example 170: (3aR,5r,6aS)-N-{2-chloro-6-[4-(propan-2-yl)piperazin-1-yl]phenyl}-5-(5-cyclopropyl-1,2,4-oxadiazol-3-yl)hexahydrocyclopenta[c]pyrrol-2(1H)-carboxamide

### Test Example 1: Measurement of agonist activity for orexin receptor 2 type

Human orexin receptor type 2 and apoaequorin were transiently expressed in CHO cells, and the agonist activity was evaluated based on intracellular calcium mobilization with ligand stimulation. The CHO cells transiently-expressed human orexin receptor type 2 and apoaequorin were seeded on a 384-well plate by 2,000 cells/well, and then incubated for 16 - 22 hours. After the plate was returned to room temperature, Coelenterazine hcp (final concentration: 1 µM) was added to the plate, and the plate was allowed to stand at room temperature for 2 hours. And then, Orexin A or each test compound was added to the plate, and the luminescence of the cells was measured with FDSS7000 (Hamamatsu Photonics K.K.), wherein Orexin A and each test compound were dissolved in DMSO (final concentration: 0.1 %), and diluted with a buffer (Hanks, 20 mM HEPES, 0.1 % BSA). The agonist activity of each test compound for orexin receptor type 2 was calculated as relative percentage of luminescence for the luminescence (100 %) of Orexin A (100 pM) .

### Test Result:

The results that each compound obtained in Examples 1 to 168 was evaluated about the agonist activity for orexin receptor type 2 showed that the present compounds have agonist activity for orexin receptor type 2. Each agonist activity of the compounds obtained in Examples 1 to 168 (10 µm) for orexin receptor type 2 is shown in the table below as relative percentage of luminescence for the luminescence (100 %) of Orexin A (100 pM).

**[Table 12]**

| Example | Agonist Activity (%) | Example | Agonist Activity (%) | Example | Agonist Activit y (%) |
|---|---|---|---|---|---|
| 1 | 77 | 57 | 171 | 113 | 157 |
| 2 | 35 | 58 | 71 | 114 | 155 |
| 3 | 27 | 59 | 158 | 115 | 159 |
| 4 | 125 | 60 | 102 | 116 | 136 |
| 5 | 156 | 61 | 156 | 117 | 158 |
| 6 | 151 | 62 | 75 | 118 | 149 |
| 7 | 154 | 63 | 26 | 119 | 154 |
| 8 | 145 | 64 | 140 | 120 | 125 |
| 9 | 132 | 65 | 93 | 121 | 130 |
| 10 | 34 | 66 | 129 | 122 | 122 |
| 11 | 142 | 67 | 154 | 123 | 105 |
| 12 | 123 | 68 | 68 | 124 | 27 |
| 13 | 99 | 69 | 129 | 125 | 141 |
| 14 | 126 | 70 | 45 | 126 | 139 |
| 15 | 101 | 71 | 88 | 127 | 145 |
| 16 | 82 | 72 | 144 | 128 | 136 |
| 17 | 133 | 73 | 141 | 129 | 138 |
| 18 | 128 | 74 | 145 | 130 | 142 |
| 19 | 133 | 75 | 131 | 131 | 150 |
| 20 | 111 | 76 | 148 | 132 | 171 |
| 21 | 156 | 77 | 158 | 133 | 156 |
| 22 | 97 | 78 | 170 | 134 | 135 |
| 23 | 92 | 79 | 156 | 135 | 103 |
| 24 | 127 | 80 | 157 | 136 | 105 |
| 25 | 136 | 81 | 145 | 137 | 126 |
| 26 | 63 | 82 | 156 | 138 | 44 |
| 27 | 129 | 83 | 146 | 139 | 121 |
| 28 | 104 | 84 | 110 | 140 | 75 |
| 29 | 77 | 85 | 145 | 141 | 121 |
| 30 | 36 | 86 | 138 | 142 | 130 |
| 31 | 164 | 87 | 142 | 143 | 109 |
| 32 | 30 | 88 | 161 | 144 | 143 |
| 33 | 150 | 89 | 152 | 145 | 148 |
| 34 | 161 | 90 | 164 | 146 | 104 |
| 35 | 163 | 91 | 195 | 147 | 167 |
| 36 | 123 | 92 | 196 | 148 | 176 |
| 37 | 47 | 93 | 203 | 149 | 146 |
| 38 | 119 | 94 | 179 | 150 | 116 |
| 39 | 106 | 95 | 178 | 151 | 97 |
| 40 | 122 | 96 | 160 | 152 | 136 |
| 41 | 47 | 97 | 69 | 153 | 54 |
| 42 | 128 | 98 | 165 | 154 | 40 |
| 43 | 135 | 99 | 75 | 155 | 55 |
| 44 | 143 | 100 | 160 | 156 | 38 |
| 45 | 127 | 101 | 157 | 157 | 94 |
| 46 | 49 | 102 | 160 | 158 | 133 |
| 47 | 165 | 103 | 178 | 159 | 150 |
| 48 | 146 | 104 | 180 | 160 | 141 |
| 49 | 196 | 105 | 165 | 161 | 90 |
| 50 | 187 | 106 | 185 | 162 | 100 |
| 51 | 151 | 107 | 163 | 163 | 110 |
| 52 | 155 | 108 | 157 | 164 | 128 |
| 53 | 140 | 109 | 161 | 165 | 96 |
| 54 | 140 | 110 | 148 | 166 | 72 |
| 55 | 154 | 111 | 146 | 167 | 183 |
| 56 | 44 | 112 | 151 | 168 | 184 |

### Test Example 2: Measurement of agonist activity for orexin receptor 2 type

Human orexin receptor type 2 and apoaequorin were transiently expressed in CHO cells, and the agonist activity was evaluated based on intracellular calcium mobilization with ligand stimulation. The CHO cells transiently-expressed human orexin receptor type 2 and apoaequorin were seeded on a 384-well plate by 2,000 cells/well, and then incubated for 16 - 22 hours. After the plate was returned to room temperature, Coelenterazine hcp (final concentration: 1 µM) was added to the plate, and the plate was allowed to stand at room temperature for 2 hours. And then, Orexin A (PEPTIDE INSTITUTE, INC., Lot. 671009) or each test compound was added to the plate, and the luminescence of the cells was measured with FDSS7000 (Hamamatsu Photonics K.K.), wherein Orexin A and each test compound were dissolved in DMSO (final concentration: 0.1 %), and diluted with a buffer (Hanks, 20 mM HEPES, 0.1 % BSA). The agonist activity of each test compound for orexin receptor type 2 was calculated as relative percentage of luminescence for the luminescence (100 %) of Orexin A (100 pM) .

### Test Result:

The results that each compound obtained in Examples 169 to 170 was evaluated about the agonist activity for orexin receptor type 2 showed that the present compounds have agonist activity for orexin receptor type 2. Each agonist activity of the compounds obtained in Examples 169 to 170 (10 um) for orexin receptor type 2 is shown in the table below as relative percentage of luminescence for the luminescence (100 %) of Orexin A (100 pM).

**[Table 13]**

| Example | Agonist Activity (%) |
|---|---|
| 169 | 444 |
| 170 | 357 |

### INDUSTRIAL APPLICABILITY

The compounds of the present invention exhibit a potent agonist activity for orexin receptor, thereby they are useful as a medicament for treating or preventing a disease such as narcolepsy, idiopathic hypersomnia, hypersomnia, sleep apnea syndrome, narcolepsy syndrome involving narcolepsy-like symptom, hypersomnia associated with Parkinson's disease, hypersomnia associated with dementia with Lewy body.

## Claims

1. A compound of formula (1): 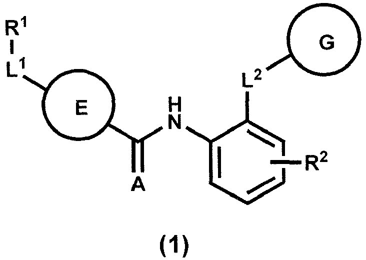 or a pharmaceutically acceptable salt thereof,
wherein
R¹ is optionally-substituted C₆₋₁₀ aromatic carbocyclyl group, optionally-substituted 5- to 10-membered aromatic heterocyclyl group, optionally-substituted C₃₋₆ saturated carbocyclyl group, optionally-substituted 4- to 10-membered saturated heterocyclyl group, or cyano;
L¹ and L² are each independently single bond, methylene (which may be optionally substituted with the same or different one or more C₁₋₆ alkyl groups), -NR⁶-, -C(=O)-, - OC(=O)-, -SO-, -SO₂-, -S-, or oxygen atom;
R² is hydrogen atom, hydroxy group, halogen atom, cyano, optionally-substituted C₁₋₆ alkyl, optionally-substituted C₁₋₆ alkoxy, C(=O)NR³R⁴ or C(=O)O-R⁵;
R³, R⁴, R⁵ and R⁶ are each independently hydrogen atom or optionally-substituted C₁₋₆ alkyl;
ring E is optionally-substituted 5- to 10-membered aromatic heterocyclyl group or optionally-substituted 4- to 10-membered saturated heterocyclyl group;
ring G is optionally-substituted C₆₋₁₀ aromatic carbocyclyl group, optionally-substituted 5- to 10-membered aromatic heterocyclyl group, optionally-substituted C₃₋₆ saturated carbocyclyl group, or optionally-substituted 4- to 10-membered saturated heterocyclyl group; and
A is oxygen atom or sulfur atom.

2. The compound according to claim 1 or a pharmaceutically acceptable salt thereof, wherein in R² to R⁶, the optional substituent of "optionally-substituted C₁₋₆ alkyl" is each independently the same or different one or more substituents selected from halogen atom, hydroxy group, C_{1 - 6} alkoxy, or C_{3 - 7} cycloalkyl, and the optional substituent of "optionally-substituted C₁₋₆ alkoxy" is each independently the same or different one or more substituents selected from halogen atom, hydroxy group, or C_{3 - 7} cycloalkyl,
in R¹, the optional substituent of C₆₋₁₀ aromatic carbocyclyl group, 5- to 10-membered aromatic heterocyclyl group, C₃₋₆ saturated carbocyclyl group, and 4- to 10-membered saturated heterocyclyl group is each independently one or more substituents selected from the group consisting of halogen atom, hydroxy group, C₆₋₁₀ aromatic carbocyclyl group (which may be optionally substituted with the same or different one or more substituents selected from halogen atom, hydroxy group, C_{1 - 6} alkyl, C₁₋₆ alkoxy or C_{3 - 7} cycloalkyl), C_{1 - 6} alkyl (which may be optionally substituted with the same or different one or more substituents selected from halogen atom, hydroxy group, C_{1 - 6} alkoxy or C_{3 - 7} cycloalkyl), C_{3 - 7} cycloalkyl (which may be optionally substituted with the same or different one or more substituents selected from halogen atom, hydroxy group, C₁₋₆ alkyl, C_{1 - 6} alkoxy or C_{3 - 7} cycloalkyl), C_{1 - 6} alkylamino (the alkyl group of which may be optionally substituted with halogen atom, hydroxy group or C_{3 - 7} cycloalkyl), C_{3 - 7} cycloalkoxy (which may be optionally substituted with the same or different one or more substituents selected from halogen atom, hydroxy group, C_{1 - 6} alkyl, C_{1 - 6} alkoxy or C₃₋₇ cycloalkyl), cyano, C_{1 - 6} alkoxy (which may be optionally substituted with the same or different one or more substituents selected from halogen atom, hydroxy group or C_{3 - 7} cycloalkyl), and 5- to 10-membered aromatic heterocyclyl group (which may be optionally substituted with the same or different one or more substituents selected from halogen atom, hydroxy group, C_{1 - 6} alkyl, C_{1 - 6} alkoxy or C₃₋₇ cycloalkyl), and
in ring E and ring G, the optional substituent of C₆₋₁₀ aromatic carbocyclyl group, 5- to 10-membered aromatic heterocyclyl group, C₃₋₆ saturated carbocyclyl group, and 4-to 10-membered saturated heterocyclyl group is each independently one or more substituents selected from the group consisting of halogen atom, C_{1 - 6} alkyl (which may be optionally substituted with the same or different one or more substituents selected from halogen atom, hydroxy group, C_{1 - 6} alkoxy or C_{3 - 7} cycloalkyl), C₆₋₁₀ aromatic carbocyclyl group (which may be optionally substituted with the same or different one or more substituents selected from halogen atom, hydroxy group, C_{1 - 6} alkyl, C_{1 - 6} alkoxy or C_{3 - 7} cycloalkyl), C_{1 - 6} alkoxy (which may be optionally substituted with the same or different one or more substituents selected from halogen atom, hydroxy group or C_{3 - 7} cycloalkyl), C_{1 - 6} alkylamino (the alkyl group of which may be optionally substituted with halogen atom, hydroxy group or C_{3 - 7} cycloalkyl), C_{3 - 7} cycloalkyl (which may be optionally substituted with the same or different one or more substituents selected from halogen atom, hydroxy group, C_{1 - 6} alkyl, C_{1 - 6} alkoxy or C₃₋₇ cycloalkyl), and C₃₋₇ cycloalkoxy (which may be optionally substituted with the same or different one or more substituents selected from halogen atom, hydroxy group, C_{1 - 6} alkyl, C_{1 - 4} alkoxy or C₃₋₇ cycloalkyl), or when there are plural optional substituents, two of them may be taken together via C₁₋₆ alkylene to form a chemically-possible bicyclic structure selected from a fused ring, a spiro ring, or bridged ring.

3. The compound according to claim 1 or 2 or a pharmaceutically acceptable salt thereof, wherein the formula is represented by formula (2): 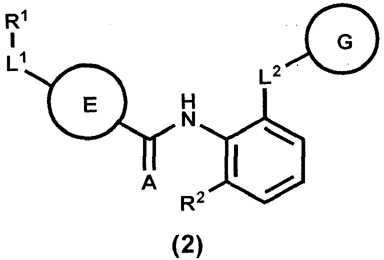 wherein
R² is hydroxy group, halogen atom, cyano, optionally-substituted C_{1 - 4} alkyl, optionally-substituted C_{1 - 4} alkoxy, C(=O)NR³R⁴ or C(=O)OR⁵; and
R¹, L¹, L², R³, R⁴, R⁵, ring E, ring G, and A are as defined in claim 1.

4. The compound according to any one of claims 1 to 3 or a pharmaceutically acceptable salt thereof, wherein R¹ is selected from cyano or the following formulae (1a-1) to (1a-6) : 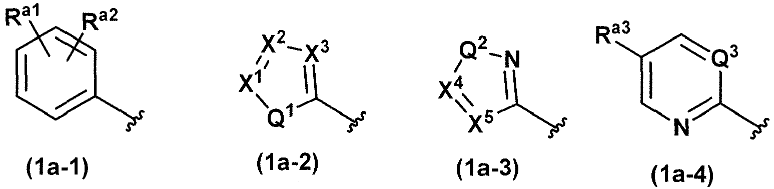 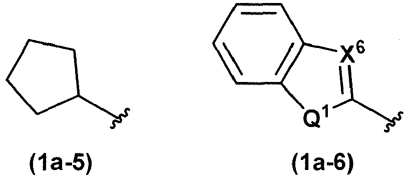 wherein
X¹ to X⁶ are each independently nitrogen atom or CR^{a4};
Q¹ and Q² are oxygen atom, -NR^{a5}- or sulfur atom;
Q³ is CH or nitrogen atom; and
R^{a1} to R^{a5} are each independently (if there are plural CR^{a4}, each R^{a4} is also independently) hydrogen atom, halogen atom, C₆₋₁₀ aromatic carbocyclyl group (which may be optionally substituted with the same or different one or more substituents selected from halogen atom, hydroxy group, C_{1 - 6} alkyl or C_{1 - 6} alkoxy), C_{1 - 6} alkyl (which may be optionally substituted with the same or different one or more substituents selected from halogen atom, hydroxy group, C_{1 - 4} alkoxy or C_{3 - 7} cycloalkyl), C_{3 - 7} cycloalkyl (which may be optionally substituted with the same or different one or more substituents selected from halogen atom, hydroxy group, C_{1 - 6} alkyl which may be optionally substituted with the same or different one or more halogen atoms, or C_{1 - 6} alkoxy), cyano, C_{1 - 6} alkoxy (which may be optionally substituted with the same or different one or more substituents selected from halogen atom, hydroxy group or C_{1 - 6} alkoxy), C_{3 - 7} cycloalkoxy (which may be optionally substituted with the same or different one or more substituents selected from halogen atom, hydroxy group, C_{1 - 6} alkyl or C_{1 - 6} alkoxy), or 5- to 10-membered aromatic heterocyclyl group (which may be optionally substituted with the same or different one or more substituents selected from halogen atom, hydroxy group, C_{1 - 6} alkyl or C_{1 - 6} alkoxy).

5. The compound according to any one of claims 1 to 4 or a pharmaceutically acceptable salt thereof, wherein ring G is selected from the following formulae (1b-1) to (1b-7): 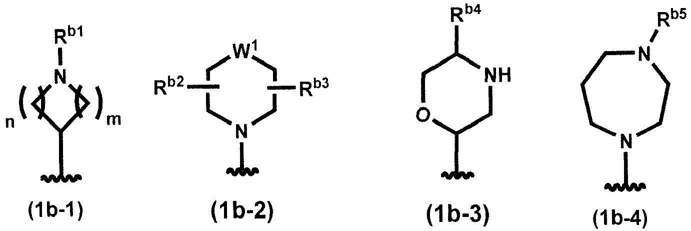 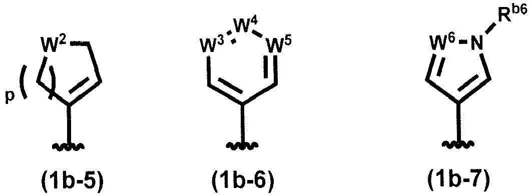 wherein
W¹ and W² are each independently NR^{b7}, oxygen atom or CR^{b8}R^{b9};
W³, W⁴, W⁵ and W⁶ are each independently nitrogen atom or CR^{b10};
R^{b1} to R^{b10} are each independently hydrogen atom, halogen atom, C_{1 - 6} alkyl (which may be optionally substituted with the same or different one or more substituents selected from halogen atom or C_{1 - 4} alkoxy), C_{1 - 6} alkoxy (which may be optionally substituted with the same or different one or more halogen atoms), C₆₋₁₀ aromatic carbocyclyl group (which may be optionally substituted with the same or different one or more substituents selected from halogen atom, C_{1 - 6} alkyl or C_{1 - 6} alkoxy), C_{3 - 7} cycloalkyl (which may be optionally substituted with the same or different one or more substituents selected from halogen atom, C_{1 - 6} alkyl or C_{1 - 6} alkoxy), 5- to 10-membered aromatic heterocyclyl group (which may be optionally substituted with the same or different one or more substituents selected from halogen atom, C_{1 - 6} alkyl or C_{1 - 6} alkoxy), or C_{3 - 7} cycloalkoxy (which may be optionally substituted with the same or different one or more substituents selected from halogen atom, C_{1 - 6} alkyl or C_{1 - 6} alkoxy), and R^{b2} and R^{b3} may bind to the same carbon atom if chemically possible;
wherein R^{b2} and R^{b3} may be taken together via C₁₋₆ alkylene to form a fused ring or a bridged ring; and
n, m and p are each independently an integer of 1 or 2.

6. The compound according to any one of claims 1 to 5 or a pharmaceutically acceptable salt thereof, wherein ring E is selected from the following formulae (1c-1) to (1c-4): 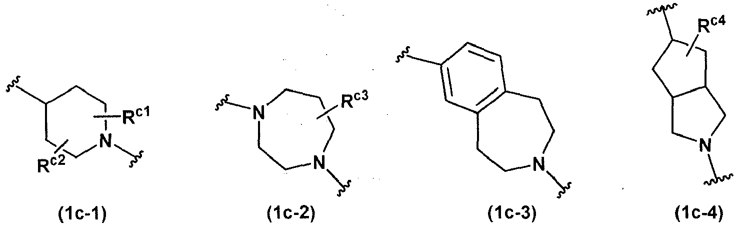 wherein
R^{c1}, R^{c2}, R^{c3} and R^{c4} are each independently hydrogen atom, halogen atom, cyano, C_{1 - 6} alkyl (which may be optionally substituted with the same or different one or more substituents selected from halogen atom or C_{1 - 6} alkoxy), C_{1 - 6} alkoxy (which may be optionally substituted with the same or different one or more halogen atoms), C₆₋₁₀ aromatic carbocyclyl group (which may be optionally substituted with the same or different one or more substituents selected from halogen atom, C_{1 - 6} alkyl or C_{1 - 6} alkoxy), C_{3 - 7} cycloalkyl (which may be optionally substituted with the same or different one or more substituents selected from halogen atom, C_{1 - 6} alkyl or C_{1 - 6} alkoxy), or C_{3 - 7} cycloalkoxy (which may be optionally substituted with the same or different one or more substituents selected from halogen atom, C_{1 - 6} alkyl or C_{1 - 6} alkoxy), and R^{c1} and R^{c2} may bind to the same carbon atom if chemically possible; and
wherein R^{c1} and R^{c2} may be taken together via C₁₋₆ alkylene to form a fused ring or a bridged ring.

7. The compound according to any one of claims 1 to 6 or a pharmaceutically acceptable salt thereof, wherein ring E is selected from the following formulae (1c-11) to (1c-41): 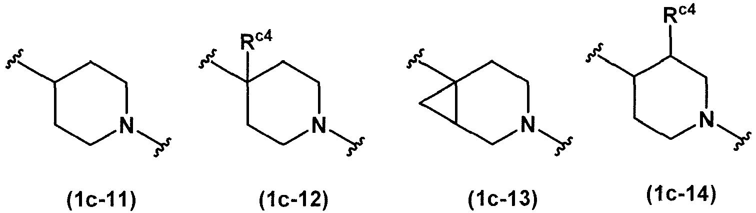 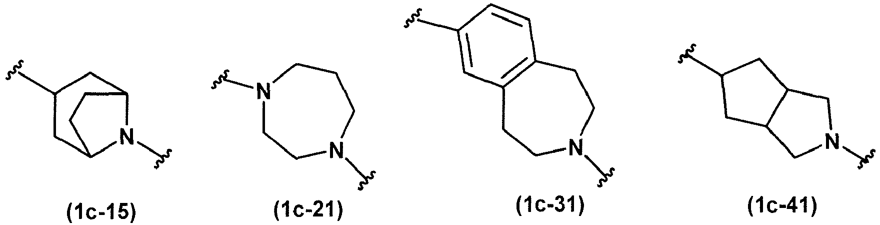 wherein
R^{c4} is each independently halogen atom, cyano, C_{1 - 6} alkyl (which may be optionally substituted with the same or different one or more substituents selected from halogen atom or C_{1 - 6} alkoxy), C_{1 - 6} alkoxy (which may be optionally substituted with the same or different one or more halogen atoms), C₆₋₁₀ aromatic carbocyclyl group (which may be optionally substituted with one or more substituents selected from halogen atom, C_{1 - 6} alkyl or C_{1 - 6} alkoxy), C₃₋₇ cycloalkyl (which may be optionally substituted with the same or different one or more substituents selected from halogen atom, C_{1 - 6} alkyl or C_{1 - 6} alkoxy), or C_{3 - 7} cycloalkoxy (which may be optionally substituted with the same or different one or more substituents selected from halogen atom, C_{1 - 6} alkyl or C_{1 - 6} alkoxy).

8. The compound according to any one of claims 1 to 7 or a pharmaceutically acceptable salt thereof, wherein the formula is represented by formula (3): 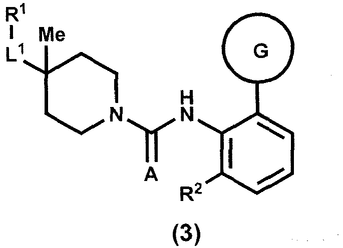 wherein
R² is C_{1 - 6} alkoxy which may be optionally substituted with halogen atom; and
R¹, A, L¹ and ring G are as defined in claim 1.

9. The compound according to any one of claims 1 to 8 or a pharmaceutically acceptable salt thereof, wherein L¹ is single bond, -CH₂- or oxygen atom.

10. The compound according to any one of claims 1 to 9 or a pharmaceutically acceptable salt thereof, wherein R¹ is selected from the following formulae (1a-1) to (1a-3): 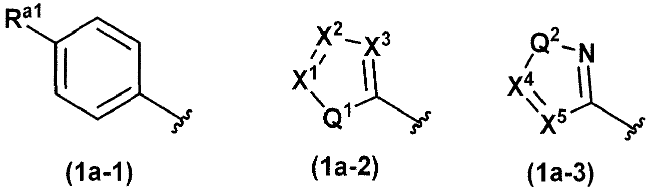 wherein
X¹ to X⁵ are each independently nitrogen atom or CR^{a4};
Q¹ and Q² are each independently oxygen atom or sulfur atom;
R^{a1} is hydrogen atom, halogen atom, cyano, C_{1 - 6} alkyl (which may be optionally substituted with the same or different one or more substituents selected from halogen atom, hydroxy group or C_{1 - 6} alkoxy), or C_{1 - 6}. alkoxy (which may be optionally substituted with the same or different one or more substituents selected from halogen atom, hydroxy group or C_{1 - 6} alkoxy); and
R^{a 4} is each independently (if there are plural CR^{a 4}, each R^{a 4} is independently) hydrogen atom, halogen atom, C₆₋₁₀ aromatic carbocyclyl group (which may be optionally substituted with the same or different one or more substituents selected from halogen atom, hydroxy group, C₁₋₆ alkyl or C_{1 - 6} alkoxy), C_{1 - 6} alkyl (which may be optionally substituted with the same or different one or more substituents selected from halogen atom, hydroxy group, C₁₋₆ alkoxy or C_{3 - 7} cycloalkyl), C_{3 - 7} cycloalkyl (which may be optionally substituted with the same or different one or more substituents selected from halogen atom, hydroxy group, C_{1 - 6} alkyl or C_{1 - 6} alkoxy), C_{1 - 6} alkoxy (which may be optionally substituted with the same or different one or more substituents selected from halogen atom, hydroxy group or C_{1 - 6} alkoxy), C_{3 - 7} cycloalkoxy (which may be optionally substituted with the same or different one or more substituents selected from halogen atom, hydroxy group, C₁₋₆ alkyl or C_{1 - 6} alkoxy), or 5- to 10-membered aromatic heterocyclyl group (which may be optionally substituted with the same or different one or more substituents selected from halogen atom, hydroxy group, C_{1 - 6} alkyl or C_{1 - 6} alkoxy).

11. The compound according to any one of claims 1 to 10 or a pharmaceutically acceptable salt thereof, wherein ring G is selected from the following formulae (1b-1) to (1b-3): 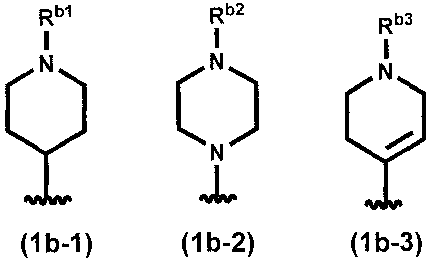 wherein
R^{b1} to R^{b3} are each independently hydrogen atom, C_{1 - 6} alkyl (which may be optionally substituted with the same or different one or more substituents selected from halogen atom or C_{1 - 6} alkoxy), C_{1 - 6} alkoxy (which may be optionally substituted with the same or different one or more halogen atoms), 5- to 10-membered aromatic heterocyclyl group (which may be optionally substituted with the same or different one or more substituents selected from halogen atom, C_{1 - 6} alkyl or C_{1 - 6} alkoxy), C₆₋₁₀ aromatic carbocyclyl group (which may be optionally substituted with the same or different one or more substituents selected from halogen atom, C_{1 - 6} alkyl or C_{1 - 6} alkoxy), or C_{3 - 7} cycloalkyl (which may be optionally substituted with the same or different one or more substituents selected from halogen atom, C_{1 - 6} alkyl or C_{1 - 6} alkoxy).

12. The compound according to any one of claims 1 to 11 or a pharmaceutically acceptable salt thereof, wherein R² is halogen atom selected from F, Cl or Br, and A is oxygen atom.

13. The compound according to any one of claims 1 to 12 or a pharmaceutically acceptable salt thereof, wherein R¹ is the following formula (1a-1): 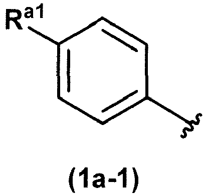 wherein
R^{a 1} is hydrogen atom, halogen atom, cyano, C_{1 - 6} alkyl (which may be optionally substituted with the same or different one or more substituents selected from halogen atom, hydroxy group or C_{1 - 6} alkoxy), or C₁₋₄ alkoxy (which may be optionally substituted with the same or different one or more substituents selected from halogen atom, hydroxy group or C_{1 - 6} alkoxy).

14. The compound according to any one of claims 1 to 12 or a pharmaceutically acceptable salt thereof, wherein R¹ is the following formula (1a-2): 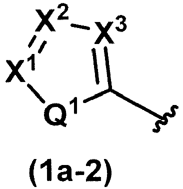 wherein
X¹ to X³ are each independently nitrogen atom or CR^{a 4} ;
Q¹ is oxygen atom or sulfur atom;
R^{a 4} is as defined in claim 10; and
L¹ is single bond.

15. The compound according to any one of claims 1 to 12 or a pharmaceutically acceptable salt thereof, wherein R¹ is selected from the following formulae (1a-21) to (1a-25): 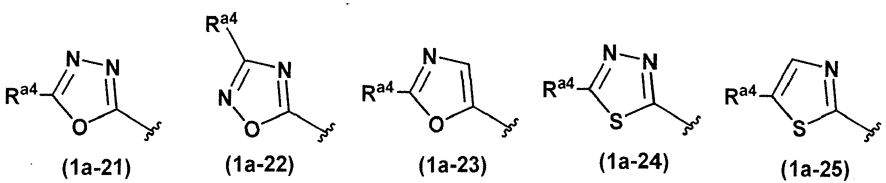 wherein R^{a4} is as defined in claim 10; and
L¹ is single bond.

16. The compound according to claim 15 or a pharmaceutically acceptable salt thereof, wherein R^{a 4} is (if there are plural CR^{a 4} , each R^{a 4} is independently) C₆₋₁₀ aromatic carbocyclyl group (which may be optionally substituted with the same or different one or more substituents selected from halogen atom, hydroxy group, C_{1 - 6} alkyl or C_{1 - 6} alkoxy), C_{1 - 6} alkyl (which may be optionally substituted with the same or different one or more substituents selected from halogen atom, hydroxy group, C_{1 - 6} alkoxy or C_{3 - 7} cycloalkyl), C_{3 - 7} cycloalkyl (which may be optionally substituted with the same or different one or more substituents selected from halogen atom, hydroxy group, C_{1 - 6} alkyl or C_{1 - 6} alkoxy), or 5- to 10-membered aromatic heterocyclyl group (which may be optionally substituted with the same or different one or more substituents selected from halogen atom, hydroxy group, C_{1 - 6} alkyl or C_{1 - 6} alkoxy).

17. The compound according to any one of claims 1 to 12 or a pharmaceutically acceptable salt thereof, wherein R¹ is the following formula (1a-3): 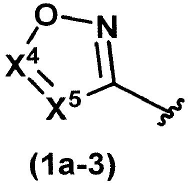 wherein X⁴ and X⁵ are as defined in claim 10; and
L¹ is single bond.

18. The compound according to claim 17 or a pharmaceutically acceptable salt thereof, wherein R¹ is selected from the following formulae (1a-31) to (1a-34): 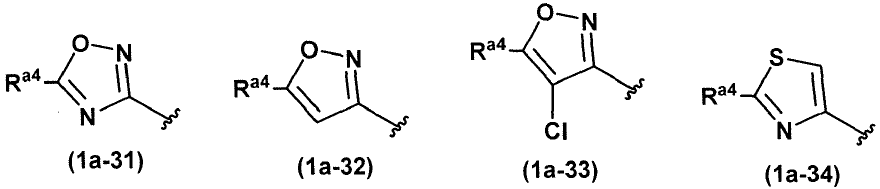 wherein R^{a 4} is as defined in claim 10.

19. The compound according to claim 17 or 18 or a pharmaceutically acceptable salt thereof, wherein R^{a 4} is (if there are plural CR^{a 4}, each R^{a 4} is independently) C_{1 - 6} alkyl (which may be optionally substituted with the same or different one or more substituents selected from halogen atom, hydroxy group, C_{1 - 6} alkoxy or C_{3 - 7} cycloalkyl), C_{3 - 7} cycloalkyl (which may be optionally substituted with the same or different one or more substituents selected from halogen atom, hydroxy group, C_{1 - 6} alkyl or C_{1 - 6} alkoxy), or C_{1 - 6} alkoxy (which may be optionally substituted with the same or different one or more substituents selected from halogen atom, hydroxy group or C_{1 - 6} alkoxy).

20. The compound according to any one of claims 1 to 19 or a pharmaceutically acceptable salt thereof, wherein ring G is the following formula (1b-2): 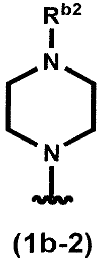 wherein R^{b2} is as defined in claim 11.

21. The compound according to any one of claims 1 to 19 or a pharmaceutically acceptable salt thereof, wherein ring G is the following formula (1b-1): 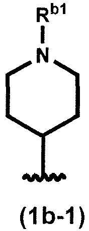 wherein
R^{b1} is C_{1 - 6} alkyl (which may be optionally substituted with the same or different one or more substituents selected from halogen atom or C_{1 - 6} alkoxy), 5- to 10-membered aromatic heterocyclyl group (which may be optionally substituted with the same or different one or more substituents selected from halogen atom, C_{1 - 6} alkyl or C_{1 - 6} alkoxy), or C_{3 - 7} cycloalkyl (which may be optionally substituted with the same or different one or more substituents selected from halogen atom, C_{1 - 6} alkyl or C_{1 - 6} alkoxy).

22. The compound according to any one of Items 1 to 19 or a pharmaceutically acceptable salt thereof, wherein ring G is the following formula (1b-3): 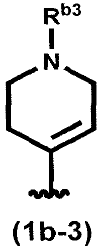 wherein
R^{b 3} is C_{1 - 6} alkyl (which may be optionally substituted with the same or different one or more substituents selected from halogen atom or C_{1 - 6} alkoxy, 5- to 10-membered aromatic heterocyclyl group (which may be optionally substituted with the same or different one or more substituents selected from halogen atom, C_{1 - 6} alkyl or C_{1 - 6} alkoxy), or C_{3 - 7} cycloalkyl (which may be optionally substituted with the same or different one or more substituents selected from halogen atom, C_{1 - 6} alkyl or C_{1 - 6} alkoxy).

23. The compound according to claim 1 or a pharmaceutically acceptable salt thereof, wherein the compound is selected from the following compounds:
N-{2-fluoro-6-[4-(propan-2-yl)piperazin-1-yl]phenyl}-4-methyl-4-[5-(propan-2-yl)-1,2,4-oxadiazol-3-yl]piperidine-1-carboxamide
N-{2-fluoro-6-[4-(propan-2-yl)piperazin-1-yl]phenyl}-4-methyl-4-(5-methyl-1,2,4-oxadiazol-3-yl)piperidine-1-carboxamide
4-(5-cyclopropyl-1,2,4-oxadiazol-3-yl)-N-{2-fluoro-6-[1-(propan-2-yl)-1,2,3,6-tetrahydropyridin-4-yl]phenyl}-4-methylpiperidine-1-carboxamide
4-(5-cyclopropyl-1,2,4-oxadiazol-3-yl)-N-{2-fluoro-6-[4-(propan-2-yl)piperazin-1-yl]phenyl}-4-methylpiperidine-1-carboxamide
4-(5-cyclopropyl-1,2-oxazol-3-yl)-N-{2-fluoro-6-[1-(propan-2-yl)piperidin-4-yl]phenyl}-4-methylpiperidine-1-carboxamide
4-(5-cyclopropyl-1,2-oxazol-3-yl)-N-{2-fluoro-6-[4-(propan-2-yl)piperazin-1-yl]phenyl}-4-methylpiperidine-1-carboxamide
4-(5-cyclopropyl-1,2-oxazol-3-yl)-N-{2-fluoro-6-[1-(propan-2-yl)-1,2,3,6-tetrahydropyridin-4-yl]phenyl}-4-methylpiperidine-1-carboxamide
N-{2-chloro-6-[4-(propan-2-yl)piperazin-1-yl]phenyl}-4-(5-cyclopropyl-1,2-oxazol-3-yl)-4-methylpiperidine-1-carboxamide
N-{2-chloro-6-[4-(propan-2-yl)piperazin-l-yl]phenyl}-4-(5-cyclopropyl-1,2,4-oxadiazol-3-yl)-4-methylpiperidine-1-carboxamide
N-{2-chloro-6-[4-(propan-2-yl)piperazin-1-yl]phenyl}-4-methyl-4-(4-methylphenyl)piperidine-1-carboxamide.

24. The compound according to claim 1 or a pharmaceutically acceptable salt thereof, wherein the compound is selected from the following compounds:
4-(5-cyclopropyl-1,2,4-oxadiazol-3-yl)-4-ethyl-N-{2-fluoro-6-[4-(propan-2-yl)piperazin-1-yl]phenyl}piperidine-1-carboxamide
N-{2-chloro-6-[4-(propan-2-yl)piperazin-l-yl]phenyl}-4-(5-cyclopropyl-1,2,4-oxadiazol-3-yl)-4-ethylpiperidine-1-carboxamide
N-{2-chloro-6-[4-(propan-2-yl)piperazin-1-yl]phenyl}-4-(5-cyclopropyl-1,3-thiazol-2-yl)-4-methylpiperidine-1-carboxamide
N-{2-chloro-6-[4-(propan-2-yl)piperazin-1-yl]phenyl}-4-{5-[(1S,2S)-2-fluorocyclopropyl)-1,2,4-oxadiazol-3-yl}-4-methylpiperidine-1-carboxamide
N-{2-chloro-6-[4-(propan-2-yl)piperazin-1-yl]phenyl}-4-{5-[(1R,2R)-2-fluorocyclopropyl]-1,2,4-oxadiazol-3-yl}-4-methylpiperidine-1-carboxamide
N-{2-chloro-6-[4-(propan-2-yl)piperazin-1-yl]phenyl}-4-methyl-4-{5-[(1S,2R)-2-methylcyclopropyl]-1,2,4-oxadiazol-3-yl}piperidine-1-carboxamide
N-(2-chloro-6-[4-(propan-2-yl)piperazin-1-yl]phenyl}-4-methyl-4-{5-[(1R,2S)-2-methylcyclopropyl]-1,2,4-oxadiazol-3-yl}piperidine-1-carboxamide
N-{2-chloro-6-[1-(propan-2-yl)-1,2,3,6-tetrahydropyridin-4-yl]phenyl}-4-(5-cyclopropyl-1,2,4-oxadiazol-3-yl)-4-methylpiperidine-1-carboxamide
N-{2-chloro-6-[1-(propan-2-yl)-1,2,3,6-tetrahydropyridin-4-yl)phenyl}-4-(5-cyclopropyl-1,2-oxazol-3-yl)-4-methylpiperidine-1-carboxamide
N-{2-chloro-6-[1-(propan-2-yl)-1,2,3,6-tetrahydropyridin-4-yl]phenyl}-4-methyl-4-(4-methylphenyl)piperidine-1-carboxamide.

25. A pharmaceutical composition comprising the compound according to any one of claims 1 to 24 or a pharmaceutically acceptable salt thereof.

26. A medicament for treating a disease associated with orexin receptor type 2, comprising the compound according to any one of claims 1 to 24 or a pharmaceutically acceptable salt thereof.

27. A medicament for treating narcolepsy, idiopathic hypersomnia, hypersomnia, sleep apnea syndrome, narcolepsy syndrome involving narcolepsy-like symptom, hypersomnia associated with Parkinson's disease, hypersomnia associated with dementia with Lewy body, hypersomnia syndrome involving daytime hypersomnia (for example, Kleine-Levin syndrome, major depression accompanied by hypersomnia, dementia with Lewy body, Parkinson's disease, progressive supranuclear palsy, Prader-Willi syndrome, Moebius syndrome, hypoventilation syndrome, Niemann-Pick disease type C, brain contusion, cerebral infarction, brain tumor, muscular dystrophy, multiple sclerosis, acute disseminated encephalomyelitis, Guillain-Barre syndrome, Rasmussen's encephalitis, Wernicke's encephalopathy, limbic encephalitis, Hashimoto encephalopathy), coma, loss of consciousness, obesity (for example, malignant mast cell, extrinsic obesity, hyperinsulinar obesity, hyperplasmic obesity, hypophysial obesity, hypoplasmic obesity, hypothyroid obesity, hypothalamic obesity, symptomatic obesity, childhood obesity, upper body obesity, alimentary obesity, gonadal obesity, systemic mastocytosis, primary obesity, central obesity), insulin resistance syndrome, Alzheimer, impaired consciousness such as coma, side effect or complication caused by anesthesia, sleep disturbance, sleep problem, insomnia, intermittent sleep, night myoclonus, REM sleep interruption, jet lag, jet lag syndrome, sleep disorder of shift workers, dyssomnia, sleep terror, depression, major depression, sleepwalking, enuresis, sleep disorder, Alzheimer's sundown syndrome, disease associated with circadian rhythm, fibromyalgia, condition resulting from decrease in sleeping quality, bulimia, obsessive eating disorder, obesity-related diseases, hypertension, diabetes, elevated plasma insulin level/insulin resistance, hyperlipemia, hyperlipidaemia, endometrial cancer, breast cancer, prostate cancer, colon cancer, cancer, osteoarthritis, obstructive sleep apnea, cholelithiasis, gallstone, heart disease, abnormal heartbeat, arrhythmia, myocardial infarction, congestive heart failure, coronary heart disease, cardiovascular disease, sudden death, polycystic ovary, craniopharyngioma, Prader-Willi syndrome, Froehlich syndrome, growth hormone deficiency, normal variant short stature, Turner syndrome, children suffering from acute lymphoblastic leukemia, syndrome X, reproductive hormone abnormality, decrease of fecundability, infertility, hypogonadism in men, sexual/reproductive-function dysfunction such as hirsutism in women, fetal defect associated with maternity obesity, gastrointestinal motility disorder such as obesity-related gastroesophageal reflux, obesity hypoventilation syndrome (Pickwickian syndrome), respiratory disease such as respiratory distress, inflammation such as vascular systemic inflammation, arteriosclerosis, hypercholesterolemia, hyperuricemia, low back pain, gallbladder disease, gout, renal cancer, secondary risk of obesity such as risk of left ventricle hypertrophy, migraine, headache, neuropathic pain, Parkinson's disease, psychosis, schizophrenia, facial flushing, night sweat, disease in genitalium/urinary system, disease associated with sexual function or fecundability, dysthymic disorder, bipolar disorder, bipolar I disorder, bipolar II disorder, cyclothymic disorder, acute stress disorder, agoraphobia, generalized anxiety disorder, obsessive-compulsive disorder, panic attack, panic disorder, posttraumatic stress disorder, separation anxiety disorder, social phobia, anxiety disorder, acute neurological and psychiatric disorder such as cerebral deficiency developed after heart bypass surgery or heart transplant, stroke, ischemic stroke, cerebral ischemia, spinal cord trauma, head injury, periparturient hypoxia, cardiac arrest, hypoglycemic nerve injury, Huntington's disease, amyotrophic lateral sclerosis, multiple sclerosis, eye damage, retinopathy, cognitive impairment, muscle spasm, tremor, epilepsy, disorder associated with muscle spasm, delirium, amnestic disorder, age-associated cognitive decline, schizoaffective disorder, paranoia, drug addiction, movement disorder, chronic fatigue syndrome, fatigue, medication-induced parkinsonian syndrome, Gilles de la Tourette syndrome, chorea, myoclonus, tic, restless legs syndrome, dystonia, dyskinesia, attention deficit hyperactivity disorder (ADHD), conduct disorder, urinary incontinence, withdrawal symptom, trigeminal neuralgia, hearing loss, tinnitus, nerve injury, retinopathy, macular degeneration, vomiting, cerebral edema, pain, bone pain, arthralgia, toothache, cataplexy, or traumatic brain injury, comprising the compound according to any one of claims 1 to 24 or a pharmaceutically acceptable salt thereof.

28. A medicament for treating narcolepsy, idiopathic hypersomnia, hypersomnia, sleep apnea syndrome, narcolepsy syndrome involving narcolepsy-like symptom, hypersomnia associated with Parkinson's disease, or hypersomnia associated with dementia with Lewy body, comprising the compound according to any one of claims 1 to 24 or a pharmaceutically acceptable salt thereof.

29. A medicament for treating narcolepsy, comprising the compound according to any one of claims 1 to 24 or a pharmaceutically acceptable salt thereof.

30. A medicament for treating idiopathic hypersomnia, comprising the compound according to any one of claims 1 to 24 or a pharmaceutically acceptable salt thereof.

31. A medicament for treating hypersomnia associated with Parkinson's disease, comprising the compound according to any one of claims 1 to 24 or a pharmaceutically acceptable salt thereof.

32. A medicament for treating hypersomnia associated with dementia with Lewy body, comprising the compound according to any one of claims 1 to 24 or a pharmaceutically acceptable salt thereof.

33. A method of treating narcolepsy, idiopathic hypersomnia, hypersomnia, sleep apnea syndrome, narcolepsy syndrome involving narcolepsy-like symptom, hypersomnia associated with Parkinson's disease, or hypersomnia associated with dementia with Lewy body, which comprises administering a therapeutically effective amount of the compound of any one of claims 1 to 24 or a pharmaceutically acceptable salt thereof to a patient in need thereof.

34. Use of the compound according to any one of claims 1 to 24 or a pharmaceutically acceptable salt thereof, in the manufacture of a medicament for treating narcolepsy, idiopathic hypersomnia, hypersomnia, sleep apnea syndrome, narcolepsy syndrome involving narcolepsy-like symptom, hypersomnia associated with Parkinson's disease, or hypersomnia associated with dementia with Lewy body.

35. A pharmaceutical composition comprising the compound according to any one of claims 1 to 24 or a pharmaceutically acceptable salt thereof for use in the treatment of narcolepsy, idiopathic hypersomnia, hypersomnia, sleep apnea syndrome, narcolepsy syndrome involving narcolepsy-like symptom, hypersomnia associated with Parkinson's disease, or hypersomnia associated with dementia with Lewy body.
